(19) Europäisches Patentamt
European Patent Office
Office européen des brevets

(11) EP 3 037 432 B1

(12) # EUROPEAN PATENT SPECIFICATION

(45) Date of publication and mention of the grant of the patent:
**17.06.2020 Bulletin 2020/25**

(51) Int Cl.:
***C07K 14/435*** (2006.01) ***C12N 15/82*** (2006.01)

(21) Application number: **15202058.2**

(22) Date of filing: **22.12.2015**

(54) **NUCAMPHOLIN NUCLEIC ACID MOLECULES TO CONTROL COLEOPTERAN INSECT PESTS**

NUCAMPHOLIN-NUKLEINSÄURE-MOLEKÜLE ZUR BEKÄMPFUNG VON KÄFERINSEKTENSCHÄDLINGEN

MOLÉCULES D'ACIDE NUCLÉIQUE DE NUCAMPHOLINE AFIN DE LUTTER CONTRE LES INSECTES NUISIBLES DE L'ORDRE DES COLÉOPTÈRES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.12.2014 US 201462095487 P**

(43) Date of publication of application:
**29.06.2016 Bulletin 2016/26**

(73) Proprietor: **Dow AgroSciences LLC**
**Indianapolis, IN 46268 (US)**

(72) Inventors:
- **Narva, Kenneth E.**
  **Indianapolis, IN 46268 (US)**
- **Worden, Sarah E.**
  **Indianapolis, IN 46268 (US)**
- **Frey, Megan L.**
  **Indianapolis, IN 46268 (US)**
- **Geng, Chaoxian**
  **Indianapolis, IN 46268 (US)**
- **Rangasamy, Murugesan**
  **Indianapolis, IN 46268 (US)**
- **Arora, Kanika**
  **West New York, NJ 07093 (US)**
- **Veeramani, Balaji**
  **Indianapolis, IN 46268 (US)**
- **Gandra, Premchand**
  **Indianapolis, IN 46268 (US)**
- **Vilcinskas, Andreas**
  **35394 Giessen (DE)**
- **Knorr, Eileen**
  **35392 Giessen (DE)**

(74) Representative: **Zwicker, Jörk**
**ZSP Patentanwälte PartG mbB**
**Hansastraße 32**
**80686 München (DE)**

(56) References cited:
**WO-A2-2004/039999 WO-A2-2012/092544**

- **William G Kelly ET AL: "Copyright 0 1997 by the Genetics Society of America Distinct Requirements for Somatic and Germline Expression of a Generally Expressed C a m h a b d i t i s elegam Gene", Genetics, 1 March 1997 (1997-03-01), pages 227-238, XP055265733, Retrieved from the Internet: URL:http://www.genetics.org/content/146/1/227.full.pdf [retrieved on 2016-04-15]**
- **DATABASE EMBL [Online] 25 April 2012 (2012-04-25), "TSA: Pogonus chalceus Pc_comp7513_c0_seq1 mRNA sequence.", XP002756662, retrieved from EBI accession no. EM_TSA:JU423906 Database accession no. JU423906 & VAN BELLEGHEM STEVEN M ET AL: "De novo Transcriptome Assembly and SNP Discovery in the Wing Polymorphic Salt Marsh Beetle Pogonus chalceus (Coleoptera, Carabidae)", PLOS ONE, vol. 7, no. 8, August 2012 (2012-08), page Article No.: e42605, ISSN: 1932-6203**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **DATABASE UniProt [Online] 26 June 2013 (2013-06-26), "SubName: Full=Uncharacterized protein {ECO:0000313|EMBL:ENN81549.1, ECO:0000313|EnsemblMetazoa:ENN81549}; Flags: Fragment;", XP002756663, retrieved from EBI accession no. UNIPROT:N6TM71 Database accession no. N6TM71 & KEELING CHRISTOPHER I ET AL: "Draft genome of the mountain pine beetle, Dendroctonus ponderosae Hopkins, a major forest pest", GENOME BIOLOGY, vol. 14, no. 3, 2013, page Article No.: R27, ISSN: 1465-6906(print)**
- **Renata Bolognesi ET AL: "Characterizing the Mechanism of Action of Double-Stranded RNA Activity against Western Corn Rootworm (Diabrotica virgifera virgifera LeConte) (e47534)", PLoS ONE, vol. 7, no. 10, 11 October 2012 (2012-10-11), pages 1-11, XP055268106, DOI: 10.1371/journal.pone.0047534**

**Description**

## TECHNICAL FIELD OF THE DISCLOSURE

The present invention relates generally to genetic control of plant damage caused by insect pests (*e.g.,* coleopteran pests). The present invention relates to target coding and non-coding polynucleotides, and the use of RNAi technologies for post-transcriptionally repressing or inhibiting expression of target coding and non-coding polynucleotides in the cells of an insect pest to provide a plant protective effect.

## BACKGROUND

The western corn rootworm (WCR), *Diabrotica virgifera virgifera* LeConte, is one of the most devastating corn rootworm species in North America and is a particular concern in corn-growing areas of the Midwestern United States. The northern corn rootworm (NCR), *Diabrotica barberi* Smith and Lawrence, is a closely-related species that co-inhabits much of the same range as WCR. There are several other related subspecies of *Diabrotica* that are significant pests in North America: the Mexican corn rootworm (MCR), *D. virgifera zeae* Krysan and Smith; the southern corn rootworm (SCR), *D. undecimpunctata howardi* Barber; *D. balteata* LeConte; *D. undecimpunctata tenella*; *D. speciosa* Germar; and *D. u. undecimpunctata* Mannerheim. The United States Department of Agriculture has estimated that corn rootworms cause $1 billion in lost revenue each year, including $800 million in yield loss and $200 million in treatment costs.

Both WCR and NCR are deposited in the soil as eggs during the summer. The insects remain in the egg stage throughout the winter. The eggs are oblong, white, and less than 0.004 inches in length. The larvae hatch in late May or early June, with the precise timing of egg hatching varying from year to year due to temperature differences and location. The newly hatched larvae are white worms that are less than 0.125 inches in length. Once hatched, the larvae begin to feed on corn roots. Corn rootworms go through three larval instars. After feeding for several weeks, the larvae molt into the pupal stage. They pupate in the soil, and then they emerge from the soil as adults in July and August. Adult rootworms are about 0.25 inches in length.

Corn rootworm larvae complete development on corn and several other species of grasses. Larvae reared on yellow foxtail emerge later and have a smaller head capsule size as adults than larvae reared on corn. Ellsbury et al. (2005) Environ. Entomol. 34:627-34. WCR adults feed on corn silk, pollen, and kernels on exposed ear tips. If WCR adults emerge before corn reproductive tissues are present, they may feed on leaf tissue, thereby slowing plant growth and occasionally killing the host plant. However, the adults will quickly shift to preferred silks and pollen when they become available. NCR adults also feed on reproductive tissues of the corn plant, but in contrast rarely feed on corn leaves.

Most of the rootworm damage in corn is caused by larval feeding. Newly hatched rootworms initially feed on fine corn root hairs and burrow into root tips. As the larvae grow larger, they feed on and burrow into primary roots. When corn rootworms are abundant, larval feeding often results in the pruning of roots all the way to the base of the corn stalk. Severe root injury interferes with the roots' ability to transport water and nutrients into the plant, reduces plant growth, and results in reduced grain production, thereby often drastically reducing overall yield. Severe root injury also often results in lodging of corn plants, which makes harvest more difficult and further decreases yield. Furthermore, feeding by adults on the corn reproductive tissues can result in pruning of silks at the ear tip. If this "silk clipping" is severe enough during pollen shed, pollination may be disrupted.

Control of corn rootworms may be attempted by crop rotation, chemical insecticides, biopesticides (*e.g.,* the spore-forming gram-positive bacterium, *Bacillus thuringiensis*), or a combination thereof. Crop rotation suffers from the significant disadvantage of placing unwanted restrictions upon the use of farmland. Moreover, oviposition of some rootworm species may occur in soybean fields, thereby mitigating the effectiveness of crop rotation practiced with corn and soybean.

Chemical insecticides are the most heavily relied upon strategy for achieving corn rootworm control. Chemical insecticide use, though, is an imperfect corn rootworm control strategy; over $1 billion may be lost in the United States each year due to corn rootworm when the costs of the chemical insecticides are added to the costs of the rootworm damage that may occur despite the use of the insecticides. High populations of larvae, heavy rains, and improper application of the insecticide(s) may all result in inadequate corn rootworm control. Furthermore, the continual use of insecticides may select for insecticide-resistant rootworm strains, as well as raise significant environmental concerns due to the toxicity of many of them to non-target species.

European pollen beetles (PB) are serious pests in oilseed rape, both the larvae and adults feed on flowers and pollen. Pollen beetle damage to the crop can cause 20-40% yield loss. The primary pest species is *Meligethes aeneus* Fabricius. Currently, pollen beetle control in oilseed rape relies mainly on pyrethroids which are expected to be phased out soon because of their environmental and regulatory profile. Moreover, pollen beetle resistance to existing chemical insecticides has been reported. Therefore, urgently needed are environmentally friendly pollen beetle control solutions with novel modes of action.

In nature, pollen beetles overwinter as adults in the soil or under leaf litter. In spring the adults emerge from hibernation and start feeding on flowers of weeds, and migrate onto flowering oilseed rape plants. The eggs are laid in oilseed rape flower buds. The larvae feed and develop in the buds and flowers. Late stage larvae find a pupation site in the soil. The second generation adults emerge in July and August and feed on various flowering plants before finding sites for overwintering.

RNA interference (RNAi) is a process utilizing endogenous cellular pathways, whereby an interfering RNA (iRNA) molecule (*e.g.,* a dsRNA molecule) that is specific for all, or any portion of adequate size, of a target gene results in the degradation of the mRNA encoded thereby. In recent years, RNAi has been used to perform gene "knockdown" in a number of species and experimental systems; for example, *Caenorhabditis elegans,* plants, insect embryos, and cells in tissue culture. *See, e.g.,* Fire et al. (1998) Nature 391:806-11; Martinez et al. (2002) Cell 110:563-74; McManus and Sharp (2002) Nature Rev. Genetics 3:737-47.

RNAi accomplishes degradation of mRNA through an endogenous pathway including the DICER protein complex. DICER cleaves long dsRNA molecules into short fragments of approximately 20 nucleotides, termed small interfering RNA (siRNA). The siRNA is unwound into two single-stranded RNAs: the passenger strand and the guide strand. The passenger strand is degraded, and the guide strand is incorporated into the RNA-induced silencing complex (RISC). Micro ribonucleic acids (miRNAs) are structurally very similar molecules that are cleaved from precursor molecules containing a polynucleotide "loop" connecting the hybridized passenger and guide strands, and they may be similarly incorporated into RISC. Post-transcriptional gene silencing occurs when the guide strand binds specifically to a complementary mRNA molecule and induces cleavage by Argonaute, the catalytic component of the RISC complex. This process is known to spread systemically throughout the organism despite initially limited concentrations of siRNA and/or miRNA in some eukaryotes such as plants, nematodes, and some insects.

U.S. Patent 7,612,194 and U.S. Patent Publication Nos. 2007/0050860, 2010/0192265, and 2011/0154545 disclose a library of 9112 expressed sequence tag (EST) sequences isolated from *D. v. virgifera* LeConte pupae. It is suggested in U.S. Patent 7,612,194 and U.S. Patent Publication No. 2007/0050860 to operably link to a promoter a nucleic acid molecule that is complementary to one of several particular partial sequences of *D. v. virgifera* vacuolar-type $H^+$-ATPase (V-ATPase) disclosed therein for the expression of anti-sense RNA in plant cells. U.S. Patent Publication No. 2010/0192265 suggests operably linking a promoter to a nucleic acid molecule that is complementary to a particular partial sequence of a *D. v. virgifera* gene of unknown and undisclosed function (the partial sequence is stated to be 58% identical to C56C10.3 gene product in *C. elegans*) for the expression of anti-sense RNA in plant cells. U.S. Patent Publication No. 2011/0154545 suggests operably linking a promoter to a nucleic acid molecule that is complementary to two particular partial sequences of *D. v. virgifera* coatomer beta subunit genes for the expression of anti-sense RNA in plant cells. Further, U.S. Patent 7,943,819 discloses a library of 906 expressed sequence tag (EST) sequences isolated from *D. v. virgifera* LeConte larvae, pupae, and dissected midguts, and suggests operably linking a promoter to a nucleic acid molecule that is complementary to a particular partial sequence of a *D. v. virgifera* charged multivesicular body protein 4b gene for the expression of double-stranded RNA in plant cells.

No further suggestion is provided in U.S. Patent 7,612,194, and U.S. Patent Publication Nos. 2007/0050860, 2010/0192265, and 2011/0154545 to use any particular sequence of the more than nine thousand sequences listed therein for RNA interference, other than the several particular partial sequences of V-ATPase and the particular partial sequences of genes of unknown function. Furthermore, none of U.S. Patent 7,612,194, and U.S. Patent Publication Nos. 2007/0050860 and 2010/0192265, and 2011/0154545 provides any guidance as to which other of the over nine thousand sequences provided would be lethal, or even otherwise useful, in species of corn rootworm when used as dsRNA or siRNA. U.S. Patent 7,943,819 provides no suggestion to use any particular sequence of the more than nine hundred sequences listed therein for RNA interference, other than the particular partial sequence of a charged multivesicular body protein 4b gene. Furthermore, U.S. Patent 7,943,819 provides no guidance as to which other of the over nine hundred sequences provided would be lethal, or even otherwise useful, in species of corn rootworm when used as dsRNA or siRNA. U.S. Patent Application Publication No. U.S. 2013/040173 and PCT Application Publication No. WO 2013/169923 describes the use of a sequence derived from a *Diabrotica virgifera* Snf7 gene for RNA interference in maize. (Also disclosed in Bolognesi et al. (2012) PLoS ONE 7(10): e47534. doi:10.1371/journal.pone.0047534).

The overwhelming majority of sequences complementary to corn rootworm DNAs (such as the foregoing) do not provide a plant protective effect from species of corn rootworm when used as dsRNA or siRNA. For example, Baum et al. (2007) Nature Biotechnology 25:1322-1326, describe the effects of inhibiting several WCR gene targets by RNAi. These authors reported that 8 of the 26 target genes they tested were not able to provide experimentally significant coleopteran pest mortality at a very high iRNA (*e.g.,* dsRNA) concentration of more than 520 $ng/cm^2$.

The authors of U.S. Patent 7,612,194 and U.S. Patent Publication No. 2007/0050860 made the first report of *in planta* RNAi in corn plants targeting the western corn rootworm. Baum et al. (2007) Nat. Biotechnol. 25(11):1322-6. These authors describe a high-throughput *in vivo* dietary RNAi system to screen potential target genes for developing transgenic RNAi maize. Of an initial gene pool of 290 targets, only 14 exhibited larval control potential. One of the most effective double-stranded RNAs (dsRNA) targeted a gene encoding vacuolar ATPase subunit A (V-ATPase), resulting in a rapid suppression of corresponding endogenous mRNA and triggering a specific RNAi response with low concentrations of dsRNA. Thus, these authors documented for the first time the potential for *in planta* RNAi as a possible pest management tool, while simultaneously demonstrating that effective targets could not be accurately identified *a priori,* even from a relatively small set of candidate genes.

WO 2012/092544 A2 discloses nucleic acid sequences of *Diabrotica virgifera* for the control of coleopteran pest through RNA interference.

## SUMMARY OF THE DISCLOSURE

**In** a first aspect, the invention relates to an isolated nucleic acid molecule comprising a polynucleotide encoding a hairpin ribonucleic acid (hpRNA) molecule with a stem and loop structure that is capable of inhibiting the expression of an endogenous target gene in a cell of a coleopteran insect upon ingestion, wherein the polynucleotide is operably linked to a heterologous promoter, and wherein the polynucleotide comprises: a first nucleotide sequence encoding a polyribonucleotide of the hpRNA molecule, wherein the first nucleotide sequence is selected from the group consisting of at least 50 contiguous nucleotides of SEQ ID NO:84, the complement of at least 50 contiguous nucleotides of SEQ ID NO:84, at least 50 contiguous nucleotides of SEQ ID NO:86, the complement of at least 50 contiguous nucleotides of SEQ ID NO:86, at least 50 contiguous nucleotides of SEQ ID NO:88, the complement of at least 50 contiguous nucleotides of SEQ ID NO:88, at least 50 contiguous nucleotides of SEQ ID NO:93 and the complement of at least 50 contiguous nucleotides of SEQ ID NO:93; a second nucleotide sequence encoding a polyribonucleotide of the hpRNA molecule; and a third nucleotide sequence encoding a polyribonucleotide of the hpRNA molecule, wherein the third nucleotide sequence is substantially the reverse complement of the first nucleotide sequence, such that the polyribonucleotides encoded by the first and the third nucleotide sequences hybridize in a stem structure of the hpRNA molecule, wherein a loop structure of the hpRNA molecule comprises the polyribonucleotide encoded by the second nucleotide sequence.

In a second aspect, the invention relates to the hairpin ribonucleic acid (hpRNA) molecule encoded by the polynucleotide of the nucleic acid molecule according to the first aspect of the invention that is capable of inhibiting the expression of an endogenous target gene in a cell of a coleopteran insect upon ingestion, the hpRNA molecule comprising: the first polyribonucleotide encoded by the first nucleotide sequence; the second polyribonucleotide encoded by the second nucleotide sequence; and the third polyribonucleotide encoded by the third nucleotide sequence, wherein the first and third polyribonucleotides are hybridized in a stem structure of the hpRNA molecule.

In another aspect, the invention relates to a transgenic cell comprising the nucleic acid molecule according to the first aspect of the invention.

In further aspects, the invention relates to a transgenic plant or a transgenic plant seed comprising the nucleic acid molecule according to the first aspect of the invention, wherein the heterologous promoter is functional in a plant cell.

In another aspect, the invention relates to a method for controlling a coleopteran insect population, the method comprising feeding insects of the population with an agent comprising the hpRNA according to the second aspect of the invention.

In yet another aspect, the invention relates to a method for producing a transgenic plant cell, the method comprising: transforming a plant cell with a nucleic acid molecule according to the first aspect of the invention, wherein the heterologous promoter is functional in a plant cell and wherein the molecule is a plant transformation vector; culturing the transformed plant cell under conditions sufficient to allow for development of a plant cell culture comprising a plurality of transformed plant cells; selecting for transformed plant cells that have integrated the polynucleotide into their genomes; screening the transformed plant cells for expression of the hpRNA molecule encoded by the polynucleotide; and selecting a plant cell that expresses the hpRNA molecule.

In yet another aspect, the invention relates to a method for producing a coleopteran pest-resistant transgenic plant, the method comprising: regenerating a transgenic plant from the transgenic plant cell according to invention, wherein expression of the RNA molecule encoded by the polynucleotide is sufficient to reduce the expression of a target gene in a coleopteran pest that ingests a cell of the transgenic plant.

Disclosed herein are nucleic acid molecules (*e.g.,* target genes, DNAs, dsRNAs, siRNAs, miRNAs, shRNAs, and hpRNAs), and methods of use thereof, for the control of insect pests, including, for example, coleopteran pests, such as *D. v. virgifera* LeConte (western corn rootworm, "WCR"); *D. barberi* Smith and Lawrence (northern corn rootworm, "NCR"); *D. u. howardi* Barber (southern corn rootworm, "SCR"); *D. v. zeae* Krysan and Smith (Mexican corn rootworm, "MCR"); *D. balteata* LeConte; *D. u. tenella; D. speciosa* Germar; *D. u. undecimpunctata* Mannerheim; and *Meligethes aeneus* Fabricius (pollen beetle, "PB"). In particular examples, exemplary nucleic acid molecules are disclosed that may be homologous to at least a portion of one or more native nucleic acids in an insect pest.

In these and further examples, the native nucleic acid sequence may be a target gene, the product of which may be, for example and without limitation: involved in a metabolic process; involved in a reproductive process; or involved in larval development. In some examples, post-transcriptional inhibition of the expression of a target gene by a nucleic acid molecule comprising a polynucleotide homologous thereto may be lethal to an insect pest or result in reduced growth and/or development of an insect pest. In specific examples, *nucampholin* (referred to herein as *ncm*) may be selected as a target gene for post-transcriptional silencing. In particular examples, a target gene useful for post-transcriptional inhibition is a novel gene referred to herein as a *Diabrotica ncm* (*e.g.,* SEQ ID NO:1 and SEQ ID NO:77). In particular examples, a target gene useful for post-transcriptional inhibition is the novel gene referred to herein as *Meligethes ncm* (*e.g.,* SEQ ID NO:84, SEQ ID NO:86, SEQ ID NO:88, and SEQ ID NO:93). An isolated nucleic acid molecule comprising the polynucleotide of SEQ ID NO:1; the complement of SEQ ID NO:1; SEQ ID NO:77; the complement of SEQ ID NO:77; SEQ ID NO:84; the complement of SEQ ID NO:84; SEQ ID NO:86; the complement of SEQ ID NO:86; SEQ ID NO:88; the complement of SEQ ID NO:88; SEQ ID NO:93; the complement of SEQ ID NO:93; and/or fragments of any of the foregoing (*e.g.*, SEQ ID NOs:3-6 and 90) is therefore disclosed herein.

Also disclosed are nucleic acid molecules comprising a polynucleotide that encodes a polypeptide that is at least about 85% identical to an amino acid sequence within a target gene product (for example, the product of a *ncm* gene). For example, a nucleic acid molecule may comprise a polynucleotide encoding a polypeptide that is at least 85% identical to a *Diabrotica* NCM (*e.g.,* SEQ ID NO:2); a *Meligethes* NCM (*e.g.,* SEQ ID NO:85, SEQ ID NO:87, SEQ ID NO:89, and SEQ ID NO:94); and/or an amino acid sequence within a product of a *Diabrotica ncm* or a *Meligethes ncm.* Further disclosed are nucleic acid molecules comprising a polynucleotide that is the reverse complement of a polynucleotide that encodes a polypeptide at least 85% identical to an amino acid sequence within a target gene product.

Also disclosed are cDNA polynucleotides that may be used for the production of iRNA (*e.g.,* dsRNA, siRNA, shRNA, miRNA, and hpRNA) molecules that are complementary to all or part of an insect pest target gene, for example, an *ncm* gene. In particular embodiments, hpRNAs may be produced *in vitro* or *in vivo* by a genetically-modified organism, such as a plant or bacterium. In particular examples, cDNA molecules are disclosed that may be used to produce iRNA molecules that are complementary to all or part of a *Diabrotica ncm* (*e.g.,* SEQ ID NO:1 and SEQ ID NO:77) or a *Meligethes ncm* (*e.g.,* SEQ ID NO:84, SEQ ID NO:86, SEQ ID NO:88, and SEQ ID NO:93).

Further disclosed are means for inhibiting expression of an essential gene in a coleopteran pest, and means for providing coleopteran pest resistance to a plant. A means for inhibiting expression of an essential gene in a coleopteran pest is a single- or double-stranded RNA molecule consisting of a polynucleotide selected from the group consisting of SEQ ID NOs:79-82; and the complements thereof. Functional equivalents of means for inhibiting expression of an essential gene in a coleopteran pest include single- or double-stranded RNA molecules that are substantially homologous to all or part of the complement of the RNA expression product of a *ncm* gene from an organism of the order coleoptera, comprising SEQ ID NO:1, SEQ ID NO:84, SEQ ID NO:86, SEQ ID NO:88, or SEQ ID NO:93. A means for providing coleopteran pest resistance to a plant is a DNA molecule comprising a polynucleotide encoding a means for inhibiting expression of an essential gene in a coleopteran pest operably linked to a promoter, wherein the DNA molecule is capable of being integrated into the genome of a maize plant.

Disclosed are methods for controlling a population of an insect pest (*e.g.,* a coleopteran pest), comprising providing to an insect pest (*e.g.,* a coleopteran pest) an iRNA (*e.g.,* dsRNA, siRNA, shRNA, miRNA, and hpRNA) molecule that functions upon being taken up by the pest to inhibit a biological function within the pest, wherein the iRNA molecule comprises all or part of a polynucleotide selected from the group consisting of: SEQ ID NO:78; the complement of SEQ ID NO:78; SEQ ID NO:79; the complement of SEQ ID NO:79; SEQ ID NO:80; the complement of SEQ ID N0:80; SEQ ID NO:81; the complement of SEQ ID NO:81; SEQ ID NO:82; the complement of SEQ ID NO:82; SEQ ID NO:83; the complement of SEQ ID NO:83; a polynucleotide that hybridizes to a native coding polynucleotide of a *Diabrotica* organism (*e.g.,* WCR) comprising all or part of SEQ ID NO:1 or SEQ ID NO:77; the complement of a polynucleotide that hybridizes to a native coding polynucleotide of a *Diabrotica* organism comprising all or part of SEQ ID NO:1 or SEQ ID NO:77; SEQ ID NO:95; the complement of SEQ ID NO:95; SEQ ID NO:96; the complement of SEQ ID NO:96; SEQ ID NO:97; the complement of SEQ ID NO:97; SEQ ID NO:98; the complement of SEQ ID NO:98; SEQ ID NO:99; the complement of SEQ ID NO:99; a polynucleotide that hybridizes to a native coding polynucleotide of a *Meligethes* organism (*e.g.,* PB) comprising all or part of any of SEQ ID NOs:84, 86, 88, and 93; and the complement of a polynucleotide that hybridizes to a native coding polynucleotide of a *Meligethes* organism comprising all or part of any of SEQ ID NOs:84, 86, 88, and 93.

In particular embodiments, an iRNA that functions upon being taken up by an insect pest to inhibit a biological function within the pest is transcribed from a DNA comprising all or part of a polynucleotide selected from the group consisting of: ; SEQ ID NO:84; the complement of SEQ ID NO:84; SEQ ID NO:86; the complement of SEQ ID NO:86; SEQ ID NO:88; the complement of SEQ ID NO:88; SEQ ID NO:93; the complement of SEQ ID NO:93; a native coding polynucleotide of a *Meligethes* organism (*e.g.,* PB) comprising all or part of any of SEQ ID NOs:84, 86, 88, and 93; and the complement of a native coding polynucleotide of a *Meligethes* organism comprising all or part of any of SEQ ID NOs:84, 86, 88, and 93.

Also disclosed herein are methods wherein dsRNAs, siRNAs, shRNAs, miRNAs, and/or hpRNAs may be provided to an insect pest in a diet-based assay, or in genetically-modified plant cells expressing the dsRNAs, siRNAs, shRNAs, miRNAs, and/or hpRNAs. In these and further examples, the dsRNAs, siRNAs, shRNAs, miRNAs, and/or hpRNAs may be ingested by the pest. Ingestion of dsRNAs, siRNA, shRNAs, miRNAs, and/or hpRNAs as disclosed herein may then result in RNAi in the pest, which in turn may result in silencing of a gene essential for viability of the pest and leading ultimately to mortality. Thus, methods are disclosed wherein nucleic acid molecules comprising exemplary polynucleotide(s) useful for parental control of insect pests are provided to an insect pest. In particular examples, a coleopteran pest controlled by use of nucleic acid molecules of the invention may be WCR, NCR, SCR, *D. undecimpunctata howardi, D. balteata, D. undecimpunctata tenella, D. speciosa, D. u. undecimpunctata,* or *Meligethes aeneus.*

The foregoing and other features will become more apparent from the following Detailed Description of several embodiments, which proceeds with reference to the accompanying **FIGs. 1-2.**

## BRIEF DESCRIPTION OF THE FIGURES

**FIG. 1** includes a depiction of a strategy used to provide dsRNA from a single transcription template with a single pair of primers.

**FIG. 2** includes a depiction of a strategy used to provide dsRNA from two transcription templates.

## SEQUENCE LISTING

The nucleic acid sequences listed in the accompanying sequence listing are shown using standard letter abbreviations for nucleotide bases, as defined in 37 C.F.R. § 1.822. The nucleic acid and amino acid sequences listed define molecules (*i.e.*, polynucleotides and polypeptides, respectively) having the nucleotide and amino acid monomers arranged in the manner described. The nucleic acid and amino acid sequences listed also each define a genus of polynucleotides or polypeptides that comprise the nucleotide and amino acid monomers arranged in the manner described. In view of the redundancy of the genetic code, it will be understood that a nucleotide sequence including a coding sequence also describes the genus of polynucleotides encoding the same polypeptide as a polynucleotide consisting of the reference sequence. It will further be understood that an amino acid sequence describes the genus of polynucleotide ORFs encoding that polypeptide.

Only one strand of each nucleic acid sequence is shown, but the complementary strand is understood as included by any reference to the displayed strand. As the complement and reverse complement of a primary nucleic acid sequence are necessarily disclosed by the primary sequence, the complementary sequence and reverse complementary sequence of a nucleic acid sequence are included by any reference to the nucleic acid sequence, unless it is explicitly stated to be otherwise (or it is clear to be otherwise from the context in which the sequence appears). Furthermore, as it is understood in the art that the nucleotide sequence of an RNA strand is determined by the sequence of the DNA from which it was transcribed (but for the substitution of uracil (U) nucleobases for thymine (T)), an RNA sequence is included by any reference to the DNA sequence encoding it. In the accompanying sequence listing:

SEQ ID NO:1 shows an exemplary *Diabrotica ncm* DNA open reading frame:

ATGCCAGATACCAAGGATGCCAAGGATACCAAGGATGCTAATTTGAGTTCTCCTGA
ACGTAAAAGACGAAGAAAGAGTAGATCTAAATCTCCAGAACGAAAAGAGAAAAAGT
CTTCCAAAAAGAAAGCCCACAATAGTAGAGACAGAGATTCATCAGAGGAAGGTTAC
AACCCTAAAGATTATCAGAGATACTATGGGGAAGATCGCCCAAACAGTGACAAATA
TTGGAATAAATATCCAAGGAAAGATACTACCAAAGTTGGCCAAAGATACTATGATG
CGGCTCCCGAAGAATCTGGCAAGAAGGGGCCAGATAGAAATTCAGAGGAGAAGGAG
TTACCAAAGCCAATGGAGTCTGTTCCTGATAAATCAGTCATCAAACCAAGAGAAAG
AAAAACTGTAGATATGTTAACATCGAGGACTGGTGGTGCTTATATTCCCCCAGCTA
AGCTACGATTGTTACAAGCCAGTATTACAGACAAAACATCAGCAGCCTATCAGCGT
ATAGCATGGGAAGCCTTAAAGAAATCCGTTCATGGTTACATTAATAAAATTAACAC
CTCGAATATTGGCATCATCGCCAGAGAATTATTGCATGAAAATATAGTAAGAGGTA
GAGGTTTGCTGTGCAAGTCAATAATACAAGCACAAGCAGCATCTCCTACTTTTACA
AACGTTTACGCAGCCTTAGTAGCTGTTATTAATTCGAAGTTTCCAAGTATAGGAGA
GCTTTTATTGAAGAGGTTGGTTTTGCAGTTCAAAAGAGGGTTTAAACAAAATAATA
AGTCTATTTGCATATCGGCTACTACTTTCGTAGCTCATTTAGTAAATCAGAGAGTG
GCACATGAAATTTTGGCTTTGGAGATACTTACATTGTTGGTGGAGACTCCTACAGA
TGATTCTGTGGAGGTGGCCATTTCATTTTTGAAGGAATGTGGACAAAAACTGACAG
AAGTTTCAAGTAGAGGTATTACTGCTATATTTGAGATGTTAAGAAACATTTTACAT
GAAGGCCAGCTAGAAAAAAAGAATTCAGTACATGATTGA

SEQ ID NO:2 shows the amino acid sequence of a *Diabrotica* NCM polypeptide encoded by an exemplary *Diabrotica ncm* DNA:

MRGGVSDDMTSTCVQGGIRPIGRYQPNMLMEPSSPQSAWQFHPAMPKREPVDHDGR
NDSGLASGGEFISSSPGSDNSEHFSASYSSPTSCHTVISTNTYYPTNLRRPSQAQT
SIPTHMMYTGDHNPLTPPNSEPMISPKSVLSRNNEGEHQTTLTPCASPEDASVDAT
DSVNCDGALKKLQATFEKNAFSEGSGDDDTKSDGEAEEYDEQGLRVPKVNSHGKIK
TFKCKQCDFVAITKLVFWEHTKLHIKADKLLKCPKCPFVTEYKHHLEYHLRNHYGS
KPFKCNQCSYSCVNKSMLNSHLKSHSNIYQYRCSDCSYATKYCHSLKLHLRKYSHK
PAMVLNPDGTPNPLPIIDVYGTRRGPKMKSEQKSSEEMSPKPEQVLPFPFNQFLPQ
MQLPFPGFPLFGGFPGGIPNPLLLQNLEKLARERRESMNSSERFSPAQSEQMDTDA
GVLDLSKPDDSSQTNRRKDSAYKLSTGDNSSDEEDDEATTTMFGNVEVVENKELED
TSSGKQTPTSAKKDDYSCQYCQINFGDPVLYTMHMGYHGYKNPFICNMCGEECNDK
VSFFLHIARNPHS

GATGCGGCTCCCGAAGAATCTGGCAAGAAGGGGCCAGATAGAAATTCAGAGGAGAA
GGAGTTACCAAAGCCAATGGAGTCTGTTCCTGATAAATCAGTCATCAAACCAAGAG
AAAGAAAAACTGTAGATATGTTAACATCGAGGACTGGTGGTGCTTATATTCCCCCA
GCTAAGCTACGATTGTTACAAGCCAGTATTACAGACAAAACATCAGCAGCCTATCA
GCGTATAGCATGGGAAGCCTTAAAGAAATCCGTTCATGGTTACATTAATAAAATTA
ACACCTCGAATATTGGCATCATCGCCAGAGAATTATTGCATGAAAATATAGTAAGA
GGTAGAGGTTTGCTGTGCAAGTCAATAATACAAGCACAAGCAGCATCTCCTACTTT
TACAAACGTTTACGCAGCC

CCCTTAGTAAAGAAATCTTAGGCAGTGATGGTGAGTCTGAATCAGGTTCCGAAGGT
TCAGAAGAGGAATCTGATAATGAAAATGAGGACGAAGTCAAGGACCAGGGAACAAT
TATTGACAATACTGAAACGAATTTAATTTCTCTTAGAAGAACCATATATTTGACTA
TTCAGTCTAGTTTAGATTTTGAAGAATGTGCACATAAGCTACTGAAGATGGAGTTG
AAACCTGGACAAGAAATAGAATTGTGTCACATGTTTCTTGACTGCTGCGCAGAACA
AAGAACCTACGAAAAGTTTTATGGTCTTTTGGCTCAAAGATTTTGTCAAATCAACA
AAGTGTATATCGAGCCTTTCCAACAAATTTTTAAAGATACCTATTCTACCACTCAC
AGACTAGATGCTAAC

CAGTCATCAAACCAAGAGAAAGAAAAACTGTAGATATGTTAACATCGAGGACTGGT
GGTGCTTATATTCCCCCAGCTAAGCTACGATTGTTACAAGCCAGTATTACAGACAA
AACATCAGCAGCCTATCAGCGTATAGCATGGGAAGCCTTAAAGAAATCCGTTCATG
GTTACATTAA

ATTGGCATCATCGCCAGAGAATTATTGCATGAAAATATAGTAAGAGGTAGAGGTTT
GCTGTGCAAGTCAATAATACAAGCACAAGCAGCATCTCCTACTTTTACAAACGTTT
ACGCAGCC

SEQ ID NO:7 shows the nucleotide sequence of a T7 phage promoter.

SEQ ID NO:8 shows an exemplary *YFP* gene.

SEQ ID NOs:9-16 show primers used for PCR amplification of *ncm* sequences comprising *ncm* reg1, *ncm* reg2, *ncm* v1, and *ncm* v2, used in some examples for dsRNA production.

SEQ ID NO:17 shows an exemplary DNA encoding a *Diabrotica ncm* v2 RNA; containing a sense polynucleotide, a loop sequence (underlined), and an antisense polynucleotide (bold font):

GGCTGCGTAAACGTTTGTAAAAGTAGGAGATGCTGCTTGTGCTTGTATTATTGACT
TGCACAGCAAACCTCTACCTCTTACTATATTTTCATGCAATAATTCTCTGGCGATG
ATGCCAATGAAACTAGTACCAGTCATCACGCTGGAGCGCACATATAGGCCCTCCAT
CAGAAAGTCATTGTGTATATCTCTCATAGGGAACGAGCTGCTTGCGTATTTCCCTT
CCGTAGTCAGAGTCATCAATCAGCTGCACCGTGTCGTAAAGCGGGACGTTCGCAAG
CTCGTCCGCGGTTA**ATTGGCATCATCGCCAGAGAATTATTGCATGAAAATATAGTA**
**AGAGGTAGAGGTTTGCTGTGCAAGTCAATAATACAAGCACAAGCAGCATCTCCTAC**
**TTTTACAAACGTTTACGCAGCC**

**[0039]** SEQ ID NO:18 shows an exemplary DNA encoding a *YFP v2*:

ATGTCATCTGGAGCACTTCTCTTTCATGGGAAGATTCCTTACGTTGTGGAGATGGA
AGGGAATGTTGATGGCCACACCTTTAGCATACGTGGGAAAGGCTACGGAGATGCCT
CAGTGGGAAAGGTTGATGCACAGTTCATCTGCACAACTGGTGATGTTCCTGTGCCT
TGGAGCACACTTGTCACCACTCTCACCTATGGAGCACAGTGCTTTGCCAAGTATGG
TCCAGAGTTGAAGGACTTCTACAAGTCCTGTATGCCAGATGGCTATGTGCAAGAGC
GCACAATCACCTTTGAAGGAGATGGCAACTTCAAGACTAGGGCTGAAGTCACCTTT
GAGAATGGGTCTGTCTACAATAGGGTCAAACTCAATGGTCAAGGCTTCAAGAAAGA
TGGTCATGTGTTGGGAAAGAACTTGGAGTTCAACTTCACTCCCCACTGCCTCTACA
TCTGGGGTGACCAAGCCAACCACGGTCTCAAGTCAGCCTTCAAGATCTGTCATGAG
ATTACTGGCAGCAAAGGCGACTTCATAGTGGCTGACCACACCCAGATGAACACTCC
CATTGGTGGAGGTCCAGTTCATGTTCCAGAGTATCATCACATGTCTTACCATGTGA
AACTTTCCAAAGATGTGACAGACCACAGAGACAACATGTCCTTGAAAGAAACTGTC
AGAGCTGTTGACTGTCGCAAGACCTACCTTTGA

SEQ ID NO:19 shows an exemplary DNA comprising a loop:

AGTCATCACGCTGGAGCGCACATATAGGCCCTCCATCAGAAAGTCATTGTGTATAT
CTCTCATAGGGAACGAGCTGCTTGCGTATTTCCCTTCCGTAGTCAGAGTCATCAAT
CAGCTGCACCGTGTCGTAAAGCGGGACGTTCGCAAGCTCGT

SEQ ID NO:20 shows an exemplary *YFP* gene.
SEQ ID NO:21 shows a DNA sequence of *annexin* region 1.
SEQ ID NO:22 shows a DNA sequence of *annexin* region 2.
SEQ ID NO:23 shows a DNA sequence of *beta spectrin 2* region 1.
SEQ ID NO:24 shows a DNA sequence of *beta spectrin 2* region 2.
SEQ ID NO:25 shows a DNA sequence of *mtRP-L4* region 1.
SEQ ID NO:26 shows a DNA sequence of *mtRP-L4* region 2.
SEQ ID NOs:27-54 show primers used to amplify gene regions of *annexin*, *beta spectrin 2, mtRP-L4,* and *YFP* for dsRNA synthesis.
SEQ ID NO:55 shows a maize DNA sequence encoding a TIP41-like protein.
SEQ ID NO:56 shows the nucleotide sequence of a T20VN primer oligonucleotide.
SEQ ID NOs:57-61 show primers and probes used for dsRNA transcript expression analyses.
SEQ ID NO:62 shows a nucleotide sequence of a portion of a *SpecR* coding region used for binary vector backbone detection.
SEQ ID NO:63 shows a nucleotide sequence of an *AAD1* coding region used for genomic copy number analysis.
SEQ ID NO:64 shows a DNA sequence of a maize *invertase* gene.
SEQ ID NOs:65-73 show the nucleotide sequences of DNA oligonucleotides used for gene copy number determinations and binary vector backbone detection.
SEQ ID NOs:74-76 show primers and probes used for dsRNA transcript maize expression analyses.
SEQ ID NO:77 shows a contig comprising an exemplary *Diabrotica ncm* DNA:

ATTACCAAATGTCAATGTCACTCATTACTCATTACCAAATGTCAATGTCACTGTCA
GGTAACGTGCAATGCAAATTGTCAATGTCAAACTTAAAAATATTTTCCTGCAACTG
CATCAAATTGTAAATTTTATTTTTTTTAAATATGCCAGATACCAAGGATGCCAAGG
ATACCAAGGATGCTAATTTGAGTTCTCCTGAACGTAAAAGACGAAGAAAGAGTAGA
TCTAAATCTCCAGAACGAAAAGAGAAAAAGTCTTCCAAAAAGAAAGCCCACAATAG
TAGAGACAGAGATTCATCAGAGGAAGGTTACAACCCTAAAGATTATCAGAGATACT
ATGGGGAAGATCGCCCAAACAGTGACAAATATTGGAATAAATATCCAAGGAAAGAT
ACTACCAAAGTTGGCCAAAGATACTATGATGCGGCTCCCGAAGAATCTGGCAAGAA
GGGGCCAGATAGAAATTCAGAGGAGAAGGAGTTACCAAAGCCAATGGAGTCTGTTC
CTGATAAATCAGTCATCAAACCAAGAGAAAGAAAAACTGTAGATATGTTAACATCG
AGGACTGGTGGTGCTTATATTCCCCCAGCTAAGCTACGATTGTTACAAGCCAGTAT
TACAGACAAAACATCAGCAGCCTATCAGCGTATAGCATGGGAAGCCTTAAAGAAT
CCGTTCATGGTTACATTAATAAAATTAACACCTCGAATATTGGCATCATCGCCAGA
GAATTATTGCATGAAAATATAGTAAGAGGTAGAGGTTTGCTGTGCAAGTCAATAAT

ACAAGCACAAGCAGCATCTCCTACTTTTACAAACGTTTACGCAGCCTTAGTAGCTG
TTATTAATTCGAAGTTTCCAAGTATAGGAGAGCTTTTATTGAAGAGGTTGGTTTTG
CAGTTCAAAAGAGGGTTTAAACAAAATAATAAGTCTATTTGCATATCGGCTACTAC
TTTCGTAGCTCATTTAGTAAATCAGAGAGTGGCACATGAAATTTTGGCTTTGGAGA
TACTTACATTGTTGGTGGAGACTCCTACAGATGATTCTGTGGAGGTGGCCATTTCA
TTTTTGAAGGAATGTGGACAAAAACTGACAGAAGTTTCAAGTAGAGGTATTACTGC
TATATTTGAGATGTTAAGAAACATTTTACATGAAGGCCAGCTAGAAAAAAAGAATT
CAGTACATGATTGAAGTTATGTTTCAAATAAGGAAAGACGGATTTAAGGATCATGC
TGCTGTCGTAGAAGAATTAGATTTAGTAGAAGAGGAAGATCAATTCACTCATCTTA
TTATGTTAGATGATGTTAAAGAGGCTGATGCAGAGGATATATTGAATGTGTTCAAA
TTTGATGAGAGTTATGAAGAAAATGAAGATAAATACAAAACCCTTAGTAAAGAAAT
CTTAGGCAGTGATGGTGAGTCTGAATCAGGTTCCGAAGGTTCAGAAGAGGAATCTG
ATAATGAAAATGAGGACGAAGTCAAGGACCAGGGAACAATTATTGACAATACTGAA
ACGAATTTAATTTCTCTTAGAAGAACCATATATTTGACTATTCAGTCTAGTTTAGA
TTTTGAAGAATGTGCACATAAGCTACTGAAGATGGAGTTGAAACCTGGACAAGAAA
TAGAATTGTGTCACATGTTTCTTGACTGCTGCGCAGAACAAAGAACCTACGAAAAG
TTTTATGGTCTTTTGGCTCAAAGATTTTGTCAAATCAACAAAGTGTATATCGAGCC
TTTCCAACAAATTTTTAAAGATACCTATTCTACCACTCACAGACTAGATGCTAACA
GGTTAAGAAACGTCAGCAAATTTTTTGCGCATTTACTTTTTACGGATGCCATTGGA
TGGGAAGTCCTTGACATCATGAAATTGAATGAAGAGGATACCAATAGTTCTAGTAG
GATTTTCATAAAAATCTTGTTTCAAGAATTGGCTGAATATATGGGACTAGGAAAAT
TAAACGCAAGGCTAAAGGATGAGACCCTGCAGGCTTATTTTTCAGGACTGTTTCCT
AGAGATAACCCAAAGAATACCAGATTTTCTATTAATTTTTTTACCTCTATCGGTTT
GGGAGGATTAACTGATGAACTCAGAGAACATTTAAAAAATATTCCAAAAATGATGG
AAATGAAGTTAGCCACTAAAGAAAAGGAAAGCAGTGGTAGTAGTAGTTCAGAAAGT
AGTTCTGAGGAAGATAGTAGTGACGACAGCTCTGAAGATTCATCAAGTTCTGAAGA
CGATAGAGGCAAAAAGAAGAAAAAAACCAAAAAGCTAGAAGAAAAAAATTCGAAAG
TAAATTCTAAGTCTAGACCTAGATCAAAAGAGAAAGAACACGCAGACAAACCTAGA
GACAAACATAGAAAGGAAGATAGACATAAATCTGACAAAAATCCCGATAGATCCTC
GTCCAAAAAATATTCTAAAGAAGATAACAAGAGGAAGAAAGACTACGAATGGATGA
AGAGCAGATATGAAGATGATATTAAGCAATTAAAAAACGATAAAAGGGTATTTGAA
AAGTCTTCCAAACGACGATCAAGATCTAGAGACAGGGTAAAAGTCAAGGAAGAGCG
TAGACGTAGAAGCAGAGAAAGAAGAAGGAGCTAAGATAATTTTTTAATAAGGATCT
ATGTATATTTATGTAAACATTTATTTAATACATGTTTTTTAAAAAAAAA

SEQ ID NOs:78-83 show exemplary RNAs transcribed from nucleic acids comprising exemplary *Diabrotica ncm* polynucleotides and fragments thereof.

SEQ ID NO:84 shows a contig comprising an exemplary *Meligethes aeneus ncm* DNA:

GCAATTTCGTTTTTGAAGGAAAGTGGTCAAAAACTCACTGAGGTGTCGAGTAAAGG
TATCAATGCCATATTTGAGATGTTGAGGAATATTCTGCATGAAGGACAGTTGGAGA
AGAGAATACAGTACATGATTGAAGTCATGTTCCAAGTTCGGAAAGATGGTTTCAAG
GATCATGCTGCTGTTACTGAAGAACTAGATATTGTTGAAGAGGAAGATCAGTTTAC
TCACCTAATCACATTGGATGATGTTAAACAAGCTAACTCAGAGGATATATTGAATG
TGTTTAAATTTGATGATAAATATGAGGAAAATGAGGGTAAATACAAAACTTTAAGT
AAGGAAATTCTCCAGTCAGACAGTGAATCAGGCGAATCTGGTTCAGAGGGGTCTGA
AGAAGACTCGGAAGATGAAGAAGGTGAAGAAGATGAAACCAAAAATCAAACCATTA
TTGATAACACAGAAACTAATTTAATCACCTTAAGGAGAACCATCTATCTCACAATA
CAATCCAGTTTGGATTTTGAGGAATGTGCCCATAAATTGATGAAAATGGAGATCAA
ACCTGGACAAGAGATTGAATTGTGTCACATGTTCCTTGATTGTTGCGCTGAACAGC
GTACCTACGAAAAATTCTTCGGCCTCCTCTCGCAGCGCTTCTGCCAAATAAACAAG
ACTTTCATCGAACCGTTCCAACAAATTTTCAAAGATACCTATTCCACAACTCACAG
ACTTGACGCCAATCGATTGAGAAACGTTAGCAAATTCTTCGCGCATTTATTGTTCA
CCGACGCCATCGGCTGGGAAGTGCTCGATATCATGAAATTAAACGAGGAAGACACC
AACAGTTCCAGCAGGATTTTCATCAAGATTTTGTTCCAGGAGTTGTCCGAATATAT
GGGATTAGCGAAGTTGAATAAAAGGCTAAAGGATGAAACTTTACAGGAATATTTCG
CGGGGCTATTTCCGAGGGATAACCCGAAGAACACGCGTTTCGCCATCAATTTTTTC
ACGTCGATCGGTTTAGGAGGTCTAACGGACGAGTTGAGGGAGCACTTGAAAAACGT
GCCAAAACATCTGGAAGTGATGGCTTTGAAAGCAGATTCGAGCAGCTCTAGCAGCA
GTAGCAGCAGTTCCAGTAACGATTCCAGCAGCAGTTCAGATTCTTCCGATGACGAG
GGTTCCAGGAAGAAGAAAACAAAAAAATTGAAAACCCCGGACAAAAAGAAGAAACA
GAAAGAAGATGAAAAACCCAAAAAGAAAAGCGAGGATAAACCGAGGAACAAACCAG
ACTATAGAGATAGAAGAAACGACGACAGGGAAAAGTTTAAAAAATACAGAAACAAC
GACGAAGAAAGCCACAGAAGAAGCAGAGAAGATGCAAGAGAAAAATACAGAGGTCA
CGAGGAAAGAAGAAGCGACCACAGAGAAGAATACCGGCCGAGAGAACATAGAGGTA
GAGATAGACGTTAGTTGTATAATAATGTATATTTTTTCGTATTTAATAAAATAAAT
TATACATTTTATAGTGTTTCGGAGCATTCACCAAGCAAGGGTTTTACTTTCGGATA
GCAATGGTGTAGTACGTTTTTGAAGGTGTCCACACACACCAAGCCGGTTTTATCTA
AATCTAGAGCTATCTTCCAAAAGTCTTCAAAGGA

SEQ ID NO:85 shows the amino acid sequence of a *Meligethes aeneus* NCM polypeptide encoded by an exemplary *Meligethes aeneus ncm* DNA:

AISFLKESGQKLTEVSSKGINAIFEMLRNILHEGQLEKRIQYMIEVMFQVRKDGFK
DHAAVTEELDIVEEEDQFTHLITLDDVKQANSEDILNVFKFDDKYEENEGKYKTLS
KEILQSDSESGESGSEGSEEDSEDEEGEEDETKNQTIIDNTETNLITLRRTIYLTI
QSSLDFEECAHKLMKMEIKPGQEIELCHMFLDCCAEQRTYEKFFGLLSQRFCQINK
TFIEPFQQIFKDTYSTTHRLDANRLRNVSKFFAHLLFTDAIGWEVLDIMKLNEEDT
NSSSRIFIKILFQELSEYMGLAKLNKRLKDETLQEYFAGLFPRDNPKNTRFAINFF
TSIGLGGLTDELREHLKNVPKHLEVMALKADSSSSSSSSSSSNDSSSSSDSSDDE

GSRKKKTKKLKTPDKKKKQKEDEKPKKKSEDKPRNKPDYRDRRNDDREKFKKYRNN
DEESHRRSREDAREKYRGHEERRSDHREEYRPREHRGRDRR

SEQ ID NO:86 shows a contig comprising an exemplary *Meligethes aeneus ncm* DNA:

CCACACGAATTGACTGGTTAATTAAAAATAAGCACAAGAAACGAATAACACTACAA
TGGTTTGAATTTACACAAAAAAAAAAATGTAGTCACTACCATTGTAGTGTTATTCG
TTTCTTGTGCTTATTTTTAATTAACCAGTCAATTCGTGTGGTGTAGTGAACAAAGG
TTATAGTTATGACGACGGATTCCGAGAGAGGTTCCCCTACAGCTGCGGCTCCACGC
AGAAGCGCCTCGAAATCGCCAGAACCAAAAAAAGCAAAGTACGATAAGAAAGAGAA
GGGCGATAAAGATCGCAAGAGGAGATCCCACAGATCCAGATCTAGATCCAGGGATA
GAGACCATAGGGACAAACATGGTGGAAAAAAACGTTACCACGACCTGGACGACCCT
TCTGAAGACTACCCAAGATATTATGGCGAGGATAGAAAACAGAACAGTGACAGATA
TTGGTCCAAGTACCCAAAGAAAGACAGGGACGAATATGTTATTGGTAGCCGGTATT
ATGATGTTGAGGAAAAGAAGGAGAAAAAAGAAAAAGAGGATGAAAATAAGGATAAA
TCCGTCATCACTCCAAGGGAAAGGAAAACAGTGGACTTACTAACATCTCGAACAGG
TGGGGCTTATATACCTCCAGCTAAATTACGTATGATGCAGGCTGAGATAACTGATA
AATCATCAGCTGCATATCAAAGAATTGCCTGGGAAGCTTTAAAAAAGTCCATTCAT
GGTTACATCAACAAAATTAACACTTCCAATATTGGTCTTATTGCTAGAGAATTACT
GCATGAAAACATTGTAAGAGGTAGAGGTTTGCTGTGTAAATCTATAATACAAGCAC
AGGCAGCTTCCCCGACGTTCACCAATGTTTATGCAGCTTTAGTTGCAGTCATAAAT
TCAAAATTCCCCAACATTGGAGAACTGTTACTGAAAAGGTTGGTTTTGCAGTTTAA
AAGGGGTTTCAAGCAGAACAACAAGTCTATCTGTATATCGGCTGCTACCTTTGTCG
CGCATTTAGTAAACCAAAGAGTGGCCCACGAAATTTTAGCATTGGAAATTCTTACT
TTACTTGTTGAGTCCCCCACAGATGATTCAGTGGAAGTAGCAATTTCGTTTTTGAA
GGAAAGTGGTCAAAAACTCACTGAGGTGTCGAGTAAAGGTATCAATGCCATATTTG
AGATGTTGAGGAATATTCTGCATGAAGGACAGTTGGAGAAGAGAATACAGTACATG
ATTGAAGTCATGTTCCAAGTTCGGAAAGATGGTTTCAAGGATCATGCTGCTGTTAC
TGAAGAACTAGATATTGTTGAAGAGGAAGATCAGTTTACTCACCTAATCACATTGG
ATGATGTTAAACAAGCTAACTCAGAGGATATATTGAATGTGTTTAAATTTGATGAT
AAATATGAGGAAAATGAGGGTAAATACAAAACTTTAAGTAAGGAAATTCTCCAGTC
AGACAGTGAATCAGGCGAATCTGGTTCAGAGGGGTCTGAAGAAGACTCGGAAGATG
AAGAAGGTGAAGAAGATGAAACCAAAAATCAAACCATTATTGATAACACAGAAACT
AATTTAATCACCTTAAGGAGAACCATCTATCTCACAATACAATCCAGTTTGGATTT
TGAGGAATGTGCCCATAAATTGATGAAAATGGAGATCAAACCTGGACAAGAGATTG
AATTGTGTCACATGTTCCTTGATTGTTGCGCTGAACAGCGTACCTACGAAAAATTC
TTCGGCCTCCTCTCGCAGCGCTTCTGCCAAATAAACAAGACTTTCATCGAACCGTT
CCAACAAATTTTCAAAGATACCTATTCCACAACTCACAGACTTGACGCCAATCGAT
TGAGAAACGTTAGCAAATTCTTCGCGCATTTATTGTTCACCGACGCCATCGGCTGG
GAAGTGCTCGATATCATGAAATTAAACGAGGAAGACACCAACAGTTCCAGCAGGAT

TTTTATCAAGATTTTGTTCCAGGAGTTGTCCGAATATATGGGATTAGCGAAGTTGA
ATAAAAGGCTAAAGGATGAAACTTTACAGGAATATTTCGCGGGGCTATTTCCGAGG
GATAACCCGAAGAACACGCGTTTCGCCATCAATTTTTTCACGTCGATCGGTTTAGG
AGGTCTAACGGACGAGTTGAGGGAGCACTTGAAAAACGTGCCAAAACATCTGGAAG
TGATGGCTTTGAAAGCAGATTCGAGCAGCTCTAGCAGCAGTAGCAGCAGTTCCAGT
AACGATTCCAGCAGCAGTTCAGATTCTTCCGATGACGAGGGTTCCAGGAAGAAGAA
AACAAAAAAATTGAAAACCCCGGACAAAAAGAAGAAACAGAAAGAAGATGAAAAAC
CCAAAAAGAAAAGCGAGGATAAACCGAGGAACAAACCAGACTATAGAGATAGGAGA
AACGACGACAGGGAAAAGTTTAAAAAATACAGAAACAACGACGAAGAAAGCCACAG
AAGAAGCAGAGAAGATGCAAGAGAAAAATACAGAGGTCACGAGGAAAGAAGACGAG
GCCGAGAAGACCAAACGCGAAGAGGACAAGACCAAGTTCCAAGGTTTGTGC

SEQ ID NO:87 shows the amino acid sequence of a *Meligethes aeneus* NCM polypeptide encoded by an exemplary *Meligethes aeneus ncm* DNA:

MTTDSERGSPTAAAPRRSASKSPEPKKAKYDKKEKGDKDRKRRSHRSRSRSRDRDH
RDKHGGKKRYHDLDDPSEDYPRYYGEDRKQNSDRYWSKYPKKDRDEYVIGSRYYDV
EEKKEKKEKEDENKDKSVITPRERKTVDLLTSRTGGAYIPPAKLRMMQAEITDKSS
AAYQRIAWEALKKSIHGYINKINTSNIGLIARELLHENIVRGRGLLCKSIIQAQAA
SPTFTNVYAALVAVINSKFPNIGELLLKRLVLQFKRGFKQNNKSICISAATFVAHL
VNQRVAHEILALEILTLLVESPTDDSVEVAISFLKESGQKLTEVSSKGINAIFEML
RNILHEGQLEKRIQYMIEVMFQVRKDGFKDHAAVTEELDIVEEEDQFTHLITLDDV
KQANSEDILNVFKFDDKYEENEGKYKTLSKEILQSDSESGESGSEGSEEDSEDEEG
EEDETKNQTIIDNTETNLITLRRTIYLTIQSSLDFEECAHKLMKMEIKPGQEIELC
HMFLDCCAEQRTYEKFFGLLSQRFCQINKTFIEPFQQIFKDTYSTTHRLDANRLRN
VSKFFAHLLFTDAIGWEVLDIMKLNEEDTNSSSRIFIKILFQELSEYMGLAKLNKR
LKDETLQEYFAGLFPRDNPKNTRFAINFFTSIGLGGLTDELREHLKNVPKHLEVMA
LKADSSSSSSSSSSSSSNDSSSSSDSSDDEGSRKKKTKKLKTPDKKKKQKEDEKPKK
KSEDKPRNKPDYRDRRNDDREKFKKYRNNDEESHRRSREDAREKYRGHEERRRGRE
DQTRRGQDQVPRFV

SEQ ID NO:88 shows a contig comprising an exemplary *Meligethes aeneus ncm* DNA:

AAATGTAGTCACTACCATTGTAGTGTTATTCGTTTCTTGTGCTTATTTTTAATTAA
CCAGTCAATTCGTGTGGTGTAGTGAACAAAGGTTATAGTTATGACGACGGATTCCG
AGAGAGGTTCCCCTACAGCTGCGGCTCCACGCAGAAGCGCCTCGAAATCGCCAGAA
CCAAAAAAAGCAAAGTACGATAAGAAAGAGAAGGGCGATAAAGATCGCAAGAGGAG
ATCCCACAGATCCAGATCTAGATCCAGGGATAGAGACCATAGGGACAAACATGGTG
GAAAAAAACGTTACCACGACCTGGACGACCCTTCTGAAGACTACCCAAGATATTAT
GGCGAGGATAGAAAACAGAACAGTGACAGATATTGGTCCAAGTACCCAAAAGAAAG

ACAGGGACGAATATGTTATTGGTAACCGGTATTATGATGTTGAGGAAAAGAAGGAG
AAAAAGGAAAAAGAGGATGAAAATAAGGATAAATCCGTCATTACTCCAAGGGAAAG
GAAAACAGTGGACTTACTAACATCTCGAACAGGTGGGGCTTATATACCTCCAGCTA
AATTACGTATGATGCAGGCTGAGATAACTGATAAATCATCAGCTGCATATCAAAGA
ATTGCCTGGGAAGCTTTAAAAAAGTCCATTCATGGTTACATCAACAAAATTAACAC
TTCCAATATTGGTCTTATTGCTAGAGAATTACTGCATGAAAACATTGTAAGAGGTA
GAGGTTTGCTGTGTAAATCTATAATACAAGCACAGGCAGCTTCCCCGACGTTCACC
AATGTTTATGCAGCTTTAGTTGCAGTCATAAATTCAAAATTCCCCAACATTGGAGA
ACTGTTACTGAAAAGGTTGGTTTTGCAGTTTAAAAGGGGTTTCAAGCAGAACAACA
AGTCTATCTGTATATCGGCTGCTACCTTTGTCGCGCATTTAGTAAACCAAAGAGTG
GCTCATGAAATTTTAGCATTGGAAATTCTTACTTTACTTGTTGAGTCCCCCACAGA
TGATTCAGTAGAAGTGAGCAGAACAACAAGTCTATCTGTATATCGGCTGCTACCTT
TGTCGCGCATTTAGTAAACCAAAGAGTGGCCCACGAAATTTTAGCATTGGAAATTC
TTACTTTACTTGTTGAGTCCCCCACAGATGATTCAGTGGAAGTAGCAATTTCGTTT
TTGAAGGAAAGTGGTCAAAAACTCACTGAGGTGTCGAGTAAAGGTATCAATGCCAT
ATTTGAGATGTTGAGGAATATTCTGCATGAAGGACAGTTGGAGAAGAGAATACAGT
ACATGATTGAAGTCATGTTCCAAGTTCGGAAAGATGGTTTCAAGGATCATGCTGCT
GTTACTGAAGAACTAGATATTGTTGAAGAGGAAGATCAGTTTACTCACCTAATCAC
ATTGGATGATGTTAAACAAGCTAACTCAGAGGATATATTGAATGTGTTTAAATTTG
ATGATAAATATGAGGAAAATGAGGGTAAATACAAAACTTTAAGTAAGGAAATTCTC
CAGTCAGACAGTGAATCAGGCGAATCTGGTTCAGAGGGGTCTGAAGAAGACTCGGA
AGATGAAGAAGGTGAAGAAGATGAAACCAAAAATCAAACCATTATTGATAACACAG
AAACTAATTTAATCACCTTAAGGAGAACCATCTATCTCACAATACAATCCAGTTTG
GATTTTGAGGAATGTGCCCATAAATTGATGAAAATGGAGATCAAACCTGGACAAGA
GATTGAATTGTGTCACATGTTCCTTGATTGTTGCGCTGAACAGCGTACCTACGAAA
AATTCTTCGGCCTCCTCTCGCAGCGCTTCTGCCAAATAAACAAGACTTTCATCGAA
CCGTTCCAACAAATTTTCAAAGATACCTATTCCACAACTCACAGACTTGACGCCAA
TCGATTGAGAAACGTTAGCAAATTCTTCGCGCATTTATTGTTCACCGACGCCATCG
GCTGGGAAGTGCTCGATATCATGAAATTAAACGAGGAAGACACCAACAGTTCCAGC
AGGATTTTCATCAAGATTTTGTTCCAGGAGTTGTCCGAATATATGGGATTAGCGAA
GTTGAATAAAAGGCTAAAGGATGAAACTTTACAGGAATATTTCGCGGGGCTATTTC
CGAGGGATAACCCGAAGAACACGCGTTTCGCCATCAATTTTTTCACGTCGATCGGT
TTAGGAGGTCTAACGGACGAGTTGAGGGAGCACTTGAAAAACGTGCCAAAACATCT
GGAAGTGATGGCTTTGAAAGCAGATTCGAGCAGCTCTAGCAGCAGTAGCAGCAGTT
CCAGTAACGATTCCAGCAGCAGTTCAGATTCTTCCGATGACGAGGGTTCCAGGAAG
AAGAAAACAAAAAAATTGAAAACCCCGGACAAAAAGAAGAAACAGAAAGAAGATGA
AAAACCCAAAAAGAAAAGCGAGGATAAACCGAGGAACAAAGTAAATGGTGATAAGA
ATATAGAAACAGAAGATACACAAGGAGTTGAGGACACAAAAAAGACTG

SEQ ID NO:89 shows the amino acid sequence of a *Meligethes aeneus* NCM polypeptide encoded by an exemplary *Meligethes aeneus ncm* DNA:

MLRNILHEGQLEKRIQYMIEVMFQVRKDGFKDHAAVTEELDIVEEEDQFTHLITL
DDVKQANSEDILNVFKFDDKYEENEGKYKTLSKEILQSDSESGESGSEGSEEDSE
DEEGEEDETKNQTIIDNTETNLITLRRTIYLTIQSSLDFEECAHKLMKMEIKPGQ
EIELCHMFLDCCAEQRTYEKFFGLLSQRFCQINKTFIEPFQQIFKDTYSTTHRLD
ANRLRNVSKFFAHLLFTDAIGWEVLDIMKLNEEDTNSSSRIFIKILFQELSEYMG
LAKLNKRLKDETLQEYFAGLFPRDNPKNTRFAINFFTSIGLGGLTDELREHLKNV
PKHLEVMALKADSSSSSSSSSSSSNDSSSSSDSSDDEGSRKKKTKKLKTPDKKKK
QKEDEKPKKKSEDKPRNKVNGDKNIETEDTQGVEDTKKT

SEQ ID NO:90 shows an exemplary *Meligethes aeneus ncm* DNA, referred to herein in some places as *ncm* reg1 (region 1), which is used in some examples for the production of a dsRNA:

GTTCCTTGATTGTTGCGCTGAACAGCGTACCTACGAAAAATTCTTCGGCCTCCTCT
CGCAGCGCTTCTGCCAAATAAACAAGACTTTCATCGAACCGTTCCAACAAATTTTC
AAAGATACCTATTCCACAACTCACAGACTTGACGCCAATCGATTGAGAAACGTTAG
CAAATTCTTCGCGCATTTATTGTTCACCGACGCCATCGGCTGGGAAGTGCTCGATA
TCATGAAATTAAACGAGGAAGACACCAACAGTTCCAGCAGGATTTTCATCAAGATT
TTGTTCCAGGAGTTGTCCGAATATATGGGATTAGCGAAGTTGAATAAAAGGCTAAA
GGATGAAACTTTACAGGAATATTTCGCGGGGCTATTTCCGAGGGATAACCCGAAGA
ACACGCGTTTCGCCATCAATTTTTTCACGTCGATCGGTTTAGGAGGTCTAACGGAC
GAGTTGAGGGAGCACTTGAAAAACGTGCCAAAACATCTGGA

SEQ ID NOs:91-92 show primers used to amplify portions of a *Meligethes aeneus ncm* sequence comprising *ncm* reg1.

SEQ ID NO:93 shows a contig comprising an exemplary *Meligethes aeneus ncm* DNA:

TTTTTGTTACCAACCAAACGGCCTAAATTTCCCTAGATAACCTAAAATAAAAACAA
CACTTCGTCATTTGTGTAAATTCAAACAAATGTAGTCACTACCATTGTAGTGTTAT
TCGTTTCTTGTGCTTATTTTTAATTAACCAGTCAATTCGTGTGGTGTAGTGAACAA
AGGTTATAGTTATGACGACGGATTCCGAGAGAGGTTCCCCTACAGCTGCGGCTCCA
CGCAGAAGCGCCTCGAAATCGCCAGAACCAAAAAAAGCAAAGTACGATAAGAAAGA
GAAGGGCGATAAAGATCGCAAGAGGAGATCCCACAGATCCAGATCTAGATCCAGGG
ATAGAGACCATAGGGACAAACATGGTGGAAAAAAACGTTACCACGACCTGGACGAC
CCTTCTGAAGACTACCCAAGATATTATGGCGAGGATAGAAAACAGAACAGTGACAG
ATATTGGTCCAAGTACCCAAAGAAAGACAGGGACGAATATGTTATTGGTAGCCGGT
ATTATGATGTTGAGGAAAAGAAGGAGAAAAAGGAAAAAGAGGATGAAAATAAGGAT

AAATCCGTCATTACTCCAAGGGAAAGGAAAACAGTGGACTTACTAACATCTCGAAC
AGGTGGGGCTTATATACCTCCAGCTAAATTACGTATGATGCAGGCTGAGATAACTG
ATAAATCATCAGCTGCATATCAAAGAATTGCCTGGGAAGCTTTAAAAAAGTCCATT
CATGGTTACATCAACAAAATTAACACTTCCAATATTGGTCTTATTGCTAGAGAATT
ACTGCATGAAAACATTGTAAGAGGTAGAGGTTTGCTGTGTAAATCTATAATACAAG
CACAGGCAGCTTCCCCGACATTCACCAATGTTTATGCAGCTTTAGTTGCAGTCATA
AATTCAAAATTCCCCAACATTGGAGAACTGTTACTGAAAAGGTTGGTTTTGCAGTT
TAAAAGGGGTTTCAAGCAGAACAACAAGTCTATCTGTATATCGGCTGCTACCTTTG
TCGCGCATTTAGTAAACCAAAGAGTGGCCCATGAAATTTTAGCATTGGAAATTCTT
ACTTTACTTGTTGAGTCCCCCACAGATGATTCAGTGGAAGTAGCAATTTCGTTTTT
GAAGGAAAGTGGTCAAAAACTCACTGAGGTGTCGAGTAAAGGTATCAATGCCATAT
TTGAGATGTTGAGGAATATTCTGCATGAAGGACAGTTGGAGAAGAGAATACAGTAC
ATGATTGAAGTCATGTTCCAAGTTCGGAAAGATGGTTTCAAGGATCATGCTGCTGT
TACTGAAGAACTAGATATTGTTGAAGAGGAAGATCAGTTTACTCACCTAATCACAT
TGGATGATGTTAAACAAGCTAACTCAGAGGATATATTGAATGTGTTTAAATTTGAT
GATAAATATGAGGAAAATGAGGGTAAATACAAAACTTTAAGTAAGGAAATTCTCCA
GTCAGACAGTGAATCAGGCGAATCTGGTTCAGAGGGGTCTGAAGAAGACTCGGAAG
ATGAAGAAGGTGAAGAAGATGAAACCAAAAATCAAACCATTATTGATAACACAGAA
ACTAATTTAATCACCTTAAGGAGAACCATCTATCTCACAATACAATCCAGTTTGGA
TTTTGAGGAATGTGCCCATAAATTGATGAAAATGGAGATCAAACCTGGACAAGAGA
TTGAATTGTGTCACATGTTCCTTGATTGTTGCGCTGAACAGCGTACCTACGAAAAA
TTCTTCGGCCTCCTCTCGCAGCGCTTCTGCCAAATAAACAAGACTTTCATCGAACC
GTTCCAACAAATTTTCAAAGATACCTATTCCACAACTCACAGACTTGACGCCAATC
GATTGAGAAACGTTAGCAAATTCTTCGCGCATTTATTGTTCACCGACGCCATCGGC
TGGGAAGTGCTCGATATCATGAAATTAAACGAGGAAGACACCAACAGTTCCAGCAG
GATTTTCATCAAGATTTTGTTCCAGGAGTTGTCCGAATATATGGGATTAGCGAAGT
TGAATAAAAGGCTAAAGGATGAAACTTTACAGGAATATTTCGCGGGGCTATTTCCG
AGGGATAACCCGAAGAACACGCGTTTCGCCATCAATTTTTTCACGTCGATCGGTTT
AGGAGGTCTAACGGACGAGTTGAGGGAGCACTTGAAAAACGTGCCAAAACATCTGG
AAGTGATGGCTTTGAAAGCAGATTCGAGCAGCTCTAGCAGCAGTAGCAGCAGTTCC
AGTAACGATTCCAGCAGCAGTTCAGATTCTTCCGATGACGAGGGTTCCAGGAAGAA
GAAAACAAAAAAATTGAAAACCCCGGACAAAAAGAAGAAACAGAAAGAAGATGAAA
AACCCAAAAAGAAAAGCGAGGATAAACCGAGGAACAAACCAGACTATAGAGATAGA
AGAAACGACGACAGGGAAAAGTTTAAAAAATACAGAAACAACGACGAAGAAAGCCA
CAGAAGAAGCAGAGAAGATGCAAGAGAAAAATACAGAGGTCACGAGGAAAGAAGAA
GCGACCACAGAGAAGAATACCGGCCGAGAGAACATAGAGGTAGAGATAGACGTTAG
TTGTATAATAATGTATATTTTTT

SEQ ID NO:94 shows the amino acid sequence of a *Meligethes aeneus* NCM polypeptide encoded by an exemplary *Meligethes aeneus ncm* DNA:

MTTDSERGSPTAAAPRRSASKSPEPKKAKYDKKEKGDKDRKRRSHRSRSRSRDRDH
RDKHGGKKRYHDLDDPSEDYPRYYGEDRKQNSDRYWSKYPKKDRDEYVIGSRYYDV
EEKKEKKEKEDENKDKSVITPRERKTVDLLTSRTGGAYIPPAKLRMMQAEITDKSS
AAYQRIAWEALKKSIHGYINKINTSNIGLIARELLHENIVRGRGLLCKSIIQAQAA
SPTFTNVYAALVAVINSKFPNIGELLLKRLVLQFKRGFKQNNKSICISAATFVAHL
VNQRVAHEILALEILTLLVESPTDDSVEVAISFLKESGQKLTEVSSKGINAIFEML
RNILHEGQLEKRIQYMIEVMFQVRKDGFKDHAAVTEELDIVEEEDQFTHLITLDDV
KQANSEDILNVFKFDDKYEENEGKYKTLSKEILQSDSESGESGSEGSEEDSEDEEG
EEDETKNQTIIDNTETNLITLRRTIYLTIQSSLDFEECAHKLMKMEIKPGQEIELC
HMFLDCCAEQRTYEKFFGLLSQRFCQINKTFIEPFQQIFKDTYSTTHRLDANRLRN
VSKFFAHLLFTDAIGWEVLDIMKLNEEDTNSSSRIFIKILFQELSEYMGLAKLNKR
LKDETLQEYFAGLFPRDNPKNTRFAINFFTSIGLGGLTDELREHLKNVPKHLEVMA
LKADSSSSSSSSSSSSNDSSSSSDSSDDEGSRKKKTKKLKTPDKKKKQKEDEKPKK
KSEDKPRNKPDYRDRRNDDREKFKKYRNNDEESHRRSREDAREKYRGHEERRSDHR
EEYRPREHRGRDRR

SEQ ID NOs:95-99 show exemplary RNAs transcribed from nucleic acids comprising exemplary *Meligethes ncm* polynucleotides and fragments thereof.

## DETAILED DESCRIPTION

### *1. Overview* of *several embodiments*

We developed RNA interference (RNAi) as a tool for insect pest management, using one of the most likely target pest species for transgenic plants that express dsRNA; the western corn rootworm. Thus far, most genes proposed as targets for RNAi in rootworm larvae do not actually achieve their purpose. Herein, we describe RNAi-mediated knockdown of *nucampholin* (*ncm*) in the exemplary insect pest, western corn rootworm, which is shown to have a lethal phenotype when, for example, iRNA are molecules delivered *via* ingested *ncm* dsRNA. In embodiments herein, the ability to deliver *ncm* dsRNA by feeding to insects confers a RNAi effect that is very useful for insect (*e.g.,* coleopteran) pest management. By combining *ncm*-mediated RNAi with other useful RNAi targets (*e.g., ROP* RNAi targets, as described in U.S. Patent Application No. 14/577,811; *RNAPII140* RNAi targets, as described in U.S. Patent Application No. 14/577,854; *Dre4* RNAi targets, as described in U.S. Patent Application No. 14/705,807; *COPI alpha* RNAi targets, as described in U.S. Patent Application No. 62/063,199; *COPI beta* RNAi targets, as described in U.S. Patent Application No. 62/063,203; *COPI gamma* RNAi targets, as described in U.S. Patent Application No. 62/063,192; *COPI delta* RNAi targets, as described in U.S. Patent Application No. 62/063,216) the potential to affect multiple target sequences, for example, in coleoptera (*e.g.,* larval rootworms), may increase opportunities to develop sustainable approaches to insect pest management involving RNAi technologies.

Disclosed herein are methods and compositions for genetic control of insect (*e.g.,* coleopteran) pest infestations. Methods for identifying one or more gene(s) essential to the lifecycle of an insect pest for use as a target gene for RNAi-mediated control of an insect pest population are also provided. DNA plasmid vectors encoding an RNA molecule may be designed to suppress one or more target gene(s) essential for growth, survival, and/or development. In some aspects of the disclosure, the RNA molecule may be capable of forming dsRNA molecules. In some embodiments, methods are provided for post-transcriptional repression of expression or inhibition of a target gene *via* nucleic acid molecules that are complementary to a coding or non-coding sequence of the target gene in an insect pest. In these and further embodiments, a pest may ingest one or more hpRNA molecules transcribed from all or a portion of a nucleic acid molecule that is complementary to a coding or non-coding sequence of a target gene, thereby providing a plant-protective effect.

Thus, some embodiments involve sequence-specific inhibition of expression of target gene products, using hpRNA that is complementary to coding and/or non-coding sequences of the target gene(s) to achieve at least partial control of an insect (*e.g.,* coleopteran) pest. Disclosed is a set of isolated and purified nucleic acid molecules comprising a polynucleotide, for example, as set forth in one of SEQ ID NOs: 1; 77; 84; 86; 88; and 93, and fragments thereof. In some embodiments, a stabilized dsRNA molecule may be expressed from these polynucleotides, fragments thereof, or a gene comprising one of these polynucleotides, for the post-transcriptional silencing or inhibition of a target gene. In certain embodiments, isolated and purified nucleic acid molecules comprise all or part of any of SEQ ID NOs: 84; 86; 88; and 93.

Some embodiments involve a recombinant host cell (*e.g.,* a plant cell) having in its genome at least one recombinant DNA encoding at least one iRNA (*e.g.,* dsRNA) molecule(s). In particular embodiments, the dsRNA molecule(s) may be provided when ingested by an insect (*e.g.,* coleopteran) pest to post-transcriptionally silence or inhibit the expression of a target gene in the pest. The recombinant DNA may comprise, for example, fragments of any of SEQ ID NOs: 84, 86, 88, and 93; and a polynucleotide consisting of a partial sequence of a gene comprising one of SEQ ID NOs: 84, 86, 88, and 93, and/or complements thereof.

Some aspects of the disclosure involve a recombinant host cell having in its genome a recombinant DNA encoding at least one iRNA (*e.g.,* dsRNA) molecule(s) comprising all or part of SEQ ID NO:78 or SEQ ID NO:83 (*e.g.,* at least one polynucleotide selected from a group comprising SEQ ID NOs:78-83). Some aspects of the disclosure involve a recombinant host cell having in its genome a recombinant DNA encoding at least one iRNA (*e.g.,* dsRNA) molecule(s) comprising all or part of any of SEQ ID NOs:95-97 and 99 (*e.g.,* SEQ ID NO:98). When ingested by an insect (*e.g.,* coleopteran) pest, the iRNA molecule(s) may silence or inhibit the expression of a target *ncm* DNA (*e.g.,* a DNA comprising all or part of a polynucleotide selected from the group consisting of SEQ ID NOs:1; 3-6; 77, 84, 86, 88, 90, and 93) in the pest, and thereby result in cessation of growth, development, and/or feeding in the pest.

In some embodiments, a recombinant host cell having in its genome at least one recombinant DNA encoding at least one RNA molecule capable of forming a dsRNA molecule may be a transformed plant cell. Some embodiments involve transgenic plants comprising such a transformed plant cell. In addition to such transgenic plants, progeny plants of any transgenic plant generation, transgenic seeds, and transgenic plant products, are all provided, each of which comprises recombinant DNA(s). In particular embodiments, an RNA molecule capable of forming a dsRNA molecule may be expressed in a transgenic plant cell. Therefore, in these and other embodiments, a dsRNA molecule may be isolated from a transgenic plant cell. In particular embodiments, the transgenic plant is a plant selected from the group comprising corn (*Zea mays*), soybean (*Glycine max*), rapeseed (*Brassica sp.*), and plants of the family *Poaceae.*

Some embodiments involve a method for modulating the expression of a target gene in an insect (*e.g.,* coleopteran) pest cell. In these and other embodiments, a nucleic acid molecule may be provided, wherein the nucleic acid molecule comprises a polynucleotide encoding an RNA molecule capable of forming a dsRNA molecule. In embodiments, a polynucleotide encoding an RNA molecule capable of forming a dsRNA molecule is operatively linked to a promoter, and may also be operatively linked to a transcription termination sequence. In particular embodiments, a method for modulating the expression of a target gene in an insect pest cell may comprise: (a) transforming a plant cell with a vector comprising a polynucleotide encoding an RNA molecule capable of forming a dsRNA molecule; (b) culturing the transformed plant cell under conditions sufficient to allow for development of a plant cell culture comprising a plurality of transformed plant cells; (c) selecting for a transformed plant cell that has integrated the vector into its genome; and (d) determining that the selected transformed plant cell comprises the RNA molecule capable of forming a dsRNA molecule encoded by the polynucleotide of the vector. A plant may be regenerated from a plant cell that has the vector integrated in its genome and comprises the dsRNA molecule encoded by the polynucleotide of the vector.

Thus, also disclosed is a transgenic plant comprising a vector having a polynucleotide encoding an RNA molecule capable of forming a dsRNA molecule integrated in its genome, wherein the transgenic plant comprises the dsRNA molecule encoded by the polynucleotide of the vector. In particular embodiments, expression of an RNA molecule capable of forming a dsRNA molecule in the plant is sufficient to modulate the expression of a target gene in a cell of an insect (*e.g.,* coleopteran) pest that contacts the transformed plant or plant cell (for example, by feeding on the transformed plant, a part of the plant (*e.g.,* root) or plant cell), such that growth and/or survival of the pest is inhibited. Transgenic plants disclosed herein may display resistance and/or enhanced tolerance to insect pest infestations. Particular transgenic plants may display resistance and/or enhanced tolerance to one or more coleopteran pest(s) selected from the group consisting of: WCR; NCR; SCR; MCR; *D. balteata* LeConte; *D. u. tenella; D. speciosa* Germar; *D. u. undecimpunctata* Mannerheim; and *Meligethes aeneus.*

Also disclosed herein are methods for delivery of control agents, such as an iRNA molecule, to an insect (*e.g.,* coleopteran) pest. Such control agents may cause, directly or indirectly, an impairment in the ability of an insect pest population to feed, grow or otherwise cause damage in a host. In some embodiments, a method is provided comprising delivery of a stabilized dsRNA molecule to an insect pest to suppress at least one target gene in the pest, thereby causing RNAi and reducing or eliminating plant damage in a pest host. In some embodiments, a method of inhibiting expression of a target gene in the insect pest may result in cessation of growth, survival, and/or development, in the pest.

In some aspects of the disclosure, compositions (*e.g.,* a topical composition) are provided that comprise an iRNA (*e.g.,* dsRNA) molecule for use in plants, animals, and/or the environment of a plant or animal to achieve the elimination or reduction of an insect (*e.g.,* coleopteran) pest infestation. In particular aspects of the disclosure, the composition may be a nutritional composition or food source to be fed to the insect pest. Some aspects of the disclosure comprise making the nutritional composition or food source available to the pest. Ingestion of a composition comprising iRNA molecules may result in the uptake of the molecules by one or more cells of the pest, which may in turn result in the inhibition of expression of at least one target gene in cell(s) of the pest. Ingestion of or damage to a plant or plant cell by an insect pest infestation may be limited or eliminated in or on any host tissue or environment in which the pest is present by providing one or more compositions comprising an iRNA molecule in the host of the pest.

RNAi baits are formed when the dsRNA is mixed with food or an attractant or both. When the pests eat the bait, they also consume the dsRNA. Baits may take the form of granules, gels, flowable powders, liquids, or solids. In another embodiment, *ncm* may be incorporated into a bait formulation such as that described in U.S. Patent No. 8,530,440 which is hereby incorporated by reference. Generally, with baits, the baits are placed in or around the environment of the insect pest, for example, WCR can come into contact with, and/or be attracted to, the bait.

The compositions and methods disclosed herein may be used together in combinations with other methods and compositions for controlling damage by insect (*e.g.,* coleopteran) pests. For example, an iRNA molecule as described herein for protecting plants from insect pests may be used in a method comprising the additional use of one or more chemical agents effective against an insect pest, biopesticides effective against such a pest, crop rotation, recombinant genetic techniques that exhibit features different from the features of RNAi-mediated methods and RNAi compositions (*e.g.,* recombinant production of proteins in plants that are harmful to an insect pest (*e.g., Bt* toxins)).

### *II. Abbreviations*

| | |
|---|---|
| dsRNA | double-stranded ribonucleic acid |
| GI | growth inhibition |
| NCBI | National Center for Biotechnology Information |
| gDNA | genomic deoxyribonucleic acid |
| iRNA | inhibitory ribonucleic acid |
| ORF | open reading frame |
| RNAi | ribonucleic acid interference |
| miRNA | micro ribonucleic acid |
| shRNA | short hairpin ribonucleic acid |
| siRNA | small inhibitory ribonucleic acid |
| hpRNA | hairpin ribonucleic acid |
| UTR | untranslated region |
| WCR | western corn rootworm (*Diabrotica virgifera virgifera* LeConte) |
| NCR | northern corn rootworm (*Diabrotica barberi* Smith and Lawrence) |
| MCR | Mexican corn rootworm (*Diabrotica virgifera zeae* Krysan and Smith) |
| PCR | Polymerase chain reaction |
| qPCR | quantitative polymerase chain reaction |
| RISC | RNA-induced Silencing Complex |
| SCR | southern corn rootworm (*Diabrotica undecimpunctata* howardi Barber) |
| YFP | yellow fluorescent protein |
| SEM | standard error of the mean |
| PB | Pollen beetle (*Meligethes aeneus* Fabricius) |

### *III. Terms*

In the description and tables which follow, a number of terms are used. In order to provide a clear and consistent understanding of the specification and claims, including the scope to be given such terms, the following definitions are provided:

Coleopteran pest: As used herein, the term "coleopteran pest" refers to pest insects of the order Coleoptera, including pest insects in the genus *Diabrotica,* which feed upon agricultural crops and crop products, including corn and other true grasses. In particular examples, a coleopteran pest is selected from a list comprising *D.* v. *virgifera* LeConte (WCR); *D. barberi* Smith and Lawrence (NCR); *D. u. howardi* (SCR); *D. v. zeae* (MCR); *D. balteata* LeConte; *D. u. tenella*; *D. speciosa* Germar; *D. u. undecimpunctata* Mannerheim; and *Meligethes aeneus* Fabricius.

Contact (with an organism): As used herein, the term "contact with" or "uptake by" an organism (*e.g.,* a coleopteran), with regard to a nucleic acid molecule, includes internalization of the nucleic acid molecule into the organism, for example and without limitation: ingestion of the molecule by the organism (*e.g.,* by feeding); contacting the organism with a composition comprising the nucleic acid molecule; and soaking of organisms with a solution comprising the nucleic acid molecule.

Contig: As used herein the term "contig" refers to a DNA sequence that is reconstructed from a set of overlapping DNA segments derived from a single genetic source.

Corn plant: As used herein, the term "corn plant" refers to a plant of the species, *Zea mays* (maize).

Expression: As used herein, "expression" of a coding polynucleotide (for example, a gene or a transgene) refers to the process by which the coded information of a nucleic acid transcriptional unit (including, *e.g.*, gDNA or cDNA) is converted into an operational, non-operational, or structural part of a cell, often including the synthesis of a protein. Gene expression can be influenced by external signals; for example, exposure of a cell, tissue, or organism to an agent that increases or decreases gene expression. Expression of a gene can also be regulated anywhere in the pathway from DNA to RNA to protein. Regulation of gene expression occurs, for example, through controls acting on transcription, translation, RNA transport and processing, degradation of intermediary molecules such as mRNA, or through activation, inactivation, compartmentalization, or degradation of specific protein molecules after they have been made, or by combinations thereof. Gene expression can be measured at the RNA level or the protein level by any method known in the art, including, without limitation, northern blot, RT-PCR, western blot, or *in vitro*, *in situ*, or *in vivo* protein activity assay(s).

Genetic material: As used herein, the term "genetic material" includes all genes, and nucleic acid molecules, such as DNA and RNA.

Inhibition: As used herein, the term "inhibition," when used to describe an effect on a coding polynucleotide (for example, a gene), refers to a measurable decrease in the cellular level of mRNA transcribed from the coding polynucleotide and/or peptide, polypeptide, or protein product of the coding polynucleotide. In some examples, expression of a coding polynucleotide may be inhibited such that expression is approximately eliminated. "Specific inhibition" refers to the inhibition of a target coding polynucleotide without consequently affecting expression of other coding polynucleotides (*e.g.,* genes) in the cell wherein the specific inhibition is being accomplished.

Insect: As used herein with regard to pests, the term "insect pest" specifically includes coleopteran insect pests.

Isolated: An "isolated" biological component (such as a nucleic acid or protein) has been substantially separated, produced apart from, or purified away from other biological components in the cell of the organism in which the component naturally occurs (*i.e.,* other chromosomal and extra-chromosomal DNA and RNA, and proteins), while effecting a chemical or functional change in the component (*e.g.,* a nucleic acid may be isolated from a chromosome by breaking chemical bonds connecting the nucleic acid to the remaining DNA in the chromosome). Nucleic acid molecules and proteins that have been "isolated" include nucleic acid molecules and proteins purified by standard purification methods. The term also embraces nucleic acids and proteins prepared by recombinant expression in a host cell, as well as chemically-synthesized nucleic acid molecules, proteins, and peptides.

Nucleic acid molecule: As used herein, the term "nucleic acid molecule" may refer to a polymeric form of nucleotides, which may include both sense and anti-sense strands of RNA, cDNA, gDNA, and synthetic forms and mixed polymers of the above. A nucleotide or nucleobase may refer to a ribonucleotide, deoxyribonucleotide, or a modified form of either type of nucleotide. A "nucleic acid molecule" as used herein is synonymous with "nucleic acid" and "polynucleotide." A nucleic acid molecule is usually at least 10 bases in length, unless otherwise specified. By convention, the nucleotide sequence of a nucleic acid molecule is read from the 5' to the 3' end of the molecule. The "complement" of a nucleic acid molecule refers to a polynucleotide having nucleobases that may form base pairs with the nucleobases of the nucleic acid molecule (*i.e.,* A-T/U, and G-C).

Some embodiments include nucleic acids comprising a template DNA that is transcribed into an RNA molecule that is the complement of an mRNA molecule. In these embodiments, the complement of the nucleic acid transcribed into the mRNA molecule is present in the 5' to 3' orientation, such that RNA polymerase (which transcribes DNA in the 5' to 3' direction) will transcribe a nucleic acid from the complement that can hybridize to the mRNA molecule. Unless explicitly stated otherwise, or it is clear to be otherwise from the context, the term "complement" therefore refers to a polynucleotide having nucleobases, from 5' to 3', that may form base pairs with the nucleobases of a reference nucleic acid. Similarly, unless it is explicitly stated to be otherwise (or it is clear to be otherwise from the context), the "reverse complement" of a nucleic acid refers to the complement in reverse orientation. The foregoing is demonstrated in the following illustration:

| | |
|---|---|
| ATGATGATG | polynucleotide |
| TACTACTAC | "complement" of the polynucleotide |
| CATCATCAT | "reverse complement" of the polynucleotide |

Embodiments of the invention relate to hairpin RNA-forming RNAi molecules. In these RNAi molecules, both the complement of a nucleic acid to be targeted by RNA interference and the reverse complement may be found in the same molecule, such that the single-stranded RNA molecule may "fold over" and hybridize to itself over the region comprising the complementary and reverse complementary polynucleotides.

"Nucleic acid molecules" include all polynucleotides, for example: single- and double-stranded forms of DNA; single-stranded forms of RNA; and double-stranded forms of RNA (dsRNA). The term "nucleotide sequence" or "nucleic acid sequence" refers to both the sense and antisense strands of a nucleic acid as either individual single strands or in the duplex. The term "ribonucleic acid" (RNA) is inclusive of iRNA (inhibitory RNA), dsRNA (double stranded RNA), siRNA (small interfering RNA), shRNA (small hairpin RNA), mRNA (messenger RNA), miRNA (micro-RNA), hpRNA (hairpin RNA), tRNA (transfer RNAs, whether charged or discharged with a corresponding acylated amino acid), and cRNA (complementary RNA). The term "deoxyribonucleic acid" (DNA) is inclusive of cDNA, gDNA, and DNA-RNA hybrids. The terms "polynucleotide" and "nucleic acid," and "fragments" thereof will be understood by those in the art as a term that includes both gDNAs, ribosomal RNAs, transfer RNAs, messenger RNAs, operons, and smaller engineered polynucleotides that encode or may be adapted to encode, peptides, polypeptides, or proteins.

Oligonucleotide: An oligonucleotide is a short nucleic acid polymer. Oligonucleotides may be formed by cleavage of longer nucleic acid segments, or by polymerizing individual nucleotide precursors. Automated synthesizers allow the synthesis of oligonucleotides up to several hundred bases in length. Because oligonucleotides may bind to a complementary nucleic acid, they may be used as probes for detecting DNA or RNA. Oligonucleotides composed of DNA (oligodeoxyribonucleotides) may be used in PCR, a technique for the amplification of DNAs. In PCR, the oligonucleotide is typically referred to as a "primer," which allows a DNA polymerase to extend the oligonucleotide and replicate the complementary strand.

A nucleic acid molecule may include either or both naturally occurring and modified nucleotides linked together by naturally occurring and/or non-naturally occurring nucleotide linkages. Nucleic acid molecules may be modified chemically or biochemically, or may contain non-natural or derivatized nucleotide bases, as will be readily appreciated by those of skill in the art. Such modifications include, for example, labels, methylation, substitution of one or more of the naturally occurring nucleotides with an analog, internucleotide modifications (*e.g.,* uncharged linkages: for example, methyl phosphonates, phosphotriesters, phosphoramidates, carbamates, *etc*.; charged linkages: for example, phosphorothioates, phosphorodithioates, *etc*.; pendent moieties: for example, peptides; intercalators: for example, acridine, psoralen, *etc*.; chelators; alkylators; and modified linkages: for example, alpha anomeric nucleic acids, *etc.*). The term "nucleic acid molecule" also includes any topological conformation, including single-stranded, double-stranded, partially duplexed, triplexed, hairpinned, circular, and padlocked conformations.

As used herein with respect to DNA, the term "coding polynucleotide," "structural polynucleotide," or "structural nucleic acid molecule" refers to a polynucleotide that is ultimately translated into a polypeptide, *via* transcription and mRNA, when placed under the control of appropriate regulatory elements. With respect to RNA, the term "coding polynucleotide" refers to a polynucleotide that is translated into a peptide, polypeptide, or protein. The boundaries of a coding polynucleotide are determined by a translation start codon at the 5'-terminus and a translation stop codon at the 3'-terminus. Coding polynucleotides include, but are not limited to: gDNA; cDNA; EST; and recombinant polynucleotides.

As used herein, "transcribed non-coding polynucleotide" refers to segments of mRNA molecules such as 5'UTR, 3'UTR and intron segments that are not translated into a peptide, polypeptide, or protein. Further, "transcribed non-coding polynucleotide" refers to a nucleic acid that is transcribed into an RNA that functions in the cell, for example, structural RNAs (*e.g.,* ribosomal RNA (rRNA) as exemplified by 5S rRNA, 5.8S rRNA, 16S rRNA, 18S rRNA, 23S rRNA, and 28S rRNA, and the like); transfer RNA (tRNA); and snRNAs such as U4, U5, U6, and the like. Transcribed non-coding polynucleotides also include, for example and without limitation, small RNAs (sRNA), which term is often used to describe small bacterial non-coding RNAs; small nucleolar RNAs (snoRNA); microRNAs; small interfering RNAs (siRNA); Piwi-interacting RNAs (piRNA); and long non-coding RNAs. Further still, "transcribed non-coding polynucleotide" refers to a polynucleotide that may natively exist as an intragenic "spacer" in a nucleic acid and which is transcribed into an RNA molecule.

Lethal RNA interference: As used herein, the term "lethal RNA interference" refers to RNA interference that results in death or a reduction in viability of the subject individual to which, for example, a dsRNA, miRNA, siRNA, shRNA, and/or hpRNA is delivered.

Genome: As used herein, the term "genome" refers to chromosomal DNA found within the nucleus of a cell, and also refers to organelle DNA found within subcellular components of the cell. In some embodiments of the invention, a DNA molecule may be introduced into a plant cell, such that the DNA molecule is integrated into the genome of the plant cell. In these and further embodiments, the DNA molecule may be either integrated into the nuclear DNA of the plant cell, or integrated into the DNA of the chloroplast or mitochondrion of the plant cell. The term "genome," as it applies to bacteria, refers to both the chromosome and plasmids within the bacterial cell. In some embodiments of the invention, a DNA molecule may be introduced into a bacterium such that the DNA molecule is integrated into the genome of the bacterium. In these and further embodiments, the DNA molecule may be either chromosomally-integrated or located as or in a stable plasmid.

Sequence identity: The term "sequence identity" or "identity," as used herein in the context of two polynucleotides or polypeptides, refers to the residues in the sequences of the two molecules that are the same when aligned for maximum correspondence over a specified comparison window.

As used herein, the term "percentage of sequence identity" may refer to the value determined by comparing two optimally aligned sequences (*e.g.,* nucleic acid sequences or polypeptide sequences) of a molecule over a comparison window, wherein the portion of the sequence in the comparison window may comprise additions or deletions (*i.e.,* gaps) as compared to the reference sequence (which does not comprise additions or deletions) for optimal alignment of the two sequences. The percentage is calculated by determining the number of positions at which the identical nucleotide or amino acid residue occurs in both sequences to yield the number of matched positions, dividing the number of matched positions by the total number of positions in the comparison window, and multiplying the result by 100 to yield the percentage of sequence identity. A sequence that is identical at every position in comparison to a reference sequence is said to be 100% identical to the reference sequence, and vice-versa.

Methods for aligning sequences for comparison are well-known in the art. Various programs and alignment algorithms are described in, for example: Smith and Waterman (1981) Adv. Appl. Math. 2:482; Needleman and Wunsch (1970) J. Mol. Biol. 48:443; Pearson and Lipman (1988) Proc. Natl. Acad. Sci. U.S.A. 85:2444; Higgins and Sharp (1988) Gene 73:237-44; Higgins and Sharp (1989) CABIOS 5:151-3; Corpet et al. (1988) Nucleic Acids Res. 16:10881-90; Huang et al. (1992) Comp. Appl. Biosci. 8:155-65; Pearson et al. (1994) Methods Mol. Biol. 24:307-31; Tatiana et al. (1999) FEMS Microbiol. Lett. 174:247-50. A detailed consideration of sequence alignment methods and homology calculations can be found in, *e.g.,* Altschul et al. (1990) J. Mol. Biol. 215:403-10.

The National Center for Biotechnology Information (NCBI) Basic Local Alignment Search Tool (BLAST™; Altschul *et al.* (1990)) is available from several sources, including the National Center for Biotechnology Information (Bethesda, MD), and on the internet, for use in connection with several sequence analysis programs. A description of how to determine sequence identity using this program is available on the internet under the "help" section for BLAST™. For comparisons of nucleic acid sequences, the "Blast 2 sequences" function of the BLAST™ (Blastn) program may be employed using the default BLOSUM62 matrix set to default parameters. Nucleic acids with even greater sequence similarity to the sequences of the reference polynucleotides will show increasing percentage identity when assessed by this method.

Specifically hybridizable/Specifically complementary: As used herein, the terms "Specifically hybridizable" and "Specifically complementary" are terms that indicate a sufficient degree of complementarity such that stable and specific binding occurs between the nucleic acid molecule and a target nucleic acid molecule. Hybridization between two nucleic acid molecules involves the formation of an anti-parallel alignment between the nucleobases of the two nucleic acid molecules. The two molecules are then able to form hydrogen bonds with corresponding bases on the opposite strand to form a duplex molecule that, if it is sufficiently stable, is detectable using methods well known in the art. A polynucleotide need not be 100% complementary to its target nucleic acid to be specifically hybridizable. However, the amount of complementarity that must exist for hybridization to be specific is a function of the hybridization conditions used.

Hybridization conditions resulting in particular degrees of stringency will vary depending upon the nature of the hybridization method of choice and the composition and length of the hybridizing nucleic acids. Generally, the temperature of hybridization and the ionic strength (especially the $Na^+$ and/or $Mg^{++}$ concentration) of the hybridization buffer will determine the stringency of hybridization, though wash times also influence stringency. Calculations regarding hybridization conditions required for attaining particular degrees of stringency are known to those of ordinary skill in the art, and are discussed, for example, in Sambrook et al. (ed.) Molecular Cloning: A Laboratory Manual, 2nd ed., vol. 1-3, Cold Spring Harbor Laboratory Press, Cold Spring Harbor, NY, 1989, chapters 9 and 11; and Hames and Higgins (eds.) Nucleic Acid Hybridization, IRL Press, Oxford, 1985. Further detailed instruction and guidance with regard to the hybridization of nucleic acids may be found, for example, in Tijssen, "Overview of principles of hybridization and the strategy of nucleic acid probe assays," in Laboratory Techniques in Biochemistry and Molecular Biology-Hybridization with Nucleic Acid Probes, Part I, Chapter 2, Elsevier, NY, 1993; and Ausubel et al., Eds., Current Protocols in Molecular Biology, Chapter 2, Greene Publishing and Wiley-Interscience, NY, 1995.

As used herein, "stringent conditions" encompass conditions under which hybridization will only occur if there is less than 20% mismatch between the sequence of the hybridization molecule and a homologous polynucleotide within the target nucleic acid molecule. "Stringent conditions" include further particular levels of stringency. Thus, as used herein, "moderate stringency" conditions are those under which molecules with more than 20% sequence mismatch will not hybridize; conditions of "high stringency" are those under which sequences with more than 10% mismatch will not hybridize; and conditions of "very high stringency" are those under which sequences with more than 5% mismatch will not hybridize.

The following are representative, non-limiting hybridization conditions.

High Stringency condition (detects polynucleotides that share at least 90% sequence identity): Hybridization in 5x SSC buffer at 65 °C for 16 hours; wash twice in 2x SSC buffer at room temperature for 15 minutes each; and wash twice in 0.5x SSC buffer at 65 °C for 20 minutes each.

Moderate Stringency condition (detects polynucleotides that share at least 80% sequence identity): Hybridization in 5x-6x SSC buffer at 65-70 °C for 16-20 hours; wash twice in 2x SSC buffer at room temperature for 5-20 minutes each; and wash twice in 1x SSC buffer at 55-70 °C for 30 minutes each.

Non-stringent control condition (polynucleotides that share at least 50% sequence identity will hybridize): Hybridization in 6x SSC buffer at room temperature to 55 °C for 16-20 hours; wash at least twice in 2x-3x SSC buffer at room temperature to 55 °C for 20-30 minutes each.

As used herein, the term "substantially homologous" or "substantial homology," with regard to a nucleic acid, refers to a polynucleotide having contiguous nucleobases that hybridize under stringent conditions to the reference nucleic acid. For example, nucleic acids that are substantially homologous to a reference nucleic acid of any of SEQ ID NOs:1, 3-6, 17, 77, 84, 86, 88, 90, and 93 are those nucleic acids that hybridize under stringent conditions (*e.g.,* the Moderate Stringency conditions set forth, *supra*) to the reference nucleic acid of any of SEQ ID NOs:1, 3-6, 17, 77, 84, 86, 88, 90, and 93. Substantially homologous polynucleotides may have at least 80% sequence identity. For example, substantially homologous polynucleotides may have from about 80% to 100% sequence identity, such as 79%; 80%; about 81%; about 82%; about 83%; about 84%; about 85%; about 86%; about 87%; about 88%; about 89%; about 90%; about 91%; about 92%; about 93%; about 94% about 95%; about 96%; about 97%; about 98%; about 98.5%; about 99%; about 99.5%; and about 100%. Moderate Stringency conditions set forth, *supra*) to the reference nucleic acid of any of SEQ ID NOs:1, 3-6, 17, 77, 84, 86, 88, 90, and 93. Substantially homologous polynucleotides may have at least 80% sequence identity. For example, substantially homologous polynucleotides may have from about 80% to 100% sequence identity to the reference nucleic acid of SEQ ID NO: 1, such as 79%; 80%; about 81%; about 82%; about 83%; about 84%; about 85%; about 86%; about 87%; about 88%; about 89%; about 90%; about 91%; about 92%; about 93%; about 94% about 95%; about 96%; about 97%; about 98%; about 98.5%; about 99%; about 99.5%; and about 100%. For example, substantially homologous polynucleotides may have from about 80% to 100% sequence identity to the reference nucleic acid of SEQ ID NO: 3, such as 79%; 80%; about 81%; about 82%; about 83%; about 84%; about 85%; about 86%; about 87%; about 88%; about 89%; about 90%; about 91%; about 92%; about 93%; about 94% about 95%; about 96%; about 97%; about 98%; about 98.5%; about 99%; about 99.5%; and about 100%. For example, substantially homologous polynucleotides may have from about 80% to 100% sequence identity to the reference nucleic acid of SEQ ID NO: 4, such as 79%; 80%; about 81%; about 82%; about 83%; about 84%; about 85%; about 86%; about 87%; about 88%; about 89%; about 90%; about 91%; about 92%; about 93%; about 94% about 95%; about 96%; about 97%; about 98%; about 98.5%; about 99%; about 99.5%; and about 100%. For example, substantially homologous polynucleotides may have from about 80% to 100% sequence identity to the reference nucleic acid of SEQ ID NO: 5, such as 79%; 80%; about 81%; about 82%; about 83%; about 84%; about 85%; about 86%; about 87%; about 88%; about 89%; about 90%; about 91%; about 92%; about 93%; about 94% about 95%; about 96%; about 97%; about 98%; about 98.5%; about 99%; about 99.5%; and about 100%. For example, substantially homologous polynucleotides may have from about 80% to 100% sequence identity to the reference nucleic acid of SEQ ID NO: 6, such as 79%; 80%; about 81%; about 82%; about 83%; about 84%; about 85%; about 86%; about 87%; about 88%; about 89%; about 90%; about 91%; about 92%; about 93%; about 94% about 95%; about 96%; about 97%; about 98%; about 98.5%; about 99%; about 99.5%; and about 100%. For example, substantially homologous polynucleotides may have from about 80% to 100% sequence identity to the reference nucleic acid of SEQ ID NO: 17, such as 79%; 80%; about 81%; about 82%; about 83%; about 84%; about 85%; about 86%; about 87%; about 88%; about 89%; about 90%; about 91%; about 92%; about 93%; about 94% about 95%; about 96%; about 97%; about 98%; about 98.5%; about 99%; about 99.5%; and about 100%. For example, substantially homologous polynucleotides may have from about 80% to 100% sequence identity to the reference nucleic acid of SEQ ID NO: 77, such as 79%; 80%; about 81%; about 82%; about 83%; about 84%; about 85%; about 86%; about 87%; about 88%; about 89%; about 90%; about 91%; about 92%; about 93%; about 94% about 95%; about 96%; about 97%; about 98%; about 98.5%; about 99%; about 99.5%; and about 100%. For example, substantially homologous polynucleotides may have from about 80% to 100% sequence identity to the reference nucleic acid of SEQ ID NO: 84, such as 79%; 80%; about 81%; about 82%; about 83%; about 84%; about 85%; about 86%; about 87%; about 88%; about 89%; about 90%; about 91%; about 92%; about 93%; about 94% about 95%; about 96%; about 97%; about 98%; about 98.5%; about 99%; about 99.5%; and about 100%. For example, substantially homologous polynucleotides may have from about 80% to 100% sequence identity to the reference nucleic acid of SEQ ID NO: 86, such as 79%; 80%; about 81%; about 82%; about 83%; about 84%; about 85%; about 86%; about 87%; about 88%; about 89%; about 90%; about 91%; about 92%; about 93%; about 94% about 95%; about 96%; about 97%; about 98%; about 98.5%; about 99%; about 99.5%; and about 100%. For example, substantially homologous polynucleotides may have from about 80% to 100% sequence identity to the reference nucleic acid of SEQ ID NO: 88, such as 79%; 80%; about 81%; about 82%; about 83%; about 84%; about 85%; about 86%; about 87%; about 88%; about 89%; about 90%; about 91%; about 92%; about 93%; about 94% about 95%; about 96%; about 97%; about 98%; about 98.5%; about 99%; about 99.5%; and about 100%. For example, substantially homologous polynucleotides may have from about 80% to 100% sequence identity to the reference nucleic acid of SEQ ID NO: 90, such as 79%; 80%; about 81%; about 82%; about 83%; about 84%; about 85%; about 86%; about 87%; about 88%; about 89%; about 90%; about 91%; about 92%; about 93%; about 94% about 95%; about 96%; about 97%; about 98%; about 98.5%; about 99%; about 99.5%; and about 100%. For example, substantially homologous polynucleotides may have from about 80% to 100% sequence identity to the reference nucleic acid of SEQ ID NO: 93, such as 79%; 80%; about 81%; about 82%; about 83%; about 84%; about 85%; about 86%; about 87%; about 88%; about 89%; about 90%; about 91%; about 92%; about 93%; about 94% about 95%; about 96%; about 97%; about 98%; about 98.5%; about 99%; about 99.5%; and about 100%. The property of substantial homology is closely related to specific hybridization. For example, a nucleic acid molecule is specifically hybridizable when there is a sufficient degree of complementarity to avoid non-specific binding of the nucleic acid to non-target polynucleotides under conditions where specific binding is desired, for example, under stringent hybridization conditions.

As used herein, the term "ortholog" refers to a gene in two or more species that has evolved from a common ancestral nucleic acid, and may retain the same function in the two or more species.

As used herein, two nucleic acid molecules are said to exhibit "complete complementarity" when every nucleotide of a polynucleotide read in the 5' to 3' direction is complementary to every nucleotide of the other polynucleotide when read in the 3' to 5' direction. A polynucleotide that is complementary to a reference polynucleotide will exhibit a sequence identical to the reverse complement of the reference polynucleotide. These terms and descriptions are well defined in the art and are easily understood by those of ordinary skill in the art.

Operably linked: A first polynucleotide is operably linked with a second polynucleotide when the first polynucleotide is in a functional relationship with the second polynucleotide. When recombinantly produced, operably linked polynucleotides are generally contiguous, and, where necessary to join two protein-coding regions, in the same reading frame (*e.g.,* in a translationally fused ORF). However, nucleic acids need not be contiguous to be operably linked.

The term, "operably linked," when used in reference to a regulatory genetic element and a coding polynucleotide, means that the regulatory element affects the expression of the linked coding polynucleotide. "Regulatory elements," or "control elements," refer to polynucleotides that influence the timing and level/amount of transcription, RNA processing or stability, or translation of the associated coding polynucleotide. Regulatory elements may include promoters; translation leaders; introns; enhancers; stem-loop structures; repressor binding polynucleotides; polynucleotides with a termination sequence; polynucleotides with a polyadenylation recognition sequence; *etc.* Particular regulatory elements may be located upstream and/or downstream of a coding polynucleotide operably linked thereto. Also, particular regulatory elements operably linked to a coding polynucleotide may be located on the associated complementary strand of a double-stranded nucleic acid molecule.

Promoter: As used herein, the term "promoter" refers to a region of DNA that may be upstream from the start of transcription, and that may be involved in recognition and binding of RNA polymerase and other proteins to initiate transcription. A promoter may be operably linked to a coding polynucleotide for expression in a cell, or a promoter may be operably linked to a polynucleotide encoding a signal peptide which may be operably linked to a coding polynucleotide for expression in a cell. A "plant promoter" may be a promoter capable of initiating transcription in plant cells. Examples of promoters under developmental control include promoters that preferentially initiate transcription in certain tissues, such as leaves, roots, seeds, fibers, xylem vessels, tracheids, or sclerenchyma. Such promoters are referred to as "tissue-preferred". Promoters which initiate transcription only in certain tissues are referred to as "tissue-specific". A "cell type-specific" promoter primarily drives expression in certain cell types in one or more organs, for example, vascular cells in roots or leaves. An "inducible" promoter may be a promoter which may be under environmental control. Examples of environmental conditions that may initiate transcription by inducible promoters include anaerobic conditions and the presence of light. Tissue-specific, tissue-preferred, cell type specific, and inducible promoters constitute the class of "non-constitutive" promoters. A "constitutive" promoter is a promoter which may be active under most environmental conditions or in most tissue or cell types.

Any inducible promoter can be used in some embodiments of the invention. *See* Ward et al. (1993) Plant Mol. Biol. 22:361-366. With an inducible promoter, the rate of transcription increases in response to an inducing agent. Exemplary inducible promoters include, but are not limited to: Promoters from the ACEI system that respond to copper; *In2* gene from maize that responds to benzenesulfonamide herbicide safeners; Tet repressor from Tn10; and the inducible promoter from a steroid hormone gene, the transcriptional activity of which may be induced by a glucocorticosteroid hormone (Schena et al. (1991) Proc. Natl. Acad. Sci. USA 88:0421).

Exemplary constitutive promoters include, but are not limited to: Promoters from plant viruses, such as the 35S promoter from Cauliflower Mosaic Virus (CaMV); promoters from rice actin genes; ubiquitin promoters; pEMU; MAS; maize H3 histone promoter; and the ALS promoter, *Xba1*/*NcoI* fragment 5' to the *Brassica napus ALS3* structural gene (or a polynucleotide similar to said *Xba1*/*NcoI* fragment) (International PCT Publication No. WO96/30530).

Additionally, any tissue-specific or tissue-preferred promoter may be utilized in some embodiments of the invention. Plants transformed with a nucleic acid molecule comprising a coding polynucleotide operably linked to a tissue-specific promoter may produce the product of the coding polynucleotide exclusively, or preferentially, in a specific tissue. Exemplary tissue-specific or tissue-preferred promoters include, but are not limited to: A seed-preferred promoter, such as that from the phaseolin gene; a leaf-specific and light-induced promoter such as that from *cab* or *rubisco*; an anther-specific promoter such as that from *LAT52*; a pollen-specific promoter such as that from *Zm13*; and a microspore-preferred promoter such as that from *apg.*

*Brassica* plant: As used herein, the terms "rape," "oilseed rape," "rapeseed," and "canola" are used interchangeably, and refer to a plant of the species *Brassica*; for example, *B. napus.*

Transformation: As used herein, the term "transformation" or "transduction" refers to the transfer of one or more nucleic acid molecule(s) into a cell. A cell is "transformed" by a nucleic acid molecule transduced into the cell when the nucleic acid molecule becomes stably replicated by the cell, either by incorporation of the nucleic acid molecule into the cellular genome, or by episomal replication. As used herein, the term "transformation" encompasses all techniques by which a nucleic acid molecule can be introduced into such a cell. Examples include, but are not limited to: transfection with viral vectors; transformation with plasmid vectors; electroporation (Fromm et al. (1986) Nature 319:791-3); lipofection (Felgner et al. (1987) Proc. Natl. Acad. Sci. USA 84:7413-7); microinjection (Mueller et al. (1978) Cell 15:579-85); *Agrobacterium*-mediated transfer (Fraley et al. (1983) Proc. Natl. Acad. Sci. USA 80:4803-7); direct DNA uptake; and microprojectile bombardment (Klein et al. (1987) Nature 327:70).

Transgene: An exogenous nucleic acid. In some examples, a transgene may be a DNA that encodes one or both strand(s) of an RNA capable of forming a dsRNA molecule that comprises a polynucleotide that is complementary to a nucleic acid molecule found in a coleopteran pest. In further examples, a transgene may be an antisense polynucleotide, wherein expression of the antisense polynucleotide inhibits expression of a target nucleic acid. In still further examples, a transgene may be a gene (*e.g.,* a herbicide-tolerance gene, a gene encoding an industrially or pharmaceutically useful compound, or a gene encoding a desirable agricultural trait). In these and other examples, a transgene may contain regulatory elements operably linked to a coding polynucleotide of the transgene (*e.g.,* a promoter).

Vector: A nucleic acid molecule as introduced into a cell, for example, to produce a transformed cell. A vector may include genetic elements that permit it to replicate in the host cell, such as an origin of replication. Examples of vectors include, but are not limited to: a plasmid; cosmid; bacteriophage; or virus that carries exogenous DNA into a cell. A vector may also include one or more genes, including ones that produce antisense molecules, and/or selectable marker genes and other genetic elements known in the art. A vector may transduce, transform, or infect a cell, thereby causing the cell to express the nucleic acid molecules and/or proteins encoded by the vector. A vector optionally includes materials to aid in achieving entry of the nucleic acid molecule into the cell (*e.g.,* a liposome, protein coating, *etc.*).

Yield: A stabilized yield of about 100% or greater relative to the yield of check varieties in the same growing location growing at the same time and under the same conditions. In particular embodiments, "improved yield" or "improving yield" means a cultivar having a stabilized yield of 105% or greater relative to the yield of check varieties in the same growing location containing significant densities of the coleopteran pests that are injurious to that crop growing at the same time and under the same conditions, which are targeted by the compositions and methods herein.

Unless specifically indicated or implied, the terms "a," "an," and "the" signify "at least one," as used herein.

Unless otherwise specifically explained, all technical and scientific terms used herein have the same meaning as commonly understood by those of ordinary skill in the art to which this disclosure belongs. Definitions of common terms in molecular biology can be found in, for example, Lewin's Genes X, Jones & Bartlett Publishers, 2009 (ISBN 10 0763766321); Krebs et al. (eds.), The Encyclopedia of Molecular Biology, Blackwell Science Ltd., 1994 (ISBN 0-632-02182-9); and Meyers R.A. (ed.), Molecular Biology and Biotechnology: A Comprehensive Desk Reference, VCH Publishers, Inc., 1995 (ISBN 1-56081-569-8). All percentages are by weight and all solvent mixture proportions are by volume unless otherwise noted. All temperatures are in degrees Celsius.

## *IV. Nucleic Acid Molecules Comprising an Insect Pest Sequence*

### A. Overview

Described herein are nucleic acid molecules useful for the control of insect pestsThe insect pest is a coleopteran insect pest. Described nucleic acid molecules include target polynucleotides (*e.g.,* native genes, and non-coding polynucleotides), dsRNAs, siRNAs, shRNAs, hpRNAs, and miRNAs. For example, hpRNA molecules are described in some embodiments that may be specifically complementary to all or part of one or more native nucleic acids in a coleopteran pest. In these and further embodiments, the native nucleic acid(s) may be one or more target gene(s), the product of which may be, for example and without limitation: involved in a metabolic process or involved in larval development. Nucleic acid molecules described herein, when introduced into a cell comprising at least one native nucleic acid(s) to which the nucleic acid molecules are specifically complementary, may initiate RNAi in the cell, and consequently reduce or eliminate expression of the native nucleic acid(s). In some examples, reduction or elimination of the expression of a target gene by a nucleic acid molecule specifically complementary thereto may result in reduction or cessation of growth, development, and/or feeding in the coleopteran pest.

In some embodiments, at least one target gene in an insect pest may be selected, wherein the target gene comprises a *ncm* polynucleotide. In particular examples, a target gene in a coleopteran pest is selected, wherein the target gene comprises a polynucleotide selected from among SEQ ID NOs:1, 3-6, 77, 84, 86, 88, 90, and 93.

In some embodiments, a target gene may be a nucleic acid molecule comprising a polynucleotide that can be reverse translated *in silico* to a polypeptide comprising a contiguous amino acid sequence that is at least about 85% identical (*e.g.,* at least 84%, 85%, about 90%, about 95%, about 96%, about 97%, about 98%, about 99%, about 100%, or 100% identical) to the amino acid sequence of a protein product of a *ncm* polynucleotide. A target gene may be any *ncm* polynucleotide in an insect pest, the post-transcriptional inhibition of which has a deleterious effect on the growth, and/or survival of the pest, for example, to provide a protective benefit against the pest to a plant. In particular examples, a target gene is a nucleic acid molecule comprising a polynucleotide that can be reverse translated *in silico* to a polypeptide comprising a contiguous amino acid sequence that is at least about 85% identical, about 90% identical, about 95% identical, about 96% identical, about 97% identical, about 98% identical, about 99% identical, about 100% identical, or 100% identical to the amino acid sequence of SEQ ID NOs:2, 85, 87, 89, and 94. In particular examples, a target gene is a nucleic acid molecule comprising a polynucleotide that can be reverse translated *in silico* to a polypeptide comprising a contiguous amino acid sequence that is at least about 85% identical, about 90% identical, about 95% identical, about 96% identical, about 97% identical, about 98% identical, about 99% identical, about 100% identical, or 100% identical to the amino acid sequence of SEQ ID NO:2. In particular examples, a target gene is a nucleic acid molecule comprising a polynucleotide that can be reverse translated *in silico* to a polypeptide comprising a contiguous amino acid sequence that is at least about 85% identical, about 90% identical, about 95% identical, about 96% identical, about 97% identical, about 98% identical, about 99% identical, about 100% identical, or 100% identical to the amino acid sequence of SEQ ID NO:85. In particular examples, a target gene is a nucleic acid molecule comprising a polynucleotide that can be reverse translated *in silico* to a polypeptide comprising a contiguous amino acid sequence that is at least about 85% identical, about 90% identical, about 95% identical, about 96% identical, about 97% identical, about 98% identical, about 99% identical, about 100% identical, or 100% identical to the amino acid sequence of SEQ ID NO:87. In particular examples, a target gene is a nucleic acid molecule comprising a polynucleotide that can be reverse translated *in silico* to a polypeptide comprising a contiguous amino acid sequence that is at least about 85% identical, about 90% identical, about 95% identical, about 96% identical, about 97% identical, about 98% identical, about 99% identical, about 100% identical, or 100% identical to the amino acid sequence of SEQ ID NO:89. In particular examples, a target gene is a nucleic acid molecule comprising a polynucleotide that can be reverse translated *in silico* to a polypeptide comprising a contiguous amino acid sequence that is at least about 85% identical, about 90% identical, about 95% identical, about 96% identical, about 97% identical, about 98% identical, about 99% identical, about 100% identical, or 100% identical to the amino acid sequence of SEQ ID NO:94.

Provided according to the invention are DNAs, the expression of which results in an RNA molecule comprising a polynucleotide that is specifically complementary to all or part of a native RNA molecule that is encoded by a coding polynucleotide in an insect (*e.g.,* coleopteran) pest. In some embodiments, after ingestion of the expressed RNA molecule by an insect pest, down-regulation of the coding polynucleotide in cells of the pest may be obtained. In particular embodiments, down-regulation of the coding sequence in cells of the insect pest may result in a deleterious effect on the growth and/or development of the pest.

In some embodiments, target polynucleotides include transcribed non-coding RNAs, such as 5'UTRs; 3'UTRs; spliced leaders; introns; outrons (*e.g.,* 5'UTR RNA subsequently modified in trans splicing); donatrons (*e.g.,* non-coding RNA required to provide donor sequences for *trans* splicing); and other non-coding transcribed RNA of target insect pest genes. Such polynucleotides may be derived from both mono-cistronic and poly-cistronic genes.

Thus, also described herein in connection with some embodiments are iRNA molecules (hpRNAs) that comprise at least one polynucleotide that is specifically complementary to all or part of a target nucleic acid in an insect (*e.g.,* coleopteran) pest. In some embodiments an iRNA molecule may comprise polynucleotide(s) that are complementary to all or part of a plurality of target nucleic acids; for example, 2, 3, 4, 5, 6, 7, 8, 9, 10, or more target nucleic acids. In particular embodiments, an iRNA molecule may be produced *in vitro,* or *in vivo* by a genetically-modified organism, such as a plant or bacterium. Also disclosed are cDNAs that may be used for the production of dsRNA molecules, siRNA molecules, miRNA molecules, shRNA molecules, and/or hpRNA molecules that are specifically complementary to all or part of a target nucleic acid in an insect pest. Further described are recombinant DNA constructs for use in achieving stable transformation of particular host targets. Transformed host targets may express effective levels of dsRNA, siRNA, miRNA, shRNA, and/or hpRNA molecules from the recombinant DNA constructs. Therefore, also described is a plant transformation vector comprising at least one polynucleotide operably linked to a heterologous promoter functional in a plant cell, wherein expression of the polynucleotide(s) results in an RNA molecule comprising a string of contiguous nucleobases that is specifically complementary to all or part of a target nucleic acid in an insect pest.

In particular examples, nucleic acid molecules useful for the control of insect (*e.g.,* coleopteran) pests may include: all or part of a native nucleic acid isolated from *Diabrotica* comprising a *ncm* polynucleotide (*e.g.,* any of SEQ ID NOs:1, 3-6, and 77); all or part of a native nucleic acid isolated from *Meligethes aeneus* comprising a *ncm* polynucleotide (*e.g.,* any of SEQ ID NOs:84, 86, 88, 90, and 93; DNAs that when expressed result in a RNA molecule comprising a polynucleotide that is specifically complementary to all or part of a native RNA molecule that is encoded by *ncm*; iRNA molecules (e.*g.,* dsRNAs, siRNAs, miRNAs, shRNAs, and hpRNAs) that comprise at least one polynucleotide that is specifically complementary to all or part of *ncm*; cDNAs that may be used for the production of dsRNA molecules, siRNA molecules, miRNA molecules, shRNA molecules, and/or hpRNA molecules that are specifically complementary to all or part of *ncm*; and recombinant DNA constructs for use in achieving stable transformation of particular host targets, wherein a transformed host target comprises one or more of the foregoing nucleic acid molecules.

### B. Nucleic Acid Molecules

The present invention provides, *inter alia,* iRNA (hpRNA) molecules that inhibit target gene expression in a cell, tissue, or organ of an insect (*e.g.,* coleopteran) pest; and DNA molecules capable of being expressed as an iRNA molecule in a cell or microorganism to inhibit target gene expression in a cell, tissue, or organ of an insect pest.

Some embodiments of the invention provide an isolated nucleic acid molecule comprising at least one (*e.g.,* one, two, three, or more) polynucleotide(s) selected from the group consisting of: SEQ ID NOs:1, 77, 84, 86, 88, and 93; the complement of SEQ ID NO:1, 77, 84, 86, 88, or 93; a fragment of at least 50 contiguous nucleotides of SEQ ID NO:84, 86, 88, or 93; the complement of at least 50 contiguous nucleotides of SEQ ID NO: 84, 86, 88, or 93; a native coding polynucleotide of a *Meligethes* organism (*e.g.,* PB) comprising SEQ ID NO:84, 86, 88, or 93; the complement of a native coding polynucleotide of a *Meligethes* organism comprising SEQ ID NO:84, 86, 88, or 93; a fragment of at least 15 contiguous nucleotides of a native coding polynucleotide of a *Meligethes* organism comprising SEQ ID NO:84, 86, 88, or 93; and the complement of a fragment of at least 15 contiguous nucleotides of a native coding polynucleotide of a *Meligethes* organism comprising SEQ ID NO:84, 86, 88, or 93. In particular embodiments, contact with or uptake by an insect (*e.g.,* coleopteran) pest of an iRNA transcribed from the isolated polynucleotide inhibits the growth, development, and/or feeding of the pest.

In some embodiments, an isolated nucleic acid molecule of the invention may comprise at least one (*e.g.,* one, two, three, or more) polynucleotide(s) selected from the group consisting of: a native coding polynucleotide of a *Meligethes* organism comprising SEQ ID NO:84, 86, 88, and/or 93; the complement of a native coding polynucleotide of a *Meligethes* organism comprising SEQ ID NO:84, 86, 88, and/or 93; a fragment of at least 50 contiguous nucleotides of a native coding polynucleotide of a *Meligethes* organism comprising SEQ ID NO:84, 86, 88, and/or 93; and the complement of a fragment of at least 50 contiguous nucleotides of a native coding polynucleotide of a *Meligethes* organism comprising SEQ ID NO:84, 86, 88, and/or 93. In particular embodiments, contact with or uptake by a coleopteran pest of the isolated polynucleotide inhibits the growth, development, and/or feeding of the pest.

In certain embodiments, dsRNA molecules provided by the invention comprise polynucleotides complementary to a transcript from a target gene comprising any of SEQ ID NOs: 84, 86, 88, and 93, and fragments thereof, the inhibition of which target gene in an insect pest results in the reduction or removal of a polypeptide or polynucleotide agent that is essential for the pest's growth, development, or other biological function. A selected target gene may exhibit from about 80% to about 100% sequence identity to any of SEQ ID NOs:1, 77, 84, 86, 88, and 93; a contiguous fragment of SEQ ID NO:1, 77, 84, 86, 88, and/or 93; and the complement of any of the foregoing. For example, a selected target gene may exhibit 79%; 80%; about 81%; about 82%; about 83%; about 84%; about 85%; about 86%; about 87%; about 88%; about 89%; about 90%; about 91%; about 92%; about 93%; about 94% about 95%; about 96%; about 97%; about 98%; about 98.5%; about 99%; about 99.5%; or about 100% sequence identity to any of SEQ ID NOs:1, 3-6, 77, 84, 86, 88, 90, and 93; a contiguous fragment of any of SEQ ID NOs:1, 3-6, 77, 84, 86, 88, 90, and 93; and the complement of any of the foregoing.

In some embodiments, a DNA molecule capable of being expressed as an iRNA molecule in a cell or microorganism to inhibit target gene expression may comprise a single polynucleotide that is specifically complementary to all or part of a native polynucleotide found in one or more target insect pest species (*e.g.,* a coleopteran pest species), or the DNA molecule can be constructed as a chimera from a plurality of such specifically complementary polynucleotides.

In some embodiments, a nucleic acid molecule may comprise a first and a second polynucleotide separated by a "spacer." A spacer may be a region comprising any sequence of nucleotides that facilitates secondary structure formation between the first and second polynucleotides, where this is desired. In one embodiment, the spacer is part of a sense or antisense coding polynucleotide for mRNA. The spacer may alternatively comprise any combination of nucleotides or homologues thereof that are capable of being linked covalently to a nucleic acid molecule.

For example, in some embodiments, the DNA molecule may comprise a polynucleotide coding for one or more different iRNA molecules, wherein each of the different iRNA molecules comprises a first polynucleotide and a second polynucleotide, wherein the first and second polynucleotides are complementary to each other. The first and second polynucleotides may be connected within an RNA molecule by a spacer. The spacer may constitute part of the first polynucleotide or the second polynucleotide. Expression of an RNA molecule comprising the first and second nucleotide polynucleotides may lead to the formation of a dsRNA molecule, by specific intramolecular base-pairing of the first and second nucleotide polynucleotides. The first polynucleotide or the second polynucleotide may be substantially identical to a polynucleotide (*e.g.,* a target gene, or transcribed non-coding polynucleotide) native to an insect pest (*e.g.,* a coleopteran pest), a derivative thereof, or a complementary polynucleotide thereto.

dsRNA nucleic acid molecules comprise double strands of polymerized ribonucleotides, and may include modifications to either the phosphate-sugar backbone or the nucleoside. Modifications in RNA structure may be tailored to allow specific inhibition. In one embodiment, dsRNA molecules may be modified through a ubiquitous enzymatic process so that siRNA molecules may be generated. This enzymatic process may utilize an RNase III enzyme, such as DICER in eukaryotes, either *in vitro* or *in vivo*. *See* Elbashir et al. (2001) Nature 411:494-8; and Hamilton and Baulcombe (1999) Science 286(5441):950-2. DICER or functionally-equivalent RNase III enzymes cleave larger dsRNA strands and/or hpRNA molecules into smaller oligonucleotides (*e.g.,* siRNAs), each of which is about 19-25 nucleotides in length. The siRNA molecules produced by these enzymes have 2 to 3 nucleotide 3' overhangs, and 5' phosphate and 3' hydroxyl termini. The siRNA molecules generated by RNase III enzymes are unwound and separated into single-stranded RNA in the cell. The siRNA molecules then specifically hybridize with RNAs transcribed from a target gene, and both RNA molecules are subsequently degraded by an inherent cellular RNA-degrading mechanism. This process may result in the effective degradation or removal of the RNA encoded by the target gene in the target organism. The outcome is the post-transcriptional silencing of the targeted gene. In some embodiments, siRNA molecules produced by endogenous RNase III enzymes from heterologous nucleic acid molecules may efficiently mediate the down-regulation of target genes in insect pests.

In some embodiments, a nucleic acid molecule may include at least one non-naturally occurring polynucleotide that can be transcribed into a single-stranded RNA molecule capable of forming a dsRNA molecule *in vivo* through intermolecular hybridization. Such dsRNAs typically self-assemble, and can be provided in the nutrition source of an insect (*e.g.,* coleopteran) pest to achieve the post-transcriptional inhibition of a target gene. In these and further embodiments, a nucleic acid molecule may comprise two different non-naturally occurring polynucleotides, each of which is specifically complementary to a different target gene in an insect pest. When such a nucleic acid molecule is provided as a dsRNA molecule to, for example, a coleopteran pest, the dsRNA molecule inhibits the expression of at least two different target genes in the pest.

### C. Obtaining Nucleic Acid Molecules

A variety of polynucleotides in insect (*e.g.,* coleopteran) pests may be used as targets for the design of nucleic acid molecules, such as iRNAs and DNA molecules encoding iRNAs. Selection of native polynucleotides is not, however, a straight-forward process. For example, only a small number of native polynucleotides in a coleopteran pest will be effective targets. It cannot be predicted with certainty whether a particular native polynucleotide can be effectively down-regulated by nucleic acid molecules of the invention, or whether down-regulation of a particular native polynucleotide will have a detrimental effect on the growth, development, and/or viability of an insect pest. The vast majority of native coleopteran pest polynucleotides, such as ESTs isolated therefrom (for example, the coleopteran pest polynucleotides listed in U.S. Patent 7,612,194), do not have a detrimental effect on the growth and/or viability of the pest. Neither is it predictable which of the native polynucleotides that may have a detrimental effect on an insect pest are able to be used in recombinant techniques for expressing nucleic acid molecules complementary to such native polynucleotides in a host plant and providing the detrimental effect on the pest upon feeding without causing harm to the host plant.

In some embodiments, nucleic acid molecules (*e.g.,* dsRNA molecules to be provided in the host plant of an insect (*e.g.,* coleopteran) pest) are selected to target cDNAs that encode proteins or parts of proteins essential for pest development, such as polypeptides involved in metabolic or catabolic biochemical pathways, cell division, energy metabolism, digestion, host plant recognition, and the like. As described herein, ingestion of compositions by a target pest organism containing one or more dsRNAs, at least one segment of which is specifically complementary to at least a substantially identical segment of RNA produced in the cells of the target pest organism, can result in the death or other inhibition of the target. A polynucleotide, either DNA or RNA, derived from an insect pest can be used to construct plant cells resistant to infestation by the pests. The host plant of the coleopteran pest (*e.g., Z. mays* or *Brassica*), for example, can be transformed to contain one or more polynucleotides derived from the coleopteran pest as provided herein. The polynucleotide transformed into the host may encode one or more RNAs that form into a dsRNA structure in the cells or biological fluids within the transformed host, thus making the dsRNA available if/when the pest forms a nutritional relationship with the transgenic host. This may result in the suppression of expression of one or more genes in the cells of the pest, and ultimately death or inhibition of its growth or development.

Thus, in some embodiments, a gene is targeted that is essentially involved in the growth and/or development of an insect (*e.g.,* coleopteran) pest. Other target genes for use in the present invention may include, for example, those that play important roles in pest viability, movement, migration, growth, development, infectivity, and establishment of feeding sites. A target gene may therefore be a housekeeping gene or a transcription factor. Additionally, a native insect pest polynucleotide for use in the present invention may also be derived from a homolog (*e.g.,* an ortholog), of a plant, viral, bacterial or insect gene, the function of which is known to those of skill in the art, and the polynucleotide of which is specifically hybridizable with a target gene in the genome of the target pest. Methods of identifying a homolog of a gene with a known nucleotide sequence by hybridization are known to those of skill in the art.

Some aspects of the disclosure provide methods for obtaining a nucleic acid molecule comprising a polynucleotide for producing an iRNA (*e.g.,* dsRNA, siRNA, miRNA, shRNA, and hpRNA) molecule. One such aspect of the disclosure comprises: (a) analyzing one or more target gene(s) for their expression, function, and phenotype upon dsRNA-mediated gene suppression in an insect (*e.g.,* coleopteran) pest; (b) probing a cDNA or gDNA library with a probe comprising all or a portion of a polynucleotide or a homolog thereof from a targeted pest that displays an altered (*e.g.,* reduced) growth or development phenotype in a dsRNA-mediated suppression analysis; (c) identifying a DNA clone that specifically hybridizes with the probe; (d) isolating the DNA clone identified in step (b); (e) sequencing the cDNA or gDNA fragment that comprises the clone isolated in step (d), wherein the sequenced nucleic acid molecule comprises all or a substantial portion of the RNA or a homolog thereof; and (f) chemically synthesizing all or a substantial portion of a gene, or an siRNA, miRNA, hpRNA, mRNA, shRNA, or dsRNA.

In further aspects of the disclosure, a method for obtaining a nucleic acid fragment comprising a polynucleotide for producing a substantial portion of an iRNA (*e.g.,* dsRNA, siRNA, miRNA, shRNA, and hpRNA) molecule includes: (a) synthesizing first and second oligonucleotide primers specifically complementary to a portion of a native polynucleotide from a targeted insect (e.g., coleopteran) pest; and (b) amplifying a cDNA or gDNA insert present in a cloning vector using the first and second oligonucleotide primers of step (a), wherein the amplified nucleic acid molecule comprises a substantial portion of a siRNA, miRNA, hpRNA, mRNA, shRNA, or dsRNA molecule.

Nucleic acids can be isolated, amplified, or produced by a number of approaches. For example, an iRNA (*e.g.,* dsRNA, siRNA, miRNA, shRNA, and hpRNA) molecule may be obtained by PCR amplification of a target polynucleotide (*e.g.,* a target gene or a target transcribed non-coding polynucleotide) derived from a gDNA or cDNA library, or portions thereof. DNA or RNA may be extracted from a target organism, and nucleic acid libraries may be prepared therefrom using methods known to those ordinarily skilled in the art. gDNA or cDNA libraries generated from a target organism may be used for PCR amplification and sequencing of target genes. A confirmed PCR product may be used as a template for *in vitro* transcription to generate sense and antisense RNA with minimal promoters. Alternatively, nucleic acid molecules may be synthesized by any of a number of techniques (*See, e.g.,* Ozaki et al. (1992) Nucleic Acids Research, 20: 5205-5214; and Agrawal et al. (1990) Nucleic Acids Research, 18: 5419-5423), including use of an automated DNA synthesizer (for example, a P.E. Biosystems, Inc. (Foster City, Calif.) model 392 or 394 DNA/RNA Synthesizer), using standard chemistries, such as phosphoramidite chemistry. *See, e.g.,* Beaucage et al. (1992) Tetrahedron, 48: 2223-2311; U.S. Patents 4,980,460, 4,725,677, 4,415,732, 4,458,066, and 4,973,679. Alternative chemistries resulting in non-natural backbone groups, such as phosphorothioate, phosphoramidate, and the like, can also be employed.

A hpRNA molecule of the present invention may be produced chemically or enzymatically by one skilled in the art through manual or automated reactions, or *in vivo* in a cell comprising a nucleic acid molecule comprising a polynucleotide encoding the hpRNA molecule. RNA may also be produced by partial or total organic synthesis- any modified ribonucleotide can be introduced by *in vitro* enzymatic or organic synthesis. An RNA molecule may be synthesized by a cellular RNA polymerase or a bacteriophage RNA polymerase (*e.g.,* T3 RNA polymerase, T7 RNA polymerase, and SP6 RNA polymerase). Expression constructs useful for the cloning and expression of polynucleotides are known in the art. *See, e.g.,* International PCT Publication No. WO97/32016; and U.S. Patents 5,593,874, 5,698,425, 5,712,135, 5,789,214, and 5,804,693. RNA molecules that are synthesized chemically or by *in vitro* enzymatic synthesis may be purified prior to introduction into a cell. For example, RNA molecules can be purified from a mixture by extraction with a solvent or resin, precipitation, electrophoresis, chromatography, or a combination thereof. Alternatively, RNA molecules that are synthesized chemically or by *in vitro* enzymatic synthesis may be used with no or a minimum of purification, for example, to avoid losses due to sample processing. The RNA molecules may be dried for storage or dissolved in an aqueous solution. The solution may contain buffers or salts to promote annealing, and/or stabilization of dsRNA molecule duplex strands.

In embodiments, a dsRNA molecule may be formed by a single self-complementary RNA strand or from two complementary RNA strands. dsRNA molecules may be synthesized either *in vivo* or *in vitro.* An endogenous RNA polymerase of the cell may mediate transcription of the one or two RNA strands *in vivo,* or cloned RNA polymerase may be used to mediate transcription *in vivo* or *in vitro.* Post-transcriptional inhibition of a target gene in an insect pest may be host-targeted by specific transcription in an organ, tissue, or cell type of the host (*e.g.,* by using a tissue-specific promoter); stimulation of an environmental condition in the host (*e.g.,* by using an inducible promoter that is responsive to infection, stress, temperature, and/or chemical inducers); and/or engineering transcription at a developmental stage or age of the host (*e.g.,* by using a developmental stage-specific promoter). RNA strands that form a dsRNA molecule, whether transcribed *in vitro* or *in vivo,* may or may not be polyadenylated, and may or may not be capable of being translated into a polypeptide by a cell's translational apparatus.

## D. Recombinant Vectors and Host Cell Transformation

In some embodiments, the invention also provides a DNA molecule for introduction into a cell (*e.g.,* a bacterial cell, a yeast cell, or a plant cell), wherein the DNA molecule comprises a polynucleotide that, upon expression to RNA and ingestion by an insect (*e.g.,* coleopteran) pest, achieves suppression of a target gene in a cell, tissue, or organ of the pest. Thus, some embodiments provide a recombinant nucleic acid molecule comprising a polynucleotide capable of being expressed as an iRNA (hpRNA) molecule in a plant cell to inhibit target gene expression in an insect pest. In order to initiate or enhance expression, such recombinant nucleic acid molecules may comprise one or more regulatory elements, which regulatory elements may be operably linked to the polynucleotide capable of being expressed as an iRNA. Methods to express a gene suppression molecule in plants are known, and may be used to express a polynucleotide of the present invention. *See*, *e.g.,* International PCT Publication No. WO06/073727; and U.S. Patent Publication No. 2006/0200878 A1)

In embodiments, a recombinant DNA molecule of the invention comprises a polynucleotide encoding an RNA that may form a dsRNA molecule. Such recombinant DNA molecules may encode RNAs that may form dsRNA molecules capable of inhibiting the expression of endogenous target gene(s) in an insect (*e.g.,* coleopteran) pest cell upon ingestion. In embodiments, a transcribed RNA forms a dsRNA molecule that may be provided in a stabilized form; *e.g.,* as a hairpin and stem and loop structure.

In some embodiments, one strand of a dsRNA molecule may be formed by transcription from a polynucleotide which is substantially homologous to a polynucleotide selected from the group consisting of SEQ ID NOs: 84, 86, 88, and 93; the complements of SEQ ID NOs: 84, 86, 88, and 93; a fragment of at least 50 contiguous nucleotides of any of SEQ ID NOs: 84, 86, 88, and 93 (*e.g.,* SEQ ID NO:90); the complement of a fragment of at least 50 contiguous nucleotides of any of SEQ ID NOs: 84, 86, 88, and 93; a native coding polynucleotide of a *Meligethes* organism (*e.g.,* PB) comprising any of SEQ ID NOs:84, 86, 88, 90, and/or 93; the complement of a native coding polynucleotide of a *Meligethes* organism comprising any of SEQ ID NOs:84, 86, 88, 90, and/or 93; a fragment of at least 50 contiguous nucleotides of a native coding polynucleotide of a *Meligethes* organism comprising any of SEQ ID NOs:84, 86, 88, 90, and/or 93; and the complement of a fragment of at least 50 contiguous nucleotides of a native coding polynucleotide of a *Meligethes* organism comprising any of SEQ ID NOs:84, 86, 88, 90, and/or 93.

In some aspects of the disclosure, one strand of a dsRNA molecule may be formed by transcription from a polynucleotide that is substantially homologous to a polynucleotide selected from the group consisting of SEQ ID NOs:3-6, and 90; the complements of SEQ ID NOs:3-6, and 90; fragments of at least 15 contiguous nucleotides of SEQ ID NOs:3-6, and 90; and the complements of fragments of at least 15 contiguous nucleotides of SEQ ID NOs:3-6, and 90.

In particular embodiments, a recombinant DNA molecule encoding an RNA that may form a dsRNA molecule may comprise a coding region wherein at least two polynucleotides are arranged such that one polynucleotide is in a sense orientation, and the other polynucleotide is in an antisense orientation, relative to at least one promoter, wherein the sense polynucleotide and the antisense polynucleotide are linked or connected by a spacer of, for example, from about five (~5) to about one thousand (~1000) nucleotides. The spacer may form a loop between the sense and antisense polynucleotides. The sense polynucleotide or the antisense polynucleotide may be substantially homologous to a target gene (*e.g.,* a *ncm* gene comprising SEQ ID NO:1, SEQ ID NO:77, SEQ ID NO:84, SEQ ID NO:86, SEQ ID NO:88, and/or SEQ ID NO:93) or fragment thereof. In some embodiments, however, a recombinant DNA molecule may encode an RNA that may form a dsRNA molecule without a spacer. In embodiments, a sense coding polynucleotide and an antisense coding polynucleotide may be different lengths.

Polynucleotides identified as having a deleterious effect on an insect pest or a plant-protective effect with regard to the pest may be readily incorporated into expressed dsRNA molecules through the creation of appropriate expression cassettes in a recombinant nucleic acid molecule of the invention. For example, such polynucleotides may be expressed as a hairpin with stem and loop structure by taking a first segment corresponding to a target gene polynucleotide (*e.g.,* a *ncm* gene comprising SEQ ID NO:1, SEQ ID NO:77, SEQ ID NO:84, SEQ ID NO:86, SEQ ID NO:88, and/or SEQ ID NO:93, and fragments of either of the foregoing); linking this polynucleotide to a second segment spacer region that is not homologous or complementary to the first segment; and linking this to a third segment, wherein at least a portion of the third segment is substantially complementary to the first segment. Such a construct forms a stem and loop structure by intramolecular base-pairing of the first segment with the third segment, wherein the loop structure forms comprising the second segment. *See, e.g.,* U.S. Patent Publication Nos. 2002/0048814 and 2003/0018993; and International PCT Publication Nos. WO94/01550 and WO98/05770. A dsRNA molecule may be generated, for example, in the form of a double-stranded structure such as a stem-loop structure (*e.g.,* hairpin), whereby production of siRNA targeted for a native insect (*e.g.,* coleopteran) pest polynucleotide is enhanced by co-expression of a fragment of the targeted gene, for instance on an additional plant expressible cassette, that leads to enhanced siRNA production, or reduces methylation to prevent transcriptional gene silencing of the dsRNA hairpin promoter.

Embodiments of the invention include introduction of a recombinant nucleic acid molecule of the present invention into a plant (*i.e*., transformation) to achieve insect (*e.g.,* coleopteran) pest-inhibitory levels of expression of one or more iRNA molecules. A recombinant DNA molecule may, for example, be a vector, such as a linear or a closed circular plasmid. The vector system may be a single vector or plasmid, or two or more vectors or plasmids that together contain the total DNA to be introduced into the genome of a host. In addition, a vector may be an expression vector. Nucleic acids of the invention can, for example, be suitably inserted into a vector under the control of a suitable promoter that functions in one or more hosts to drive expression of a linked coding polynucleotide or other DNA element. Many vectors are available for this purpose, and selection of the appropriate vector will depend mainly on the size of the nucleic acid to be inserted into the vector and the particular host cell to be transformed with the vector. Each vector contains various components depending on its function (*e.g.,* amplification of DNA or expression of DNA) and the particular host cell with which it is compatible.

To impart insect (*e.g.,* coleopteran) pest resistance to a transgenic plant, a recombinant DNA may, for example, be transcribed into an iRNA molecule (*e.g.,* an RNA molecule that forms a dsRNA molecule) within the tissues or fluids of the recombinant plant. An iRNA molecule may comprise a polynucleotide that is substantially homologous and specifically hybridizable to a corresponding transcribed polynucleotide within an insect pest that may cause damage to the host plant species. The pest may contact the iRNA molecule that is transcribed in cells of the transgenic host plant, for example, by ingesting cells or fluids of the transgenic host plant that comprise the iRNA molecule. Thus, in particular examples, expression of a target gene is suppressed by the iRNA molecule within coleopteran pests that infest the transgenic host plant. In some embodiments, suppression of expression of the target gene in a target coleopteran pest may result in the plant being resistant to attack by the pest.

In order to enable delivery of iRNA molecules to an insect pest in a nutritional relationship with a plant cell that has been transformed with a recombinant nucleic acid molecule of the invention, expression (*i.e.*, transcription) of iRNA molecules in the plant cell is required. Thus, a recombinant nucleic acid molecule may comprise a polynucleotide of the invention operably linked to one or more regulatory elements, such as a heterologous promoter element that functions in a host cell, such as a bacterial cell wherein the nucleic acid molecule is to be amplified, and a plant cell wherein the nucleic acid molecule is to be expressed.

Promoters suitable for use in nucleic acid molecules of the invention include those that are inducible, viral, synthetic, or constitutive, all of which are well known in the art. Non-limiting examples describing such promoters include U.S. Patents 6,437,217 (maize RS81 promoter); 5,641,876 (rice actin promoter); 6,426,446 (maize RS324 promoter); 6,429,362 (maize PR-1 promoter); 6,232,526 (maize A3 promoter); 6,177,611 (constitutive maize promoters); 5,322,938, 5,352,605,5,359,142, and 5,530,196 (CaMV 35S promoter); 6,433,252 (maize L3 oleosin promoter); 6,429,357 (rice actin 2 promoter, and rice actin 2 intron); 6,294,714 (light-inducible promoters); 6,140,078 (salt-inducible promoters); 6,252,138 (pathogen-inducible promoters); 6,175,060 (phosphorous deficiency-inducible promoters); 6,388,170 (bidirectional promoters); 6,635,806 (gamma-coixin promoter); and U.S. Patent Publication No. 2009/757,089 (maize chloroplast aldolase promoter). Additional promoters include the nopaline synthase (NOS) promoter (Ebert et al. (1987) Proc. Natl. Acad. Sci. USA 84(16):5745-9) and the octopine synthase (OCS) promoters (which are carried on tumor-inducing plasmids of *Agrobacterium tumefaciens*); the caulimovirus promoters such as the cauliflower mosaic virus (CaMV) 19S promoter (Lawton et al. (1987) Plant Mol. Biol. 9:315-24); the CaMV 35S promoter (Odell et al. (1985) Nature 313:810-2; the figwort mosaic virus 35S-promoter (Walker et al. (1987) Proc. Natl. Acad. Sci. USA 84(19):6624-8); the sucrose synthase promoter (Yang and Russell (1990) Proc. Natl. Acad. Sci. USA 87:4144-8); the R gene complex promoter (Chandler et al. (1989) Plant Cell 1:1175-83); the chlorophyll a/b binding protein gene promoter; CaMV 35S (U.S. Patents 5,322,938, 5,352,605, 5,359,142, and 5,530,196); FMV 35S (U.S. Patents 6,051,753, and 5,378,619); a PC1SV promoter (U.S. Patent 5,850,019); the SCP1 promoter (U.S. Patent 6,677,503); and AGRtu.nos promoters (GenBank™ Accession No. V00087; Depicker et al. (1982) J. Mol. Appl. Genet. 1:561-73; Bevan et al. (1983) Nature 304:184-7).

In particular embodiments, nucleic acid molecules of the invention comprise a tissue-specific promoter, such as a root-specific promoter. Root-specific promoters drive expression of operably-linked coding polynucleotides exclusively or preferentially in root tissue. Examples of root-specific promoters are known in the art. *See, e.g.,* U.S. Patents 5,110,732; 5,459,252 and 5,837,848; and Opperman et al. (1994) Science 263:221-3; and Hirel et al. (1992) Plant Mol. Biol. 20:207-18. In some embodiments, a polynucleotide or fragment for coleopteran pest control according to the invention may be cloned between two root-specific promoters oriented in opposite transcriptional directions relative to the polynucleotide or fragment, and which are operable in a transgenic plant cell and expressed therein to produce RNA molecules in the transgenic plant cell that subsequently may form dsRNA molecules, as described, *supra.* The iRNA molecules expressed in plant tissues may be ingested by an insect pest so that suppression of target gene expression is achieved.

Additional regulatory elements that may optionally be operably linked to a nucleic acid include 5'UTRs located between a promoter element and a coding polynucleotide that function as a translation leader element. The translation leader element is present in fully-processed mRNA, and it may affect processing of the primary transcript, and/or RNA stability. Examples of translation leader elements include maize and petunia heat shock protein leaders (U.S. Patent 5,362,865), plant virus coat protein leaders, plant rubisco leaders, and others. *See, e.g.,* Turner and Foster (1995) Molecular Biotech. 3(3):225-36. Non-limiting examples of 5'UTRs include GmHsp (U.S. Patent 5,659,122); PhDnaK (U.S. Patent 5,362,865); AtAnt1; TEV (Carrington and Freed (1990) J. Virol. 64:1590-7); and AGRtunos (GenBank™ Accession No. V00087; and Bevan et al. (1983) Nature 304:184-7).

Additional regulatory elements that may optionally be operably linked to a nucleic acid also include 3' non-translated elements, 3' transcription termination regions, or polyadenylation regions. These are genetic elements located downstream of a polynucleotide, and include polynucleotides that provide polyadenylation signal, and/or other regulatory signals capable of affecting transcription or mRNA processing. The polyadenylation signal functions in plants to cause the addition of polyadenylate nucleotides to the 3' end of the mRNA precursor. The polyadenylation element can be derived from a variety of plant genes, or from T-DNA genes. A non-limiting example of a 3' transcription termination region is the nopaline synthase 3' region (nos 3'; Fraley et al. (1983) Proc. Natl. Acad. Sci. USA 80:4803-7). An example of the use of different 3' non-translated regions is provided in Ingelbrecht et al., (1989) Plant Cell 1:671-80. Non-limiting examples of polyadenylation signals include one from a *Pisum sativum* RbcS2 gene (Ps.RbcS2-E9; Coruzzi et al. (1984) EMBO J. 3:1671-9) and AGRtu.nos (GenBank™ Accession No. E01312).

Some embodiments may include a plant transformation vector that comprises an isolated and purified DNA molecule comprising at least one of the above-described regulatory elements operatively linked to one or more polynucleotides of the present invention. When expressed, the one or more polynucleotides result in one or more iRNA molecule(s) comprising a polynucleotide that is specifically complementary to all or part of a native RNA molecule in an insect *(e.g.,* coleopteran) pest. Thus, the polynucleotide(s) may comprise a segment encoding all or part of a polyribonucleotide present within a targeted coleopteran pest RNA transcript, and may comprise inverted repeats of all or a part of a targeted pest transcript. A plant transformation vector may contain polynucleotides specifically complementary to more than one target polynucleotide, thus allowing production of more than one dsRNA for inhibiting expression of two or more genes in cells of one or more populations or species of target insect pests. Segments of polynucleotides specifically complementary to polynucleotides present in different genes can be combined into a single composite nucleic acid molecule for expression in a transgenic plant. Such segments may be contiguous or separated by a spacer.

In some embodiments, a plasmid of the present invention already containing at least one polynucleotide(s) of the invention can be modified by the sequential insertion of additional polynucleotide(s) in the same plasmid, wherein the additional polynucleotide(s) are operably linked to the same regulatory elements as the original at least one polynucleotide(s). In some embodiments, a nucleic acid molecule may be designed for the inhibition of multiple target genes. In some embodiments, the multiple genes to be inhibited can be obtained from the same insect (*e.g.,* coleopteran) pest species, which may enhance the effectiveness of the nucleic acid molecule. In other embodiments, the genes can be derived from different insect pests, which may broaden the range of pests against which the agent(s) is/are effective. When multiple genes are targeted for suppression or a combination of expression and suppression, a polycistronic DNA element can be engineered.

A recombinant nucleic acid molecule or vector of the present invention may comprise a selectable marker that confers a selectable phenotype on a transformed cell, such as a plant cell. Selectable markers may also be used to select for plants or plant cells that comprise a recombinant nucleic acid molecule of the invention. The marker may encode biocide resistance, antibiotic resistance (*e.g.,* kanamycin, Geneticin (G418), bleomycin, hygromycin, *etc.*), or herbicide tolerance (*e.g.,* glyphosate, *etc.*). Examples of selectable markers include, but are not limited to: a *neo* gene which codes for kanamycin resistance and can be selected for using kanamycin, G418, *etc.;* a *bar* gene which codes for bialaphos resistance; a mutant EPSP synthase gene which encodes glyphosate tolerance; a *nitrilase* gene which confers resistance to bromoxynil; a mutant acetolactate synthase (*ALS*) gene which confers imidazolinone or sulfonylurea tolerance; and a methotrexate resistant *DHFR* gene. Multiple selectable markers are available that confer resistance to ampicillin, bleomycin, chloramphenicol, gentamycin, hygromycin, kanamycin, lincomycin, methotrexate, phosphinothricin, puromycin, spectinomycin, rifampicin, streptomycin and tetracycline, and the like. Examples of such selectable markers are illustrated in, *e.g.,* U.S. Patents 5,550,318; 5,633,435; 5,780,708 and 6,118,047.

A recombinant nucleic acid molecule or vector of the present invention may also include a screenable marker. Screenable markers may be used to monitor expression. Exemplary screenable markers include a β-glucuronidase or *uidA* gene (GUS) which encodes an enzyme for which various chromogenic substrates are known (Jefferson et al. (1987) Plant Mol. Biol. Rep. 5:387-405); an R-locus gene, which encodes a product that regulates the production of anthocyanin pigments (red color) in plant tissues (Dellaporta et al. (1988) "Molecular cloning of the maize R-nj allele by transposon tagging with Ac." In 18th Stadler Genetics Symposium, P. Gustafson and R. Appels, eds. (New York: Plenum), pp. 263-82); a β-lactamase gene (Sutcliffe et al. (1978) Proc. Natl. Acad. Sci. USA 75:3737-41); a gene which encodes an enzyme for which various chromogenic substrates are known (*e.g.,* PADAC, a chromogenic cephalosporin); a luciferase gene (Ow et al. (1986) Science 234:856-9); an *xylE* gene that encodes a catechol dioxygenase that can convert chromogenic catechols (Zukowski et al. (1983) Gene 46(2-3):247-55); an amylase gene (Ikatu et al. (1990) Bio/Technol. 8:241-2); a tyrosinase gene which encodes an enzyme capable of oxidizing tyrosine to DOPA and dopaquinone which in turn condenses to melanin (Katz et al. (1983) J. Gen. Microbiol. 129:2703-14); and an α-galactosidase.

In some embodiments, recombinant nucleic acid molecules, as described, *supra,* may be used in methods for the creation of transgenic plants and expression of heterologous nucleic acids in plants to prepare transgenic plants that exhibit reduced susceptibility to insect *(e.g.,* coleopteran) pests. Plant transformation vectors can be prepared, for example, by inserting nucleic acid molecules encoding iRNA molecules into plant transformation vectors and introducing these into plants.

Suitable methods for transformation of host cells include any method by which DNA can be introduced into a cell, such as by transformation of protoplasts (*See, e.g.,* U.S. Patent 5,508,184), by desiccation/inhibition-mediated DNA uptake (*See, e.g.,* Potrykus et al. (1985) Mol. Gen. Genet. 199:183-8), by electroporation (*See, e.g.,* U.S. Patent 5,384,253), by agitation with silicon carbide fibers (*See, e.g.,* U.S. Patents 5,302,523 and 5,464,765), by *Agrobacterium*-mediated transformation (*See, e.g.,* U.S. Patents 5,563,055; 5,591,616; 5,693,512; 5,824,877; 5,981,840; and 6,384,301) and by acceleration of DNA-coated particles (*See, e.g.,* U.S. Patents 5,015,580; 5,550,318; 5,538,880; 6,160,208; 6,399,861; and 6,403,865), *etc.* Techniques that are particularly useful for transforming corn are described, for example, in U.S. Patents 7,060,876 and 5,591,616; and International PCT Publication WO95/06722. Through the application of techniques such as these, the cells of virtually any species may be stably transformed. In some embodiments, transforming DNA is integrated into the genome of the host cell. In the case of multicellular species, transgenic cells may be regenerated into a transgenic organism. Any of these techniques may be used to produce a transgenic plant, for example, comprising one or more nucleic acids encoding one or more iRNA molecules in the genome of the transgenic plant.

The most widely utilized method for introducing an expression vector into plants is based on the natural transformation system of *Agrobacterium. A. tumefaciens* and *A. rhizogenes* are plant pathogenic soil bacteria which genetically transform plant cells. The Ti and Ri plasmids of *A. tumefaciens* and *A. rhizogenes,* respectively, carry genes responsible for genetic transformation of the plant. The Ti (tumor-inducing)-plasmids contain a large segment, known as T-DNA, which is transferred to transformed plants. Another segment of the Ti plasmid, the Vir region, is responsible for T-DNA transfer. The T-DNA region is bordered by terminal repeats. In modified binary vectors, the tumor-inducing genes have been deleted, and the functions of the Vir region are utilized to transfer foreign DNA bordered by the T-DNA border elements. The T-region may also contain a selectable marker for efficient recovery of transgenic cells and plants, and a multiple cloning site for inserting polynucleotides for transfer such as a dsRNA encoding nucleic acid.

Thus, in some embodiments, a plant transformation vector is derived from a Ti plasmid of *A. tumefaciens* (*See, e.g.,* U.S. Patents 4,536,475, 4,693,977, 4,886,937, and 5,501,967; and European Patent No. EP 0 122 791) or a Ri plasmid of *A. rhizogenes.* Additional plant transformation vectors include, for example and without limitation, those described by Herrera-Estrella et al. (1983) Nature 303:209-13; Bevan et al. (1983) Nature 304:184-7; Klee et al. (1985) Bio/Technol. 3:637-42; and in European Patent No. EP 0 120 516, and those derived from any of the foregoing. Other bacteria such as *Sinorhizobium, Rhizobium,* and *Mesorhizobium* that interact with plants naturally can be modified to mediate gene transfer to a number of diverse plants. These plant-associated symbiotic bacteria can be made competent for gene transfer by acquisition of both a disarmed Ti plasmid and a suitable binary vector.

After providing exogenous DNA to recipient cells, transformed cells are generally identified for further culturing and plant regeneration. In order to improve the ability to identify transformed cells, one may desire to employ a selectable or screenable marker gene, as previously set forth, with the transformation vector used to generate the transformant. In the case where a selectable marker is used, transformed cells are identified within the potentially transformed cell population by exposing the cells to a selective agent or agents. In the case where a screenable marker is used, cells may be screened for the desired marker gene trait.

Cells that survive the exposure to the selective agent, or cells that have been scored positive in a screening assay, may be cultured in media that supports regeneration of plants. In some embodiments, any suitable plant tissue culture media (*e.g*., MS and N6 media) may be modified by including further substances, such as growth regulators. Tissue may be maintained on a basic medium with growth regulators until sufficient tissue is available to begin plant regeneration efforts, or following repeated rounds of manual selection, until the morphology of the tissue is suitable for regeneration (*e.g*., at least 2 weeks), then transferred to media conducive to shoot formation. Cultures are transferred periodically until sufficient shoot formation has occurred. Once shoots are formed, they are transferred to media conducive to root formation. Once sufficient roots are formed, plants can be transferred to soil for further growth and maturation.

To confirm the presence of a nucleic acid molecule of interest (for example, a DNA encoding one or more iRNA molecules that inhibit target gene expression in a coleopteran pest) in the regenerating plants, a variety of assays may be performed. Such assays include, for example: molecular biological assays, such as Southern and northern blotting, PCR, and nucleic acid sequencing; biochemical assays, such as detecting the presence of a protein product, *e.g.,* by immunological means (ELISA and/or western blots) or by enzymatic function; plant part assays, such as leaf or root assays; and analysis of the phenotype of the whole regenerated plant.

Integration events may be analyzed, for example, by PCR amplification using, *e.g*., oligonucleotide primers specific for a nucleic acid molecule of interest. PCR genotyping is understood to include, but not be limited to, polymerase-chain reaction (PCR) amplification of gDNA derived from isolated host plant callus tissue predicted to contain a nucleic acid molecule of interest integrated into the genome, followed by standard cloning and sequence analysis of PCR amplification products. Methods of PCR genotyping have been well described (for example, Rios, G. et al. (2002) Plant J. 32:243-53) and may be applied to gDNA derived from any plant species (*e.g., Z. mays* or *Brassica napus*) or tissue type, including cell cultures.

A transgenic plant formed using *Agrobacterium*-dependent transformation methods typically contains a single recombinant DNA inserted into one chromosome. The polynucleotide of the single recombinant DNA is referred to as a "transgenic event" or "integration event". Such transgenic plants are heterozygous for the inserted exogenous polynucleotide. In some embodiments, a transgenic plant homozygous with respect to a transgene may be obtained by sexually mating (selfing) an independent segregant transgenic plant that contains a single exogenous gene to itself, for example a $T_0$ plant, to produce $T_1$ seed. One fourth of the $T_1$ seed produced will be homozygous with respect to the transgene. Germinating $T_1$ seed results in plants that can be tested for heterozygosity, typically using an SNP assay or a thermal amplification assay that allows for the distinction between heterozygotes and homozygotes (*i.e.,* a zygosity assay).

In particular embodiments, at least 2, 3, 4, 5, 6, 7, 8, 9 or 10 or more different iRNA molecules are produced in a plant cell that have an insect (*e.g.*, coleopteran) pest-inhibitory effect. The iRNA molecules (*e.g.*, dsRNA molecules) may be expressed from multiple nucleic acids introduced in different transformation events, or from a single nucleic acid introduced in a single transformation event. In some embodiments, a plurality of iRNA molecules are expressed under the control of a single promoter. In other embodiments, a plurality of iRNA molecules are expressed under the control of multiple promoters. Single iRNA molecules may be expressed that comprise multiple polynucleotides that are each homologous to different loci within one or more insect pests (for example, the loci defined by SEQ ID NOs:1, 77, 84, 86, 88, and 93), both in different populations of the same species of insect pest, or in different species of insect pests.

In addition to direct transformation of a plant with a recombinant nucleic acid molecule, transgenic plants can be prepared by crossing a first plant having at least one transgenic event with a second plant lacking such an event. For example, a recombinant nucleic acid molecule comprising a polynucleotide that encodes an iRNA molecule may be introduced into a first plant line that is amenable to transformation to produce a transgenic plant, which transgenic plant may be crossed with a second plant line to introgress the polynucleotide that encodes the iRNA molecule into the second plant line.

In some aspects, seeds and commodity products produced by transgenic plants derived from transformed plant cells are included, wherein the seeds or commodity products comprise a detectable amount of a nucleic acid of the invention. In some embodiments, such commodity products may be produced, for example, by obtaining transgenic plants and preparing food or feed from them. Commodity products comprising one or more of the polynucleotides of the invention includes, for example and without limitation: meals, oils, crushed or whole grains or seeds of a plant, and any food product comprising any meal, oil, or crushed or whole grain of a recombinant plant or seed comprising one or more of the nucleic acids of the invention. The detection of one or more of the polynucleotides of the invention in one or more commodity or commodity products is *de facto* evidence that the commodity or commodity product is produced from a transgenic plant designed to express one or more of the iRNA molecules of the invention for the purpose of controlling insect (*e.g.*, coleopteran) pests.

In some embodiments, a transgenic plant or seed comprising a nucleic acid molecule of the invention also may comprise at least one other transgenic event in its genome, including without limitation: a transgenic event from which is transcribed an iRNA molecule targeting a locus in a coleopteran pest other than the ones defined by SEQ ID NOs:1, 77, 84, 86, 88, and 93, such as, for example, one or more loci selected from the group consisting of Cafl-180 (U.S. Patent Application Publication No. 2012/0174258), VatpaseC (U.S. Patent Application Publication No. 2012/0174259), Rho1 (U.S. Patent Application Publication No. 2012/0174260), VatpaseH (U.S. Patent Application Publication No. 2012/0198586), PPI-87B (U.S. Patent Application Publication No. 2013/0091600), RPA70 (U.S. Patent Application Publication No. 2013/0091601), RPS6 (U.S. Patent Application Publication No. 2013/0097730), *ROP* (U.S. Patent Application No. 14/577,811), *RNAPII140* (U.S. Patent Application No. 14/577,854), *Dre4* (U.S. Patent Application No. 14/705,807), *COPI alpha* (U.S. Patent Application No. 62/063,199), *COPI beta* (U.S. Patent Application No. 62/063,203), *COPI gamma* (U.S. Patent Application No. 62/063,192), and *COPI delta* (U.S. Patent Application No. 62/063,216); a transgenic event from which is transcribed an iRNA molecule targeting a gene in an organism other than a coleopteran pest (*e.g.,* a plant-parasitic nematode); a gene encoding an insecticidal protein (*e.g.,* a *Bacillus thuringiensis* insecticidal protein); an herbicide tolerance gene (*e.g.,* a gene providing tolerance to glyphosate); and a gene contributing to a desirable phenotype in the transgenic plant, such as increased yield, altered fatty acid metabolism, or restoration of cytoplasmic male sterility. In particular embodiments, polynucleotides encoding iRNA molecules of the invention may be combined with other insect control and disease traits in a plant to achieve desired traits for enhanced control of plant disease and insect damage. Combining insect control traits that employ distinct modes-of-action may provide protected transgenic plants with superior durability over plants harboring a single control trait, for example, because of the reduced probability that resistance to the trait(s) will develop in the field.

### *V. Target Gene Suppression in an Insect Pest*

#### A. Overview

In some embodiments of the invention, at least one nucleic acid molecule useful for the control of insect (*e.g.*, coleopteran) pests may be provided to an insect pest, wherein the nucleic acid molecule leads to RNAi-mediated gene silencing in the pest. In particular embodiments, an iRNA molecule (hpRNA) may be provided to a coleopteran pest. In some embodiments, a nucleic acid molecule useful for the control of insect pests may be provided to a pest by contacting the nucleic acid molecule with the pest. In these and further embodiments, a nucleic acid molecule useful for the control of insect pests may be provided in a feeding substrate of the pest, for example, a nutritional composition. In these and further embodiments, a nucleic acid molecule useful for the control of an insect pest may be provided through ingestion of plant material comprising the nucleic acid molecule that is ingested by the pest. In certain embodiments, the nucleic acid molecule is present in plant material through expression of a recombinant nucleic acid introduced into the plant material, for example, by transformation of a plant cell with a vector comprising the recombinant nucleic acid and regeneration of a plant material or whole plant from the transformed plant cell.

**B. RNAi-mediated Target Gene Suppression**

In embodiments, the invention provides iRNA molecules (hpRNA) that may be designed to target essential native polynucleotides (*e.g.*, essential genes) in the transcriptome of an insect pest (for example, a coleopteran (*e.g.,* WCR, NCR, SCR, and *Meligethes aeneus*) pest), for example by designing an iRNA molecule that comprises at least one strand comprising a polynucleotide that is specifically complementary to the target polynucleotide. The sequence of an iRNA molecule so designed may be identical to that of the target polynucleotide, or may incorporate mismatches that do not prevent specific hybridization between the iRNA molecule and its target polynucleotide.

iRNA molecules of the invention may be used in methods for gene suppression in an insect (*e.g.*, coleopteran) pest, thereby reducing the level or incidence of damage caused by the pest on a plant (for example, a protected transformed plant comprising an iRNA molecule). As used herein the term "gene suppression" refers to any of the well-known methods for reducing the levels of protein produced as a result of gene transcription to mRNA and subsequent translation of the mRNA, including the reduction of protein expression from a gene or a coding polynucleotide including post-transcriptional inhibition of expression and transcriptional suppression. Post-transcriptional inhibition is mediated by specific homology between all or a part of an mRNA transcribed from a gene targeted for suppression and the corresponding iRNA molecule used for suppression. Additionally, post-transcriptional inhibition refers to the substantial and measurable reduction of the amount of mRNA available in the cell for binding by ribosomes.

In embodiments, the dsRNA molecule may be cleaved by the enzyme, DICER, into short siRNA molecules (approximately 20 nucleotides in length). The double-stranded siRNA molecule generated by DICER activity upon the dsRNA molecule may be separated into two single-stranded siRNAs; the "passenger strand" and the "guide strand". The passenger strand may be degraded, and the guide strand may be incorporated into RISC. Post-transcriptional inhibition occurs by specific hybridization of the guide strand with a specifically complementary polynucleotide of an mRNA molecule, and subsequent cleavage by the enzyme, Argonaute (catalytic component of the RISC complex).

Disclosed is the use of any form of iRNA molecule. Those of skill in the art will understand that dsRNA molecules typically are more stable during preparation and during the step of providing the iRNA molecule to a cell than are single-stranded RNA molecules, and are typically also more stable in a cell. Thus, while siRNA and miRNA molecules, for example, may be equally effective, a dsRNA molecule may be chosen due to its stability.

In particular embodiments, a nucleic acid molecule is provided that comprises a polynucleotide, which polynucleotide may be expressed *in vitro* to produce an iRNA molecule that is substantially homologous to a nucleic acid molecule encoded by a polynucleotide within the genome of an insect (*e.g.,* coleopteran) pest. In embodiments, the *in vitro* transcribed iRNA molecule is a stabilized dsRNA molecule that comprises a stem-loop structure. After an insect pest contacts the *in vitro* transcribed iRNA molecule, post-transcriptional inhibition of a target gene in the pest (for example, an essential gene) may occur.

In some embodiments of the invention, expression of a nucleic acid molecule comprising at least 50 contiguous nucleotides of a polynucleotide are used in a method for post-transcriptional inhibition of a target gene in an insect (*e.g.*, coleopteran) pest, wherein the polynucleotide is selected from the group consisting of: SEQ ID NO:84; the complement of SEQ ID NO:84, SEQ ID NO:86; the complement of SEQ ID NO:86, SEQ ID NO:88; the complement of SEQ ID NO:88; SEQ ID NO:90; the complement of SEQ ID NO:90; SEQ ID NO:93; the complement of SEQ ID NO:93; a fragment of at least 50 contiguous nucleotides of SEQ ID NO:84; the complement of a fragment of at least 50 contiguous nucleotides of SEQ ID NO:84; a fragment of at least 50 contiguous nucleotides of SEQ ID NO:86; the complement of a fragment of at least 50 contiguous nucleotides of SEQ ID NO:86; a fragment of at least 50 contiguous nucleotides of SEQ ID NO:88; the complement of a fragment of at least 50 contiguous nucleotides of SEQ ID NO:88; a fragment of at least 50 contiguous nucleotides of SEQ ID NO:90; the complement of a fragment of at least 50 contiguous nucleotides of SEQ ID NO:90; ; a fragment of at least 50 contiguous nucleotides of SEQ ID NO:93; the complement of a fragment of at least 50 contiguous nucleotides of SEQ ID NO:93; a native coding polynucleotide of a *Meligethes* organism comprising SEQ ID NO:84; the complement of a native coding polynucleotide of a *Meligethes* organism comprising SEQ ID NO:84; a native coding polynucleotide of a *Meligethes* organism comprising SEQ ID NO:86; the complement of a native coding polynucleotide of a *Meligethes* organism comprising SEQ ID NO:86; a native coding polynucleotide of a *Meligethes* organism comprising SEQ ID NO:88; the complement of a native coding polynucleotide of a *Meligethes* organism comprising SEQ ID NO:88; a native coding polynucleotide of a *Meligethes* organism comprising SEQ ID NO:90; the complement of a native coding polynucleotide of a *Meligethes* organism comprising SEQ ID NO:90; a fragment of at least 50 contiguous nucleotides of a native coding polynucleotide of a *Meligethes* organism comprising SEQ ID NO:84; the complement of a fragment of at least 50 contiguous nucleotides of a native coding polynucleotide of a *Meligethes* organism comprising SEQ ID NO:84; a fragment of at least 50 contiguous nucleotides of a native coding polynucleotide of a *Meligethes* organism comprising SEQ ID NO:86; the complement of a fragment of at least 50 contiguous nucleotides of a native coding polynucleotide of a *Meligethes* organism comprising SEQ ID NO:86; a fragment of at least 50 contiguous nucleotides of a native coding polynucleotide of a *Meligethes* organism comprising SEQ ID NO:88; the complement of a fragment of at least 50 contiguous nucleotides of a native coding polynucleotide of a *Meligethes* organism comprising SEQ ID NO:88; a fragment of at least 50 contiguous nucleotides of a native coding polynucleotide of a *Meligethes* organism comprising SEQ ID NO:90; the complement of a fragment of at least 50 contiguous nucleotides of a native coding polynucleotide of a *Meligethes* organism comprising SEQ ID NO:90; a fragment of at least 50 contiguous nucleotides of a native coding polynucleotide of a *Meligethes* organism comprising SEQ ID NO:93; the complement of a fragment of at least 50 contiguous nucleotides of a native coding polynucleotide of a *Meligethes* organism comprising SEQ ID NO:93. In certain embodiments, expression of a nucleic acid molecule that is at least about 80% identical (*e.g.*, 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, about 100%, and 100%) with any of the foregoing may be used. In these and further embodiments, a nucleic acid molecule may be expressed that specifically hybridizes to an RNA molecule present in at least one cell of an insect (*e.g.*, coleopteran) pest.

It is an important feature of some embodiments herein that the RNAi post-transcriptional inhibition system is able to tolerate sequence variations among target genes that might be expected due to genetic mutation, strain polymorphism, or evolutionary divergence. The introduced nucleic acid molecule may not need to be absolutely homologous to either a primary transcription product or a fully-processed mRNA of a target gene, so long as the introduced nucleic acid molecule is specifically hybridizable to either a primary transcription product or a fully-processed mRNA of the target gene. Moreover, the introduced nucleic acid molecule may not need to be full-length, relative to either a primary transcription product or a fully processed mRNA of the target gene.

Inhibition of a target gene using the iRNA technology of the present invention is sequence-specific; *i.e.,* polynucleotides substantially homologous to the iRNA molecule(s) are targeted for genetic inhibition. In some embodiments, an RNA molecule comprising a polynucleotide with a nucleotide sequence that is identical to that of a portion of a target gene may be used for inhibition. In these and further embodiments, an RNA molecule comprising a polynucleotide with one or more insertion, deletion, and/or point mutations relative to a target polynucleotide may be used. In particular embodiments, an iRNA molecule and a portion of a target gene may share, for example, at least from about 80%, at least from about 81%, at least from about 82%, at least from about 83%, at least from about 84%, at least from about 85%, at least from about 86%, at least from about 87%, at least from about 88%, at least from about 89%, at least from about 90%, at least from about 91%, at least from about 92%, at least from about 93%, at least from about 94%, at least from about 95%, at least from about 96%, at least from about 97%, at least from about 98%, at least from about 99%, at least from about 100%, and 100% sequence identity. Alternatively, the duplex region of a dsRNA molecule may be specifically hybridizable with a portion of a target gene transcript. In specifically hybridizable molecules, a less than full length polynucleotide exhibiting a greater homology compensates for a longer, less homologous polynucleotide. The length of the polynucleotide of a duplex region of a dsRNA molecule that is identical to a portion of a target gene transcript may be at least about 25, 50, 100, 200, 300, 400, 500, or at least about 1000 bases. In some embodiments, a polynucleotide of greater than 50-100 nucleotides may be used. In particular embodiments, a polynucleotide of greater than about 200-300 nucleotides may be used. In particular embodiments, a polynucleotide of greater than about 500-1000 nucleotides may be used, depending on the size of the target gene.

In certain embodiments, expression of a target gene in an insect pest (*e.g.,* a coleopteran insect pest) may be inhibited by at least 10%; at least 33%; at least 50%; or at least 80% within a cell of the pest, such that a significant inhibition takes place. Significant inhibition refers to inhibition over a threshold that results in a detectable phenotype (*e.g.*, cessation of growth, cessation of feeding, cessation of development, induced mortality, *etc.*), or a detectable decrease in RNA and/or gene product corresponding to the target gene being inhibited. Although, in certain embodiments of the invention, inhibition occurs in substantially all cells of the pest, in other embodiments inhibition occurs only in a subset of cells expressing the target gene.

In some embodiments, transcriptional suppression is mediated by the presence in a cell of a dsRNA molecule exhibiting substantial sequence identity to a promoter DNA or the complement thereof to effect what is referred to as "promoter trans suppression." Gene suppression may be effective against target genes in an insect pest that may ingest or contact such dsRNA molecules, for example, by ingesting or contacting plant material containing the dsRNA molecules. dsRNA molecules for use in promoter trans suppression may be specifically designed to inhibit or suppress the expression of one or more homologous or complementary polynucleotides in the cells of the insect pest. Post-transcriptional gene suppression by antisense or sense oriented RNA to regulate gene expression in plant cells is disclosed in U.S. Patents 5,107,065; 5,759,829; 5,283,184; and 5,231,020.

### C. Expression of iRNA Molecules Provided to an Insect Pest

Expression of iRNA molecules for RNAi-mediated gene inhibition in an insect (*e.g.,* coleopteran) pest may be carried out in any one of many *in vitro* or *in vivo* formats. The iRNA molecules may then be provided to an insect pest, for example, by contacting the iRNA molecules with the pest, or by causing the pest to ingest or otherwise internalize the iRNA molecules. Some embodiments include transformed host plants of a coleopteran pest, transformed plant cells, and progeny of transformed plants. The transformed plant cells and transformed plants may be engineered to express one or more of the iRNA molecules, for example, under the control of a heterologous promoter, to provide a pest-protective effect. Thus, when a transgenic plant or plant cell is consumed by an insect pest during feeding, the pest may ingest iRNA molecules expressed in the transgenic plants or cells. The polynucleotides of the present invention may also be introduced into a wide variety of prokaryotic and eukaryotic microorganism hosts to produce iRNA molecules. The term "microorganism" includes prokaryotic and eukaryotic species, such as bacteria and fungi.

Modulation of gene expression may include partial or complete suppression of such expression. In another embodiment, a method for suppression of gene expression in an insect (*e.g.,* coleopteran) pest comprises providing in the tissue of the host of the pest a gene-suppressive amount of at least one dsRNA molecule formed following transcription of a polynucleotide as described herein, at least one segment of which is complementary to an mRNA within the cells of the insect pest. A hpRNA molecule, ingested by an insect pest may be at least from about 80%, 81%, 82%, 83%, 84%, 85%, 86%, 87%, 88%, 89%, 90%, 91%, 92%, 93%, 94%, 95%, 96%, 97%, 98%, 99%, or about 100% identical to an RNA molecule transcribed from a *ncm* DNA molecule, for example, comprising a polynucleotide selected from the group consisting of SEQ ID NOs: 84, 86, 88, 90, and 93. In particular a hpRNA molecule, ingested by an insect pest may be at least from about 80%, 81%, 82%, 83%, 84%, 85%, 86%, 87%, 88%, 89%, 90%, 91%, 92%, 93%, 94%, 95%, 96%, 97%, 98%, 99%, or about 100% identical to an RNA molecule transcribed from polynucleotide according to SEQ ID NO:84. In particular hpRNA molecule, ingested by an insect pest may be at least from about 80%, 81%, 82%, 83%, 84%, 85%, 86%, 87%, 88%, 89%, 90%, 91%, 92%, 93%, 94%, 95%, 96%, 97%, 98%, 99%, or about 100% identical to an RNA molecule transcribed from polynucleotide according to SEQ ID NO:86. In particular a hpRNA molecule, ingested by an insect pest may be at least from about 80%, 81%, 82%, 83%, 84%, 85%, 86%, 87%, 88%, 89%, 90%, 91%, 92%, 93%, 94%, 95%, 96%, 97%, 98%, 99%, or about 100% identical to an RNA molecule transcribed from polynucleotide according to SEQ ID NO:88. In particular hpRNA molecule, ingested by an insect pest may be at least from about 80%, 81%, 82%, 83%, 84%, 85%, 86%, 87%, 88%, 89%, 90%, 91%, 92%, 93%, 94%, 95%, 96%, 97%, 98%, 99%, or about 100% identical to an RNA molecule transcribed from polynucleotide according to SEQ ID NO:90. particular a hpRNA molecule, ingested by an insect pest may be at least from about 80%, 81%, 82%, 83%, 84%, 85%, 86%, 87%, 88%, 89%, 90%, 91%, 92%, 93%, 94%, 95%, 96%, 97%, 98%, 99%, or about 100% identical to an RNA molecule transcribed from polynucleotide according to SEQ ID NO:93. Isolated and substantially purified nucleic acid molecules including, but not limited to, non-naturally occurring polynucleotides and recombinant DNA constructs for providing dsRNA molecules are therefore provided, which suppress or inhibit the expression of an endogenous coding polynucleotide or a target coding polynucleotide in an insect pest when introduced thereto.

Particular embodiments provide a delivery system for the delivery of iRNA molecules for the post-transcriptional inhibition of one or more target gene(s) in an insect (*e.g.,* coleopteran) plant pest and control of a population of the plant pest. In some embodiments, the delivery system comprises ingestion of a host transgenic plant cell or contents of the host cell comprising RNA molecules transcribed in the host cell. In these and further embodiments, a transgenic plant cell or a transgenic plant is created that contains a recombinant DNA construct providing a stabilized dsRNA molecule of the invention. Transgenic plant cells and transgenic plants comprising nucleic acids encoding a particular iRNA molecule may be produced by employing recombinant DNA technologies (which basic technologies are well-known in the art) to construct a plant transformation vector comprising a polynucleotide encoding an iRNA molecule of the invention (a hpRNA molecule); to transform a plant cell or plant; and to generate the transgenic plant cell or the transgenic plant that contains the transcribed iRNA molecule.

To impart insect (*e.g.*, coleopteran) pest resistance to a transgenic plant, a recombinant DNA molecule may, for example, be transcribed into an iRNA molecule, such as a dsRNA molecule, a siRNA molecule, a miRNA molecule, a shRNA molecule, or a hpRNA molecule. In some embodiments, a RNA molecule transcribed from a recombinant DNA molecule may form a dsRNA molecule within the tissues or fluids of the recombinant plant. Such a dsRNA molecule may be comprised in part of a polynucleotide that is identical to a corresponding polynucleotide transcribed from a DNA within an insect pest of a type that may infest the host plant. Expression of a target gene within the pest is suppressed by the dsRNA molecule, and the suppression of expression of the target gene in the pest results in the transgenic plant being resistant to the pest. The modulatory effects of dsRNA molecules have been shown to be applicable to a variety of genes expressed in pests, including, for example, endogenous genes responsible for cellular metabolism or cellular transformation, including house-keeping genes; transcription factors; molting-related genes; and other genes which encode polypeptides involved in cellular metabolism or normal growth and development.

For transcription from a transgene *in vivo* or an expression construct, a regulatory region (*e.g.*, promoter, enhancer, silencer, and polyadenylation signal) may be used in some embodiments to transcribe the RNA strand (or strands). Therefore, in some embodiments, as set forth, *supra,* a polynucleotide for use in producing iRNA molecules may be operably linked to one or more promoter elements functional in a plant host cell. The promoter may be an endogenous promoter, normally resident in the host genome. The polynucleotide of the present invention, under the control of an operably linked promoter element, may further be flanked by additional elements that advantageously affect its transcription and/or the stability of a resulting transcript. Such elements may be located upstream of the operably linked promoter, downstream of the 3' end of the expression construct, and may occur both upstream of the promoter and downstream of the 3' end of the expression construct.

Some embodiments provide methods for reducing the damage to a host plant (*e.g.,* a corn plant or canola plant) caused by an insect (*e.g.*, coleopteran) pest that feeds on the plant, wherein the method comprises providing in the host plant a transformed plant cell expressing at least one nucleic acid molecule of the invention, wherein the nucleic acid molecule(s) functions upon being taken up by the pest(s) to inhibit the expression of a target polynucleotide within the pest(s), which inhibition of expression results in mortality and/or reduced growth of the pest(s), thereby reducing the damage to the host plant caused by the pest(s). In some embodiments, the nucleic acid molecule(s) comprise dsRNA molecules. In these and further embodiments, the nucleic acid molecule(s) comprise dsRNA molecules that each comprise more than one polynucleotide that is specifically hybridizable to a nucleic acid molecule expressed in a coleopteran pest cell. In some embodiments, the nucleic acid molecule(s) consist of one polynucleotide that is specifically hybridizable to a nucleic acid molecule expressed in an insect pest cell.

In some embodiments, a method for increasing the yield of a corn crop is provided, wherein the method comprises introducing into a corn plant at least one nucleic acid molecule of the invention; cultivating the corn plant to allow the expression of an iRNA molecule comprising the nucleic acid, wherein expression of an iRNA molecule comprising the nucleic acid inhibits insect (e.g., coleopteran) pest damage and/or growth, thereby reducing or eliminating a loss of yield due to pest infestation. In embodiments, the iRNA molecule is a dsRNA molecule. In these and further embodiments, the nucleic acid molecule(s) comprise dsRNA molecules that each comprise more than one polynucleotide that is specifically hybridizable to a nucleic acid molecule expressed in an insect pest cell. In some examples, the nucleic acid molecule(s) comprises a polynucleotide that is specifically hybridizable to a nucleic acid molecule expressed in a coleopteran pest cell.

In some embodiments, a method for modulating the expression of a target gene in an insect (e.g., coleopteran) pest is provided, the method comprising: transforming a plant cell with a vector comprising a polynucleotide encoding at least one iRNA molecule of the invention, wherein the polynucleotide is operatively-linked to a promoter and a transcription termination element; culturing the transformed plant cell under conditions sufficient to allow for development of a plant cell culture including a plurality of transformed plant cells; selecting for transformed plant cells that have integrated the polynucleotide into their genomes; screening the transformed plant cells for expression of an iRNA molecule encoded by the integrated polynucleotide; selecting a transgenic plant cell that expresses the iRNA molecule; and feeding the selected transgenic plant cell to the insect pest. Plants may also be regenerated from transformed plant cells that express an iRNA molecule encoded by the integrated nucleic acid molecule. In embodiments, the iRNA molecule is a dsRNA molecule. In these and further embodiments, the nucleic acid molecule(s) comprise dsRNA molecules that each comprise more than one polynucleotide that is specifically hybridizable to a nucleic acid molecule expressed in an insect pest cell. In some examples, the nucleic acid molecule(s) comprises a polynucleotide that is specifically hybridizable to a nucleic acid molecule expressed in a coleopteran pest cell.

iRNA molecules of the invention can be incorporated within the seeds of a plant species (e.g., corn or canola), either as a product of expression from a recombinant gene incorporated into a genome of the plant cells, or as incorporated into a coating or seed treatment that is applied to the seed before planting. A plant cell comprising a recombinant gene is considered to be a transgenic event. Also included in embodiments of the invention are delivery systems for the delivery of iRNA molecules to insect (e.g., coleopteran) pests. For example, the iRNA molecules of the invention may be directly introduced into the cells of a pest(s). Methods for introduction may include direct mixing of iRNA with plant tissue from a host for the insect pest(s), as well as application of compositions comprising iRNA molecules of the invention to host plant tissue. For example, iRNA molecules may be sprayed onto a plant surface. Alternatively, an iRNA molecule may be expressed by a microorganism, and the microorganism may be applied onto the plant surface, or introduced into a root or stem by a physical means such as an injection. As discussed, *supra,* a transgenic plant may also be genetically engineered to express at least one iRNA molecule in an amount sufficient to kill the insect pests known to infest the plant. iRNA molecules produced by chemical or enzymatic synthesis may also be formulated in a manner consistent with common agricultural practices, and used as spray-on products for controlling plant damage by an insect pest. The formulations may include the appropriate stickers and wetters required for efficient foliar coverage, as well as UV protectants to protect iRNA molecules (e.g., dsRNA molecules) from UV damage. Such additives are commonly used in the bioinsecticide industry, and are well known to those skilled in the art. Such applications may be combined with other spray-on insecticide applications (biologically based or otherwise) to enhance plant protection from the pests.

All references, including publications, patents, and patent applications, cited herein are hereby incorporated by reference to the extent they are not inconsistent with the explicit details of this disclosure, and are so incorporated to the same extent as if each reference were individually and specifically indicated to be incorporated by reference and were set forth in its entirety herein. The references discussed herein are provided solely for their disclosure prior to the filing date of the present application. Nothing herein is to be construed as an admission that the inventors are not entitled to antedate such disclosure by virtue of prior invention.

The following EXAMPLES are provided to illustrate certain particular features and/or aspects. These EXAMPLES should not be construed to limit the disclosure to the particular features or aspects described.

## EXAMPLES

### Example 1: Materials and Methods

Sample preparation and bioassays.

A number of dsRNA molecules (including those corresponding to *ncm* reg1 (SEQ ID NO:3), *ncm* reg2 (SEQ ID NO:4), *ncm* v1 (SEQ ID NO:5), and *ncm* v2 (SEQ ID NO:6) were synthesized and purified using a MEGASCRIPT® RNAi kit or HiScribe® T7 In Vitro Transcription Kit. The purified dsRNA molecules were prepared in TE buffer, and all bioassays contained a control treatment consisting of this buffer, which served as a background check for mortality or growth inhibition of WCR (*Diabrotica virgifera virgifera* LeConte). The concentrations of dsRNA molecules in the bioassay buffer were measured using a NANODROP™ 8000 spectrophotometer (THERMO SCIENTIFIC, Wilmington, DE).

Samples were tested for insect activity in bioassays conducted with neonate insect larvae on artificial insect diet. WCR eggs were obtained from CROP CHARACTERISTICS, INC. (Farmington, MN).

The bioassays were conducted in 128-well plastic trays specifically designed for insect bioassays (C-D INTERNATIONAL, Pitman, NJ). Each well contained approximately 1.0 mL of an artificial diet designed for growth of coleopteran insects. A 60 μL aliquot of dsRNA sample was delivered by pipette onto the surface of the diet of each well (40 $\mu L/cm^2$). dsRNA sample concentrations were calculated as the amount of dsRNA per square centimeter ($ng/cm^2$) of surface area (1.5 $cm^2$) in the well. The treated trays were held in a fume hood until the liquid on the diet surface evaporated or was absorbed into the diet.

Within a few hours of eclosion, individual larvae were picked up with a moistened camel hair brush and deposited on the treated diet (one or two larvae per well). The infested wells of the 128-well plastic trays were then sealed with adhesive sheets of clear plastic, and vented to allow gas exchange. Bioassay trays were held under controlled environmental conditions (28 °C, ~40% Relative Humidity, 16:8 (Light:Dark)) for 9 days, after which time the total number of insects exposed to each sample, the number of dead insects, and the weight of surviving insects were recorded. Average percent mortality and average growth inhibition were calculated for each treatment. Growth inhibition (GI) was calculated as follows:

$$GI = [1 - (TWIT/TNIT)/(TWIBC/TNIBC)],$$

where TWIT is the Total Weight of live Insects in the Treatment;
TNIT is the Total Number of Insects in the Treatment;
TWIBC is the Total Weight of live Insects in the Background Check (Buffer control); and
TNIBC is the Total Number of Insects in the Background Check (Buffer control).

The statistical analysis was done using JMP™ software (SAS, Cary, NC).

The $LC_{50}$ (Lethal Concentration) is defined as the dosage at which 50% of the test insects are killed. The $GI_{50}$ (Growth Inhibition) is defined as the dosage at which the mean growth (*e.g.* live weight) of the test insects is 50% of the mean value seen in Background Check samples.

Replicated bioassays demonstrated that ingestion of particular samples resulted in a surprising and unexpected mortality and growth inhibition of corn rootworm larvae.

### Example 2: Identification of Candidate Target Genes

Multiple stages of WCR (*Diabrotica virgifera virgifera* LeConte) development were selected for pooled transcriptome analysis to provide candidate target gene sequences for control by RNAi transgenic plant insect resistance technology.

In one exemplification, total RNA was isolated from about 0.9 gm whole first-instar WCR larvae; (4 to 5 days post-hatch; held at 16 °C), and purified using the following phenol/TRI REAGENT®-based method (MOLECULAR RESEARCH CENTER, Cincinnati, OH):

Larvae were homogenized at room temperature in a 15 mL homogenizer with 10 mL of TRI REAGENT® until a homogenous suspension was obtained. Following 5 min. incubation at room temperature, the homogenate was dispensed into 1.5 mL microfuge tubes (1 mL per tube), 200 μL of chloroform was added, and the mixture was vigorously shaken for 15 seconds. After allowing the extraction to sit at room temperature for 10 min, the phases were separated by centrifugation at 12,000 x g at 4 °C. The upper phase (comprising about 0.6 mL) was carefully transferred into another sterile 1.5 mL tube, and an equal volume of room temperature isopropanol was added. After incubation at room temperature for 5 to 10 min, the mixture was centrifuged 8 min at 12,000 x g (4 °C or 25 °C).

The supernatant was carefully removed and discarded, and the RNA pellet was washed twice by vortexing with 75% ethanol, with recovery by centrifugation for 5 min at 7,500 x g (4 °C or 25 °C) after each wash. The ethanol was carefully removed, the pellet was allowed to air-dry for 3 to 5 min, and then was dissolved in nuclease-free sterile water. RNA concentration was determined by measuring the absorbance (A) at 260 nm and 280 nm. A typical extraction from about 0.9 gm of larvae yielded over 1 mg of total RNA, with an $A_{260}/A_{280}$ ratio of 1.9. The RNA thus extracted was stored at -80 °C until further processed.

RNA quality was determined by running an aliquot through a 1% agarose gel. The agarose gel solution was made using autoclaved 10x TAE buffer (Tris-acetate EDTA; 1x concentration is 0.04 M Tris-acetate, 1 mM EDTA (ethylenediamine tetra-acetic acid sodium salt), pH 8.0) diluted with DEPC (diethyl pyrocarbonate)-treated water in an autoclaved container. 1x TAE was used as the running buffer. Before use, the electrophoresis tank and the well-forming comb were cleaned with RNaseAway™ (INVITROGEN INC., Carlsbad, CA). Two μL of RNA sample were mixed with 8 μL of TE buffer (10 mM Tris HCl pH 7.0; 1 mM EDTA) and 10 μL of RNA sample buffer (NOVAGEN® Catalog No 70606; EMD4 Bioscience, Gibbstown, NJ). The sample was heated at 70 °C for 3 min, cooled to room temperature, and 5 μL (containing 1 μg to 2 μg RNA) were loaded per well. Commercially available RNA molecular weight markers were simultaneously run in separate wells for molecular size comparison. The gel was run at 60 volts for 2 hrs.

A normalized cDNA library was prepared from the larval total RNA by a commercial service provider (EUROFINS MWG Operon, Huntsville, AL), using random priming. The normalized larval cDNA library was sequenced at 1/2 plate scale by GS FLX 454 Titanium™ series chemistry at EUROFINS MWG Operon, which resulted in over 600,000 reads with an average read length of 348 bp. 350,000 reads were assembled into over 50,000 contigs. Both the unassembled reads and the contigs were converted into BLASTable databases using the publicly available program, FORMATDB (available from NCBI).

Total RNA and normalized cDNA libraries were similarly prepared from materials harvested at other WCR developmental stages. A pooled transcriptome library for target gene screening was constructed by combining cDNA library members representing the various developmental stages.

Candidate genes for RNAi targeting were selected using information regarding lethal RNAi effects of particular genes in other insects such as *Drosophila* and *Tribolium.* These genes were hypothesized to be essential for survival and growth in coleopteran insects. Selected target gene homologs were identified in the transcriptome sequence database as described below. Full-length or partial sequences of the target genes were amplified by PCR to prepare templates for double-stranded RNA (dsRNA) production.

TBLASTN searches using candidate protein coding sequences were run against BLASTable databases containing the unassembled *Diabrotica* sequence reads or the assembled contigs. Significant hits to a *Diabrotica* sequence (defined as better than $e^{-20}$ for contigs homologies and better than $e^{-10}$ for unassembled sequence reads homologies) were confirmed using BLASTX against the NCBI non-redundant database. The results of this BLASTX search confirmed that the *Diabrotica* homolog candidate gene sequences identified in the TBLASTN search indeed comprised *Diabrotica* genes, or were the best hit to the non-*Diabrotica* candidate gene sequence present in the *Diabrotica* sequences. In a few cases, it was clear that some of the *Diabrotica* contigs or unassembled sequence reads selected by homology to a non-*Diabrotica* candidate gene overlapped, and that the assembly of the contigs had failed to join these overlaps. In those cases, Sequencher™ v4.9 (GENE CODES CORPORATION, Ann Arbor, MI) was used to assemble the sequences into longer contigs.

A candidate target gene encoding *Diabrotica ncm* (SEQ ID NO:1 and SEQ ID NO:77) was identified as a gene that may lead to coleopteran pest mortality, inhibition of growth or inhibition of development in WCR.

*Ncm* dsRNA transgenes can be combined with other dsRNA molecules to provide redundant RNAi targeting and synergistic RNAi effects. Transgenic corn events expressing dsRNA that targets *ncm* are useful for preventing root feeding damage by corn rootworm. *Ncm* dsRNA transgenes represent new modes of action for combining with *Bacillus thuringiensis* insecticidal protein technology in Insect Resistance Management gene pyramids to mitigate against the development of rootworm populations resistant to either of these rootworm control technologies.

Full-length or partial clones of sequences of a *Diabrotica* candidate gene, herein referred to as *ncm,* were used to generate PCR amplicons for dsRNA synthesis.

**EXAMPLE 3: Amplification of Target Genes to produce dsRNA**

Primers were designed to amplify portions of coding regions of each target gene by PCR. *See* **Table 1.** Where appropriate, a T7 phage promoter sequence (TTAATACGACTCACTATAGGGAGA; SEQ ID NO:7) was incorporated into the 5' ends of the amplified sense or antisense strands. *See* **Table 1.** Total RNA was extracted from WCR using TRIzol® (Life Technologies, Grand Island, NY), where WCR larvae and adults were homogenized at room temperature in a 1.5 mL microfuge tube with 1 mL of TRIzol® using a Pestle Motor Mixer (Cole-Parmer, Vernon Hills, IL) until a homogenous suspension was obtained. Following 5 min. incubation at room temperature, the homogenate was centrifuged to remove cell debris and 1 mL supernatant was transferred to a new tube. 200 μL of chloroform was added, and the mixture was vigorously shaken for 15 seconds. After allowing the extraction to sit at room temperature for 2-3 min, the phases were separated by centrifugation at 12,000 x g at 4 °C. The upper phase (comprising about 0.6 mL) was carefully transferred into another sterile 1.5 mL tube, and 500 uL of room temperature isopropanol was added. After incubation at room temperature for 10 min, the mixture was centrifuged 10 min at 12,000 x g at 4 °C. The supernatant was carefully removed and discarded, and the RNA pellet was washed twice by vortexing with 75% ethanol, with recovery by centrifugation for 5 min at 7,500 x g (4 °C or 25 °C) after each wash. The ethanol was carefully removed, the pellet was allowed to air-dry for 3 to 5 min, and then was dissolved in nuclease-free sterile water.

Total RNA was then used to make first-strand cDNA with SuperScriptIII® First-Strand Synthesis System and manufacturers Oligo dT primed instructions (Life Technologies, Grand Island, NY). This first-strand cDNA was used as template for PCR reactions using opposing primers positioned to amplify all or part of the native target gene sequence. dsRNA was also amplified from a DNA clone comprising the coding region for a yellow fluorescent protein (YFP) (SEQ ID NO:8; Shagin et al. (2004) Mol. Biol. Evol. 21(5):841-50).

**Table 1.** Primers and Primer Pairs used to amplify portions of coding regions of exemplary *ncm* target gene and YFP negative control gene.

| | Gene ID | Primer ID | Sequence |
|---|---|---|---|
| **Pair 1** | *ncm* reg 1 | Dvv_ncm-1-411 | TTAATACGACTCACTATAGGGAGAGGCTGCGTAAACGTTTGTAAAAG (SEQ ID NO:9) |
| | | Dvv_ncm-1-411_Rev | TTAATACGACTCACTATAGGGAGAGATGCGGCTCCCGAAGAATCTG (SEQ ID NO:10) |
| **Pair 2** | *ncm* reg2 | Dvv_ncm-2-407_For | TTAATACGACTCACTATAGGGAGAGTTAGCATCTAGTCTGTGAGTGG (SEQ ID NO:11) |
| | | Dvv_ncm-2-407_Rev | TTAATACGACTCACTATAGGGAGACCCTTAGTAAAGAAATCTTAGGCAG (SEQ ID NO:12) |
| **Pair 3** | *ncm* v1 | Dvv_ncm-1v1_For | TTAATACGACTCACTATAGGGAGATTAATGTAACCATGAACGGATTTC (SEQ ID NO:13) |
| | | Dvv_ncm-1v1_Rev | TTAATACGACTCACTATAGGGAGACAGTCATCAAACCAAGAGAAAG (SEQ ID NO:14) |
| **Pair 4** | *ncm* v2 | Dvv_ncm-1v2_For | TTAATACGACTCACTATAGGGAGAGGCTGCGTAAACGTTTGTAAAAG (SEQ ID NO:15) |
| | | Dvv_ncm-1v2_Rev | TTAATACGACTCACTATAGGGAGAATTGGCATCATCGCCAGAGAATTATTG (SEQ ID NO:16) |

(continued)

| | Gene ID | Primer ID | Sequence |
|---|---|---|---|
| Pair 5 | YFP | YFP-F_T7 | TTAATACGACTCACTATAGGGAGACACCATGGG CTCCAGCGGCGCCC (SEQ ID NO:27) |
| | | YFP-R T7 | TTAATACGACTCACTATAGGGAGAAGATCTTGA AGGCGCTCTTCAGG (SEQ ID NO:30) |

**EXAMPLE 4: RNAi Constructs**

Template preparation by PCR and dsRNA synthesis.

A strategy used to provide specific templates for *ncm* and YFP dsRNA production is shown in **FIG. 1.** Template DNAs intended for use in *ncm* dsRNA synthesis were prepared by PCR using the primer pairs in **Table 1** and (as PCR template) first-strand cDNA prepared from total RNA isolated from WCR eggs, first-instar larvae, or adults. For each selected *ncm* and YFP target gene region, PCR amplifications introduced a T7 promoter sequence at the 5' ends of the amplified sense and antisense strands (the YFP segment was amplified from a DNA clone of the YFP coding region). The two PCR amplified fragments for each region of the target genes were then mixed in approximately equal amounts, and the mixture was used as transcription template for dsRNA production. *See* **FIG. 1.** The sequences of the dsRNA templates amplified with the particular primer pairs were: SEQ ID NO:3 (*ncm* reg1), SEQ ID NO:4 (*ncm* reg2), SEQ ID NO:5 (*ncm* v1), SEQ ID NO:6 (*ncm* v2) and YFP (SEQ ID NO:8). Double-stranded RNA for insect bioassay was synthesized and purified using an AMBION® MEGASCRIPT® RNAi kit following the manufacturer's instructions (INVITROGEN) or HiScribe® T7 In Vitro Transcription Kit following the manufacturer's instructions (New England Biolabs, Ipswich, MA). The concentrations of dsRNAs were measured using a NANODROP™ 8000 spectrophotometer (THERMO SCIENTIFIC, Wilmington, DE).

Construction of plant transformation vectors

Entry vectors harboring a target gene construct for hairpin formation comprising segments of *ncm* (SEQ ID NO:1 and/or SEQ ID NO:77) are assembled using a combination of chemically synthesized fragments (DNA2.0, Menlo Park, CA) and standard molecular cloning methods. Intramolecular hairpin formation by RNA primary transcripts is facilitated by arranging (within a single transcription unit) two copies of a target gene segment in opposite orientation to one another, the two segments being separated by a linker sequence (*e.g.* SEQ ID NO:19). Thus, the primary mRNA transcript contains the two *ncm* gene segment sequences as large inverted repeats of one another, separated by the linker sequence. A copy of a promoter (*e.g.* maize ubiquitin 1, U.S. Patent No. 5,510,474; 35S from Cauliflower Mosaic Virus (CaMV); Sugarcane bacilliform badnavirus (ScBV) promoter; promoters from rice actin genes; ubiquitin promoters; pEMU; MAS; maize H3 histone promoter; ALS promoter; phaseolin gene promoter; *cab; rubisco; LAT52; Zm13;* and/or *apg*) is used to drive production of the primary mRNA hairpin transcript, and a fragment comprising a 3' untranslated region for example but not limited to a maize peroxidase 5 gene (ZmPer5 3'UTR v2; U.S. Patent No. 6,699,984), AtUbi10, AtEfl, or StPinII is used to terminate transcription of the hairpin-RNA-expressing gene.

Entry vectors pDAB126948 and pDAB126954 comprise a *ncm* v2-RNA construct (SEQ ID NO:17) that comprises a segment of *ncm* (SEQ ID NO:1).

Entry vectors described above are used in standard GATEWAY® recombination reactions with a typical binary destination vector to produce *ncm* hairpin RNA expression transformation vectors for *Agrobacterium*-mediated maize embryo transformations.

The Binary destination vector comprises a herbicide tolerance gene (aryloxyalknoate dioxygenase; *AAD-1* v3) (U.S. Patent 7,838,733, and Wright et al. (2010) Proc. Natl. Acad. Sci. U.S.A. 107:20240-5) under the regulation of a plant operable promoter (e.g., sugarcane bacilliform badnavirus (ScBV) promoter (Schenk et al. (1999) Plant Mol. Biol. 39:1221-30) or ZmUbi1 (U.S. Patent 5,510,474)). A 5'UTR and intron are positioned between the 3' end of the promoter segment and the start codon of the *AAD-1* coding region. A fragment comprising a 3' untranslated region from a maize lipase gene (ZmLip 3'UTR; U.S. Patent 7,179,902) is used to terminate transcription of the *AAD-1* mRNA.

A negative control binary vector that comprises a gene that expresses a YFP protein, is constructed by means of standard GATEWAY® recombination reactions with a typical binary destination vector and entry vector. The binary destination vector comprises a herbicide tolerance gene (aryloxyalknoate dioxygenase; *AAD-1* v3) (as above) under the expression regulation of a maize ubiquitin 1 promoter (as above) and a fragment comprising a 3' untranslated region from a maize lipase gene (ZmLip 3'UTR; as above). The entry Vector comprises a YFP coding region (SEQ ID NO:20) under the expression control of a maize ubiquitin 1 promoter (as above) and a fragment comprising a 3' untranslated region from a maize peroxidase 5 gene (as above).

**EXAMPLE 5: Screening of Candidate Target Genes**

Synthetic dsRNA designed to inhibit target gene sequences identified in EXAMPLE 2 caused mortality and growth inhibition when administered to WCR in diet-based assays. *Ncm* reg1, *ncm* reg2, *ncm* v1 and *ncm* v2 were observed to exhibit greatly increased efficacy in this assay over other dsRNAs screened.

Replicated bioassays demonstrated that ingestion of dsRNA preparations derived from *ncm* reg1, *ncm* reg2, *ncm* v1 and *ncm* v2 each resulted in mortality and/or growth inhibition of western corn rootworm larvae. **Table 2** and **Table 3** show the results of diet-based feeding bioassays of WCR larvae following 9-day exposure to these dsRNAs, as well as the results obtained with a negative control sample of dsRNA prepared from a yellow fluorescent protein (YFP) coding region (SEQ ID NO:8).

**Table 2.** Results of *ncm* dsRNA diet feeding assays obtained with western corn rootworm larvae after 9 days of feeding. ANOVA analysis found significance differences in Mean % Mortality and Mean % Growth Inhibition (GI). Means were separated using the Tukey-Kramer test.

| Gene Name | Dose ($ng/cm^2$) | N | Mean (%Mortality) ± SEM* | Mean (GI) ± SEM |
|---|---|---|---|---|
| ***ncm* reg1** | 500 | 7 | 86.50 ± 4.35 (A) | 0.77 ± 0.06 (A) |
| ***ncm* reg2** | 500 | 8 | 76.14 ± 3.99 (A) | 0.75 ± 0.06 (A) |
| ***ncm* v1** | 500 | 8 | 85.20 ± 3.51 (A) | 0.96 ± 0.01 (A) |
| ***ncm* v2** | 500 | 4 | 87.10 ± 3.40 (A) | 0.95 ± 0.03 (A) |
| **TE**** | 0 | 10 | 19.29 ± 4.36 (B) | -0.01 ± 0.08 (B) |
| **WATER** | 0 | 10 | 12.88 ± 3.20 (B) | 0.05 ± 0.08 (B) |
| **YFP***** | 500 | 11 | 18.29 ± 1.98 (B) | 0.14 ± 0.08 (B) |

*SEM =Standard Error of the Mean. Letters in parentheses designate statistical levels. Levels not connected by same letter are significantly different (P<0.05).
**TE = Tris HCl (1 mM) plus EDTA (0.1 mM) buffer, pH 7.2.
***YFP = Yellow Fluorescent Protein

**Table 3.** Summary of oral potency of *ncm* dsRNA on WCR larvae ($ng/cm^2$).

| Gene Name | $LC_{50}$ | Range | $GI_{50}$ | Range |
|---|---|---|---|---|
| ***ncm* reg1** | 7.04 | 4.87 - 9.96 | 4.20 | 1.61 - 10.89 |
| ***ncm* reg2** | 17.32 | 11.46 - 25.99 | 8.20 | 2.21 - 30.41 |
| ***ncm* v1** | 2.39 | 2.05 - 2.73 | 17.00 | 7.60 - 38.00 |
| ***ncm* v2** | 2.52 | 2.21 - 2.85 | 18.19 | 12.82 - 25.82 |

It has previously been suggested that certain genes of *Diabrotica* spp. may be exploited for RNAi-mediated insect control. *See* U.S. Patent Publication No. 2007/0124836, which discloses 906 sequences, and U.S. Patent No. 7,612,194, which discloses 9,112 sequences. However, it was determined that many genes suggested to have utility for RNAi-mediated insect control are not efficacious in controlling *Diabrotica.* It was also determined that sequences *ncm* reg1, *ncm* reg2, *ncm* v1 and *ncm* v2 each provide surprising and unexpected superior control of *Diabrotica,* compared to other genes suggested to have utility for RNAi-mediated insect control.

For example, *annexin, beta spectrin* 2, and *mtRP-L4* were each suggested in U.S. Patent No. 7,612,194 to be efficacious in RNAi-mediated insect control. SEQ ID NO:21 is the DNA sequence of *annexin* region 1 (Reg 1) and SEQ ID NO:22 is the DNA sequence of *annexin* region 2 (Reg 2). SEQ ID NO:23 is the DNA sequence of *beta spectrin* 2 region 1 (Reg 1) and SEQ ID NO:24 is the DNA sequence of *beta spectrin* 2 region 2 (Reg2). SEQ ID NO:25 is the DNA sequence of *mtRP-L4* region 1 (Reg 1) and SEQ ID NO:26 is the DNA sequence of *mtRP-L4* region 2 (Reg 2). A YFP sequence (SEQ ID NO:8) was also used to produce dsRNA as a negative control.

Each of the aforementioned sequences was used to produce dsRNA by the methods of EXAMPLE 3. The strategy used to provide specific templates for dsRNA production is shown in FIG. 2. Template DNAs intended for use in dsRNA synthesis were prepared by PCR using the primer pairs in **Table 4** and (as PCR template) first-strand cDNA prepared from total RNA isolated from WCR first-instar larvae. (YFP was amplified from a DNA clone.) For each selected target gene region, two separate PCR amplifications were performed. The first PCR amplification introduced a T7 promoter sequence at the 5' end of the amplified sense strands. The second reaction incorporated the T7 promoter sequence at the 5' ends of the antisense strands. The two PCR amplified fragments for each region of the target genes were then mixed in approximately equal amounts, and the mixture was used as transcription template for dsRNA production. *See* **FIG. 2.** Double-stranded RNA was synthesized and purified using an AMBION® MEGAscript® RNAi kit following the manufacturer's instructions (INVITROGEN). The concentrations of dsRNAs were measured using a NANODROP™ 8000 spectrophotometer (THERMO SCIENTIFIC, Wilmington, DE) and the dsRNAs were each tested by the same diet-based bioassay methods described above. **Table 4** lists the sequences of the primers used to produce the *annexin* Reg1, *annexin* Reg2, *beta spectrin* 2 Reg1, *beta spectrin* 2 Reg2, *mtRP-L4* Reg1, and *mtRP-L4* Reg2 dsRNA molecules. YFP primer sequences for use in the method depicted in **FIG. 2** are also listed in **Table 4. Table 5** presents the results of diet-based feeding bioassays of WCR larvae following 9-day exposure to these dsRNA molecules. Replicated bioassays demonstrated that ingestion of these dsRNAs resulted in no mortality or growth inhibition of western corn rootworm larvae above that seen with control samples of TE buffer, water, or YFP protein.

**Table 4.** Primers and Primer Pairs used to amplify portions of coding regions of genes.

| | Gene (Region) | Primer ID | Sequence |
|---|---|---|---|
| **Pair 5** | YFP | YFP-F_T7 | TTAATACGACTCACTATAGGGAGACACCATGGGCTCCAGCGGCGCCC (SEQ ID NO:27) |
| | YFP | YFP-R | AGATCTTGAAGGCGCTCTTCAGG (SEQ ID NO:28) |
| **Pair 6** | YFP | YFP-F | CACCATGGGCTCCAGCGGCGCCC (SEQ ID NO:29) |
| | YFP | YFP-R_T7 | TTAATACGACTCACTATAGGGAGAAGATCTTGAAGGCGCTCTTCAGG (SEQ ID NO:30) |
| **Pair 7** | *annexin* (Reg 1) | Ann-F1_T7 | TTAATACGACTCACTATAGGGAGAGCTCCAACAGTGGTTCCTTATC (SEQ ID NO:31) |
| | *annexin* (Reg 1) | Ann-R1 | CTAATAATTCTTTTTTAATGTTCCTGAGG (SEQ ID NO:32) |
| **Pair 8** | *annexin* (Reg 1) | Ann-F1 | GCTCCAACAGTGGTTCCTTATC (SEQ ID NO:33) |
| | *annexin* (Reg 1) | Ann-R1_T7 | TTAATACGACTCACTATAGGGAGACTAATAATTCTTTTTTAATGTTCCTGAGG (SEQ ID NO:34) |
| **Pair 9** | *annexin* (Reg 2) | Ann-F2_T7 | TTAATACGACTCACTATAGGGAGATTGTTACAAGCTGGAGAACTTCTC (SEQ ID NO:35) |
| | *annexin* (Reg 2) | Ann-R2 | CTTAACCAACAACGGCTAATAAGG (SEQ ID NO:36) |
| **Pair 10** | *annexin* (Reg 2) | Ann-F2 | TTGTTACAAGCTGGAGAACTTCTC (SEQ ID NO:37) |
| | *annexin* (Reg 2) | Ann-R2T7 | TTAATACGACTCACTATAGGGAGACTTAACCAACAACGGCTAATAAGG (SEQ ID NO:38) |

(continued)

| | Gene (Region) | Primer ID | Sequence |
|---|---|---|---|
| Pair 11 | *beta-spect2* (Reg 1) | Betasp2-F1_T7 | TTAATACGACTCACTATAGGGAGAAGATGTT GGCTGCATCTAGAGAA (SEQ ID NO:39) |
| | *beta-spect2* (Reg 1) | Betasp2-R1 | GTCCATTCGTCCATCCACTGCA (SEQ ID NO:40) |
| Pair 12 | *beta-spect2* (Reg 1) | Betasp2-F1 | AGATGTTGGCTGCATCTAGAGAA (SEQ ID NO:41) |
| | *beta-spect2* (Reg 1) | Betasp2-R1_T7 | TTAATACGACTCACTATAGGGAGAGTCCATT CGTCCATCCACTGCA (SEQ ID NO:42) |
| Pair 13 | *beta-spect2* (Reg 2) | Betasp2-F2_T7 | TTAATACGACTCACTATAGGGAGAGCAGATG AACACCAGCGAGAAA (SEQ ID NO:43) |
| | *beta-spect2* (Reg 2) | Betasp2-R2 | CTGGGCAGCTTCTTGTTTCCTC (SEQ ID NO:44) |
| Pair 14 | *beta-spect2* (Reg 2) | Betasp2-F2 | GCAGATGAACACCAGCGAGAAA (SEQ ID NO:45) |
| | *beta-spect2* (Reg 2) | Betasp2-R2_T7 | TTAATACGACTCACTATAGGGAGACTGGGCA GCTTCTTGTTTCCTC (SEQ ID NO:46) |
| Pair 15 | *mtRP-L4* (Reg 1) | L4-F1_T7 | TTAATACGACTCACTATAGGGAGAAGTGAAA TGTTAGCAAATATAACATCC (SEQ ID NO:47) |
| | *mtRP-L4* (Reg 1) | L4-R1 | ACCTCTCACTTCAAATCTTGACTTTG (SEQ ID NO:48) |
| Pair 16 | *mtRP-L4* (Reg 1) | L4-F1 | AGTGAAATGTTAGCAAATATAACATCC (SEQ ID NO:49) |
| | *mtRP-L4* (Reg 1) | L4-R1_T7 | TTAATACGACTCACTATAGGGAGAACCTCTC ACTTCAAATCTTGACTTTG (SEQ ID NO:50) |
| Pair 17 | *mtRP-L4* (Reg 2) | L4-F2_T7 | TTAATACGACTCACTATAGGGAGACAAAGTC AAGATTTGAAGTGAGAGGT (SEQ ID NO:51) |
| | *mtRP-L4* (Reg 2) | L4-R2 | CTACAAATAAAACAAGAAGGACCCC (SEQ ID NO:52) |
| Pair 18 | *mtRP-L4* (Reg 2) | L4-F2 | CAAAGTCAAGATTTGAAGTGAGAGGT (SEQ ID NO:53) |
| | *mtRP-L4* (Reg 2) | L4-R2_T7 | TTAATACGACTCACTATAGGGAGACTACAAA TAAAACAAGAAGGACCCC (SEQ ID NO:54) |

**Table 5.** Results of diet feeding assays obtained with western corn rootworm larvae after 9 days.

| Gene Name | Dose (ng/cm$^2$) | Mean Live Larval Weight (mg) | Mean % Mortality | Mean Growth Inhibition |
|---|---|---|---|---|
| ***annexin-Reg* 1** | 1000 | 0.545 | 0 | -0.262 |
| ***annexin-Reg* 2** | 1000 | 0.565 | 0 | -0.301 |
| ***beta spectrin2* Reg 1** | 1000 | 0.340 | 12 | -0.014 |

(continued)

| Gene Name | Dose (ng/cm$^2$) | Mean Live Larval Weight (mg) | Mean % Mortality | Mean Growth Inhibition |
|---|---|---|---|---|
| ***beta spectrin2* Reg 2** | 1000 | 0.465 | 18 | -0.367 |
| ***mtRP-L4* Reg 1** | 1000 | 0.305 | 4 | -0.168 |
| ***mtRP-L4* Reg 2** | 1000 | 0.305 | 7 | -0.180 |
| **TE buffer*** | 0 | 0.430 | 13 | 0.000 |
| **Water** | 0 | 0.535 | 12 | 0.000 |
| **YFP**** | 1000 | 0.480 | 9 | -0.386 |
| *TE = Tris HCl (10 mM) plus EDTA (1 mM) buffer, pH8.<br>**YFP = Yellow Fluorescent Protein | | | | |

**EXAMPLE 6: Production of Transgenic Maize Tissues Comprising Insecticidal dsRNAs**

*Agrobacterium*-mediated Transformation. Transgenic maize cells, tissues, and plants that produce one or more insecticidal dsRNA molecules (for example, at least one dsRNA molecule including a dsRNA molecule targeting a gene comprising *ncm;* SEQ ID NO:1 and SEQ ID NO:77) through expression of a chimeric gene stably-integrated into the plant genome are produced following *Agrobacterium*-mediated transformation. Maize transformation methods employing superbinary or binary transformation vectors are known in the art, as described, for example, in U.S. Patent No. 8,304,604, which is herein incorporated by reference in its entirety. Transformed tissues are selected by their ability to grow on Haloxyfop-containing medium and are screened for dsRNA production, as appropriate. Portions of such transformed tissue cultures may be presented to neonate corn rootworm larvae for bioassay, essentially as described in EXAMPLE 1.

*Agrobacterium* Culture Initiation. Glycerol stocks of *Agrobacterium* strain DAt13192 cells (WO 2012/016222A2) harboring a binary transformation vector as described above (EXAMPLE 4) are streaked on AB minimal medium plates (Watson et al. (1975) J. Bacteriol. 123:255-64) containing appropriate antibiotics and are grown at 20 °C for 3 days. The cultures are then streaked onto YEP plates (gm/L: yeast extract, 10; Peptone, 10; NaCl, 5) containing the same antibiotics and are incubated at 20 °C for 1 day.

*Agrobacterium* culture. On the day of an experiment, a stock solution of Inoculation Medium and acetosyringone is prepared in a volume appropriate to the number of constructs in the experiment and pipetted into a sterile, disposable, 250 mL flask. Inoculation Medium ((Frame et al. (2011) Genetic Transformation Using Maize Immature Zygotic Embryos. IN Plant Embryo Culture Methods and Protocols: Methods in Molecular Biology. T. A. Thorpe and E. C. Yeung, (Eds), Springer Science and Business Media, LLC. pp 327-341) contained: 2.2 gm/L MS salts; IX ISU Modified MS Vitamins (Frame et al., ibid.) 68.4 gm/L sucrose; 36 gm/L glucose; 115 mg/L L-proline; and 100 mg/L myo-inositol; at pH 5.4.) Acetosyringone is added to the flask containing Inoculation Medium to a final concentration of 200 μM from a 1 M stock solution in 100% dimethyl sulfoxide and the solution is thoroughly mixed.

For each construct, 1 or 2 inoculating loops-full of *Agrobacterium* from the YEP plate are suspended in 15 mL of the Inoculation Medium/acetosyringone stock solution in a sterile, disposable, 50 mL centrifuge tube, and the optical density of the solution at 550 nm ($OD_{550}$) is measured in a spectrophotometer. The suspension is then diluted to $OD_{550}$ of 0.3 to 0.4 using additional Inoculation Medium/acetosyringone mixture. The tube of *Agrobacterium* suspension is then placed horizontally on a platform shaker set at about 75 rpm at room temperature and shaken for 1 to 4 hours while embryo dissection is performed.

Ear sterilization and embryo isolation. Maize immature embryos are obtained from plants of *Zea mays* inbred line B104 (Hallauer et al. (1997) Crop Science 37:1405-1406) grown in the greenhouse and self- or sib-pollinated to produce ears. The ears are harvested approximately 10 to 12 days post-pollination. On the experimental day, de-husked ears are surface-sterilized by immersion in a 20% solution of commercial bleach (ULTRA CLOROX® Germicidal Bleach, 6.15% sodium hypochlorite; with two drops of TWEEN 20) and shaken for 20 to 30 min, followed by three rinses in sterile deionized water in a laminar flow hood. Immature zygotic embryos (1.8 to 2.2 mm long) are aseptically dissected from each ear and randomly distributed into microcentrifuge tubes containing 2.0 mL of a suspension of appropriate *Agrobacterium* cells in liquid Inoculation Medium with 200 μM acetosyringone, into which 2 μL of 10% BREAK-THRU® S233 surfactant (EVONIK INDUSTRIES; Essen, Germany) had been added. For a given set of experiments, embryos from pooled ears are used for each transformation.

*Agrobacterium* co-cultivation. Following isolation, the embryos are placed on a rocker platform for 5 minutes.

The contents of the tube are then poured onto a plate of Co-cultivation Medium, which contains 4.33 gm/L MS salts; 1X ISU Modified MS Vitamins; 30 gm/L sucrose; 700 mg/L L-proline; 3.3 mg/L Dicamba in KOH (3,6-dichloro-o-anisic acid or 3,6-dichloro-2-methoxybenzoic acid); 100 mg/L myo-inositol; 100 mg/L Casein Enzymatic Hydrolysate; 15 mg/L $AgNO_3$; 200 μM acetosyringone in DMSO; and 3 gm/L GELZAN™, at pH 5.8. The liquid *Agrobacterium* suspension is removed with a sterile, disposable, transfer pipette. The embryos are then oriented with the scutellum facing up using sterile forceps with the aid of a microscope. The plate is closed, sealed with 3M™ MICROPORE™ medical tape, and placed in an incubator at 25 °C with continuous light at approximately 60 μmol $m^{-2}s^{-1}$ of Photosynthetically Active Radiation (PAR).

Callus Selection and Regeneration of Transgenic Events. Following the Co-Cultivation period, embryos are transferred to Resting Medium, which is composed of 4.33 gm/L MS salts; IX ISU Modified MS Vitamins; 30 gm/L sucrose; 700 mg/L L-proline; 3.3 mg/L Dicamba in KOH; 100 mg/L myo-inositol; 100 mg/L Casein Enzymatic Hydrolysate; 15 mg/L $AgNO_3$; 0.5 gm/L MES (2-(N-morpholino)ethanesulfonic acid monohydrate; PHYTOTECHNOLOGIES LABR.; Lenexa, KS); 250 mg/L Carbenicillin; and 2.3 gm/L GELZAN™; at pH 5.8. No more than 36 embryos are moved to each plate. The plates are placed in a clear plastic box and incubated at 27 °C with continuous light at approximately 50 μmol $m^{-2}s^{-1}$ PAR for 7 to 10 days. Callused embryos are then transferred (<18/plate) onto Selection Medium I, which is comprised of Resting Medium (above) with 100 nM R-Haloxyfop acid (0.0362 mg/L; for selection of calli harboring the AAD-1 gene). The plates are returned to clear boxes and incubated at 27 °C with continuous light at approximately 50 μmol $m^{-2}s^{-1}$ PAR for 7 days. Callused embryos are then transferred (<12/plate) to Selection Medium II, which is comprised of Resting Medium (above) with 500 nM R-Haloxyfop acid (0.181 mg/L). The plates are returned to clear boxes and incubated at 27 °C with continuous light at approximately 50 μmol $m^{-2}s^{-1}$ PAR for 14 days. This selection step allows transgenic callus to further proliferate and differentiate.

Proliferating, embryogenic calli are transferred (<9/plate) to Pre-Regeneration medium. Pre-Regeneration Medium contains 4.33 gm/L MS salts; IX ISU Modified MS Vitamins; 45 gm/L sucrose; 350 mg/L L-proline; 100 mg/L myo-inositol; 50 mg/L Casein Enzymatic Hydrolysate; 1.0 mg/L $AgNO_3$; 0.25 gm/L MES; 0.5 mg/L naphthaleneacetic acid in NaOH; 2.5 mg/L abscisic acid in ethanol; 1 mg/L 6-benzylaminopurine; 250 mg/L Carbenicillin; 2.5 gm/L GELZAN™; and 0.181 mg/L Haloxyfop acid; at pH 5.8. The plates are stored in clear boxes and incubated at 27 °C with continuous light at approximately 50 μmol $m^{-2}s^{-1}$ PAR for 7 days. Regenerating calli are then transferred (<6/plate) to Regeneration Medium in PHYTATRAYS™ (SIGMA-ALDRICH) and incubated at 28 °C with 16 hours light/8 hours dark per day (at approximately 160 μmol $m^{-2}s^{-1}$ PAR) for 14 days or until shoots and roots develop. Regeneration Medium contains 4.33 gm/L MS salts; IX ISU Modified MS Vitamins; 60 gm/L sucrose; 100 mg/L myo-inositol; 125 mg/L Carbenicillin; 3 gm/L GELLAN™ gum; and 0.181 mg/L R-Haloxyfop acid; at pH 5.8. Small shoots with primary roots are then isolated and transferred to Elongation Medium without selection. Elongation Medium contains 4.33 gm/L MS salts; IX ISU Modified MS Vitamins; 30 gm/L sucrose; and 3.5 gm/L GELRITE™: at pH 5.8.

Transformed plant shoots selected by their ability to grow on medium containing Haloxyfop are transplanted from PHYTATRAYS™ to small pots filled with growing medium (PROMIX BX; PREMIER TECH HORTICULTURE), covered with cups or HUMI-DOMES (ARCO PLASTICS), and then hardened-off in a CONVIRON growth chamber (27 °C day/24 °C night, 16-hour photoperiod, 50-70% RH, 200 μmol $m^{-2}s^{-1}$ PAR). In some instances, putative transgenic plantlets are analyzed for transgene relative copy number by quantitative real-time PCR assays using primers designed to detect the AAD1 herbicide tolerance gene integrated into the maize genome. Further, DNA qPCR assays are used to detect the presence of the linker sequence and/or target sequence in putative transformants. Selected transformed plantlets are then moved into a greenhouse for further growth and testing.

Transfer and establishment of To plants in the greenhouse for bioassay and seed production. When plants reach the V3-V4 stage, they are transplanted into IE CUSTOM BLEND (PROFILE/METRO MIX 160) soil mixture and grown to flowering in the greenhouse (Light Exposure Type: Photo or Assimilation; High Light Limit: 1200 PAR; 16-hour day length; 27 °C day/24 °C night).

Plants to be used for insect bioassays are transplanted from small pots to TINUS™ 350-4 ROOTRAINERS® (SPENCER-LEMAIRE INDUSTRIES, Acheson, Alberta, Canada;) (one plant per event per ROOTRAINER®). Approximately four days after transplanting to ROOTRAINERS®, plants are infested for bioassay.

Plants of the $T_1$ generation are obtained by pollinating the silks of $T_0$ transgenic plants with pollen collected from plants of non-transgenic inbred line B104 or other appropriate pollen donors, and planting the resultant seeds. Reciprocal crosses are performed when possible.

**EXAMPLE 7: Molecular Analyses of Transgenic Maize Tissues**

Molecular analyses (e.g. RNA qPCR) of maize tissues are performed on samples from leaves that are collected from greenhouse grown plants on the day before or same days that root feeding damage is assessed.

Results of RNA qPCR assays for the target gene are used to validate expression of the transgene. Results of RNA qPCR assay for intervening sequence between repeat sequences (which is integral to the formation of dsRNA hairpin molecules) in expressed RNAs can also be used to validate the presence of hairpin transcripts. Transgene RNA expression levels are measured relative to the RNA levels of an endogenous maize gene.

DNA qPCR analyses to detect a portion of the AAD1 coding region in genomic DNA are used to estimate transgene insertion copy number. Samples for these analyses are collected from plants grown in environmental chambers. Results are compared to DNA qPCR results of assays designed to detect a portion of a single-copy native gene, and simple events (having one or two copies of the transgenes) are advanced for further studies in the greenhouse. Results are compared to DNA qPCR results of assays designed to detect a portion of a single-copy native gene, and simple events (one or two copies of the transgenes) are advanced for further studies.

Additionally, qPCR assays designed to detect a portion of the spectinomycin-resistance gene (SpecR; harbored on the binary vector plasmids outside of the T-DNA) are used to determine if the transgenic plants contained extraneous integrated plasmid backbone sequences.

RNA transcript expression level: target qPCR. Callus cell events or transgenic plants are analyzed by real time quantitative PCR (qPCR) of the target sequence to determine the relative expression level of the transgene, as compared to the transcript level of an internal maize gene (SEQ ID NO:55; GENBANK Accession No. BT069734), which encodes a TIP41-like protein (*i.e.,* a maize homolog of GENBANK Accession No. AT4G34270; having a tBLASTX score of 74% identity). RNA is isolated using Norgen BioTek Total RNA Isolation Kit (Norgen, Thorold, ON). The total RNA is subjected to an On-Column DNase1 treatment according to the kit's suggested protocol. The RNA is then quantified on a NANODROP 8000 spectrophotometer (THERMO SCIENTIFIC) and concentration is normalized to 50 ng/μL. First strand cDNA is prepared using a High Capacity cDNA synthesis kit (INVITROGEN) in a 10 μL reaction volume with 5 μL denatured RNA, substantially according to the manufacturer's recommended protocol. The protocol is modified slightly to include the addition of 10 μL of 100 μM T20VN oligonucleotide (IDT) (SEQ ID NO:56; TTTTTTTTTTTTTTTTTTTTVN, where V is A, C, or G, and N is A, C, G, or T/U) into the 1 mL tube of random primer stock mix, in order to prepare a working stock of combined random primers and oligo dT.

Following cDNA synthesis, samples are diluted 1:3 with nuclease-free water, and stored at -20 °C until assayed.

Separate real-time PCR assays for the target gene and TIP41-like transcript are performed on a LIGHTCYCLER™ 480 (ROCHE DIAGNOSTICS, Indianapolis, IN) in 10 μL reaction volumes. For the target gene assay, reactions are run with Primers HpNcmlv2 FWD Set 1 (SEQ ID NO:57) and HpNCM1v2 REV Set1 (SEQ ID NO:58), and an IDT Custom Oligo probe HpNcm1v2 PRB Set1, labeled with FAM and double quenched with Zen and Iowa Black quenchers. For the TIP41-like reference gene assay, primers TIPmxF (SEQ ID NO:59) and TIPmxR (SEQ ID NO:60), and Probe HXTIP (SEQ ID NO:61) labeled with HEX (hexachlorofluorescein) are used.

All assays include negative controls of no-template (mix only). For the standard curves, a blank (water in source well) is also included in the source plate to check for sample cross-contamination. Primer and probe sequences are set forth in **Table 7.** Reaction components recipes for detection of the various transcripts are disclosed in **Table 8,** and PCR reactions conditions are summarized in **Table 9.** The FAM (6-Carboxy Fluorescein Amidite) fluorescent moiety is excited at 465 nm and fluorescence is measured at 510 nm; the corresponding values for the HEX (hexachlorofluorescein) fluorescent moiety are 533 nm and 580 nm.

**Table 7.** Oligonucleotide sequences for molecular analyses of transcript levels in transgenic maize.

| Target | Oligonucleotide | Sequence |
|---|---|---|
| Ncm | HpNcm1v2 FWD Set1 | TGCTGCTTGTGCTTGTATTATTG (SEQ ID NO:57) |
| Ncm | HpNCM1v2 REV Set1 | GGCATCATCGCCAGAGAATTA (SEQ ID NO:58) |
| Ncm | HpNcm1v2 PRB Set1 (FAM-Probe) | /56-FAM/ACTTGCACA/ZEN/GCAAACCTCTACCTCT/3IABkFQ/ |
| TIP41 | TIPmxF | TGAGGGTAATGCCAACTGGTT (SEQ ID NO:59) |
| TIP41 | TIPmxR | GCAATGTAACCGAGTGTCTCTCAA (SEQ ID NO:60) |
| TIP41 | HXTIP (HEX-Probe) | TTTTTGGCTTAGAGTTGATGGTGTACTGATGA (SEQ ID NO:61) |
| *TIP41-like protein. | | |

**Table 8.** PCR reaction recipes for transcript detection.

| | *ncm* | TIP-like Gene |
|---|---|---|
| **Component** | **Final Concentration** | |
| Roche Buffer | 1 X | 1X |
| HpNcm1v2 FWD Set1 | 0.4 μM | 0 |
| HpNcm1v2 REV Set1 | 0.4 μM | 0 |
| HpNcm1v2 PRB Set1 (FAM) | 0.2 μM | 0 |
| HEXtipZM F | 0 | 0.4 μM |
| HEXtipZM R | 0 | 0.4 μM |
| HEXtipZMP (HEX) | 0 | 0.2 μM |
| cDNA (2.0 μL) | NA | NA |
| Water | To 10 μL | To 10 μL |

**Table 9.** Thermocycler conditions for RNA qPCR.

| Target gene and TIP41-like Gene Detection | | | |
|---|---|---|---|
| **Process** | **Temp.** | **Time** | **No. Cycles** |
| Target Activation | 95 °C | 10 min | 1 |
| Denature | 95 °C | 10 sec | 40 |
| Extend | 60 °C | 40 sec | |
| Acquire FAM or HEX | 72 °C | 1 sec | |
| Cool | 40 °C | 10 sec | 1 |

Data is analyzed using LIGHTCYCLER™ Software v1.5 by relative quantification using a second derivative max algorithm for calculation of Cq values according to the supplier's recommendations. For expression analyses, expression values are calculated using the ΔΔCt method (*i.e.,* 2-(Cq TARGET - Cq REF)), which relies on the comparison of differences of Cq values between two targets, with the base value of 2 being selected under the assumption that, for optimized PCR reactions, the product doubles every cycle.

Hairpin transcript size and integrity: Northern Blot Assay. In some instances, additional molecular characterization of the transgenic plants is obtained by the use of Northern Blot (RNA blot) analysis to determine the molecular size of the *ncm* hairpin RNA in transgenic plants expressing a *ncm* hairpin dsRNA.

All materials and equipment are treated with RNASFZAP™ (AMBION/INVITROGEN) before use. Tissue samples (100 mg to 500 mg) are collected in 2 mL SAFELOCK EPPENDORF tubes, disrupted with a KLFCKO™ tissue pulverizer (GARCIA MANUFACTURING, Visalia, CA) with three tungsten beads in 1 mL TRIZOL (INVITROGEN) for 5 min, then incubated at room temperature (RT) for 10 min. Optionally, the samples are centrifuged for 10 min at 4 °C at 11,000 rpm and the supernatant is transferred into a fresh 2 mL SAFELOCK EPPENDORF tube. After 200 μL of chloroform are added to the homogenate, the tube is mixed by inversion for 2 to 5 min, incubated at RT for 10 minutes, and centrifuged at 12,000 x g for 15 min at 4 °C. The top phase is transferred into a sterile 1.5 mL EPPENDORF tube, 600 μL of 100% isopropanol are added, followed by incubation at RT for 10 min to 2 hr, then centrifuged at 12,000 x g for 10 min at 4 to 25 °C. The supernatant is discarded and the RNA pellet is washed twice with 1 mL of 70% ethanol, with centrifugation at 7,500 x g for 10 min at 4 to 25 °C between washes. The ethanol is discarded and the pellet is briefly air dried for 3 to 5 min before resuspending in 50 μL of nuclease-free water.

Total RNA is quantified using the NANODROP 8000® (THERMO-FISHER) and samples are normalized to 5 μg/10 μL. 10 μL of glyoxal (AMBION/INVITROGEN) are then added to each sample. Five to 14 ng of DIG RNA standard marker mix (ROCHE APPLIED SCIENCE, Indianapolis, IN) are dispensed and added to an equal volume of glyoxal. Samples and marker RNAs are denatured at 50 °C for 45 min and stored on ice until loading on a 1.25% SEAKEM GOLD agarose (LONZA, Allendale, NJ) gel in NORTHERNMAX 10 X glyoxal running buffer (AMBION/INVITROGEN). RNAs are separated by electrophoresis at 65 volts/30 mA for 2 hr and 15 min.

Following electrophoresis, the gel is rinsed in 2X SSC for 5 min and imaged on a GEL DOC station (BIORAD, Hercules, CA), then the RNA is passively transferred to a nylon membrane (MILLIPORE) overnight at RT, using 10X SSC as the transfer buffer (20X SSC consists of 3 M sodium chloride and 300 mM trisodium citrate, pH 7.0). Following the transfer, the membrane is rinsed in 2X SSC for 5 minutes, the RNA is UV-crosslinked to the membrane (AGILENT/STRATAGENE), and the membrane is allowed to dry at room temperature for up to 2 days.

The membrane is pre-hybridized in ULTRAHYB™ buffer (AMBION/INVITROGEN) for 1 to 2 hr. The probe consists of a PCR amplified product containing the sequence of interest, (for example, the antisense sequence portion of SEQ ID NO:17, as appropriate) labeled with digoxygenin by means of a ROCHE APPLIED SCIENCE DIG procedure. Hybridization in recommended buffer is overnight at a temperature of 60 °C in hybridization tubes. Following hybridization, the blot is subjected to DIG washes, wrapped, exposed to film for 1 to 30 minutes, then the film is developed, all by methods recommended by the supplier of the DIG kit.

Transgene copy number determination. Maize leaf pieces approximately equivalent to 2 leaf punches are collected in 96-well collection plates (QIAGEN™). Tissue disruption is performed with a KLECKO™ tissue pulverizer (GARCIA MANUFACTURING, Visalia, CA) in BIOSPRINT96™ API lysis buffer (supplied with a BIOSPRINT96™ PLANT KIT; QIAGEN™) with one stainless steel bead. Following tissue maceration, genomic DNA (gDNA) is isolated in high throughput format using a BIOSPRINT96™ PLANT KIT and a BIOSPRINT96™ extraction robot. Genomic DNA is diluted 1:3 DNA:water prior to setting up the qPCR reaction.

qPCR analysis. Transgene detection by hydrolysis probe assay is performed by real-time PCR using a LIGHTCYCLER®480 system. Oligonucleotides to be used in hydrolysis probe assays to detect the target gene (*e.g. ncm*), the linker sequence (*e.g.,* SEQ ID NO:19), and/or to detect a portion of the SpecR gene (*i.e.* the spectinomycin resistance gene borne on the binary vector plasmids; SEQ ID NO:73; SPC1 oligonucleotides in **Table 10**), are designed using LIGHTCYCLER® PROBE DESIGN SOFTWARE 2.0. Further, oligonucleotides to be used in hydrolysis probe assays to detect a segment of the AAD-1 herbicide tolerance gene (SEQ ID NO:67; GAAD1 oligonucleotides in **Table 10**) are designed using PRIMER EXPRESS software (APPLIED BIOSYSTEMS). **Table 10** shows the sequences of the primers and probes. Assays are multiplexed with reagents for an endogenous maize chromosomal gene (Invertase (SEQ ID NO:64; GENBANK Accession No: U16123; referred to herein as IVR1), which serves as an internal reference sequence to ensure gDNA is present in each assay. For amplification, LIGHTCYCLER®480 PROBES MASTER mix (ROCHE APPLIED SCIENCE) is prepared at 1x final concentration in a 10 μL volume multiplex reaction containing 0.4 μM of each primer and 0.2 μM of each probe (**Table 11**). A two-step amplification reaction is performed as outlined in **Table 12.** Fluorophore activation and emission for the FAM- and HEX-labeled probes are as described above; CY5 conjugates are excited maximally at 650 nm and fluoresce maximally at 670 nm.

Cp scores (the point at which the fluorescence signal crosses the background threshold) are determined from the real time PCR data using the fit points algorithm (LIGHTCYCLER® SOFTWARE release 1.5) and the Relative Quant module (based on the ΔΔCt method). Data are handled as described previously (above; RNA qPCR).

**Table 10.** Sequences of primers and probes (with fluorescent conjugate) for gene copy number determinations and binary vector plasmid backbone detection.

| Name | Sequence |
|---|---|
| GAAD1-F | TGTTCGGTTCCCTCTACCAA (SEQ ID NO:65) |
| GAAD1-R | CAACATCCATCACCTTGACTGA (SEQ ID NO:66) |
| GAAD1-P (FAM) | CACAGAACCGTCGCTTCAGCAACA (SEQ ID NO:67) |
| IVR1-F | TGGCGGACGACGACTTGT (SEQ ID NO:68) |
| IVR1-R | AAAGTTTGGAGGCTGCCGT (SEQ ID NO:69) |
| IVR1-P (HEX) | CGAGCAGACCGCCGTGTACTTCTACC (SEQ ID NO:70) |
| SPC1A | CTTAGCTGGATAACGCCAC (SEQ ID NO:71) |
| SPC1S | GACCGTAAGGCTTGATGAA (SEQ ID NO:72) |
| TQSPEC (CY5*) | CGAGATTCTCCGCGCTGTAGA (SEQ ID NO:73) |
| Loop_F | GGAACGAGCTGCTTGCGTAT (SEQ ID NO:74) |
| Loop_R | CACGGTGCAGCTGATTGATG (SEQ ID NO:75) |
| Loop_FAM | TCCCTTCCGTAGTCAGAG (SEQ ID NO:76) |
| CY5 = Cyanine-5 | |

**Table 11.** Reaction components for gene copy number analyses and plasmid backbone detection.

| Component | Amt. (μL) | Stock | Final Concentration |
|---|---|---|---|
| 2x Buffer | 5.0 | 2x | 1x |
| Appropriate Forward Primer | 0.4 | 10 μM | 0.4 |
| Appropriate Reverse Primer | 0.4 | 10 μM | 0.4 |
| Appropriate Probe | 0.4 | 5 μM | 0.2 |
| IVR1-Forward Primer | 0.4 | 10 μM | 0.4 |
| IVR1-Reverse Primer | 0.4 | 10 μM | 0.4 |
| IVR1-Probe | 0.4 | 5 μM | 0.2 |
| $H_2O$ | 0.6 | NA* | NA |
| gDNA | 2.0 | ND** | ND |
| **Total** | 10.0 | | |
| *NA = Not Applicable<br>**ND = Not Determined | | | |

**Table 12.** Thermocycler conditions for DNA qPCR.

| Genomic copy number analyses | | | |
|---|---|---|---|
| **Process** | **Temp.** | **Time** | **No. Cycles** |
| Target Activation | 95 °C | 10 min | 1 |
| Denature | 95 °C | 10 sec | 40 |
| Extend & Acquire FAM, HEX, or CY5 | 60 °C | 40 sec | |
| Cool | 40 °C | 10 sec | 1 |

**EXAMPLE 8: Bioassay of Transgenic Maize**

Insect Bioassays. Bioactivity of dsRNA of the subject invention produced in plant cells is demonstrated by bioassay methods. *See, e.g.,* Baum et al. (2007) Nat. Biotechnol. 25(11): 1322-1326. One is able to demonstrate efficacy, for example, by feeding various plant tissues or tissue pieces derived from a plant producing an insecticidal dsRNA to target insects in a controlled feeding environment. Alternatively, extracts are prepared from various plant tissues derived from a plant producing the insecticidal dsRNA, and the extracted nucleic acids are dispensed on top of artificial diets for bioassays as previously described herein. The results of such feeding assays are compared to similarly conducted bioassays that employ appropriate control tissues from host plants that do not produce an insecticidal dsRNA, or to other control samples. Growth and survival of target insects on the test diet is reduced compared to that of the control group.

Insect Bioassays with Transgenic Maize Events. Two western corn rootworm larvae (1 to 3 days old) hatched from washed eggs are selected and placed into each well of the bioassay tray. The wells are then covered with a "PULL N' PEEL " tab cover (BIO-CV-16, BIO-SERV) and placed in a 28 °C incubator with an 18 hr/6 hr light/dark cycle. Nine days after the initial infestation, the larvae are assessed for mortality, which is calculated as the percentage of dead insects out of the total number of insects in each treatment. Significant mortality is observed. The insect samples are frozen at -20 °C for two days, then the insect larvae from each treatment are pooled and weighed. The percent of growth inhibition is calculated as the mean weight of the experimental treatments divided by the mean of the average weight of two control well treatments. The data are expressed as a Percent Growth Inhibition (of the negative controls). Mean weights that exceed the control mean weight are normalized to zero. Significant growth inhibition is observed.

Insect bioassays in the greenhouse. Western corn rootworm (WCR, *Diabrotica virgifera virgifera* LeConte) eggs are received in soil from CROP CHARACTERISTICS (Farmington, MN). WCR eggs are incubated at 28 °C for 10 to 11 days. Eggs are washed from the soil, placed into a 0.15% agar solution, and the concentration is adjusted to approximately 75 to 100 eggs per 0.25 mL aliquot. A hatch plate is set up in a Petri dish with an aliquot of egg suspension to monitor hatch rates.

The soil around the maize plants growing in ROOTRANERS® is infested with 150 to 200 WCR eggs. The insects are allowed to feed for 2 weeks, after which time a "Root Rating" is given to each plant. A Node-Injury Scale is utilized for grading, essentially according to Oleson et al. (2005) J. Econ. Entomol. 98:1-8. Plants passing this bioassay, showing reduced injury, are transplanted to 5-gallon pots for seed production. Transplants are treated with insecticide to prevent further rootworm damage and insect release in the greenhouses. Plants are hand pollinated for seed production. Seeds produced by these plants are saved for evaluation at the $T_1$ and subsequent generations of plants.
Transgenic negative control plants are generated by transformation with vectors harboring genes designed to produce a yellow fluorescent protein (YFP). Non-transformed negative control plants are grown from seeds of parental corn varieties from which the transgenic plants are produced. Bioassays are conducted with negative controls included in each set of plant materials.

**EXAMPLE 9: Transgenic *Zea mays* Comprising Coleopteran Pest Sequences**

10-20 transgenic $T_0$ *Zea mays* plants are generated as described in EXAMPLE 6. A further 10-20 $T_1$ *Zea mays* independent lines expressing hairpin dsRNA for an RNAi construct are obtained for corn rootworm challenge. Hairpin dsRNA are as set forth in SEQ ID NO:17, or otherwise further comprising SEQ ID NO:1 or SEQ ID NO:77. Additional hairpin dsRNAs are derived, for example, from coleopteran pest sequences such as, for example, Cafl-180 (U.S. Patent Application Publication No. 2012/0174258), VatpaseC (U.S. Patent Application Publication No. 2012/0174259), Rho1 (U.S. Patent Application Publication No. 2012/0174260), VatpaseH (U.S. Patent Application Publication No. 2012/0198586), PPI-87B (U.S. Patent Application Publication No. 2013/0091600), RPA70 (U.S. Patent Application Publication No. 2013/0091601), RPS6 (U.S. Patent Application Publication No. 2013/0097730), *ROP* (U.S. Patent Application No. 14/577,811), *RNAPII140* (U.S. Patent Application No. 14/577,854), *Dre4* (U.S. Patent Application No. 14/705,807), *COPI alpha* (U.S. Patent Application No. 62/063,199), *COPI beta* (U.S. Patent Application No. 62/063,203), *COPI gamma* (U.S. Patent Application No. 62/063,192), or *COPI delta* (U.S. Patent Application No. 62/063,216). These are confirmed through RT-PCR or other molecular analysis methods.
Total RNA preparations from selected independent $T_1$ lines are optionally used for RT-PCR with primers designed to bind in the linker of the hairpin expression cassette in each of the RNAi constructs. In addition, specific primers for each target gene in an RNAi construct are optionally used to amplify and confirm the production of the pre-processed mRNA required for siRNA production *in planta.* The amplification of the desired bands for each target gene confirms the expression of the hairpin RNA in each transgenic *Zea mays* plant. Processing of the dsRNA hairpin of the target genes into siRNA is subsequently optionally confirmed in independent transgenic lines using RNA blot hybridizations.
Moreover, RNAi molecules having mismatch sequences with more than 80% sequence identity to target genes affect corn rootworms in a way similar to that seen with RNAi molecules having 100% sequence identity to the target genes. The pairing of mismatch sequence with native sequences to form a hairpin dsRNA in the same RNAi construct delivers plant-processed siRNAs capable of affecting the growth, development, and viability of feeding coleopteran pests.
*In planta* delivery of dsRNA, siRNA, or miRNA corresponding to target genes and the subsequent uptake by coleopteran pests through feeding results in down-regulation of the target genes in the coleopteran pest through RNA-mediated gene silencing. When the function of a target gene is important at one or more stages of development, the growth and/or development of the coleopteran pest is affected, and in the case of at least one of WCR, NCR, SCR, MCR, *D. balteata* LeConte, *D. u. tenella, D. speciosa* Germar, *D. u. undecimpunctata* Mannerheim, and *Meligethes aeneus* Fabricius, leads to failure to successfully infest, feed, and/or develop, or leads to death of the coleopteran pest. The choice of target genes and the successful application of RNAi are then used to control coleopteran pests.
Phenotypic comparison of transgenic RNAi lines and nontransformed *Zea mays.* Target coleopteran pest genes or sequences selected for creating hairpin dsRNA have no similarity to any known plant gene sequence. Hence, it is not expected that the production or the activation of (systemic) RNAi by constructs targeting these coleopteran pest genes or sequences will have any deleterious effect on transgenic plants. However, development and morphological characteristics of transgenic lines are compared with non-transformed plants, as well as those of transgenic lines transformed with an "empty" vector having no hairpin-expressing gene. Plant root, shoot, foliage and reproduction characteristics are compared. Plant shoot characteristics such as height, leaf numbers and sizes, time of flowering, floral size and appearance are recorded. In general, there are no observable morphological differences between transgenic lines and those without expression of target iRNA molecules when cultured *in vitro* and in soil in the glasshouse.

**EXAMPLE 10: Transgenic *Zea mays* Comprising a Coleopteran Pest Sequence and Additional RNAi Constructs**

A transgenic *Zea mays* plant comprising a heterologous coding sequence in its genome that is transcribed into an iRNA molecule that targets an organism other than a coleopteran pest is secondarily transformed *via Agrobacterium* or WHISKERS™ methodologies (*see* Petolino and Arnold (2009) Methods Mol. Biol. 526:59-67) to produce one or more insecticidal dsRNA molecules (for example, at least one dsRNA molecule including a dsRNA molecule targeting a gene comprising SEQ ID NO:1 or SEQ ID NO:77). Plant transformation plasmid vectors prepared essentially as described in EXAMPLE 4 are delivered *via Agrobacterium* or WHISKERS™-mediated transformation methods into maize suspension cells or immature maize embryos obtained from a transgenic Hi II or B104 *Zea mays* plant comprising a heterologous coding sequence in its genome that is transcribed into an iRNA molecule that targets an organism other than a coleopteran pest.

**EXAMPLE 11: Transgenic *Zea mays* Comprising an RNAi Construct and Additional Coleopteran Pest Control Sequences**

A transgenic *Zea mays* plant comprising a heterologous coding sequence in its genome that is transcribed into an iRNA molecule that targets a coleopteran pest organism (for example, at least one dsRNA molecule including a dsRNA molecule targeting a gene comprising SEQ ID NO:1 or SEQ ID NO:77) is secondarily transformed *via Agrobacterium* or WHISKERS™ methodologies (*see* Petolino and Arnold (2009) Methods Mol. Biol. 526:59-67) to produce one or more insecticidal protein molecules, for example, Cry3, Cry34 and Cry35 insecticidal proteins. Plant transformation plasmid vectors prepared essentially as described in EXAMPLE 4 are delivered *via Agrobacterium* or WHISKERS™-mediated transformation methods into maize suspension cells or immature maize embryos obtained from a transgenic B104 *Zea mays* plant comprising a heterologous coding sequence in its genome that is transcribed into an iRNA molecule that targets a coleopteran pest organism. Doubly-transformed plants are obtained that produce iRNA molecules and insecticidal proteins for control of coleopteran pests.

**EXAMPLE 12: Pollen Beetle Transcriptome**

Insects: Larvae and adult pollen beetles were collected from fields with flowering rapeseed plants (Giessen, Germany). Young adult beetles (each per treatment group: n = 20; 3 replicates) were challenged by injecting a mixture of two different bacteria (*Staphylococcus aureus* and *Pseudomonas aeruginosa*), one yeast (*Saccharomyces cerevisiae*) and bacterial LPS. Bacterial cultures were grown at 37 °C with agitation, and the optical density was monitored at 600 nm (OD600). The cells were harvested at OD600 ~1 by centrifugation and resuspended in phosphate-buffered saline. The mixture was introduced ventrolaterally by pricking the abdomen of pollen beetle imagoes using a dissecting needle dipped in an aqueous solution of 10 mg/ml LPS (purified *E. coli* endotoxin; Sigma, Taufkirchen, Germany) and the bacterial and yeast cultures. Along with the immune challenged beetles naive beetles and larvae were collected (n = 20 per and 3 replicates each) at the same time point.

RNA isolation: Total RNA was extracted 8 h after immunization from frozen beetles and larvae using TriReagent (Molecular Research Centre, Cincinnati, OH, USA) and purified using the RNeasy Micro Kit (Qiagen, Hilden, Germany) in each case following the manufacturers' guidelines. The integrity of the RNA was verified using an Agilent 2100 Bioanalyzer and a RNA 6000 Nano Kit (Agilent Technologies, Palo Alto, CA, USA). The quantity of RNA was determined using a Nanodrop ND-1000 spectrophotometer. RNA was extracted from each of the adult immune-induced treatment groups, adult control groups, and larval groups individually and equal amounts of total RNA were subsequently combined in one pool per sample (immune-challenged adults, control adults and larvae) for sequencing.

Transcriptome information: RNA-Seq data generation and assembly Single-read 100-bp RNA-Seq was carried out separately on 5 μg total RNA isolated from immune-challenged adult beetles, naive (control) adult beetles and untreated larvae. Sequencing was carried out by Eurofins MWG Operon using the Illumina HiSeq-2000 platform. This yielded 20.8 million reads for the adult control beetle sample, 21.5 million reads for the LPS-challenged adult beetle sample and 25.1 million reads for the larval sample. The pooled reads (67.5 million) were assembled using *Velvet*/*Oases* assembler software (M.H. Schulz et al. (2012) Bioinformatics. 28:1086-92; Zerbino & E. Birney (2008) Genome Research. 18:821-9). The transcriptome contained 55648 sequences.

Pollen beetle *ncm* identification: A tblastn search of the transcriptome was used to identify matching contigs. As a query the peptide sequence of *ncm* from *Tribolium castaneum* was used (Genbank XM_001811253.1). GAP5 (Bonfield JK & Whitwham (2010). Bioinformatics 26: 1699-1703) was used for verification of sequences.

**EXAMPLE 13: Mortality of Pollen Beetle *(Meligethes aeneus)***

**following treatment with *ncm* RNAi**

Gene-specific primers including the T7 polymerase promoter sequence at the 5' end were used to create PCR products of approximate 500 bp by PCR (SEQ ID NOs:91-92). PCR fragments were cloned in the pGEM T easy vector according to the manufacturer's protocol and sent to a sequencing company to verify the sequence. The dsRNA was then produced by the T7 RNA polymerase (MEGAscript® RNAi Kit, Applied Biosystems) from a PCR construct generated from the sequenced plasmid according to the manufacturer's protocol.

Injection of ~100 nL dsRNA (1 μg/μL) into larvae and adult beetles was performed with a micromanipulator under a dissecting stereomicroscope (n=10, 3 biological replications). Animals were anaesthetized on ice before they were affixed to double-stick tape. Controls received the same volume of water. A negative control dsRNA of IMPI (insect metalloproteinase inhibitor gene of the lepidopteran *Galleria mellonella*) were conducted. All controls in all stages could not be tested due to a lack of animals.

Pollen beetles were maintained in Petri dishes with dried pollen and a wet tissue. The larvae were reared in plastic boxes on inflorescence of canola in an agar/water media.

**Table 13.** Results of adult pollen beetle injection bioassay.

| Treatment | % Survival Mean ± SD* | | | | |
|---|---|---|---|---|---|
| | **Day 0** | **Day 2** | **Day 4** | **Day 6** | **Day 8** |
| *ncm* | 100 ± 0 | 83 ± 21 | 80 ± 17 | 67 ± 15 | 60 ± 17 |
| water | 100 ± 0 | 100 ± 0 | 100 ± 0 | 100 ± 0 | 100 ± 0 |
| | **Day 10** | **Day 12** | **Day 14** | **Day 16** | |
| *ncm* | 37 ± 15 | 33 ± 15 | 30 ± 17 | 13 ± 6 | |
| water | 93 ± 12 | 90 ± 10 | 87 ± 12 | 80 ± 10 | |
| * Standard deviation | | | | | |

**Table 14.** Results of larval pollen beetle injection bioassay.

| Treatment | % Survival Mean ± SD* | | | |
|---|---|---|---|---|
| | **Day 0** | **Day 2** | **Day 4** | **Day 6** |
| *ncm* | 100 ± 0 | 73 ± 15 | 50 ± 10 | 37 ± 25 |
| Negative control | 100 ± 0 | 100 ± 0 | 97 ± 6 | 73 ± 21 |
| * Standard deviation | | | | |

Controls were performed on a different date due to the limited availability of insects.

Feeding Bioassay: Beetles were kept without access to water in empty falcon tubes 24 h before treatment. A droplet of dsRNA (~5μL) was placed in a small Petri dish and 5 to 8 beetles were added to the Petri dish. Animals were observed under a stereomicroscope and those that ingested dsRNA containing diet solution were selected for the bioassay. Beetles were transferred into Petri dishes with dried pollen and a wet tissue. Controls received the same volume of water. A negative control dsRNA of IMPI (insect metalloproteinase inhibitor gene of the lepidopteran *Galleria mellonella*) was conducted. All controls in all stages could not be tested due to a lack of animals.

Table 15. Results of adult feeding bioassay.

| Treatment | % Survival Mean ± SD* | | | | |
|---|---|---|---|---|---|
| | **Day 0** | **Day 2** | **Day 4** | **Day 6** | **Day 8** |
| *ncm* | 100 ± 0 | 100 ± 0 | 100 ± 0 | 93 ± 6 | 93 ± 6 |
| Negative control | 100 ± 0 | 93 ± 5.8 | 90 ± 10 | 87 ± 5.8 | 83 ± 5.8 |
| water | 100 ± 0 | 100 ± 0 | 100 ± 0 | 93 ± 3.8 | 93 ±3.8 |
| | **Day 10** | **Day 12** | **Day 14** | **Day 16** | |
| *ncm* | 93 ± 6 | 83 ± 12 | 83 ± 12 | 83 ± 12 | |
| Negative control | 80 ± 10 | 80 ± 10 | 80 ± 10 | 77 ± 12 | |
| water | 93 ± 3.8 | 87 ± 10 | 80 ± 13 | 80 ± 13 | |
| * Standard deviation | | | | | |

Controls were performed on a different date due to the limited availability of insects.

While the present disclosure may be susceptible to various modifications and alternative forms, specific aspects of the disclosure have been described by way of example in detail herein. However, it should be understood that the present disclosure is not intended to be limited to the particular forms disclosed. Rather, the present disclosure is to cover all modifications, equivalents, and alternatives falling within the scope of the present disclosure as defined by the following appended claims and their legal equivalents.

**Example 14: *Agrobacterium*-mediated transformation of Canola (*Brassica nanus*) hypocotyls**

*Agrobacterium* Preparation

The *Agrobacterium* strain containing a binary plasmid is streaked out on YEP media (Bacto Peptone™ 20.0 gm/L and Yeast Extract 10.0 gm/L) plates containing streptomycin (100 mg/ml) and spectinomycin (50 mg/mL) and incubated for 2 days at 28 °C. The propagated *Agrobacterium* strain containing the binary plasmid is scraped from the 2-day streak plate using a sterile inoculation loop. The scraped *Agrobacterium* strain containing the binary plasmid is then inoculated into 150 mL modified YEP liquid with streptomycin (100 mg/ml) and spectinomycin (50 mg/ml) into sterile 500 mL baffled flask(s) and shaken at 200 rpm at 28 °C. The cultures are centrifuged and resuspended in M-medium (LS salts, 3% glucose, modified B5 vitamins, 1 μM kinetin, 1 μM 2,4-D, pH 5.8) and diluted to the appropriate density (50 Klett Units as measured using a spectrophotometer) prior to transformation of canola hypocotyls.

Canola Transformation

*Seed germination*: Canola seeds (var. NEXERA 710™) are surface-sterilized in 10% Clorox™ for 10 minutes and rinsed three times with sterile distilled water (seeds are contained in steel strainers during this process). Seeds are planted for germination on ½ MS Canola medium (1/2 MS, 2% sucrose, 0.8% agar) contained in Phytatrays™ (25 seeds per Phytatray™) and placed in a Percival™ growth chamber with growth regime set at 25 °C, photoperiod of 16 hours light and 8 hours dark for 5 days of germination.

*Pre-treatment*: On day 5, hypocotyl segments of about 3 mm in length are aseptically excised, the remaining root and shoot sections are discarded (drying of hypocotyl segments is prevented by immersing the hypocotyls segments into 10 mL of sterile milliQ™ water during the excision process). Hypocotyl segments are placed horizontally on sterile filter paper on callus induction medium, MSK1D1 (MS, 1 mg/L kinetin, 1 mg/L 2,4-D, 3.0% sucrose, 0.7% phytagar) for 3 days pre-treatment in a Percival™ growth chamber with growth regime of 22-23 °C, and a photoperiod of 16 hours light, 8 hours dark.

*Co-cultivation with Agrobacterium*: The day before *Agrobacterium* co-cultivation, flasks of YEP medium containing the appropriate antibiotics, are inoculated with the *Agrobacterium* strain containing the binary plasmid. Hypocotyl segments are transferred from filter paper callus induction medium, MSK1D1 to an empty 100 x 25 mm Petri™ dishes containing 10 mL of liquid M-medium to prevent the hypocotyl segments from drying. A spatula is used at this stage to scoop the segments and transfer the segments to new medium. The liquid M-medium is removed with a pipette and 40 mL of *Agrobacterium* suspension is added to the Petri™ dish (500 segments with 40 mL of *Agrobacterium* solution). The hypocotyl segments are treated for 30 minutes with periodic swirling of the Petri™ dish so that the hypocotyl segments remain immersed in the *Agrobacterium* solution. At the end of the treatment period, the *Agrobacterium* solution is pipetted into a waste beaker; autoclaved and discarded (the *Agrobacterium* solution is completely removed to prevent *Agrobacterium* overgrowth). The treated hypocotyls are transferred with forceps back to the original plates containing MSK1D1 media overlaid with filter paper (care is taken to ensure that the segments did not dry). The transformed hypocotyl segments and non-transformed control hypocotyl segments are returned to the Percival™ growth chamber under reduced light intensity (by covering the plates with aluminum foil), and the treated hypocotyl segments are co-cultivated with *Agrobacterium* for 3 days.

*Callus induction on selection medium*: After 3 days of co-cultivation, the hypocotyl segments are individually transferred with forceps onto callus induction medium, MSK1D1H1 (MS, 1 mg/L kinetin, 1 mg/L 2,4-D, 0.5 gm/L MES, 5 mg/L $AgNO_3$, 300 mg/L Timentin™, 200 mg/L carbenicillin, 1 mg/L Herbiace™, 3% sucrose, 0.7% phytagar) with growth regime set at 22-26°C. The hypocotyl segments are anchored on the medium but are not deeply embedded into the medium.

*Selection and shoot regeneration*: After 7 days on callus induction medium, the callusing hypocotyl segments are transferred to Shoot Regeneration Medium 1 with selection, MSB3Z1H1 (MS, 3 mg/L BAP, 1 mg/L zeatin, 0.5 gm/L MES, 5 mg/L $AgNO_3$, 300 mg/L Timentin™, 200 mg/L carbenicillin, 1 mg/L Herbiace™, 3% sucrose, 0.7% phytagar). After 14 days, the hypocotyl segments which develop shoots are transferred to Regeneration Medium 2 with increased selection, MSB3Z1H3 (MS, 3 mg/L BAP, 1 mg/L Zeatin, 0.5 gm/L MES, 5 mg/L $AgNO_3$, 300 mg/l Timentin™, 200 mg/L carbenicillin, 3 mg/L Herbiace™, 3% sucrose, 0.7% phytagar) with growth regime set at 22-26°C.

*Shoot elongation*: After 14 days, the hypocotyl segments that develop shoots are transferred from Regeneration Medium 2 to shoot elongation medium, MSMESH5 (MS, 300 mg/L Timentin™, 5 mg/l Herbiace™, 2% sucrose, 0.7% TC Agar) with growth regime set at 22-26 °C. Shoots that are already elongated were isolated from the hypocotyl segments and transferred to MSMESH5. After 14 days the remaining shoots which have not elongated in the first round of culturing on shoot elongation medium are transferred to fresh shoot elongation medium ,MSMESH5. At this stage all remaining hypocotyl segments which do not produce shoots are discarded.

*Root induction*: After 14 days of culturing on the shoot elongation medium, the isolated shoots are transferred to MSMEST medium (MS, 0.5 g/L MES, 300 mg/L Timentin™, 2% sucrose, 0.7% TC Agar) for root induction at 22-26 °C. Any shoots which do not produce roots after incubation in the first transfer to MSMEST medium are transferred for a second or third round of incubation on MSMEST medium until the shoots develop roots.

*PCR analysis*: Transformed canola hypocotyl segments which regenerated into shoots comprising roots are further analyzed via a PCR molecular confirmation assay. Leaf tissue is obtained from the green shoots and tested via PCR for the presence of the selectable marker gene. Any chlorotic shoots are discarded and not subjected to PCR analysis. Samples that are identified as positive for the presence of the selectable marker gene are kept and cultured on MSMEST medium to continue development and elongation of the shoots and roots. The samples that are identified as not containing the selectable marker gene negative according to PCR analysis are discarded.

The transformed canola plants comprising shoots and roots that are PCR-positive for the presence of the selectable marker gene are transplanted into soil in a greenhouse. After establishment of the canola plants within soil, the canola plants are further analyzed to quantify the copy number of the selectable marker gene expression cassette via an Invader™ quantitative PCR assay and Southern blotting. Transgenic To canola plants which are confirmed to contain at least one copy of the selectable marker gene expression cassette are advanced for further analysis of the seed. The seeds obtained from theses transgenic $T_0$ canola plants, *i.e.*, $T_1$ canola seeds, are analyzed to detect the presences of the target gene.

## Claims

1. An isolated nucleic acid molecule comprising a polynucleotide encoding a hairpin ribonucleic acid (hpRNA) molecule with a stem and loop structure that is capable of inhibiting the expression of an endogenous target gene in a cell of a coleopteran insect upon ingestion, wherein the polynucleotide is operably linked to a heterologous promoter, and wherein the polynucleotide comprises:

    a first nucleotide sequence encoding a polyribonucleotide of the hpRNA molecule, wherein the first nucleotide sequence is selected from the group consisting of at least 50 contiguous nucleotides of SEQ ID NO:84, the complement of at least 50 contiguous nucleotides of SEQ ID NO:84, at least 50 contiguous nucleotides of SEQ ID NO:86, the complement of at least 50 contiguous nucleotides of SEQ ID NO:86, at least 50 contiguous nucleotides of SEQ ID NO:88, the complement of at least 50 contiguous nucleotides of SEQ ID NO:88, at least 50 contiguous nucleotides of SEQ ID NO:93 and the complement of at least 50 contiguous nucleotides of SEQ ID NO:93;
    a second nucleotide sequence encoding a polyribonucleotide of the hpRNA molecule; and
    a third nucleotide sequence encoding a polyribonucleotide of the hpRNA molecule, wherein the third nucleotide sequence is substantially the reverse complement of the first nucleotide sequence, such that the polyribonucleotides encoded by the first and the third nucleotide sequences hybridize in a stem structure of the hpRNA molecule,
    wherein a loop structure of the hpRNA molecule comprises the polyribonucleotide encoded by the second nucleotide sequence.

2. The nucleic acid molecule of claim 1, wherein
the first nucleotide sequence is SEQ ID NO:90 or the complement of SEQ ID NO: 90.

3. The nucleic acid molecule of claim 1 or claim 2, wherein the heterologous promoter is functional in a plant cell.

4. A hairpin ribonucleic acid (hpRNA) molecule encoded by the polynucleotide of the nucleic acid molecule of claim 1 or claim 2 that is capable of inhibiting the expression of an endogenous target gene in a cell of a coleopteran insect upon ingestion, the hpRNA molecule comprising:

    the first polyribonucleotide encoded by the first nucleotide sequence;
    the second polyribonucleotide encoded by the second nucleotide sequence; and
    the third polyribonucleotide encoded by the third nucleotide sequence,
    wherein the first and third polyribonucleotides are hybridized in a stem structure of the hpRNA molecule.

5. A transgenic cell comprising the nucleic acid molecule of claim 1 or claim 2, wherein the cell is a prokaryotic cell or a eukaryotic cell.

6. A transgenic plant cell comprising the nucleic acid molecule of claim 3.

7. The transgenic plant cell of claim 6, wherein the cell further comprises a polynucleotide encoding an insecticidal polypeptide from *Bacillus thuringiensis* selected from the group consisting of Cry1B, Cry1I, Cry2A, Cry3, Cry7A, Cry8, Cry9D, Cry14, Cry18, Cry22, Cry23, Cry34, Cry35, Cry36, Cry37, Cry43, Cry55, Cyt1A, and Cyt2C.

8. A transgenic plant comprising the nucleic acid molecule of claim 3.

9. The transgenic plant of claim 8, wherein the plant further comprises a polynucleotide encoding an insecticidal polypeptide from *Bacillus thuringiensis* selected from the group consisting of Cry1B, Cry1I, Cry2A, Cry3, Cry7A, Cry8, Cry9D, Cry14, Cry18, Cry22, Cry23, Cry34, Cry35, Cry36, Cry37, Cry43, Cry55, Cyt1A, and Cyt2C.

10. A transgenic plant seed comprising the nucleic acid molecule of claim 3.

11. The nucleic acid molecule of claim 1, further comprising a polynucleotide encoding an insecticidal polypeptide from *Bacillus thuringiensis* selected from the group consisting of Cry1B, Cry1I, Cry2A, Cry3, Cry7A, Cry8, Cry9D, Cry14, Cry18, Cry22, Cry23, Cry34, Cry35, Cry36, Cry37, Cry43, Cry55, Cyt1A, and Cyt2C.

12. A method for controlling a coleopteran insect population, the method comprising feeding insects of the population with an agent comprising the hpRNA molecule of claim 4.

13. The method according to claim 12, wherein the agent is an RNA bait comprising the hpRNA molecule, or a spray-on product comprising the hpRNA molecule.

14. The method according to claim 12, wherein the agent comprises a transgenic plant cell that expresses the hpRNA molecule from a polynucleotide in the plant cell.

15. The method according to claim 12, wherein the agent is a transgenic plant or transgenic plant material that expresses the hpRNA molecule from a polynucleotide in the plant or plant material.

16. A method for producing a transgenic plant cell, the method comprising:

transforming a plant cell with the nucleic acid molecule of claim 3, wherein the molecule is a plant transformation vector;
culturing the transformed plant cell under conditions sufficient to allow for development of a plant cell culture comprising a plurality of transformed plant cells;
selecting for transformed plant cells that have integrated the polynucleotide into their genomes;
screening the transformed plant cells for expression of the hpRNA molecule encoded by the polynucleotide; and
selecting a plant cell that expresses the hpRNA molecule.

17. A method for producing a coleopteran pest-resistant transgenic plant, the method comprising:
regenerating a transgenic plant from the transgenic plant cell of claim 6 or claim 7, wherein expression of the RNA molecule encoded by the polynucleotide is sufficient to reduce the expression of a target gene in a coleopteran pest that ingests a cell of the transgenic plant.

18. The method according to claim 16, wherein the transformed plant cell further comprises an insecticidal polynucleotide encoding a polypeptide from *Bacillus thuringiensis* selected from the group consisting of Cry1B, Cry1I, Cry2A, Cry3, Cry7A, Cry8, Cry9D, Cry14, Cry18, Cry22, Cry23, Cry34, Cry35, Cry36, Cry37, Cry43, Cry55, Cyt1A, and Cyt2C.

19. The method according to claim 12, wherein the method further comprises feeding the insects of the population with an insecticidal polypeptide from *Bacillus thuringiensis.*

**Patentansprüche**

1. Ein isoliertes Nukleinsäuremolekül, umfassend ein Polynukleotid, das für ein Haarnadel-Ribonukleinsäure (hpRNA)-Molekül mit einer Stamm- und Schleifenstruktur kodiert, das in der Lage ist, die Expression eines endogenen Zielgens in einer Zelle eines Coleoptera-Insekts nach Aufnahme zu inhibieren, wobei das Polynukleotid funktionsfähig mit einem heterologen Promotor verbunden ist und wobei das Polynukleotid umfasst:

eine erste Nukleotidsequenz, die für ein Polyribonukleotid des hpRNA-Moleküls kodiert, wobei die erste Nukleotidsequenz ausgewählt ist aus der Gruppe bestehend aus mindestens 50 zusammenhängenden Nukleotiden der SEQ ID NO: 84, dem Komplement von mindestens 50 zusammenhängenden Nukleotiden der SEQ ID NO: 84, mindestens 50 zusammenhängenden Nukleotiden der SEQ ID NO: 86, dem Komplement von mindestens 50 zusammenhängenden Nukleotiden der SEQ ID NO: 86, mindestens 50 zusammenhängenden Nukleotiden der SEQ ID NO:88, dem Komplement von mindestens 50 aufeinanderfolgenden Nukleotiden der SEQ ID NO:88, mindestens 50 aufeinanderfolgenden Nukleotiden der SEQ ID NO:93 und dem Komplement von mindestens 50 aufeinanderfolgenden Nukleotiden der SEQ ID NO:93;
eine zweite Nukleotidsequenz, die für ein Polyribonukleotid des hpRNA-Moleküls kodiert; und eine dritte Nukleotidsequenz, die für ein Polyribonukleotid des hpRNA-Moleküls kodiert, wobei die dritte Nukleotidsequenz im Wesentlichen das reverse Komplement der ersten Nukleotidsequenz ist, so dass die von der ersten und der dritten Nukleotidsequenz kodierten Polyribonukleotide in einer Stammstruktur des hpRNA-Moleküls hybridisieren,
wobei eine Schleifenstruktur des hpRNA-Moleküls das von der zweiten Nukleotidsequenz kodierte Polyribonukleotid umfasst.

2. Das Nukleinsäuremolekül nach Anspruch 1, wobei
die erste Nukleotidsequenz SEQ ID NO: 90 oder das Komplement von SEQ ID NO: 90 ist.

3. Das Nukleinsäuremolekül nach Anspruch 1 oder 2, wobei der heterologe Promotor in einer Pflanzenzelle funktionell ist.

4. Ein Haarnadel-Ribonukleinsäure-(hpRNA)-Molekül, das von dem Polynukleotid des Nukleinsäuremoleküls nach Anspruch 1 oder 2 kodiert wird und das in der Lage ist, die Expression eines endogenen Zielgens in einer Zelle eines Coleoptera-Insekts nach der Aufnahme zu inhibieren, wobei das hpRNA-Molekül umfasst:

das erste Polyribonukleotid, das von der ersten Nukleotidsequenz kodiert wird;
das zweite Polyribonukleotid, das von der zweiten Nukleotidsequenz kodiert wird; und
das dritte Polyribonukleotid, das von der dritten Nukleotidsequenz kodiert wird,
wobei das erste und dritte Polyribonukleotid in einer Stammstruktur des hpRNA-Moleküls hybridisiert sind.

5. Eine transgene Zelle, umfassend das Nukleinsäuremolekül nach Anspruch 1 oder Anspruch 2, wobei die Zelle eine prokaryotische Zelle oder eine eukaryotische Zelle ist.

6. Eine transgene Pflanzenzelle, umfassend das Nukleinsäuremolekül nach Anspruch 3.

7. Die transgene Pflanzenzelle nach Anspruch 6, wobei die Zelle ferner ein Polynukleotid umfasst, das für ein insektizides Polypeptid aus *Bacillus thuringiensis* kodiert, ausgewählt aus der Gruppe bestehend aus Cry1B, Cry1I, Cry2A, Cry3, Cry7A, Cry8, Cry9D, Cry14, Cry18, Cry22, Cry23, Cry34, Cry35, Cry36, Cry37, Cry43, Cry55, Cyt1A und Cyt2C.

8. Eine transgene Pflanze, die das Nukleinsäuremolekül nach Anspruch 3 umfasst.

9. Die transgene Pflanze nach Anspruch 8, wobei die Pflanze weiterhin ein Polynukleotid umfasst, das für ein insektizides Polypeptid aus *Bacillus thuringiensis* kodiert, ausgewählt aus der Gruppe bestehend aus Cry1B, Cry1I, Cry2A, Cry3, Cry7A, Cry8, Cry9D, Cry14, Cry18, Cry22, Cry23, Cry34, Cry35, Cry36, Cry37, Cry43, Cry55, Cyt1A und Cyt2C.

10. Ein transgener Pflanzensamen, umfassend das Nukleinsäuremolekül nach Anspruch 3.

11. Das Nukleinsäuremolekül nach Anspruch 1, ferner umfassend ein Polynukleotid, das für ein insektizides Polypeptid aus *Bacillus thuringiensis* kodiert, ausgewählt aus der Gruppe bestehend aus Cry1B, Cry1I, Cry2A, Cry3, Cry7A, Cry8, Cry9D, Cry14, Cry18, Cry22, Cry23, Cry34, Cry35, Cry36, Cry37, Cry43, Cry55, Cyt1A und Cyt2C.

12. Ein Verfahren zur Bekämpfung einer Coleoptera-Insektenpopulation, wobei das Verfahren umfasst
Fütterung von Insekten der Population mit einem Mittel, das das hpRNA-Molekül nach Anspruch 4 umfasst.

13. Das Verfahren nach Anspruch 12, wobei das Mittel ein RNA-Köder, umfassend das hpRNA-Molekül, oder ein Aufsprühprodukt, umfassend das hpRNA-Molekül, ist.

14. Das Verfahren nach Anspruch 12, wobei das Mittel eine transgene Pflanzenzelle umfasst, die das hpRNA-Molekül aus einem Polynukleotid in der Pflanzenzelle exprimiert.

15. Das Verfahren nach Anspruch 12, wobei der Wirkstoff eine transgene Pflanze oder ein transgenes Pflanzenmaterial ist, die bzw. das das hpRNA-Molekül aus einem Polynukleotid in der Pflanze oder dem Pflanzenmaterial exprimiert.

16. Ein Verfahren zur Herstellung einer transgenen Pflanzenzelle, wobei das Verfahren umfasst:

das Transformieren einer Pflanzenzelle mit dem Nukleinsäuremolekül nach Anspruch 3, wobei das Molekül ein Pflanzentransformationsvektor ist;
Kultivieren der transformierten Pflanzenzelle unter Bedingungen, die ausreichen, um die Entwicklung einer Pflanzenzellkultur zu ermöglichen, die eine Vielzahl von transformierten Pflanzenzellen umfasst;
Selektion auf transformierte Pflanzenzellen, die das Polynukleotid in ihr Genom integriert haben;
Screenen der transformierten Pflanzenzellen auf Expression des durch das Polynukleotid kodierten hpRNA-Moleküls; und
Auswahl einer Pflanzenzelle, die das hpRNA-Molekül exprimiert.

17. Ein Verfahren zur Herstellung einer gegen Coleoptera-Schädlinge resistenten transgenen Pflanze, wobei das Verfahren umfasst:
die Regenerierung einer transgenen Pflanze aus der transgenen Pflanzenzelle nach Anspruch 6 oder 7, wobei die Expression des durch das Polynukleotid kodierten RNA-Moleküls ausreicht, um die Expression eines Zielgens in einem Coleoptera-Schädling, der eine Zelle der transgenen Pflanze aufnimmt, zu reduzieren.

18. Das Verfahren nach Anspruch 16, wobei die transformierte Pflanzenzelle weiterhin ein insektizides Polynukleotid umfasst, das für ein Polypeptid aus *Bacillus thuringiensis* kodiert, ausgewählt aus der Gruppe bestehend aus Cry1B, Cry1I, Cry2A, Cry3, Cry7A, Cry8, Cry9D, Cry14, Cry18, Cry22, Cry23, Cry34, Cry35, Cry36, Cry37, Cry43, Cry55, Cyt1A und Cyt2C.

19. Das Verfahren nach Anspruch 12, wobei das Verfahren ferner das Füttern der Insekten der Population mit einem insektiziden Polypeptid aus *Bacillus thuringiensis* umfasst.

## Revendications

1. Molécule d’acide nucléique isolé, qui comprend un polynucléotide codant une molécule d’acide ribonucléique en épingle à cheveux (ARNhp) doté d’une structure à tige et boucle, qui est capable d’inhiber l’expression d’un gène-cible endogène au sein d’une cellule d’un insecte coléoptère après ingestion par celui-ci, lequel polynucléotide est opérationnellement raccordé à un promoteur hétérologue et lequel polynucléotide comprend :

- une première séquence de nucléotides codant un polyribonucléotide de la molécule d’ARNhp, laquelle première séquence de nucléotides est choisie dans l’ensemble constitué par les suivantes : au moins 50 nucléotides contigus de SEQ ID NO : 84, la complémentaire d’au moins 50 nucléotides contigus de SEQ ID NO : 84, au moins 50 nucléotides contigus de SEQ ID NO : 86, la complémentaire d’au moins 50 nucléotides contigus de SEQ ID NO : 86, au moins 50 nucléotides contigus de SEQ ID NO : 88, la complémentaire d’au moins 50 nucléotides contigus de SEQ ID NO : 88, au moins 50 nucléotides contigus de SEQ ID NO : 93, et la complémentaire d’au moins 50 nucléotides contigus de SEQ ID NO : 93,
- une deuxième séquence de nucléotides codant un polyribonucléotide de la molécule d’ARNhp,
- et une troisième séquence de nucléotides codant un polyribonucléotide de la molécule d’ARNhp,

étant entendu

- que la troisième séquence de nucléotides est pratiquement l’inverse de la complémentaire de la première séquence de nucléotides, de sorte que les polyribonucléotides codés par la première séquence de nucléotides et la troisième séquence de nucléotides s’hybrident pour former la structure « tige » de la molécule d’ARNhp,
- et que la structure « boucle » de la molécule d’ARNhp comprend le polyribonucléotide codé par la deuxième séquence de nucléotides.

**2.** Molécule d'acide nucléique conforme à la revendication 1, dans laquelle la première séquence de nucléotides est SEQ ID NO : 90 ou la complémentaire de SEQ ID NO : 90.

**3.** Molécule d'acide nucléique conforme à la revendication 1 ou 2, dans laquelle le promoteur hétérologue peut fonctionner au sein d'une cellule végétale.

**4.** Molécule d'acide ribonucléique en épingle à cheveux (ARNhp) codé par le polynucléotide d'une molécule d'acide nucléique conforme à la revendication 1 ou 2, qui est capable d'inhiber l'expression d'un gène-cible endogène au sein d'une cellule d'un insecte coléoptère après ingestion par celui-ci, laquelle molécule d'ARNhp comprend :

- un premier polyribonucléotide codé par la première séquence de nucléotides,
- un deuxième polyribonucléotide codé par la deuxième séquence de nucléotides,
- et un troisième polyribonucléotide codé par la troisème séquence de nucléotides,

étant entendu que le premier polyribonucléotide et le troisième sont hybridés dans la structure « tige » de la molécule d'ARNhp.

**5.** Cellule transgénique comprenant une molécule d'acide nucléique conforme à la revendication 1 ou 2, laquelle cellule est une cellule procaryote ou une cellule eucaryote.

**6.** Cellule végétale transgénique comprenant une molécule d'acide nucléique conforme à la revendication 3.

**7.** Cellule végétale transgénique conforme à la revendication 6, laquelle cellule comprend en outre un polynucléotide qui code un polypeptide insecticide provenant de *Bacillus thuringiensis,* choisi dans l'ensemble formé par les suivants : Cry1B, Cry1I, Cry2A, Cry3, Cry7A, Cry8, Cry9D, Cry14, Cry18, Cry22, Cry23, Cry34, Cry35, Cry36, Cry37, Cry43, Cry55, Cyt1A, et Cyt2C.

**8.** Végétal transgénique comprenant une molécule d'acide nucléique conforme à la revendication 3.

**9.** Végétal transgénique conforme à la revendication 8, lequel végétal comprend en outre un polynucléotide qui code un polypeptide insecticide provenant de *Bacillus thuringiensis*, choisi dans l'ensemble formé par les suivants : Cry1B, Cry1I, Cry2A, Cry3, Cry7A, Cry8, Cry9D, Cryl4, Cryl8, Cry22, Cry23, Cry34, Cry35, Cry36, Cry37, Cry43, Cry55, Cyt1A, et Cyt2C.

**10.** Semence de végétal transgénique comprenant une molécule d'acide nucléique conforme à la revendication 3.

**11.** Molécule d'acide nucléique conforme à la revendication 1, comprenant en outre un polynucléotide qui code un polypeptide insecticide provenant de *Bacillus thuringiensis*, choisi dans l'ensemble formé par les suivants : CrylB, Cry1I, Cry2A, Cry3, Cry7A, Cry8, Cry9D, Cry14, Cryl8, Cry22, Cry23, Cry34, Cry35, Cry36, Cry37, Cry43, Cry55, Cyt1A, et Cyt2C.

**12.** Procédé de lutte contre une population d'insectes coléoptères, lequel procédé comporte le fait de fournir comme aliment aux insectes de la population un agent comprenant la molécule d'ARNhp conforme à la revendication 4.

**13.** Procédé conforme à la revendication 12, dans lequel l'agent est un appât à ARN comprenant la molécule d'ARNhp ou un produit à pulvériser comprenant la molécule d'ARNhp.

**14.** Procédé conforme à la revendication 12, dans lequel l'agent comprend une cellule végétale transgénique qui exprime la molécule d'ARNhp, à partir d'un polynucléotide présent dans la cellule végétale.

**15.** Procédé conforme à la revendication 12, dans lequel l'agent est un végétal transgénique ou un matériau végétal transgénique qui exprime la molécule d'ARNhp, à partir d'un polynucléotide présent dans le végétal ou le matériau végétal.

**16.** Procédé de production d'une cellule végétale transgénique, lequel procédé comporte les étapes suivantes :

- transformer une cellule végétale avec la molécule d'acide nucléique conforme à la revendication 3, laquelle molécule est un vecteur pour transformation de végétal,

- cultiver la cellule végétale transformée, dans des conditions suffisantes pour permettre le développement d'une culture de cellules végétales comprenant de multiples cellules végétales transformées,
- sélectionner pour les cellules végétales transformées qui ont intégré le polynucléotide dans leur génome,
- passer au crible les cellules végétales transformées pour y rechercher l'expression de la molécule d'ARNhp codée par le polynucléotide,
- et sélectionner une cellule végétale qui exprime la molécule d'ARNhp.

**17.** Procédé de production d'un végétal transgénique résistant à un coléoptère nuisible, lequel procédé comporte l'étape suivante :

- régénérer un végétal transgénique à partir d'une cellule végétale transgénique conforme à la revendication 6 ou 7,

étant entendu que l'expression de la molécule d'ARN codée par le polynucléotide suffit à réduire l'expression d'un gène cible chez un coléoptère nuisible qui ingère une cellule du végétal transgénique.

**18.** Procédé conforme à la revendication 16, dans lequel la cellule végétale transformée comprend en outre un polynucléotide insecticide qui code un polypeptide provenant de *Bacillus thuringiensis,* choisi dans l'ensemble formé par les suivants : CrylB, Cry1I, Cry2A, Cry3, Cry7A, Cry8, Cry9D, Cry14, Cry18, Cry22, Cry23, Cry34, Cry35, Cry36, Cry37, Cry43, Cry55, Cyt1A, et Cyt2C.

**19.** Procédé conforme à la revendication 12, lequel procédé comporte en outre le fait de fournir comme aliment aux insectes de la population un polypeptide insecticide provenant de *Bacillus thuringiensis.*

**FIG. 1.** Generation of dsRNA from a single transcription template with a single pair of primers

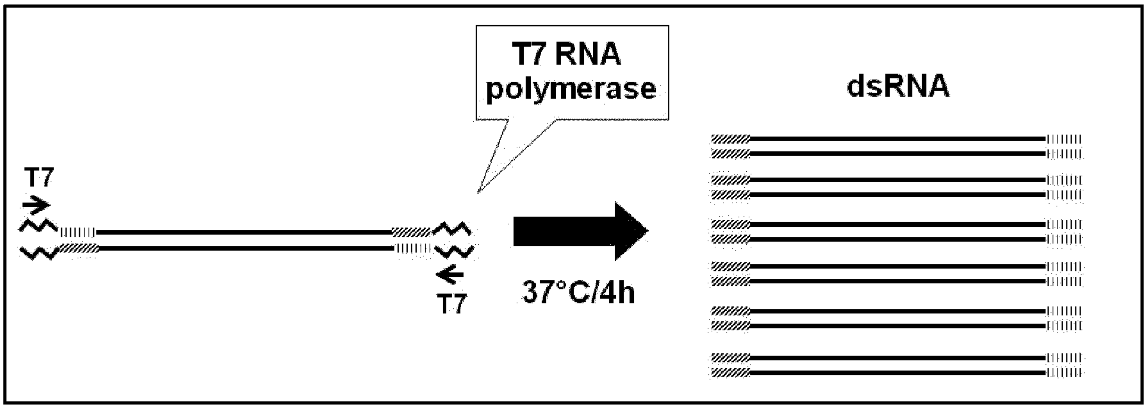

**FIG. 2.** Generation of dsRNA from two transcription templates.

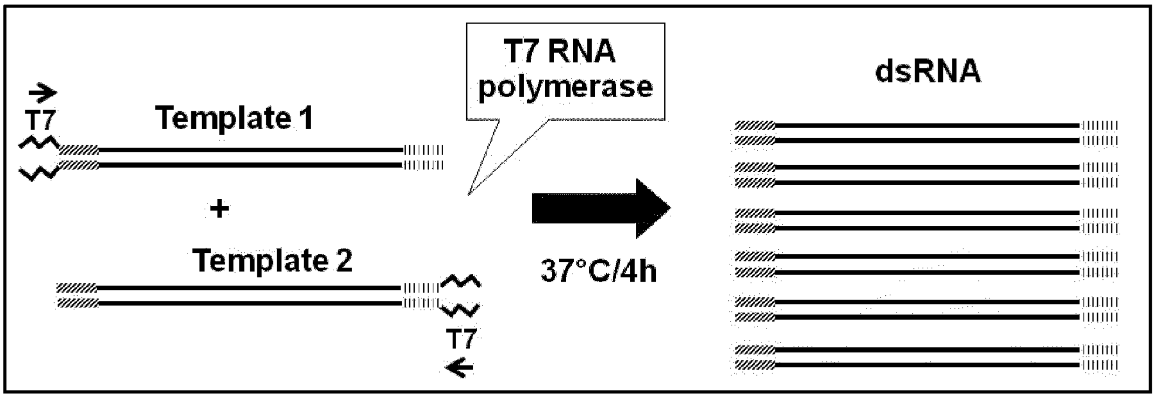

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 7612194 B
- US 20070050860 A
- US 20100192265 A
- US 20110154545 A
- US 7943819 B
- US 2013040173
- WO 2013169923 A
- WO 2012092544 A2
- US 577811
- US 577854
- US 705807
- US 62063199
- US 62063203
- US 62063192
- US 62063216
- US 8530440 B
- WO 9630530 A
- US 4980460 A
- US 4725677 A
- US 4415732 A
- US 4458066 A
- US 4973679 A
- WO 9732016 A
- US 5593874 A
- US 5698425 A
- US 5712135 A
- US 5789214 A
- US 5804693 A
- WO 06073727 A
- US 20060200878 A1
- US 20020048814 A
- US 20030018993 A
- WO 9401550 A
- WO 9805770 A
- US 6437217 B
- US 5641876 A
- US 6426446 B
- US 6429362 B
- US 6232526 B
- US 6177611 B
- US 5322938 A
- US 5352605 A
- US 5359142 A
- US 5530196 A
- US 6433252 B
- US 6429357 B
- US 6294714 B
- US 6140078 A
- US 6252138 B
- US 6175060 B
- US 6388170 B
- US 6635806 B
- US 2009757089 A
- US 6051753 A
- US 5378619 A
- US 5850019 A
- US 6677503 B
- US 5110732 A
- US 5459252 A
- US 5837848 A
- US 5362865 A
- US 5659122 A
- US 5550318 A
- US 5633435 A
- US 5780708 A
- US 6118047 A
- US 5508184 A
- US 5384253 A
- US 5302523 A
- US 5464765 A
- US 5563055 A
- US 5591616 A
- US 5693512 A
- US 5824877 A
- US 5981840 A
- US 6384301 B
- US 5015580 A
- US 5538880 A
- US 6160208 A
- US 6399861 B
- US 6403865 B
- US 7060876 B
- WO 9506722 A
- US 4536475 A
- US 4693977 A
- US 4886937 A
- US 5501967 A
- EP 0122791 A
- EP 0120516 A
- US 20120174258
- US 20120174259
- US 20120174260
- US 20120198586
- US 20130091600
- US 20130091601
- US 20130097730
- US 5107065 A

- US 5759829 A
- US 5283184 A
- US 5231020 A
- US 5510474 A
- US 6699984 B
- US 7838733 B
- US 7179902 B
- US 20070124836 A
- US 8304604 B
- WO 2012016222 A2

**Non-patent literature cited in the description**


- **ELLSBURY et al.** *Environ. Entomol.,* 2005, vol. 34, 627-34
- **FIRE et al.** *Nature,* 1998, vol. 391, 806-11
- **MARTINEZ et al.** *Cell,* 2002, vol. 110, 563-74
- **MCMANUS ; SHARP.** *Nature Rev. Genetics,* 2002, vol. 3, 737-47
- **BOLOGNESI et al.** *PLoS ONE,* 2012, vol. 7 (10), e47534
- **BAUM et al.** *Nature Biotechnology,* 2007, vol. 25, 1322-1326
- **BAUM et al.** *Nat. Biotechnol.,* 2007, vol. 25 (11), 1322-6
- **SMITH ; WATERMAN.** *Adv. Appl. Math.,* 1981, vol. 2, 482
- **NEEDLEMAN ; WUNSCH.** *J. Mol. Biol.,* 1970, vol. 48, 443
- **PEARSON ; LIPMAN.** *Proc. Natl. Acad. Sci. U.S.A.,* 1988, vol. 85, 2444
- **HIGGINS ; SHARP.** *Gene,* 1988, vol. 73, 237-44
- **HIGGINS ; SHARP.** *CABIOS,* 1989, vol. 5, 151-3
- **CORPET et al.** *Nucleic Acids Res.,* 1988, vol. 16, 10881-90
- **HUANG et al.** *Comp. Appl. Biosci.,* 1992, vol. 8, 155-65
- **PEARSON et al.** *Methods Mol. Biol.,* 1994, vol. 24, 307-31
- **TATIANA et al.** *FEMS Microbiol. Lett.,* 1999, vol. 174, 247-50
- **ALTSCHUL et al.** *J. Mol. Biol.,* 1990, vol. 215, 403-10
- **SAMBROOK et al.** Molecular Cloning: A Laboratory Manual. Cold Spring Harbor Laboratory Press, 1989, vol. 1-3
- Nucleic Acid Hybridization. IRL Press, 1985
- Overview of principles of hybridization and the strategy of nucleic acid probe assays. **TIJSSEN.** Laboratory Techniques in Biochemistry and Molecular Biology-Hybridization with Nucleic Acid Probes. Elsevier, 1993
- Current Protocols in Molecular Biology. Greene Publishing and Wiley-Interscience, 1995
- **WARD et al.** *Plant Mol. Biol.,* 1993, vol. 22, 361-366
- **SCHENA et al.** *Proc. Natl. Acad. Sci. USA,* 1991, vol. 88, 0421
- **FROMM et al.** *Nature,* 1986, vol. 319, 791-3
- **FELGNER et al.** *Proc. Natl. Acad. Sci. USA,* 1987, vol. 84, 7413-7
- **MUELLER et al.** *Cell,* 1978, vol. 15, 579-85
- **FRALEY et al.** *Proc. Natl. Acad. Sci. USA,* 1983, vol. 80, 4803-7
- **KLEIN et al.** *Nature,* 1987, vol. 327, 70
- **LEWIN'S.** Genes X. Jones & Bartlett Publishers, 2009
- The Encyclopedia of Molecular Biology. Blackwell Science Ltd, 1994
- Molecular Biology and Biotechnology: A Comprehensive Desk Reference. VCH Publishers, Inc, 1995
- **ELBASHIR et al.** *Nature,* 2001, vol. 411, 494-8
- **HAMILTON ; BAULCOMBE.** *Science,* 1999, vol. 286 (5441), 950-2
- **OZAKI et al.** *Nucleic Acids Research,* 1992, vol. 20, 5205-5214
- **AGRAWAL et al.** *Nucleic Acids Research,* 1990, vol. 18, 5419-5423
- **BEAUCAGE et al.** *Tetrahedron,* 1992, vol. 48, 2223-2311
- **EBERT et al.** *Proc. Natl. Acad. Sci. USA,* 1987, vol. 84 (16), 5745-9
- **LAWTON et al.** *Plant Mol. Biol.,* 1987, vol. 9, 315-24
- **ODELL et al.** *Nature,* 1985, vol. 313, 810-2
- **WALKER et al.** *Proc. Natl. Acad. Sci. USA,* 1987, vol. 84 (19), 6624-8
- **YANG ; RUSSELL.** *Proc. Natl. Acad. Sci. USA,* 1990, vol. 87, 4144-8
- **CHANDLER et al.** *Plant Cell,* 1989, vol. 1, 1175-83
- **DEPICKER et al.** *J. Mol. Appl. Genet.,* 1982, vol. 1, 561-73
- **BEVAN et al.** *Nature,* 1983, vol. 304, 184-7
- **OPPERMAN et al.** *Science,* 1994, vol. 263, 221-3
- **HIREL et al.** *Plant Mol. Biol.,* 1992, vol. 20, 207-18
- **TURNER ; FOSTER.** *Molecular Biotech.,* 1995, vol. 3 (3), 225-36
- **CARRINGTON ; FREED.** *J. Virol.,* 1990, vol. 64, 1590-7
- **INGELBRECHT et al.** *Plant Cell,* 1989, vol. 1, 671-80
- **CORUZZI et al.** *EMBO J.,* 1984, vol. 3, 1671-9

- **JEFFERSON et al.** *Plant Mol. Biol. Rep.,* 1987, vol. 5, 387-405
- Molecular cloning of the maize R-nj allele by transposon tagging with Ac. **DELLAPORTA et al.** 18th Stadler Genetics Symposium. Plenum, 1988, 263-82
- **SUTCLIFFE et al.** *Proc. Natl. Acad. Sci. USA,* 1978, vol. 75, 3737-41
- **OW et al.** *Science,* 1986, vol. 234, 856-9
- **ZUKOWSKI et al.** *Gene,* 1983, vol. 46 (2-3), 247-55
- **IKATU et al.** *Bio/Technol.,* 1990, vol. 8, 241-2
- **KATZ et al.** *J. Gen. Microbiol.,* 1983, vol. 129, 2703-14
- **POTRYKUS et al.** *Mol. Gen. Genet.,* 1985, vol. 199, 183-8
- **HERRERA-ESTRELLA et al.** *Nature,* 1983, vol. 303, 209-13
- **KLEE et al.** *Bio/Technol.,* 1985, vol. 3, 637-42
- **RIOS, G. et al.** *Plant J.,* 2002, vol. 32, 243-53
- **SHAGIN et al.** *Mol. Biol. Evol.,* 2004, vol. 21 (5), 841-50
- **WRIGHT et al.** *Proc. Natl. Acad. Sci. U.S.A.,* 2010, vol. 107, 20240-5
- **SCHENK et al.** *Plant Mol. Biol.,* 1999, vol. 39, 1221-30
- **WATSON et al.** *J. Bacteriol.,* 1975, vol. 123, 255-64
- IN Plant Embryo Culture Methods and Protocols: Methods in Molecular Biology. **FRAME et al.** Genetic Transformation Using Maize Immature Zygotic Embryos. Business Media, LLC, 2011, 327-341
- **FRAME et al.** *GENETIC TRANSFORMATION USING MAIZE IMMATURE ZEGOTIC EMBRYOS*
- **HALLAUER et al.** *Crop Science,* 1997, vol. 37, 1405-1406
- **BAUM et al.** *Nat. Biotechnol.,* 2007, vol. 25 (11), 1322-1326
- **OLESON et al.** *J. Econ. Entomol.,* 2005, vol. 98, 1-8
- **PETOLINO ; ARNOLD.** *Methods Mol. Biol.,* 2009, vol. 526, 59-67
- **M.H. SCHULZ et al.** *Bioinformatics,* 2012, vol. 28, 1086-92
- **ZERBINO ; E. BIRNEY.** *Genome Research.,* 2008, vol. 18, 821-9
- **BONFIELD JK ; WHITWHAM.** *Bioinformatics,* 2010, vol. 26, 1699-1703

SEQUENCE LISTING

<110> Dow AgroSciences LLC

<120> NUCAMPHOLIN NUCLEIC ACID MOLECULES TO CONTROL COLEOPTERAN INSECT PESTS

<130> 971-1

<160> 99

<170> PatentIn version 3.5

<210> 1
<211> 1047
<212> DNA
<213> Diabrotica virgifera

<400> 1

atgccagata ccaaggatgc caaggatacc aaggatgcta atttgagttc tcctgaacgt 60
aaaagacgaa gaaagagtag atctaaatct ccagaacgaa aagagaaaaa gtcttccaaa 120
aagaaagccc acaatagtag agacagagat tcatcagagg aaggttacaa ccctaaagat 180
tatcagagat actatgggga agatcgccca aacagtgaca aatattggaa taaatatcca 240
aggaaagata ctaccaaagt tggccaaaga tactatgatg cggctcccga agaatctggc 300
aagaaggggc cagatagaaa ttcagaggag aaggagttac caaagccaat ggagtctgtt 360
cctgataaat cagtcatcaa accaagagaa agaaaaactg tagatatgtt aacatcgagg 420
actggtggtg cttatattcc cccagctaag ctacgattgt tacaagccag tattacagac 480
aaaacatcag cagcctatca gcgtatagca tgggaagcct taaagaaatc cgttcatggt 540
tacattaata aaattaacac ctcgaatatt ggcatcatcg ccagagaatt attgcatgaa 600
aatatagtaa gaggtagagg tttgctgtgc aagtcaataa tacaagcaca agcagcatct 660
cctactttta caaacgttta cgcagcctta gtagctgtta ttaattcgaa gtttccaagt 720
ataggagagc ttttattgaa gaggttggtt ttgcagttca aaagagggtt taaacaaaat 780
aataagtcta tttgcatatc ggctactact ttcgtagctc atttagtaaa tcagagagtg 840
gcacatgaaa ttttggcttt ggagatactt acattgttgg tggagactcc tacagatgat 900
tctgtggagg tggccatttc atttttgaag gaatgtggac aaaaactgac agaagtttca 960
agtagaggta ttactgctat atttgagatg ttaagaaaca ttttacatga aggccagcta 1020
gaaaaaaaga attcagtaca tgattga 1047

<210> 2
<211> 348
<212> PRT
<213> Diabrotica virgifera

<400> 2

```
Met Pro Asp Thr Lys Asp Ala Lys Asp Thr Lys Asp Ala Asn Leu Ser
1               5                   10                  15

Ser Pro Glu Arg Lys Arg Arg Arg Lys Ser Arg Ser Lys Ser Pro Glu
            20                  25                  30

Arg Lys Glu Lys Lys Ser Ser Lys Lys Lys Ala His Asn Ser Arg Asp
        35                  40                  45

Arg Asp Ser Ser Glu Glu Gly Tyr Asn Pro Lys Asp Tyr Gln Arg Tyr
    50                  55                  60

Tyr Gly Glu Asp Arg Pro Asn Ser Asp Lys Tyr Trp Asn Lys Tyr Pro
65                  70                  75                  80

Arg Lys Asp Thr Thr Lys Val Gly Gln Arg Tyr Tyr Asp Ala Ala Pro
                85                  90                  95

Glu Glu Ser Gly Lys Lys Gly Pro Asp Arg Asn Ser Glu Glu Lys Glu
            100                 105                 110

Leu Pro Lys Pro Met Glu Ser Val Pro Asp Lys Ser Val Ile Lys Pro
        115                 120                 125

Arg Glu Arg Lys Thr Val Asp Met Leu Thr Ser Arg Thr Gly Gly Ala
    130                 135                 140

Tyr Ile Pro Pro Ala Lys Leu Arg Leu Leu Gln Ala Ser Ile Thr Asp
145                 150                 155                 160

Lys Thr Ser Ala Ala Tyr Gln Arg Ile Ala Trp Glu Ala Leu Lys Lys
                165                 170                 175

Ser Val His Gly Tyr Ile Asn Lys Ile Asn Thr Ser Asn Ile Gly Ile
            180                 185                 190

Ile Ala Arg Glu Leu Leu His Glu Asn Ile Val Arg Gly Arg Gly Leu
        195                 200                 205

Leu Cys Lys Ser Ile Ile Gln Ala Gln Ala Ala Ser Pro Thr Phe Thr
    210                 215                 220

Asn Val Tyr Ala Ala Leu Val Ala Val Ile Asn Ser Lys Phe Pro Ser
225                 230                 235                 240

Ile Gly Glu Leu Leu Leu Lys Arg Leu Val Leu Gln Phe Lys Arg Gly
                245                 250                 255
```

```
Phe Lys Gln Asn Asn Lys Ser Ile Cys Ile Ser Ala Thr Thr Phe Val
            260                 265                 270

Ala His Leu Val Asn Gln Arg Val Ala His Glu Ile Leu Ala Leu Glu
        275                 280                 285

Ile Leu Thr Leu Leu Val Glu Thr Pro Thr Asp Asp Ser Val Glu Val
    290                 295                 300

Ala Ile Ser Phe Leu Lys Glu Cys Gly Gln Lys Leu Thr Glu Val Ser
305                 310                 315                 320

Ser Arg Gly Ile Thr Ala Ile Phe Glu Met Leu Arg Asn Ile Leu His
                325                 330                 335

Glu Gly Gln Leu Glu Lys Lys Asn Ser Val His Asp
            340                 345
```

<210> 3
<211> 411
<212> DNA
<213> Diabrotica virgifera

<400> 3

```
gatgcggctc ccgaagaatc tggcaagaag gggccagata gaaattcaga ggagaaggag      60
ttaccaaagc caatggagtc tgttcctgat aaatcagtca tcaaaccaag agaaagaaaa     120
actgtagata tgttaacatc gaggactggt ggtgcttata ttccccccagc taagctacga     180
ttgttacaag ccagtattac agacaaaaca tcagcagcct atcagcgtat agcatgggaa     240
gccttaaaga aatccgttca tggttacatt aataaaatta acacctcgaa tattggcatc     300
atcgccagag aattattgca tgaaaatata gtaagaggta gaggtttgct gtgcaagtca     360
ataatacaag cacaagcagc atctcctact tttacaaacg tttacgcagc c              411
```

<210> 4
<211> 407
<212> DNA
<213> Diabrotica virgifera

<400> 4

cccttagtaa agaaatctta ggcagtgatg gtgagtctga atcaggttcc gaaggttcag 60

aagaggaatc tgataatgaa aatgaggacg aagtcaagga ccagggaaca attattgaca 120

atactgaaac gaatttaatt tctcttagaa gaaccatata tttgactatt cagtctagtt 180

tagattttga agaatgtgca cataagctac tgaagatgga gttgaaacct ggacaagaaa 240

tagaattgtg tcacatgttt cttgactgct gcgcagaaca aagaacctac gaaaagtttt 300

atggtctttt ggctcaaaga ttttgtcaaa tcaacaaagt gtatatcgag cctttccaac 360

aaatttttaa agatacctat tctaccactc acagactaga tgctaac 407

<210> 5
<211> 178
<212> DNA
<213> Diabrotica virgifera

<400> 5

cagtcatcaa accaagagaa agaaaaactg tagatatgtt aacatcgagg actggtggtg 60

cttatattcc cccagctaag ctacgattgt tacaagccag tattacagac aaaacatcag 120

cagcctatca gcgtatagca tgggaagcct taaagaaatc cgttcatggt tacattaa 178

<210> 6
<211> 120
<212> DNA
<213> Diabrotica virgifera

<400> 6

attggcatca tcgccagaga attattgcat gaaaatatag taagaggtag aggtttgctg 60

tgcaagtcaa taatacaagc acaagcagca tctcctactt ttacaaacgt ttacgcagcc 120

<210> 7
<211> 24
<212> DNA
<213> Artificial Sequence

<220>
<223> Promotor oligonucleotide

<400> 7
ttaatacgac tcactatagg gaga 24

<210> 8
<211> 503
<212> DNA
<213> Artificial Sequence

<220>
<223> Partial coding region

<400> 8

```
caccatgggc tccagcggcg ccctgctgtt ccacggcaag atcccctacg tggtggagat      60
ggagggcaat gtggatggcc acaccttcag catccgcggc aagggctacg gcgatgccag     120
cgtgggcaag gtggatgccc agttcatctg caccaccggc gatgtgcccg tgccctggag     180
caccctggtg accaccctga cctacggcgc ccagtgcttc gccaagtacg gccccgagct     240
gaaggatttc tacaagagct gcatgcccga tggctacgtg caggagcgca ccatcacctt     300
cgagggcgat ggcaatttca agacccgcgc cgaggtgacc ttcgagaatg gcagcgtgta     360
caatcgcgtg aagctgaatg gccagggctt caagaaggat ggccacgtgc tgggcaagaa     420
tctggagttc aatttcaccc cccactgcct gtacatctgg ggcgatcagg ccaatcacgg     480
cctgaagagc gccttcaaga tct                                             503
```

<210> 9
<211> 47
<212> DNA
<213> Artificial Sequence

<220>
<223> Primer oligonucleotide

<400> 9
ttaatacgac tcactatagg gagaggctgc gtaaacgttt gtaaaag 47

<210> 10
<211> 46
<212> DNA
<213> Artificial Sequence

<220>
<223> Primer oligonucleotide

<400> 10
ttaatacgac tcactatagg gagagatgcg gctcccgaag aatctg 46

<210> 11
<211> 47
<212> DNA
<213> Artificial Sequence

<220>
<223> Primer oligonucleotide

<400> 11
ttaatacgac tcactatagg gagagttagc atctagtctg tgagtgg 47

<210> 12
<211> 49
<212> DNA
<213> Artificial Sequence

<220>
<223> Primer oligonucleotide

<400> 12
ttaatacgac tcactatagg gagaccctta gtaaagaaat cttaggcag 49

<210> 13
<211> 48
<212> DNA
<213> Artificial Sequence

<220>
<223> Primer oligonucleotide

<400> 13
ttaatacgac tcactatagg gagattaatg taaccatgaa cggatttc 48

<210> 14
<211> 46
<212> DNA
<213> Artificial Sequence

<220>
<223> Primer oligonucleotide

<400> 14
ttaatacgac tcactatagg gagacagtca tcaaaccaag agaaag 46

<210> 15
<211> 47
<212> DNA
<213> Artificial Sequence

<220>
<223> Primer oligonucleotide

<400> 15
ttaatacgac tcactatagg gagaggctgc gtaaacgttt gtaaaag 47

<210> 16
<211> 51
<212> DNA
<213> Artificial Sequence

<220>
<223> Primer oligonucleotide

<400> 16
ttaatacgac tcactatagg gagaattggc atcatcgcca gagaattatt g 51

<210> 17
<211> 505
<212> DNA
<213> Artificial Sequence

<220>
<223> Artificial sequence

<400> 17

```
ggctgcgtaa acgtttgtaa aagtaggaga tgctgcttgt gcttgtatta ttgacttgca      60
cagcaaacct ctacctctta ctatattttc atgcaataat tctctggcga tgatgccaat     120
gaatccttgc gtcatttggt gactagtacc ggttgggaaa ggtatgtttc tgcttctacc     180
tttgatatat atataataat tatcactaat tagtagtaat atagtatttc aagtattttt     240
ttcaaaataa aagaatgtag tatatagcta ttgcttttct gtagtttata agtgtgtata     300
ttttaattta taacttttct aatatatgac caaaacatgg tgatgtgcag gttgatccgc     360
ggttaagttg tgcgtgagtc cattgattgg catcatcgcc agagaattat tgcatgaaaa     420
tatagtaaga ggtagaggtt tgctgtgcaa gtcaataata caagcacaag cagcatctcc     480

tacttttaca aacgtttacg cagcc                                           505
```

<210> 18
<211> 678
<212> DNA
<213> Artificial Sequence

<220>
<223> Artificial sequence

<400> 18

```
atgtcatctg gagcacttct ctttcatggg aagattcctt acgttgtgga gatggaaggg      60
aatgttgatg gccacacctt tagcatacgt gggaaaggct acggagatgc ctcagtggga     120
aaggttgatg cacagttcat ctgcacaact ggtgatgttc ctgtgccttg gagcacacag     180
tgctttgcca agtatggtcc agagttgaag gacttctaca agtcctgtat gccagatggc     240
tatgtgcaag agcgcacaat cacctttgaa ggagatggca acttcaagac tagggctgaa     300
gtcacctttg agaatgggtc tgtctacaat agggtcaaac tcaatggtca aggcttcaag     360
aaagatggtc atgtgttggg aaagaacttg gagttcaact tcactcccca ctgcctctac     420
atctggggtg accaagccaa ccacggtctc aagtcagcct tcaagatctg tcatgagatt     480
actggcagca aaggcgactt catagtggct gaccacaccc agatgaacac tcccattggt     540
ggaggtccag ttcatgttcc agagtatcat cacatgtctt accatgtgaa actttccaaa     600
gatgtgacag accacagaga caacatgtcc ttgaaagaaa ctgtcagagc tgttgactgt     660
cgcaagacct acctttga                                                   678
```

<210> 19
<211> 153
<212> DNA
<213> Artificial Sequence

<220>
<223> Loop

<400> 19

```
agtcatcacg ctggagcgca catataggcc ctccatcaga aagtcattgt gtatatctct        60
catagggaac gagctgcttg cgtatttccc ttccgtagtc agagtcatca atcagctgca       120
ccgtgtcgta aagcgggacg ttcgcaagct cgt                                    153
```

<210> 20
<211> 705
<212> DNA
<213> Artificial Sequence

<220>
<223> Artificial sequence

<400> 20

```
atgtcatctg gagcacttct ctttcatggg aagattcctt acgttgtgga gatggaaggg        60
aatgttgatg gccacacctt tagcatacgt gggaaaggct acggagatgc ctcagtggga       120
aaggttgatg cacagttcat ctgcacaact ggtgatgttc ctgtgccttg gagcacactt       180
gtcaccactc tcacctatgg agcacagtgc tttgccaagt atggtccaga gttgaaggac       240
ttctacaagt cctgtatgcc agatggctat gtgcaagagc gcacaatcac ctttgaagga       300
gatggcaact tcaagactag ggctgaagtc acctttgaga atgggtctgt ctacaatagg       360
gtcaaactca atggtcaagg cttcaagaaa gatggtcatg tgttgggaaa gaacttggag       420
ttcaacttca ctccccactg cctctacatc tggggtgacc aagccaacca cggtctcaag       480
tcagccttca agatctgtca tgagattact ggcagcaaag gcgacttcat agtggctgac       540
cacacccaga tgaacactcc cattggtgga ggtccagttc atgttccaga gtatcatcac       600
atgtcttacc atgtgaaact ttccaaagat gtgacagacc acagagacaa catgtccttg       660
aaagaaactg tcagagctgt tgactgtcgc aagacctacc tttga                      705
```

<210> 21
<211> 218
<212> DNA
<213> Diabrotica virgifera

<400> 21

```
tagctctgat gacagagccc atcgagtttc aagccaaaca gttgcataaa gctatcagcg        60
gattgggaac tgatgaaagt acaatmgtmg aaattttaag tgtmcacaac aacgatgaga       120
ttataagaat ttcccaggcc tatgaaggat tgtaccaacg mtcattggaa tctgatatca       180
aaggagatac ctcaggaaca ttaaaaaaga attattag                              218
```

<210> 22
<211> 424
<212> DNA
<213> Diabrotica virgifera

<220>
<221> misc_feature
<222> (393)..(393)
<223> n is a, c, g, or t

<220>
<221> misc_feature
<222> (394)..(394)
<223> n is a, c, g, or t

<220>
<221> misc_feature
<222> (395)..(395)
<223> n is a, c, g, or t

<400> 22

```
ttgttacaag ctggagaact tctctttgct ggaaccgaag agtcagtatt taatgctgta        60
ttctgtcaaa gaaataaacc acaattgaat ttgatattcg acaaatatga agaaattgtt       120
gggcatccca ttgaaaaagc cattgaaaac gagttttcag gaaatgctaa acaagccatg       180
ttacacctta tccagagcgt aagagatcaa gttgcatatt tggtaaccag gctgcatgat       240
tcaatggcag gcgtcggtac tgacgataga actttaatca gaattgttgt ttcgagatct       300
gaaatcgatc tagaggaaat caaacaatgc tatgaagaaa tctacagtaa aaccttggct       360
gataggatag cggatgacac atctggcgac tannnaaaag ccttattagc cgttgttggt       420
taag                                                                    424
```

<210> 23
<211> 397
<212> DNA
<213> Diabrotica virgifera

<400> 23

agatgttggc tgcatctaga gaattacaca agttcttcca tgattgcaag gatgtactga 60
gcagaatagt ggaaaaacag gtatccatgt ctgatgaatt gggaagggac gcaggagctg 120
tcaatgccct tcaacgcaaa caccagaact tcctccaaga cctacaaaca ctccaatcga 180
acgtccaaca aatccaagaa gaatcagcta aacttcaagc tagctatgcc ggtgatagag 240
ctaaagaaat caccaacagg gagcaggaag tggtagcagc ctgggcagcc ttgcagatcg 300
cttgcgatca gagacacgga aaattgagcg atactggtga tctattcaaa ttctttaact 360
tggtacgaac gttgatgcag tggatggacg aatggac 397

<210> 24
<211> 490
<212> DNA
<213> Diabrotica virgifera

<400> 24

gcagatgaac accagcgaga aaccaagaga tgttagtggt gttgaattgt tgatgaacaa 60
ccatcagaca ctcaaggctg agatcgaagc cagagaagac aactttacgg cttgtatttc 120
tttaggaaag gaattgttga gccgtaatca ctatgctagt gctgatatta aggataaatt 180
ggtcgcgttg acgaatcaaa ggaatgctgt actacagagg tgggaagaaa gatgggagaa 240
cttgcaactc atcctcgagg tataccaatt cgccagagat gcggccgtcg ccgaagcatg 300
gttgatcgca caagaacctt acttgatgag ccaagaacta ggacacacca ttgacgacgt 360
tgaaaacttg ataaagaaac acgaagcgtt cgaaaaatcg gcagcggcgc aagaagagag 420
attcagtgct ttggagagac tgacgacgtt cgaattgaga gaaataaaga ggaaacaaga 480
agctgcccag 490

<210> 25
<211> 330
<212> DNA
<213> Diabrotica virgifera

<400> 25

agtgaaatgt tagcaaatat aacatccaag tttcgtaatt gtacttgctc agttagaaaa 60
tattctgtag tttcactatc ttcaaccgaa aatagaataa atgtagaacc tcgcgaactt 120
gcctttcctc caaaatatca agaacctcga caagtttggt tggagagttt agatacgata 180
gacgacaaaa aattgggtat tcttgagctg catcctgatg tttttgctac taatccaaga 240
atagatatta tacatcaaaa tgttagatgg caaagtttat atagatatgt aagctatgct 300
catacaaagt caagatttga agtgagaggt 330

<210> 26
<211> 320
<212> DNA
<213> Diabrotica virgifera

<400> 26

```
caaagtcaag atttgaagtg agaggtggag gtcgaaaacc gtggccgcaa aagggattgg      60
gacgtgctcg acatggttca attagaagtc cactttggag aggtggagga gttgttcatg     120
gaccaaaatc tccaacccct catttttaca tgattccatt ctacacccgt ttgctgggtt     180
tgactagcgc actttcagta aaatttgccc aagatgactt gcacgttgtg gatagtctag     240
atctgccaac tgacgaacaa agttatatag aagagctggt caaaagccgc ttttgggggt     300
ccttcttgtt ttatttgtag                                                 320
```

<210> 27
<211> 47
<212> DNA
<213> Artificial Sequence

<220>
<223> Primer oligonucleotide

<400> 27
ttaatacgac tcactatagg gagacaccat gggctccagc ggcgccc 47

<210> 28
<211> 23
<212> DNA
<213> Artificial Sequence

<220>
<223> Primer oligonucleotide

<400> 28
agatcttgaa ggcgctcttc agg 23

<210> 29
<211> 23
<212> DNA
<213> Artificial Sequence

<220>
<223> Primer oligonucleotide

<400> 29
caccatgggc tccagcggcg ccc 23

<210> 30
<211> 47
<212> DNA
<213> Artificial Sequence

<220>

<223> Primer oligonucleotide

<400> 30
ttaatacgac tcactatagg gagaagatct tgaaggcgct cttcagg 47

<210> 31
<211> 46
<212> DNA
<213> Artificial Sequence

<220>
<223> Primer oligonucleotide

<400> 31
ttaatacgac tcactatagg gagagctcca acagtggttc cttatc 46

<210> 32
<211> 29
<212> DNA
<213> Artificial Sequence

<220>
<223> Primer oligonucleotide

<400> 32
ctaataattc ttttttaatg ttcctgagg 29

<210> 33
<211> 22
<212> DNA
<213> Artificial Sequence

<220>
<223> Primer oligonucleotide

<400> 33
gctccaacag tggttcctta tc 22

<210> 34
<211> 53
<212> DNA
<213> Artificial Sequence

<220>
<223> Primer oligonucleotide

<400> 34
ttaatacgac tcactatagg gagactaata attctttttt aatgttcctg agg 53

<210> 35
<211> 48
<212> DNA
<213> Artificial Sequence

<220>
<223> Primer oligonucleotide

<400> 35

ttaatacgac tcactatagg gagattgtta caagctggag aacttctc 48

<210> 36
<211> 24
<212> DNA
<213> Artificial Sequence

<220>
<223> Primer oligonucleotide

<400> 36
cttaaccaac aacggctaat aagg 24

<210> 37
<211> 24
<212> DNA
<213> Artificial Sequence

<220>
<223> Primer oligonucleotide

<400> 37
ttgttacaag ctggagaact tctc 24

<210> 38
<211> 48
<212> DNA
<213> Artificial Sequence

<220>
<223> Primer oligonucleotide

<400> 38
ttaatacgac tcactatagg gagacttaac caacaacggc taataagg 48

<210> 39
<211> 47
<212> DNA
<213> Artificial Sequence

<220>
<223> Primer oligonucleotide

<400> 39
ttaatacgac tcactatagg gagaagatgt tggctgcatc tagagaa 47

<210> 40
<211> 22
<212> DNA
<213> Artificial Sequence

<220>
<223> Primer oligonucleotide

<400> 40
gtccattcgt ccatccactg ca 22

<210> 41

<211> 23
<212> DNA
<213> Artificial Sequence

<220>
<223> Primer oligonucleotide

<400> 41
agatgttggc tgcatctaga gaa 23

<210> 42
<211> 46
<212> DNA
<213> Artificial Sequence

<220>
<223> Primer oligonucleotide

<400> 42
ttaatacgac tcactatagg gagagtccat tcgtccatcc actgca 46

<210> 43
<211> 46
<212> DNA
<213> Artificial Sequence

<220>
<223> Primer oligonucleotide

<400> 43
ttaatacgac tcactatagg gagagcagat gaacaccagc gagaaa 46

<210> 44
<211> 22
<212> DNA
<213> Artificial Sequence

<220>
<223> Primer oligonucleotide

<400> 44
ctgggcagct tcttgtttcc tc 22

<210> 45
<211> 22
<212> DNA
<213> Artificial Sequence

<220>
<223> Primer oligonucleotide

<400> 45
gcagatgaac accagcgaga aa 22

<210> 46
<211> 46
<212> DNA
<213> Artificial Sequence

<220>
<223> Primer oligonucleotide

<400> 46
ttaatacgac tcactatagg gagactgggc agcttcttgt ttcctc 46

<210> 47
<211> 51
<212> DNA
<213> Artificial Sequence

<220>
<223> Primer oligonucleotide

<400> 47
ttaatacgac tcactatagg gagaagtgaa atgttagcaa atataacatc c 51

<210> 48
<211> 26
<212> DNA
<213> Artificial Sequence

<220>
<223> Primer oligonucleotide

<400> 48
acctctcact tcaaatcttg actttg 26

<210> 49
<211> 27
<212> DNA
<213> Artificial Sequence

<220>
<223> Primer oligonucleotide

<400> 49
agtgaaatgt tagcaaatat aacatcc 27

<210> 50
<211> 50
<212> DNA
<213> Artificial Sequence

<220>
<223> Primer oligonucleotide

<400> 50
ttaatacgac tcactatagg gagaacctct cacttcaaat cttgactttg 50

<210> 51
<211> 50
<212> DNA
<213> Artificial Sequence

<220>
<223> Primer oligonucleotide

<400> 51
ttaatacgac tcactatagg gagacaaagt caagatttga agtgagaggt 50

<210> 52
<211> 25
<212> DNA
<213> Artificial Sequence

<220>
<223> Primer oligonucleotide

<400> 52
ctacaaataa aacaagaagg acccc 25

<210> 53
<211> 26
<212> DNA
<213> Artificial Sequence

<220>
<223> Primer oligonucleotide

<400> 53
caaagtcaag atttgaagtg agaggt 26

<210> 54
<211> 49
<212> DNA
<213> Artificial Sequence

<220>
<223> Primer oligonucleotide

<400> 54
ttaatacgac tcactatagg gagactacaa ataaaacaag aaggacccc 49

<210> 55
<211> 1150
<212> DNA
<213> Zea mays

<400> 55

```
caacggggca gcactgcact gcactgcaac tgcgaatttc cgtcagcttg gagcggtcca     60
agcgccctgc gaagcaaact acgccgatgg cttcggcggc ggcgtgggag ggtccgacgg    120
ccgcggagct gaagacagcg ggggcggagg tgattcccgg cggcgtgcga gtgaaggggt    180
gggtcatcca gtcccacaaa ggccctatcc tcaacgccgc ctctctgcaa cgctttgaag    240
atgaacttca aacaacacat ttacctgaga tggtttttgg agagagtttc ttgtcacttc    300
aacatacaca aactggcatc aaatttcatt ttaatgcgct tgatgcactc aaggcatgga    360
agaaagaggc actgccacct gttgaggttc ctgctgcagc aaaatggaag ttcagaagta    420
agccttctga ccaggttata cttgactacg actatacatt tacgacacca tattgtggga    480
gtgatgctgt ggttgtgaac tctggcactc cacaaacaag tttagatgga tgcggcactt    540
tgtgttggga ggatactaat gatcggattg acattgttgc cctttcagca aaagaaccca    600
ttcttttcta cgacgaggtt atcttgtatg aagatgagtt agctgacaat ggtatctcat    660
ttcttactgt gcgagtgagg gtaatgccaa ctggttggtt tctgcttttg cgtttttggc    720
ttagagttga tggtgtactg atgaggttga gagacactcg gttacattgc ctgtttggaa    780
acggcgacgg agccaagcca gtggtacttc gtgagtgctg ctggagggaa gcaacatttg    840
ctactttgtc tgcgaaagga tatccttcgg actctgcagc gtacgcggac ccgaacctta    900
ttgcccataa gcttcctatt gtgacgcaga agacccaaaa gctgaaaaat cctacctgac    960
tgacacaaag gcgccctacc gcgtgtacat catgactgtc ctgtcctatc gttgcctttt   1020
gtgtttgcca catgttgtgg atgtacgttt ctatgacgaa acaccatagt ccatttcgcc   1080
tgggccgaac agagatagct gattgtcatg tcacgtttga attagaccat tccttagccc   1140
tttttccccc                                                          1150
```

<210> 56
<211> 22
<212> DNA
<213> Artificial Sequence

<220>
<223> Primer oligonucleotide

<220>
<221> misc_feature
<222> (22)..(22)
<223> n is a, c, g, or t

<400> 56
tttttttttt tttttttttt vn 22

<210> 57
<211> 20
<212> DNA
<213> Artificial Sequence

<220>
<223> Primer oligonucleotide

<400> 57
ttgtgatgtt ggtggcgtat 20

<210> 58
<211> 24
<212> DNA
<213> Artificial Sequence

<220>
<223> Primer oligonucleotide

<400> 58
tgttaaataa aaccccaaag atcg 24

<210> 59
<211> 21
<212> DNA
<213> Artificial Sequence

<220>
<223> Primer oligonucleotide

<400> 59
tgagggtaat gccaactggt t 21

<210> 60
<211> 24
<212> DNA
<213> Artificial Sequence

<220>
<223> Primer oligonucleotide

<400> 60
gcaatgtaac cgagtgtctc tcaa 24

<210> 61
<211> 32
<212> DNA
<213> Artificial Sequence

<220>
<223> Probe oligonucleotide

<400> 61
ttttggctt agagttgatg gtgtactgat ga 32

<210> 62
<211> 151
<212> DNA
<213> Escherichia coli

<400> 62

gaccgtaagg cttgatgaaa caacgcggcg agctttgatc aacgaccttt tggaaacttc 60

ggcttcccct ggagagagcg agattctccg cgctgtagaa gtcaccattg ttgtgcacga 120

cgacatcatt ccgtggcgtt atccagctaa g 151

<210> 63
<211> 69
<212> DNA
<213> Artificial Sequence

<220>
<223> Partial coding region

<400> 63

tgttcggttc cctctaccaa gcacagaacc gtcgcttcag caacacctca gtcaaggtga 60

tggatgttg 69

<210> 64
<211> 4233
<212> DNA
<213> Zea mays

<400> 64

```
agcctggtgt ttccggagga gacagacatg atccctgccg ttgctgatcc gacgacgctg      60
gacggcgggg gcgcgcgcag gccgttgctc ccggagacgg accctcgggg gcgtgctgcc     120
gccggcgccg agcagaagcg gccgccggct acgccgaccg ttctcaccgc cgtcgtctcc     180
gccgtgctcc tgctcgtcct cgtggcggtc acagtcctcg cgtcgcagca cgtcgacggg     240
caggctgggg gcgttcccgc gggcgaagat gccgtcgtcg tcgaggtggc cgcctcccgt     300
ggcgtggctg agggcgtgtc ggagaagtcc acggccccgc tcctcggctc cggcgcgctc     360
caggacttct cctggaccaa cgcgatgctg gcgtggcagc gcacggcgtt ccacttccag     420
ccccccaaga actggatgaa cggttagttg gacccgtcgc catcggtgac gacgcgcgga     480
tcgttttttt ctttttttcct ctcgttctgg ctctaacttg gttccgcgtt tctgtcacgg    540
acgcctcgtg cacatggcga tacccgatcc gccggccgcg tatatctatc tacctcgacc     600
ggcttctcca gatccgaacg gtaagttgtt ggctccgata cgatcgatca catgtgagct     660
cggcatgctg cttttctgcg cgtgcatgcg gctcctagca ttccacgtcc acgggtcgtg     720
acatcaatgc acgatataat cgtatcggta cagagatatt gtcccatcag ctgctagctt     780
tcgcgtattg atgtcgtgac attttgcacg caggtccgct gtatcacaag ggctggtacc     840
acctcttcta ccagtggaac ccggactccg cggtatgggg caacatcacc tggggccacg     900
ccgtctcgcg cgacctcctc cactggctgc acctaccgct ggccatggtg cccgatcacc     960
cgtacgacgc caacggcgtc tggtccgggt cggcgacgcg cctgcccgac ggccggatcg    1020
tcatgctcta cacgggctcc acggcggagt cgtcggcgca ggtgcagaac ctcgcggagc    1080
cggccgacgc gtccgacccg ctgctgcggg agtgggtcaa gtcggacgcc aacccggtgc    1140
```

tggtgccgcc gccgggcatc gggccgacgg acttccgcga cccgacgacg gcgtgtcgga 1200
cgccggccgg caacgacacg gcgtggcggg tcgccatcgg gtccaaggac cgggaccacg 1260
cggggctggc gctggtgtac cggacggagg acttcgtgcg gtacgacccg gcgccggcgc 1320
tgatgcacgc cgtgccgggc accggcatgt gggagtgcgt ggacttctac ccggtggccg 1380
cgggatcagg cgccgcggcg ggcagcgggg acgggctgga gacgtccgcg gcgccgggac 1440
ccggggtgaa gcacgtgctc aaggctagcc tcgacgacga caagcacgac tactacgcga 1500
tcggcaccta cgacccggcg acggacacct ggacccccga cagcgcggag gacgacgtcg 1560
ggatcggcct ccggtacgac tatggcaagt actacgcgtc gaagaccttc tacgaccccg 1620
tccttcgccg gcgggtgctc tgggggtggg tcggcgagac cgacagcgag cgcgcggaca 1680
tcctcaaggg ctgggcatcc gtgcaggtac gtctcagggt ttgaggctag catggcttca 1740
atcttgctgg catcgaatca ttaatgggca gatattataa cttgataatc tgggttggtt 1800
gtgtgtggtg gggatggtga cacacgcgcg gtaataatgt agctaagctg gttaaggatg 1860
agtaatgggg ttgcgtataa acgacagctc tgctaccatt acttctgaca cccgattgaa 1920
ggagacaaca gtaggggtag ccggtagggt tcgtcgactt gccttttctt ttttcctttg 1980
ttttgttgtg gatcgtccaa cacaaggaaa ataggatcat ccaacaaaca tggaagtaat 2040
cccgtaaaac atttctcaag gaaccatcta gctagacgag cgtggcatga tccatgcatg 2100
cacaaacact agataggtct ctgcagctgt gatgttcctt tacatatacc accgtccaaa 2160
ctgaatccgg tctgaaaatt gttcaagcag agaggccccg atcctcacac ctgtacacgt 2220
ccctgtacgc gccgtcgtgg tctcccgtga tcctgccccg tcccctccac gcggccacgc 2280
ctgctgcagc gctctgtaca agcgtgcacc acgtgagaat ttccgtctac tcgagcctag 2340
tagttagacg ggaaaacgag aggaagcgca cggtccaagc acaacacttt gcgcgggccc 2400
gtgacttgtc tccggttggc tgagggcgcg cgacagagat gtatggcgcc gcggcgtgtc 2460
ttgtgtcttg tcttgcctat acaccgtagt cagagactgt gtcaaagccg tccaacgaca 2520
atgagctagg aaacgggttg gagagctggg ttcttgcctt gcctcctgtg atgtctttgc 2580
cttgcatagg gggcgcagta tgtagctttg cgttttactt cacgccaaag gatactgctg 2640
atcgtgaatt attattatta tatatatatc gaatatcgat ttcgtcgctc tcgtggggtt 2700
ttattttcca gactcaaact tttcaaaagg cctgtgtttt agttcttttc ttccaattga 2760
gtaggcaagg cgtgtgagtg tgaccaacgc atgcatggat atcgtggtag actggtagag 2820
ctgtcgttac cagcgcgatg cttgtatatg tttgcagtat tttcaaatga atgtctcagc 2880
tagcgtacag ttgaccaagt cgacgtggag ggcgcacaac agacctctga cattattcac 2940
ttttttttta ccatgccgtg cacgtgcagt caatccccag gacggtcctc ctggacacga 3000

agacgggcag caacctgctc cagtggccgg tggtggaggt ggagaacctc cggatgagcg 3060
gcaagagctt cgacggcgtc gcgctggacc gcggatccgt cgtgcccctc gacgtcggca 3120
aggcgacgca ggtgacgccg cacgcagcct gctgcagcga acgaactcgc gcgttgccgg 3180
cccgcggcca gctgacttag tttctctggc tgatcgaccg tgtgcctgcg tgcgtgcagt 3240
tggacatcga ggctgtgttc gaggtggacg cgtcggacgc ggcgggcgtc acggaggccg 3300
acgtgacgtt caactgcagc accagcgcag gcgcggcggg ccggggcctg ctcggcccgt 3360
tcggccttct cgtgctggcg gacgacgact tgtccgagca gaccgccgtg tacttctacc 3420
tgctcaaggg cacggacggc agcctccaaa ctttcttctg ccaagacgag ctcaggtatg 3480
tatgttatga cttatgacca tgcatgcatg cgcatttctt agctaggctg tgaagcttct 3540
tgttgagttg tttcacagat gcttaccgtc tgctttgttt cgtatttcga ctaggcatcc 3600
aaggcgaacg atctggttaa gagagtatac gggagcttgg tccctgtgct agatggggag 3660
aatctctcgg tcagaatact ggtaagtttt tacagcgcca gccatgcatg tgttggccag 3720
ccagctgctg gtactttgga cactcgttct tctcgcactg ctcattattg cttctgatct 3780
ggatgcacta caaattgaag gttgaccact ccatcgtgga gagctttgct caaggcggga 3840
ggacgtgcat cacgtcgcga gtgtacccca cacgagccat ctacgactcc gcccgcgtct 3900
tcctcttcaa caacgccaca catgctcacg tcaaagcaaa atccgtcaag atctggcagc 3960
tcaactccgc ctacatccgg ccatatccgg caacgacgac ttctctatga ctaaattaag 4020
tgacggacag ataggcgata ttgcatactt gcatcatgaa ctcatttgta caacagtgat 4080
tgtttaattt atttgctgcc ttccttatcc ttcttgtgaa actatatggt acacacatgt 4140
atcattaggt ctagtagtgt tgttgcaaag acacttagac accagaggtt ccaggagtat 4200
cagagataag gtataagagg gagcagggag cag 4233

<210> 65
<211> 20
<212> DNA
<213> Artificial Sequence

<220>
<223> Primer oligonucleotide

<400> 65
tgttcggttc cctctaccaa 20

<210> 66
<211> 22
<212> DNA
<213> Artificial Sequence

<220>
<223> Primer oligonucleotide

<400> 66
caacatccat caccttgact ga 22

<210> 67
<211> 24
<212> DNA
<213> Artificial Sequence

<220>
<223> Probe oligonucleotide

<400> 67
cacagaaccg tcgcttcagc aaca 24

<210> 68
<211> 18
<212> DNA
<213> Artificial Sequence

<220>
<223> Primer oligonucleotide

<400> 68
tggcggacga cgacttgt 18

<210> 69
<211> 19
<212> DNA
<213> Artificial Sequence

<220>
<223> Primer oligonucleotide

<400> 69
aaagtttgga ggctgccgt 19

<210> 70
<211> 26
<212> DNA
<213> Artificial Sequence

<220>
<223> Probe oligonucleotide

<400> 70
cgagcagacc gccgtgtact tctacc 26

<210> 71
<211> 19
<212> DNA
<213> Artificial Sequence

<220>
<223> Primer oligonucleotide

<400> 71
cttagctgga taacgccac 19

<210> 72
<211> 19
<212> DNA
<213> Artificial Sequence

<220>
<223> Primer oligonucleotide

<400> 72
gaccgtaagg cttgatgaa 19

<210> 73
<211> 21
<212> DNA
<213> Artificial Sequence

<220>
<223> Probe oligonucleotide

<400> 73
cgagattctc cgcgctgtag a 21

<210> 74
<211> 25
<212> DNA
<213> Artificial Sequence

<220>
<223> Primer oligonucleotide

<400> 74
gtatgtttct gcttctacct ttgat 25

<210> 75
<211> 29
<212> DNA
<213> Artificial Sequence

<220>
<223> Primer oligonucleotide

<400> 75
ccatgttttg gtcatatatt agaaaagtt 29

<210> 76
<211> 34
<212> DNA
<213> Artificial Sequence

<220>
<223> Probe oligonucleotide

<400> 76
agtaatatag tatttcaagt atttttttca aaat 34

<210> 77
<211> 2681
<212> DNA

<213> Diabrotica virgifera

<400> 77

attaccaaat gtcaatgtca ctcattactc attaccaaat gtcaatgtca ctgtcaggta 60
acgtgcaatg caaattgtca atgtcaaact taaaaatatt ttcctgcaac tgcatcaaat 120
tgtaaatttt attttttta aatatgccag ataccaagga tgccaaggat accaaggatg 180
ctaatttgag ttctcctgaa cgtaaaagac gaagaaagag tagatctaaa tctccagaac 240
gaaaagagaa aaagtcttcc aaaaagaaag cccacaatag tagagacaga gattcatcag 300
aggaaggtta caaccctaaa gattatcaga gatactatgg ggaagatcgc ccaaacagtg 360
acaaatattg gaataaatat ccaaggaaag atactaccaa agttggccaa agatactatg 420
atgcggctcc cgaagaatct ggcaagaagg ggccagatag aaattcagag gagaaggagt 480
taccaaagcc aatggagtct gttcctgata aatcagtcat caaaccaaga gaaagaaaaa 540
ctgtagatat gttaacatcg aggactggtg gtgcttatat tccccagct aagctacgat 600
tgttacaagc cagtattaca gacaaaacat cagcagccta tcagcgtata gcatgggaag 660
ccttaaagaa atccgttcat ggttacatta ataaaattaa cacctcgaat attggcatca 720
tcgccagaga attattgcat gaaaatatag taagaggtag aggtttgctg tgcaagtcaa 780
taatacaagc acaagcagca tctcctactt ttacaaacgt ttacgcagcc ttagtagctg 840
ttattaattc gaagtttcca agtataggag agcttttatt gaagaggttg gttttgcagt 900
tcaaaagagg gtttaaacaa aataataagt ctatttgcat atcggctact actttcgtag 960
ctcatttagt aaatcagaga gtggcacatg aaattttggc tttggagata cttacattgt 1020
tggtggagac tcctacagat gattctgtgg aggtggccat ttcatttttg aaggaatgtg 1080
gacaaaaact gacagaagtt tcaagtagag gtattactgc tatatttgag atgttaagaa 1140
acattttaca tgaaggccag ctagaaaaaa agaattcagt acatgattga agttatgttt 1200
caaataagga aagacggatt taaggatcat gctgctgtcg tagaagaatt agatttagta 1260
gaagaggaag atcaattcac tcatcttatt atgttagatg atgttaaaga ggctgatgca 1320
gaggatatat tgaatgtgtt caaatttgat gagagttatg aagaaaatga agataaatac 1380
aaaaccctta gtaaagaaat cttaggcagt gatggtgagt ctgaatcagg ttccgaaggt 1440
tcagaagagg aatctgataa tgaaaatgag gacgaagtca aggaccaggg aacaattatt 1500
gacaatactg aaacgaattt aatttctctt agaagaacca tatatttgac tattcagtct 1560
agtttagatt ttgaagaatg tgcacataag ctactgaaga tggagttgaa acctggacaa 1620
gaaatagaat tgtgtcacat gtttcttgac tgctgcgcag aacaaagaac ctacgaaaag 1680
ttttatggtc ttttggctca aagattttgt caaatcaaca aagtgtatat cgagcctttc 1740
caacaaattt ttaaagatac ctattctacc actcacagac tagatgctaa caggttaaga 1800

```
aacgtcagca aattttttgc gcatttactt tttacggatg ccattggatg ggaagtcctt     1860
gacatcatga aattgaatga agaggatacc aatagttcta gtaggatttt cataaaaatc     1920
ttgtttcaag aattggctga atatatggga ctaggaaaat taaacgcaag gctaaaggat     1980
gagaccctgc aggcttattt ttcaggactg tttcctagag ataacccaaa gaataccaga     2040
ttttctatta attttttttac ctctatcggt ttgggaggat taactgatga actcagagaa     2100
catttaaaaa atattccaaa aatgatggaa atgaagttag ccactaaaga aaaggaaagc     2160
agtggtagta gtagttcaga aagtagttct gaggaagata gtagtgacga cagctctgaa     2220
gattcatcaa gttctgaaga cgatagaggc aaaaagaaga aaaaaaccaa aaagctagaa     2280
gaaaaaaatt cgaaagtaaa ttctaagtct agacctagat caaaagagaa agaacacgca     2340
gacaaaccta gagacaaaca tagaaaggaa gatagacata aatctgacaa aaatcccgat     2400
agatcctcgt ccaaaaaata ttctaaagaa gataacaaga ggaagaaaga ctacgaatgg     2460
atgaagagca gatatgaaga tgatattaag caattaaaaa acgataaaag ggtatttgaa     2520
aagtcttcca aacgacgatc aagatctaga gacagggtaa aagtcaagga agagcgtaga     2580
cgtagaagca gagaaagaag aaggagctaa gataattttt taataaggat ctatgtatat     2640
ttatgtaaac atttatttaa tacatgtttt ttaaaaaaaa a                         2681
```

<210> 78
<211> 1047
<212> RNA
<213> Diabrotica virgifera

<400> 78

```
augccagaua ccaaggaugc caaggauacc aaggaugcua auuugaguuc uccugaacgu      60
aaaagacgaa gaaagaguag aucuaaaucu ccagaacgaa aagagaaaaa gucuuccaaa     120
aagaaagccc acaauaguag agacagagau ucaucagagg aagguuacaa cccuaaagau     180
uaucagagau acuaugggga agaucgccca aacagugaca aauauuggaa uaaauaucca     240
aggaaagaua cuaccaaagu uggccaaaga uacuaugaug cggcuccccga agaaucuggc    300
aagaaggggc cagauagaaa uucagaggag aaggaguuac caaagccaau ggagucuguu     360
ccugauaaau cagucaucaa accaagagaa agaaaaacug uagauauguu aacaucgagg     420
acuggguggug cuuauauucc cccagcuaag cuacgauugu uacaagccag uauuacagac    480
aaaacaucag cagccuauca gcguauagca ugggaagccu uaaagaaauc cguucauggu     540
uacauuaaua aaauuaacac cucgaauauu ggcaucaucg ccagagaauu auugcaugaa     600
aauauaguaa gagguagagg uuugcugugc aagucaauaa uacaagcaca agcagcaucu     660
ccuacuuuua caaacguuua cgcagccuua guagcuguua uuaauucgaa guuuccaagu     720
auaggagagc uuuuauugaa gagguugguu uugcaguuca aaagaggguu uaaacaaaau     780
aauaagucua uuugcauauc ggcuacuacu uucguagcuc auuuaguaaa ucagagagug     840
gcacaugaaa uuuuggcuuu ggagauacuu acauuguugg uggagacucc uacagaugau     900
ucuguggagg uggccauuuc auuuuugaag gaauguggac aaaaacugac agaaguuuca     960
aguagaggua uuacugcuau auuugagaug uuaagaaaca uuuuacauga aggccagcua    1020
gaaaaaaaga auucaguaca ugauuga                                       1047
```

<210> 79
<211> 411
<212> RNA
<213> Diabrotica virgifera

<400> 79

```
gaugcggcuc ccgaagaauc uggcaagaag gggccagaua gaaauucaga ggagaaggag      60
uuaccaaagc caauggaguc uguuccugau aaaucaguca ucaaaccaag agaaagaaaa     120
acuguagaua uguuaacauc gaggacuggu ggugcuuaua uucccccagc uaagcuacga     180
uuguuacaag ccaguauuac agacaaaaca ucagcagccu aucagcguau agcaugggaa     240
gccuuaaaga aauccguuca ugguuacauu aauaaaauua acaccucgaa uauuggcauc     300
aucgccagag aauuauugca ugaaaauaua guaagaggua gagguuugcu gugcaaguca     360
auaauacaag cacaagcagc aucuccuacu uuuacaaacg uuuacgcagc c             411
```

<210> 80
<211> 407

<212> RNA
<213> Diabrotica virgifera

<400> 80

```
cccuuaguaa agaaaucuua ggcagugaug gugagucuga aucagguucc gaagguucag      60
aagaggaauc ugauaaugaa aaugaggacg aagucaagga ccagggaaca auuauugaca     120
auacugaaac gaauuuaauu ucucuuagaa gaaccauaua uuugacuauu cagucuaguu     180
uagauuuuga agaaugugca cauaagcuac ugaagaugga guugaaaccu ggacaagaaa     240
uagaauugug ucacauguuu cuugacugcu gcgcagaaca aagaaccuac gaaaaguuuu     300
auggucuuuu ggcucaaaga uuuugucaaa ucaacaaagu guauaucgag ccuuuccaac     360
aaauuuuuaa agauaccuau ucuaccacuc acagacuaga ugcuaac                   407
```

<210> 81
<211> 178
<212> RNA
<213> Diabrotica virgifera

<400> 81

```
cagucaucaa accaagagaa agaaaaacug uagauauguu aacaucgagg acugguggug      60
cuuauauucc cccagcuaag cuacgauugu uacaagccag uauuacagac aaaacaucag     120
cagccuauca gcguauagca ugggaagccu uaaagaaauc cguucauggu uacauuaa       178
```

<210> 82
<211> 120
<212> RNA
<213> Diabrotica virgifera

<400> 82

```
auuggcauca ucgccagaga auuauugcau gaaaauauag uaagagguag agguuugcug      60
ugcaagucaa uaauacaagc acaagcagca ucuccuacuu uuacaaacgu uuacgcagcc     120
```

<210> 83
<211> 2681
<212> RNA
<213> Diabrotica virgifera

<400> 83

```
auuaccaaau gucaauguca cucauuacuc auuaccaaau gucaauguca cugucaggua      60
acgugcaaug caaauuguca augucaaacu uaaaaauauu uuccugcaac ugcaucaaau     120
uguaaauuuu auuuuuuuua aauaugccag auaccaagga ugccaaggau accaaggaug     180
cuaauuugag uucuccugaa cguaaaagac gaagaaagag uagaucuaaa ucuccagaac     240
gaaaagagaa aaagucuucc aaaaagaaag cccacaauag uagagacaga gauucaucag     300
aggaagguua caacccuaaa gauuaucaga gauacuaugg ggaagaucgc ccaaacagug     360
acaaauauug gaauaaauau ccaaggaaag auacuaccaa aguuggccaa agauacuaug     420
augcggcucc cgaagaaucu ggcaagaagg ggccagauag aaauucagag gagaaggagu     480
uaccaaagcc aauggagucu guuccugaua aaucagucau caaaccaaga gaaagaaaaa     540
cuguagauau guuaacaucg aggacuggug gugcuuauau ucccccagcu aagcuacgau     600
uguuacaagc caguauuaca gacaaaacau cagcagccua ucagcguaua gcaugggaag     660
ccuuaaagaa auccguucau gguuacauua auaaaauuaa caccucgaau auuggcauca     720
ucgccagaga auuauugcau gaaaauauag uaagagguag agguuugcug ugcaagucaa     780
uaauacaagc acaagcagca ucuccuacuu uuacaaacgu uuacgcagcc uuaguagcug     840
uuauuaauuc gaaguuucca aguauaggag agcuuuuauu gaagagguug guuuugcagu     900
ucaaaagagg guuuaaacaa aauaauaagu cuauuugcau aucggcuacu acuuucguag     960
cucauuuagu aaaucagaga guggcacaug aaauuuuggc uuuggagaua cuuacauugu    1020
ugguggagac uccuacagau gauucugugg agguggccau uucauuuuug aaggaaugug    1080
gacaaaaacu gacagaaguu ucaaguagag guauuacugc uauauuugag auguuaagaa    1140
acauuuuaca ugaaggccag cuagaaaaaa agaauucagu acaugauuga aguuauguuu    1200
caaauaagga aagacggauu uaaggaucau gcugcugucg uagaagaauu agauuuagua    1260
```

gaagaggaag aucaauucac ucaucuuauu auguuagaug auguuaaaga ggcugaugca 1320
gaggauauau ugaauguguu caaauuugau gagaguuaug aagaaaauga agauaaauac 1380
aaaacccuua guaaagaaau cuuaggcagu gauggugagu cugaaucagg uuccgaaggu 1440
ucagaagagg aaucugauaa ugaaaaugag gacgaaguca aggaccaggg aacaauuauu 1500
gacaauacug aaacgaauuu aauuucucuu agaagaacca uauauuugac uauucagucu 1560
aguuuagauu uugaagaaug ugcacauaag cuacugaaga uggaguugaa accuggacaa 1620
gaaauagaau ugugucacau guuucuugac ugcugcgcag aacaaagaac cuacgaaaag 1680
uuuuaugguc uuuuggcuca aagauuuugu caaaucaaca aaguguauau cgagccuuuc 1740
caacaaauuu uuaaagauac cuauucuacc acucacagac uagaugcuaa cagguuaaga 1800
aacgucagca aauuuuuugc gcauuuacuu uuuacggaug ccauuggaug ggaaguccuu 1860
gacaucauga aauugaauga agaggauacc aauaguucua guaggauuuu cauaaaaauc 1920
uuguuucaag aauuggcuga auauauggga cuaggaaaau uaaacgcaag gcuaaaggau 1980
gagacccugc aggcuuauuu uucaggacug uuuccuagag auaacccaaa gaauaccaga 2040
uuuucuauua auuuuuuuac cucuaucggu uugggaggau uaacugauga acucagagaa 2100
cauuuaaaaa auauuccaaa aaugauggaa augaaguuag ccacuaaaga aaaggaaagc 2160
agugguagua guaguucaga aaguaguucu gaggaagaua guagugacga cagcucugaa 2220
gauucaucaa guucugaaga cgauagaggc aaaaagaaga aaaaaaccaa aaagcuagaa 2280
gaaaaaaauu cgaaaguaaa uucuaagucu agaccuagau caaaagagaa agaacacgca 2340
gacaaaccua gagacaaaca uagaaaggaa gauagacaua aaucugacaa aaaucccgau 2400
agauccucgu ccaaaaaaua uucuaaagaa gauaacaaga ggaagaaaga cuacgaaugg 2460
augaagagca gauaugaaga ugauauuaag caauuaaaaa acgauaaaag gguauuugaa 2520
aagucuucca aacgacgauc aagaucuaga gacaggguaa aagucaagga agagcguaga 2580
cguagaagca gagaaagaag aaggagcuaa gauaauuuuu uaauaaggau cuauguauau 2640
uuauguaaac auuuauuuaa uacauguuuu uuaaaaaaaa a 2681

<210> 84
<211> 1658
<212> DNA
<213> Meligethes aeneus

<400> 84

gcaatttcgt ttttgaagga aagtggtcaa aaactcactg aggtgtcgag taaaggtatc 60
aatgccatat ttgagatgtt gaggaatatt ctgcatgaag gacagttgga gaagagaata 120
cagtacatga ttgaagtcat gttccaagtt cggaaagatg gtttcaagga tcatgctgct 180
gttactgaag aactagatat tgttgaagag gaagatcagt ttactcacct aatcacattg 240
gatgatgtta aacaagctaa ctcagaggat atattgaatg tgtttaaatt tgatgataaa 300
tatgaggaaa atgagggtaa atacaaaact ttaagtaagg aaattctcca gtcagacagt 360
gaatcaggcg aatctggttc agaggggtct gaagaagact cggaagatga agaaggtgaa 420
gaagatgaaa ccaaaaatca aaccattatt gataacacag aaactaattt aatcacctta 480
aggagaacca tctatctcac aatacaatcc agtttggatt ttgaggaatg tgcccataaa 540
ttgatgaaaa tggagatcaa acctggacaa gagattgaat tgtgtcacat gttccttgat 600
tgttgcgctg aacagcgtac ctacgaaaaa ttcttcggcc tcctctcgca gcgcttctgc 660
caaataaaca agactttcat cgaaccgttc caacaaattt tcaaagatac ctattccaca 720
actcacagac ttgacgccaa tcgattgaga aacgttagca aattcttcgc gcatttattg 780
ttcaccgacg ccatcggctg ggaagtgctc gatatcatga aattaaacga ggaagacacc 840
aacagttcca gcaggatttt catcaagatt ttgttccagg agttgtccga atatatggga 900
ttagcgaagt tgaataaaag gctaaaggat gaaactttac aggaatattt cgcggggcta 960
tttccgaggg ataacccgaa gaacacgcgt ttcgccatca attttttcac gtcgatcggt 1020
ttaggaggtc taacggacga gttgagggag cacttgaaaa acgtgccaaa acatctggaa 1080
gtgatggctt tgaaagcaga ttcgagcagc tctagcagca gtagcagcag ttccagtaac 1140
gattccagca gcagttcaga ttcttccgat gacgagggtt ccaggaagaa gaaaacaaaa 1200
aaattgaaaa ccccggacaa aaagaagaaa cagaaagaag atgaaaaacc caaaaagaaa 1260
agcgaggata aaccgaggaa caaaccagac tatagagata gaagaaacga cgacagggaa 1320
aagtttaaaa aatacagaaa caacgacgaa gaaagccaca gaagaagcag agaagatgca 1380
agagaaaaat acagaggtca cgaggaaaga agaagcgacc acagagaaga ataccggccg 1440
agagaacata gaggtagaga tagacgttag ttgtataata atgtatattt tttcgtattt 1500
aataaaataa attatacatt ttatagtgtt tcggagcatt caccaagcaa gggttttact 1560
ttcggatagc aatggtgtag tacgtttttg aaggtgtcca cacacaccaa gccggtttta 1620
tctaaatcta gagctatctt ccaaaagtct tcaaagga 1658

<210> 85
<211> 489
<212> PRT
<213> Meligethes aeneus

<400> 85

```
Ala Ile Ser Phe Leu Lys Glu Ser Gly Gln Lys Leu Thr Glu Val Ser
1               5                   10                  15

Ser Lys Gly Ile Asn Ala Ile Phe Glu Met Leu Arg Asn Ile Leu His
            20                  25                  30
```

Glu Gly Gln Leu Glu Lys Arg Ile Gln Tyr Met Ile Glu Val Met Phe
35 40 45

Gln Val Arg Lys Asp Gly Phe Lys Asp His Ala Ala Val Thr Glu Glu
50 55 60

Leu Asp Ile Val Glu Glu Glu Asp Gln Phe Thr His Leu Ile Thr Leu
65 70 75 80

Asp Asp Val Lys Gln Ala Asn Ser Glu Asp Ile Leu Asn Val Phe Lys
85 90 95

Phe Asp Asp Lys Tyr Glu Glu Asn Glu Gly Lys Tyr Lys Thr Leu Ser
100 105 110

Lys Glu Ile Leu Gln Ser Asp Ser Glu Ser Gly Glu Ser Gly Ser Glu
115 120 125

Gly Ser Glu Glu Asp Ser Glu Asp Glu Glu Gly Glu Glu Asp Glu Thr
130 135 140

Lys Asn Gln Thr Ile Ile Asp Asn Thr Glu Thr Asn Leu Ile Thr Leu
145 150 155 160

Arg Arg Thr Ile Tyr Leu Thr Ile Gln Ser Ser Leu Asp Phe Glu Glu
165 170 175

Cys Ala His Lys Leu Met Lys Met Glu Ile Lys Pro Gly Gln Glu Ile
180 185 190

Glu Leu Cys His Met Phe Leu Asp Cys Cys Ala Glu Gln Arg Thr Tyr
195 200 205

Glu Lys Phe Phe Gly Leu Leu Ser Gln Arg Phe Cys Gln Ile Asn Lys
210 215 220

Thr Phe Ile Glu Pro Phe Gln Gln Ile Phe Lys Asp Thr Tyr Ser Thr
225 230 235 240

Thr His Arg Leu Asp Ala Asn Arg Leu Arg Asn Val Ser Lys Phe Phe
245 250 255

Ala His Leu Leu Phe Thr Asp Ala Ile Gly Trp Glu Val Leu Asp Ile
260 265 270

Met Lys Leu Asn Glu Glu Asp Thr Asn Ser Ser Ser Arg Ile Phe Ile
275 280 285

```
Lys Ile Leu Phe Gln Glu Leu Ser Glu Tyr Met Gly Leu Ala Lys Leu
    290                 295                 300

Asn Lys Arg Leu Lys Asp Glu Thr Leu Gln Glu Tyr Phe Ala Gly Leu
305                 310                 315                 320

Phe Pro Arg Asp Asn Pro Lys Asn Thr Arg Phe Ala Ile Asn Phe Phe
                325                 330                 335

Thr Ser Ile Gly Leu Gly Gly Leu Thr Asp Glu Leu Arg Glu His Leu
            340                 345                 350

Lys Asn Val Pro Lys His Leu Glu Val Met Ala Leu Lys Ala Asp Ser
        355                 360                 365

Ser Ser Ser Ser Ser Ser Ser Ser Ser Ser Ser Asn Asp Ser Ser Ser
    370                 375                 380

Ser Ser Asp Ser Ser Asp Asp Glu Gly Ser Arg Lys Lys Lys Thr Lys
385                 390                 395                 400

Lys Leu Lys Thr Pro Asp Lys Lys Lys Lys Gln Lys Glu Asp Glu Lys
                405                 410                 415

Pro Lys Lys Lys Ser Glu Asp Lys Pro Arg Asn Lys Pro Asp Tyr Arg
            420                 425                 430

Asp Arg Arg Asn Asp Asp Arg Glu Lys Phe Lys Lys Tyr Arg Asn Asn
        435                 440                 445

Asp Glu Glu Ser His Arg Arg Ser Arg Glu Asp Ala Arg Glu Lys Tyr
    450                 455                 460

Arg Gly His Glu Glu Arg Arg Ser Asp His Arg Glu Glu Tyr Arg Pro
465                 470                 475                 480

Arg Glu His Arg Gly Arg Asp Arg Arg
                485
```

<210> 86
<211> 2571
<212> DNA
<213> Meligethes aeneus

<400> 86

```
ccacacgaat tgactggtta attaaaaata agcacaagaa acgaataaca ctacaatggt      60
ttgaatttac acaaaaaaaa aaatgtagtc actaccattg tagtgttatt cgtttcttgt     120
```

gcttattttt aattaaccag tcaattcgtg tggtgtagtg aacaaaggtt atagttatga 180

cgacggattc cgagagaggt tcccctacag ctgcggctcc acgcagaagc gcctcgaaat 240

cgccagaacc aaaaaaagca aagtacgata agaaagagaa gggcgataaa gatcgcaaga 300

ggagatccca cagatccaga tctagatcca gggatagaga ccatagggac aaacatggtg 360

gaaaaaaacg ttaccacgac ctggacgacc cttctgaaga ctacccaaga tattatggcg 420

aggatagaaa acagaacagt gacagatatt ggtccaagta cccaaagaaa gacagggacg 480

aatatgttat tggtagccgg tattatgatg ttgaggaaaa gaaggagaaa aaagaaaaag 540

aggatgaaaa taaggataaa tccgtcatca ctccaaggga aaggaaaaca gtggacttac 600

taacatctcg aacaggtggg gcttatatac ctccagctaa attacgtatg atgcaggctg 660

agataactga taaatcatca gctgcatatc aaagaattgc ctgggaagct ttaaaaaagt 720

ccattcatgg ttacatcaac aaaattaaca cttccaatat tggtcttatt gctagagaat 780

tactgcatga aaacattgta agaggtagag gtttgctgtg taaatctata atacaagcac 840

aggcagcttc cccgacgttc accaatgttt atgcagcttt agttgcagtc ataaattcaa 900

aattccccaa cattggagaa ctgttactga aaaggttggt tttgcagttt aaaaggggtt 960

tcaagcagaa caacaagtct atctgtatat cggctgctac ctttgtcgcg catttagtaa 1020

accaaagagt ggcccacgaa attttagcat tggaaattct tactttactt gttgagtccc 1080

ccacagatga ttcagtggaa gtagcaattt cgtttttgaa ggaaagtggt caaaaactca 1140

ctgaggtgtc gagtaaaggt atcaatgcca tatttgagat gttgaggaat attctgcatg 1200

aaggacagtt ggagaagaga atacagtaca tgattgaagt catgttccaa gttcggaaag 1260

atggtttcaa ggatcatgct gctgttactg aagaactaga tattgttgaa gaggaagatc 1320

agtttactca cctaatcaca ttggatgatg ttaaacaagc taactcagag gatatattga 1380

atgtgtttaa atttgatgat aaatatgagg aaaatgaggg taaatacaaa actttaagta 1440

aggaaattct ccagtcagac agtgaatcag gcgaatctgg ttcagagggg tctgaagaag 1500

actcggaaga tgaagaaggt gaagaagatg aaaccaaaaa tcaaaccatt attgataaca 1560

cagaaactaa tttaatcacc ttaaggagaa ccatctatct cacaatacaa tccagtttgg 1620

attttgagga atgtgcccat aaattgatga aaatggagat caaacctgga caagagattg 1680

aattgtgtca catgttcctt gattgttgcg ctgaacagcg tacctacgaa aaattcttcg 1740

gcctcctctc gcagcgcttc tgccaaataa acaagacttt catcgaaccg ttccaacaaa 1800

ttttcaaaga tacctattcc acaactcaca gacttgacgc caatcgattg agaaacgtta 1860

gcaaattctt cgcgcattta ttgttcaccg acgccatcgg ctgggaagtg ctcgatatca 1920

tgaaattaaa cgaggaagac accaacagtt ccagcaggat ttttatcaag attttgttcc 1980

```
aggagttgtc cgaatatatg ggattagcga agttgaataa aaggctaaag gatgaaactt     2040
tacaggaata tttcgcgggg ctatttccga gggataaccc gaagaacacg cgtttcgcca     2100
tcaatttttt cacgtcgatc ggtttaggag gtctaacgga cgagttgagg gagcacttga     2160
aaaacgtgcc aaaacatctg gaagtgatgg ctttgaaagc agattcgagc agctctagca     2220
gcagtagcag cagttccagt aacgattcca gcagcagttc agattcttcc gatgacgagg     2280
gttccaggaa gaagaaaaca aaaaaattga aaccccgga caaaaagaag aaacagaaag     2340
aagatgaaaa acccaaaaag aaaagcgagg ataaaccgag gaacaaacca gactatagag     2400
ataggagaaa cgacgacagg gaaaagttta aaaaatacag aaacaacgac gaagaaagcc     2460
acagaagaag cagagaagat gcaagagaaa aatacagagg tcacgaggaa agaagacgag     2520
gccgagaaga ccaaacgcga agaggacaag accaagttcc aaggtttgtg c              2571
```

<210> 87
<211> 798
<212> PRT
<213> Meligethes aeneus

<400> 87

```
Met Thr Thr Asp Ser Glu Arg Gly Ser Pro Thr Ala Ala Ala Pro Arg
1               5                   10                  15

Arg Ser Ala Ser Lys Ser Pro Glu Pro Lys Lys Ala Lys Tyr Asp Lys
            20                  25                  30

Lys Glu Lys Gly Asp Lys Asp Arg Lys Arg Arg Ser His Arg Ser Arg
        35                  40                  45

Ser Arg Ser Arg Asp Arg Asp His Arg Asp Lys His Gly Gly Lys Lys
    50                  55                  60

Arg Tyr His Asp Leu Asp Asp Pro Ser Glu Asp Tyr Pro Arg Tyr Tyr
65                  70                  75                  80

Gly Glu Asp Arg Lys Gln Asn Ser Asp Arg Tyr Trp Ser Lys Tyr Pro
                85                  90                  95

Lys Lys Asp Arg Asp Glu Tyr Val Ile Gly Ser Arg Tyr Tyr Asp Val
            100                 105                 110

Glu Glu Lys Lys Glu Lys Lys Glu Lys Glu Asp Glu Asn Lys Asp Lys
        115                 120                 125

Ser Val Ile Thr Pro Arg Glu Arg Lys Thr Val Asp Leu Leu Thr Ser
    130                 135                 140
```

```
Arg Thr Gly Gly Ala Tyr Ile Pro Pro Ala Lys Leu Arg Met Met Gln
145                 150                 155                 160

Ala Glu Ile Thr Asp Lys Ser Ser Ala Ala Tyr Gln Arg Ile Ala Trp
                165                 170                 175

Glu Ala Leu Lys Lys Ser Ile His Gly Tyr Ile Asn Lys Ile Asn Thr
            180                 185                 190

Ser Asn Ile Gly Leu Ile Ala Arg Glu Leu Leu His Glu Asn Ile Val
        195                 200                 205

Arg Gly Arg Gly Leu Leu Cys Lys Ser Ile Ile Gln Ala Gln Ala Ala
    210                 215                 220

Ser Pro Thr Phe Thr Asn Val Tyr Ala Ala Leu Val Ala Val Ile Asn
225                 230                 235                 240

Ser Lys Phe Pro Asn Ile Gly Glu Leu Leu Leu Lys Arg Leu Val Leu
                245                 250                 255

Gln Phe Lys Arg Gly Phe Lys Gln Asn Asn Lys Ser Ile Cys Ile Ser
            260                 265                 270

Ala Ala Thr Phe Val Ala His Leu Val Asn Gln Arg Val Ala His Glu
        275                 280                 285

Ile Leu Ala Leu Glu Ile Leu Thr Leu Leu Val Glu Ser Pro Thr Asp
    290                 295                 300

Asp Ser Val Glu Val Ala Ile Ser Phe Leu Lys Glu Ser Gly Gln Lys
305                 310                 315                 320

Leu Thr Glu Val Ser Ser Lys Gly Ile Asn Ala Ile Phe Glu Met Leu
                325                 330                 335

Arg Asn Ile Leu His Glu Gly Gln Leu Glu Lys Arg Ile Gln Tyr Met
            340                 345                 350

Ile Glu Val Met Phe Gln Val Arg Lys Asp Gly Phe Lys Asp His Ala
        355                 360                 365

Ala Val Thr Glu Glu Leu Asp Ile Val Glu Glu Glu Asp Gln Phe Thr
    370                 375                 380

His Leu Ile Thr Leu Asp Asp Val Lys Gln Ala Asn Ser Glu Asp Ile
385                 390                 395                 400
```

```
Leu Asn Val Phe Lys Phe Asp Asp Lys Tyr Glu Glu Asn Glu Gly Lys
                405                 410                 415

Tyr Lys Thr Leu Ser Lys Glu Ile Leu Gln Ser Asp Ser Glu Ser Gly
            420                 425                 430

Glu Ser Gly Ser Glu Gly Ser Glu Glu Asp Ser Glu Asp Glu Glu Gly
        435                 440                 445

Glu Glu Asp Glu Thr Lys Asn Gln Thr Ile Ile Asp Asn Thr Glu Thr
    450                 455                 460

Asn Leu Ile Thr Leu Arg Arg Thr Ile Tyr Leu Thr Ile Gln Ser Ser
465                 470                 475                 480

Leu Asp Phe Glu Glu Cys Ala His Lys Leu Met Lys Met Glu Ile Lys
                485                 490                 495

Pro Gly Gln Glu Ile Glu Leu Cys His Met Phe Leu Asp Cys Cys Ala
            500                 505                 510

Glu Gln Arg Thr Tyr Glu Lys Phe Phe Gly Leu Leu Ser Gln Arg Phe
        515                 520                 525

Cys Gln Ile Asn Lys Thr Phe Ile Glu Pro Phe Gln Gln Ile Phe Lys
    530                 535                 540

Asp Thr Tyr Ser Thr Thr His Arg Leu Asp Ala Asn Arg Leu Arg Asn
545                 550                 555                 560

Val Ser Lys Phe Phe Ala His Leu Leu Phe Thr Asp Ala Ile Gly Trp
                565                 570                 575

Glu Val Leu Asp Ile Met Lys Leu Asn Glu Glu Asp Thr Asn Ser Ser
            580                 585                 590

Ser Arg Ile Phe Ile Lys Ile Leu Phe Gln Glu Leu Ser Glu Tyr Met
        595                 600                 605

Gly Leu Ala Lys Leu Asn Lys Arg Leu Lys Asp Glu Thr Leu Gln Glu
    610                 615                 620

Tyr Phe Ala Gly Leu Phe Pro Arg Asp Asn Pro Lys Asn Thr Arg Phe
625                 630                 635                 640

Ala Ile Asn Phe Phe Thr Ser Ile Gly Leu Gly Gly Leu Thr Asp Glu
```

645 650 655

Leu Arg Glu His Leu Lys Asn Val Pro Lys His Leu Glu Val Met Ala
660 665 670

Leu Lys Ala Asp Ser Ser Ser Ser Ser Ser Ser Ser Ser Ser Ser Ser
675 680 685

Asn Asp Ser Ser Ser Ser Ser Asp Ser Ser Asp Asp Glu Gly Ser Arg
690 695 700

Lys Lys Lys Thr Lys Lys Leu Lys Thr Pro Asp Lys Lys Lys Lys Gln
705 710 715 720

Lys Glu Asp Glu Lys Pro Lys Lys Lys Ser Glu Asp Lys Pro Arg Asn
725 730 735

Lys Pro Asp Tyr Arg Asp Arg Arg Asn Asp Asp Arg Glu Lys Phe Lys
740 745 750

Lys Tyr Arg Asn Asn Asp Glu Glu Ser His Arg Arg Ser Arg Glu Asp
755 760 765

Ala Arg Glu Lys Tyr Arg Gly His Glu Glu Arg Arg Arg Gly Arg Glu
770 775 780

Asp Gln Thr Arg Arg Gly Gln Asp Gln Val Pro Arg Phe Val
785 790 795

<210> 88
<211> 2512
<212> DNA
<213> Meligethes aeneus

<400> 88

```
aaatgtagtc actaccattg tagtgttatt cgtttcttgt gcttattttt aattaaccag      60
tcaattcgtg tggtgtagtg aacaaaggtt atagttatga cgacggattc cgagagaggt     120
tcccctacag ctgcggctcc acgcagaagc gcctcgaaat cgccagaacc aaaaaaagca     180
aagtacgata agaaagagaa gggcgataaa gatcgcaaga ggagatccca cagatccaga     240
tctagatcca gggatagaga ccatagggac aaacatggtg gaaaaaaacg ttaccacgac     300
ctggacgacc cttctgaaga ctacccaaga tattatggcg aggatagaaa acagaacagt     360
gacagatatt ggtccaagta cccaaaagaa agacagggac gaatatgtta ttggtaaccg     420
gtattatgat gttgaggaaa agaaggagaa aaaggaaaaa gaggatgaaa ataaggataa     480
atccgtcatt actccaaggg aaaggaaaac agtggactta ctaacatctc gaacaggtgg     540
```

ggcttatata cctccagcta aattacgtat gatgcaggct gagataactg ataaatcatc 600
agctgcatat caaagaattg cctgggaagc tttaaaaaag tccattcatg gttacatcaa 660
caaaattaac acttccaata ttggtcttat tgctagagaa ttactgcatg aaaacattgt 720
aagaggtaga ggtttgctgt gtaaatctat aatacaagca caggcagctt ccccgacgtt 780
caccaatgtt tatgcagctt tagttgcagt cataaattca aaattcccca acattggaga 840
actgttactg aaaaggttgg ttttgcagtt taaaaggggt ttcaagcaga acaacaagtc 900
tatctgtata tcggctgcta cctttgtcgc gcatttagta aaccaaagag tggctcatga 960
aattttagca ttggaaattc ttactttact tgttgagtcc cccacagatg attcagtaga 1020
agtgagcaga acaacaagtc tatctgtata tcggctgcta cctttgtcgc gcatttagta 1080
aaccaaagag tggcccacga aattttagca ttggaaattc ttactttact tgttgagtcc 1140
cccacagatg attcagtgga agtagcaatt tcgtttttga aggaaagtgg tcaaaaactc 1200
actgaggtgt cgagtaaagg tatcaatgcc atatttgaga tgttgaggaa tattctgcat 1260
gaaggacagt tggagaagag aatacagtac atgattgaag tcatgttcca agttcggaaa 1320
gatggtttca aggatcatgc tgctgttact gaagaactag atattgttga agaggaagat 1380
cagtttactc acctaatcac attggatgat gttaaacaag ctaactcaga ggatatattg 1440
aatgtgttta aatttgatga taaatatgag gaaaatgagg gtaaatacaa aactttaagt 1500
aaggaaattc tccagtcaga cagtgaatca ggcgaatctg gttcagaggg gtctgaagaa 1560
gactcggaag atgaagaagg tgaagaagat gaaaccaaaa atcaaaccat tattgataac 1620
acagaaacta atttaatcac cttaaggaga accatctatc tcacaataca atccagtttg 1680
gattttgagg aatgtgccca taaattgatg aaaatggaga tcaaacctgg acaagagatt 1740
gaattgtgtc acatgttcct tgattgttgc gctgaacagc gtacctacga aaaattcttc 1800
ggcctcctct cgcagcgctt ctgccaaata aacaagactt tcatcgaacc gttccaacaa 1860
attttcaaag atacctattc cacaactcac agacttgacg ccaatcgatt gagaaacgtt 1920
agcaaattct tcgcgcattt attgttcacc gacgccatcg gctgggaagt gctcgatatc 1980
atgaaattaa acgaggaaga caccaacagt tccagcagga ttttcatcaa gattttgttc 2040
caggagttgt ccgaatatat gggattagcg aagttgaata aaaggctaaa ggatgaaact 2100
ttacaggaat atttcgcggg gctatttccg agggataacc cgaagaacac gcgtttcgcc 2160
atcaattttt tcacgtcgat cggtttagga ggtctaacgg acgagttgag ggagcacttg 2220
aaaaacgtgc caaaacatct ggaagtgatg gctttgaaag cagattcgag cagctctagc 2280
agcagtagca gcagttccag taacgattcc agcagcagtt cagattcttc cgatgacgag 2340
ggttccagga agaagaaaac aaaaaaattg aaaaccccgg acaaaaagaa gaaacagaaa 2400
gaagatgaaa aacccaaaaa gaaaagcgag gataaaccga ggaacaaagt aaatggtgat 2460

aagaatatag aaacagaaga tacacaagga gttgaggaca caaaaaagac tg 2512

<210> 89
<211> 424
<212> PRT
<213> Meligethes aeneus

<400> 89

```
Met Leu Arg Asn Ile Leu His Glu Gly Gln Leu Glu Lys Arg Ile Gln
1               5                   10                  15

Tyr Met Ile Glu Val Met Phe Gln Val Arg Lys Asp Gly Phe Lys Asp
            20                  25                  30

His Ala Ala Val Thr Glu Glu Leu Asp Ile Val Glu Glu Glu Asp Gln
        35                  40                  45

Phe Thr His Leu Ile Thr Leu Asp Asp Val Lys Gln Ala Asn Ser Glu
    50                  55                  60

Asp Ile Leu Asn Val Phe Lys Phe Asp Asp Lys Tyr Glu Glu Asn Glu
65                  70                  75                  80

Gly Lys Tyr Lys Thr Leu Ser Lys Glu Ile Leu Gln Ser Asp Ser Glu
                85                  90                  95

Ser Gly Glu Ser Gly Ser Glu Gly Ser Glu Glu Asp Ser Glu Asp Glu
            100                 105                 110

Glu Gly Glu Glu Asp Glu Thr Lys Asn Gln Thr Ile Ile Asp Asn Thr
        115                 120                 125

Glu Thr Asn Leu Ile Thr Leu Arg Arg Thr Ile Tyr Leu Thr Ile Gln
    130                 135                 140

Ser Ser Leu Asp Phe Glu Glu Cys Ala His Lys Leu Met Lys Met Glu
145                 150                 155                 160

Ile Lys Pro Gly Gln Glu Ile Glu Leu Cys His Met Phe Leu Asp Cys
                165                 170                 175

Cys Ala Glu Gln Arg Thr Tyr Glu Lys Phe Phe Gly Leu Leu Ser Gln
            180                 185                 190

Arg Phe Cys Gln Ile Asn Lys Thr Phe Ile Glu Pro Phe Gln Gln Ile
        195                 200                 205
```

```
Phe Lys Asp Thr Tyr Ser Thr Thr His Arg Leu Asp Ala Asn Arg Leu
    210                 215                 220

Arg Asn Val Ser Lys Phe Phe Ala His Leu Leu Phe Thr Asp Ala Ile
225                 230                 235                 240

Gly Trp Glu Val Leu Asp Ile Met Lys Leu Asn Glu Glu Asp Thr Asn
                245                 250                 255

Ser Ser Ser Arg Ile Phe Ile Lys Ile Leu Phe Gln Glu Leu Ser Glu
            260                 265                 270

Tyr Met Gly Leu Ala Lys Leu Asn Lys Arg Leu Lys Asp Glu Thr Leu
        275                 280                 285

Gln Glu Tyr Phe Ala Gly Leu Phe Pro Arg Asp Asn Pro Lys Asn Thr
    290                 295                 300

Arg Phe Ala Ile Asn Phe Phe Thr Ser Ile Gly Leu Gly Gly Leu Thr
305                 310                 315                 320

Asp Glu Leu Arg Glu His Leu Lys Asn Val Pro Lys His Leu Glu Val
                325                 330                 335

Met Ala Leu Lys Ala Asp Ser Ser Ser Ser Ser Ser Ser Ser Ser Ser
            340                 345                 350

Ser Ser Asn Asp Ser Ser Ser Ser Ser Asp Ser Ser Asp Asp Glu Gly
        355                 360                 365

Ser Arg Lys Lys Lys Thr Lys Lys Leu Lys Thr Pro Asp Lys Lys Lys
    370                 375                 380

Lys Gln Lys Glu Asp Glu Lys Pro Lys Lys Lys Ser Glu Asp Lys Pro
385                 390                 395                 400

Arg Asn Lys Val Asn Gly Asp Lys Asn Ile Glu Thr Glu Asp Thr Gln
                405                 410                 415

Gly Val Glu Asp Thr Lys Lys Thr
            420
```

<210> 90
<211> 489
<212> DNA
<213> Meligethes aeneus

<400> 90

gttccttgat tgttgcgctg aacagcgtac ctacgaaaaa ttcttcggcc tcctctcgca 60

gcgcttctgc caaataaaca agactttcat cgaaccgttc caacaaattt tcaaagatac 120

ctattccaca actcacagac ttgacgccaa tcgattgaga aacgttagca aattcttcgc 180

gcatttattg ttcaccgacg ccatcggctg ggaagtgctc gatatcatga aattaaacga 240

ggaagacacc aacagttcca gcaggatttt catcaagatt ttgttccagg agttgtccga 300

atatatggga ttagcgaagt tgaataaaag gctaaaggat gaaactttac aggaatattt 360

cgcggggcta tttccgaggg ataacccgaa gaacacgcgt ttcgccatca atttttttcac 420

gtcgatcggt ttaggaggtc taacggacga gttgagggag cacttgaaaa acgtgccaaa 480

acatctgga 489

<210> 91
<211> 44
<212> DNA
<213> Artificial

<220>
<223> ncm reg1 forward primer

<400> 91
taatacgact cactataggg agagttcctt gattgttgcg ctga 44

<210> 92
<211> 45
<212> DNA
<213> Artificial

<220>
<223> ncm reg1 reverse primer

<400> 92
taatacgact cactataggg agatccagat gttttggcac gtttt 45

<210> 93
<211> 2599
<212> DNA
<213> Meligethes aeneus

<400> 93

```
tttttgttac caaccaaacg gcctaaattt ccctagataa cctaaaataa aaacaacact      60
tcgtcatttg tgtaaattca aacaaatgta gtcactacca ttgtagtgtt attcgtttct     120
tgtgcttatt tttaattaac cagtcaattc gtgtggtgta gtgaacaaag gttatagtta     180
tgacgacgga ttccgagaga ggttccccta cagctgcggc tccacgcaga agcgcctcga     240
aatcgccaga accaaaaaaa gcaaagtacg ataagaaaga gaagggcgat aaagatcgca     300
agaggagatc ccacagatcc agatctagat ccagggatag agaccatagg gacaaacatg     360
gtggaaaaaa acgttaccac gacctggacg acccttctga agactaccca agatattatg     420
gcgaggatag aaaacagaac agtgacagat attggtccaa gtacccaaag aaagacaggg     480
```

acgaatatgt tattggtagc cggtattatg atgttgagga aaagaaggag aaaaaggaaa 540

aagaggatga aaataaggat aaatccgtca ttactccaag ggaaaggaaa acagtggact 600

tactaacatc tcgaacaggt ggggcttata tacctccagc taaattacgt atgatgcagg 660

ctgagataac tgataaatca tcagctgcat atcaaagaat tgcctgggaa gctttaaaaa 720

agtccattca tggttacatc aacaaaatta acacttccaa tattggtctt attgctagag 780

aattactgca tgaaaacatt gtaagaggta gaggtttgct gtgtaaatct ataatacaag 840

cacaggcagc ttccccgaca ttcaccaatg tttatgcagc tttagttgca gtcataaatt 900

caaaattccc caacattgga gaactgttac tgaaaaggtt ggttttgcag tttaaaaggg 960

gtttcaagca gaacaacaag tctatctgta tatcggctgc tacctttgtc gcgcatttag 1020

taaaccaaag agtggcccat gaaattttag cattggaaat tcttacttta cttgttgagt 1080

cccccacaga tgattcagtg gaagtagcaa tttcgttttt gaaggaaagt ggtcaaaaac 1140

tcactgaggt gtcgagtaaa ggtatcaatg ccatatttga gatgttgagg aatattctgc 1200

atgaaggaca gttggagaag agaatacagt acatgattga agtcatgttc caagttcgga 1260

aagatggttt caaggatcat gctgctgtta ctgaagaact agatattgtt gaagaggaag 1320

atcagtttac tcacctaatc acattggatg atgttaaaca agctaactca gaggatatat 1380

tgaatgtgtt taaatttgat gataaatatg aggaaaatga gggtaaatac aaaactttaa 1440

gtaaggaaat tctccagtca gacagtgaat caggcgaatc tggttcagag gggtctgaag 1500

aagactcgga agatgaagaa ggtgaagaag atgaaaccaa aaatcaaacc attattgata 1560

acacagaaac taatttaatc accttaagga gaaccatcta tctcacaata caatccagtt 1620

tggattttga ggaatgtgcc cataaattga tgaaaatgga gatcaaacct ggacaagaga 1680

ttgaattgtg tcacatgttc cttgattgtt gcgctgaaca gcgtacctac gaaaaattct 1740

tcggcctcct ctcgcagcgc ttctgccaaa taaacaagac tttcatcgaa ccgttccaac 1800

aaattttcaa agatacctat tccacaactc acagacttga cgccaatcga ttgagaaacg 1860

ttagcaaatt cttcgcgcat ttattgttca ccgacgccat cggctgggaa gtgctcgata 1920

tcatgaaatt aaacgaggaa gacaccaaca gttccagcag gattttcatc aagattttgt 1980

tccaggagtt gtccgaatat atgggattag cgaagttgaa taaaaggcta aaggatgaaa 2040

ctttacagga atatttcgcg gggctatttc cgagggataa cccgaagaac acgcgtttcg 2100

ccatcaattt tttcacgtcg atcggtttag gaggtctaac ggacgagttg agggagcact 2160

tgaaaaacgt gccaaaacat ctggaagtga tggctttgaa agcagattcg agcagctcta 2220

gcagcagtag cagcagttcc agtaacgatt ccagcagcag ttcagattct tccgatgacg 2280

agggttccag gaagaagaaa acaaaaaaat tgaaaacccc ggacaaaaag aagaaacaga 2340

```
aagaagatga aaaacccaaa aagaaaagcg aggataaacc gaggaacaaa ccagactata     2400
gagatagaag aaacgacgac agggaaaagt ttaaaaaata cagaaacaac gacgaagaaa     2460
gccacagaag aagcagagaa gatgcaagag aaaaatacag aggtcacgag gaaagaagaa     2520
gcgaccacag agaagaatac cggccgagag aacatagagg tagagataga cgttagttgt     2580
ataataatgt atattttt                                                   2599
```

<210> 94
<211> 798
<212> PRT
<213> Meligethes aeneus

<400> 94

```
Met Thr Thr Asp Ser Glu Arg Gly Ser Pro Thr Ala Ala Ala Pro Arg
1               5                   10                  15

Arg Ser Ala Ser Lys Ser Pro Glu Pro Lys Lys Ala Lys Tyr Asp Lys
            20                  25                  30

Lys Glu Lys Gly Asp Lys Asp Arg Lys Arg Arg Ser His Arg Ser Arg
        35                  40                  45

Ser Arg Ser Arg Asp Arg Asp His Arg Asp Lys His Gly Gly Lys Lys
    50                  55                  60

Arg Tyr His Asp Leu Asp Asp Pro Ser Glu Asp Tyr Pro Arg Tyr Tyr
65                  70                  75                  80

Gly Glu Asp Arg Lys Gln Asn Ser Asp Arg Tyr Trp Ser Lys Tyr Pro
                85                  90                  95

Lys Lys Asp Arg Asp Glu Tyr Val Ile Gly Ser Arg Tyr Tyr Asp Val
            100                 105                 110

Glu Glu Lys Lys Glu Lys Lys Glu Lys Glu Asp Glu Asn Lys Asp Lys
        115                 120                 125

Ser Val Ile Thr Pro Arg Glu Arg Lys Thr Val Asp Leu Leu Thr Ser
    130                 135                 140

Arg Thr Gly Gly Ala Tyr Ile Pro Pro Ala Lys Leu Arg Met Met Gln
145                 150                 155                 160

Ala Glu Ile Thr Asp Lys Ser Ser Ala Ala Tyr Gln Arg Ile Ala Trp
                165                 170                 175

Glu Ala Leu Lys Lys Ser Ile His Gly Tyr Ile Asn Lys Ile Asn Thr
```

180 185 190

Ser Asn Ile Gly Leu Ile Ala Arg Glu Leu Leu His Glu Asn Ile Val
195 200 205

Arg Gly Arg Gly Leu Leu Cys Lys Ser Ile Ile Gln Ala Gln Ala Ala
210 215 220

Ser Pro Thr Phe Thr Asn Val Tyr Ala Ala Leu Val Ala Val Ile Asn
225 230 235 240

Ser Lys Phe Pro Asn Ile Gly Glu Leu Leu Leu Lys Arg Leu Val Leu
245 250 255

Gln Phe Lys Arg Gly Phe Lys Gln Asn Asn Lys Ser Ile Cys Ile Ser
260 265 270

Ala Ala Thr Phe Val Ala His Leu Val Asn Gln Arg Val Ala His Glu
275 280 285

Ile Leu Ala Leu Glu Ile Leu Thr Leu Leu Val Glu Ser Pro Thr Asp
290 295 300

Asp Ser Val Glu Val Ala Ile Ser Phe Leu Lys Glu Ser Gly Gln Lys
305 310 315 320

Leu Thr Glu Val Ser Ser Lys Gly Ile Asn Ala Ile Phe Glu Met Leu
325 330 335

Arg Asn Ile Leu His Glu Gly Gln Leu Glu Lys Arg Ile Gln Tyr Met
340 345 350

Ile Glu Val Met Phe Gln Val Arg Lys Asp Gly Phe Lys Asp His Ala
355 360 365

Ala Val Thr Glu Glu Leu Asp Ile Val Glu Glu Glu Asp Gln Phe Thr
370 375 380

His Leu Ile Thr Leu Asp Asp Val Lys Gln Ala Asn Ser Glu Asp Ile
385 390 395 400

Leu Asn Val Phe Lys Phe Asp Asp Lys Tyr Glu Glu Asn Glu Gly Lys
405 410 415

Tyr Lys Thr Leu Ser Lys Glu Ile Leu Gln Ser Asp Ser Glu Ser Gly
420 425 430

Glu Ser Gly Ser Glu Gly Ser Glu Glu Asp Ser Glu Asp Glu Glu Gly
435 440 445

Glu Glu Asp Glu Thr Lys Asn Gln Thr Ile Ile Asp Asn Thr Glu Thr
450 455 460

Asn Leu Ile Thr Leu Arg Arg Thr Ile Tyr Leu Thr Ile Gln Ser Ser
465 470 475 480

Leu Asp Phe Glu Glu Cys Ala His Lys Leu Met Lys Met Glu Ile Lys
485 490 495

Pro Gly Gln Glu Ile Glu Leu Cys His Met Phe Leu Asp Cys Cys Ala
500 505 510

Glu Gln Arg Thr Tyr Glu Lys Phe Phe Gly Leu Leu Ser Gln Arg Phe
515 520 525

Cys Gln Ile Asn Lys Thr Phe Ile Glu Pro Phe Gln Gln Ile Phe Lys
530 535 540

Asp Thr Tyr Ser Thr Thr His Arg Leu Asp Ala Asn Arg Leu Arg Asn
545 550 555 560

Val Ser Lys Phe Phe Ala His Leu Leu Phe Thr Asp Ala Ile Gly Trp
565 570 575

Glu Val Leu Asp Ile Met Lys Leu Asn Glu Glu Asp Thr Asn Ser Ser
580 585 590

Ser Arg Ile Phe Ile Lys Ile Leu Phe Gln Glu Leu Ser Glu Tyr Met
595 600 605

Gly Leu Ala Lys Leu Asn Lys Arg Leu Lys Asp Glu Thr Leu Gln Glu
610 615 620

Tyr Phe Ala Gly Leu Phe Pro Arg Asp Asn Pro Lys Asn Thr Arg Phe
625 630 635 640

Ala Ile Asn Phe Phe Thr Ser Ile Gly Leu Gly Gly Leu Thr Asp Glu
645 650 655

Leu Arg Glu His Leu Lys Asn Val Pro Lys His Leu Glu Val Met Ala
660 665 670

Leu Lys Ala Asp Ser Ser Ser Ser Ser Ser Ser Ser Ser Ser Ser Ser
675 680 685

```
Asn Asp Ser Ser Ser Ser Ser Asp Ser Ser Asp Asp Glu Gly Ser Arg
    690                 695                 700

Lys Lys Lys Thr Lys Lys Leu Lys Thr Pro Asp Lys Lys Lys Lys Gln
705                 710                 715                 720

Lys Glu Asp Glu Lys Pro Lys Lys Lys Ser Glu Asp Lys Pro Arg Asn
                725                 730                 735

Lys Pro Asp Tyr Arg Asp Arg Arg Asn Asp Asp Arg Glu Lys Phe Lys
            740                 745                 750

Lys Tyr Arg Asn Asn Asp Glu Glu Ser His Arg Arg Ser Arg Glu Asp
        755                 760                 765

Ala Arg Glu Lys Tyr Arg Gly His Glu Glu Arg Arg Ser Asp His Arg
    770                 775                 780

Glu Glu Tyr Arg Pro Arg Glu His Arg Gly Arg Asp Arg Arg
785                 790                 795
```

<210> 95
<211> 1658
<212> RNA
<213> Meligethes aeneus

<400> 95

gcaauuucgu uuuugaagga aaguggucaa aaacucacug aggugucgag uaaagguauc 60
aaugccauau uugagauguu gaggaauauu cugcaugaag gacaguugga gaagagaaua 120
caguacauga uugaagucau guuccaaguu cggaaagaug guuucaagga ucaugcugcu 180
guuacugaag aacuagauau uguugaagag gaagaucagu uuacucaccu aaucacauug 240
gaugauguua aacaagcuaa cucagaggau auauugaaug uguuuaaauu ugaugauaaa 300
uaugaggaaa augaggguaa auacaaaacu uuaaguaagg aaauucucca gucagacagu 360
gaaucaggcg aaucugguuc agaggggucu gaagaagacu cggaagauga agaaggugaa 420
gaagaugaaa ccaaaaauca aaccauuauu gauaacacag aaacuaauuu aaucaccuua 480
aggagaacca ucuaucucac aauacaaucc aguuuggauu uugaggaaug ugcccauaaa 540
uugaugaaaa uggagaucaa accuggacaa gagauugaau ugugucacau guuccuugau 600
uguugcgcug aacagcguac cuacgaaaaa uucuucggcc uccucucgca gcgcuucugc 660
caaauaaaca agacuuucau cgaaccguuc caacaaauuu ucaaagauac cuauuccaca 720
acucacagac uugacgccaa ucgauugaga aacguuagca aauucuucgc gcauuuauug 780
uucaccgacg ccaucggcug ggaagugcuc gauaucauga aauuaaacga ggaagacacc 840

aacaguucca gcaggauuuu caucaagauu uuguuccagg aguuguccga auauaugga 900
uuagcgaagu ugaauaaaag gcuaaaggau gaaacuuuac aggaauauuu cgcggggcua 960
uuuccgaggg auaacccgaa gaacacgcgu uucgccauca auuuuuucac gucgaucggu 1020
uuaggagguc uaacggacga guugagggag cacuugaaaa acgugccaaa acaucuggaa 1080
gugauggcuu ugaaagcaga uucgagcagc ucuagcagca guagcagcag uuccaguaac 1140
gauuccagca gcaguucaga uucuuccgau gacgaggguu ccaggaagaa gaaaacaaaa 1200
aaauugaaaa ccccggacaa aaagaagaaa cagaaagaag augaaaaacc caaaaagaaa 1260
agcgaggaua aaccgaggaa caaaccagac uauagagaua gaagaaacga cgacagggaa 1320
aaguuuaaaa aauacagaaa caacgacgaa gaaagccaca gaagaagcag agaagaugca 1380
agagaaaaau acagagguca cgaggaaaga agaagcgacc acagagaaga auaccggccg 1440
agagaacaua gagguagaga uagacguuag uuguauaaua auguauauuu uuucguauuu 1500
aauaaaauaa auuauacauu uuauaguguu ucggagcauu caccaagcaa ggguuuuacu 1560
uucggauagc aaugguguag uacguuuuug aaggugucca cacacaccaa gccgguuuua 1620
ucuaaaucua gagcuaucuu ccaaaagucu ucaaagga 1658

<210> 96
<211> 2571
<212> RNA
<213> Meligethes aeneus

<400> 96

ccacacgaau ugacugguua auuaaaaaua agcacaagaa acgaauaaca cuacaauggu 60

uugaauuuac acaaaaaaaa aaauguaguc acuaccauug uaguguuauu cguuucuugu 120

gcuuauuuuu aauuaaccag ucaauucgug ugguguagug aacaaagguu auaguuauga 180

cgacggauuc cgagagaggu uccccuacag cugcggcucc acgcagaagc gccucgaaau 240

cgccagaacc aaaaaaagca aaguacgaua agaaagagaa gggcgauaaa gaucgcaaga 300

ggagaucccca cagauccaga ucuagaucca gggauagaga ccauagggac aaacauggug 360

gaaaaaaacg uuaccacgac cuggacgacc cuucugaaga cuacccaaga uauuauggcg 420

aggauagaaa acagaacagu gacagauauu gguccaagua cccaaagaaa gacagggacg 480

aauauguuau ugguagccgg uauuaugaug uugaggaaaa gaaggagaaa aaagaaaaag 540

aggaugaaaa uaaggauaaa uccgucauca cuccaaggga aaggaaaaca guggacuuac 600

uaacaucucg aacaggu gggg gcuuauauac cuccagcuaa auuacguaug augcaggcug 660

agauaacuga uaaaucauca gcugcauauc aaagaauugc cugggaagcu uuaaaaaagu 720

ccauucaugg uuacaucaac aaaauuaaca cuuccaauau uggucuuauu gcuagagaau 780

uacugcauga aaacauugua agagguagag guuugcugug uaaaucuaua auacaagcac 840

aggcagcuuc cccgacguuc accaauguuu augcagcuuu aguugcaguc auaaauucaa 900
aauuccccaa cauuggagaa cuguuacuga aaagguuggu uuugcaguuu aaaaggggu u 960
ucaagcagaa caacaagucu aucuguauau cggcugcuac cuuugucgcg cauuuaguaa 1020
accaaagagu ggcccacgaa auuuuagcau uggaaauucu uacuuuacuu guugaguccc 1080
ccacagauga uucaguggaa guagcaauuu cguuuuugaa ggaaaguggu caaaaacuca 1140
cugagguguc gaguaaaggu aucaaugcca uauuugagau guugaggaau auucugcaug 1200
aaggacaguu ggagaagaga auacaguaca ugauugaagu cauguuccaa guucggaaag 1260
augguuucaa ggaucaugcu gcuguuacug aagaacuaga uauuguugaa gaggaagauc 1320
aguuuacuca ccuaaucaca uuggaugaug uuaaacaagc uaacucagag gauauauuga 1380
auguguuuaa auuugaugau aaauaugagg aaaaugaggg uaaauacaaa acuuuaagua 1440
aggaaauucu ccagucagac agugaaucag gcgaaucugg uucagagggg ucugaagaag 1500
acucggaaga ugaagaaggu gaagaagaug aaaccaaaaa ucaaaccauu auugauaaca 1560
cagaaacuaa uuuaaucacc uuaaggagaa ccaucuaucu cacaauacaa uccaguuugg 1620
auuuugagga augugcccau aaauugauga aaauggagau caaaccugga caagagauug 1680
aauuguguca cauguuccuu gauuguugcg cugaacagcg uaccuacgaa aaauucuucg 1740
gccuccucuc gcagcgcuuc ugccaaauaa acaagacuuu caucgaaccg uuccaacaaa 1800
uuuucaaaga uaccuauucc acaacucaca gacuugacgc caaucgauug agaaacguua 1860
gcaaauucuu cgcgcauuua uuguucaccg acgccaucgg cugggaagug cucgauauca 1920
ugaaauuaaa cgaggaagac accaacaguu ccagcaggau uuuuaucaag auuuuguucc 1980
aggaguuguc cgaauauaug ggauuagcga aguugaauaa aaggcuaaag gaugaaacuu 2040
uacaggaaua uuucgcgggg cuauuuccga gggauaaccc gaagaacacg cguuucgcca 2100
ucaauuuuuu cacgucgauc gguuuaggag gucuaacgga cgaguugagg gagcacuuga 2160
aaaacgugcc aaaacaucug gaagugaugg cuuugaaagc agauucgagc agcucuagca 2220
gcaguagcag caguuccagu aacgauucca gcagcaguuc agauucuucc gaugacgagg 2280
guuccaggaa gaagaaaaca aaaaaauuga aaccccgga caaaaagaag aaacagaaag 2340
aagaugaaaa acccaaaaag aaaagcgagg auaaaccgag gaacaaacca gacuauagag 2400
auaggagaaa cgacgacagg gaaaaguuua aaaaauacag aaacaacgac gaagaaagcc 2460
acagaagaag cagagaagau gcaagagaaa aauacagagg ucacgaggaa agaagacgag 2520
gccgagaaga ccaaacgcga agaggacaag accaaguucc aagguuugug c 2571

<210> 97
<211> 2512
<212> RNA

<213> Meligethes aeneus

<400> 97

aaauguaguc acuaccauug uaguguuauu cguuucuugu gcuuauuuuu aauuaaccag 60
ucaauucgug ugguguagug aacaaagguu auaguuauga cgacggauuc cgagagaggu 120
uccccuacag cugcggcucc acgcagaagc gccucgaaau cgccagaacc aaaaaaagca 180
aaguacgaua agaaagagaa gggcgauaaa gaucgcaaga ggagauccca cagauccaga 240
ucuagaucca gggauagaga ccauagggac aaacauggug gaaaaaaacg uuaccacgac 300
cuggacgacc cuucugaaga cuacccaaga uauuauggcg aggauagaaa acagaacagu 360
gacagauauu gguccaagua cccaaaagaa agacagggac gaauauguua uugguaaccg 420
guauuaugau guugaggaaa agaaggagaa aaaggaaaaa gaggaugaaa auaaggauaa 480
auccgucauu acuccaaggg aaaggaaaac aguggacuua cuaacaucuc gaacaggugg 540
ggcuuauaua ccuccagcua aauuacguau gaugcaggcu gagauaacug auaaaucauc 600
agcugcauau caaagaauug ccugggaagc uuuaaaaaag uccauucaug guuacaucaa 660
caaaauuaac acuuccaaua uuggucuuau ugcuagagaa uuacugcaug aaaacauugu 720
aagagguaga gguuugcugu guaaaucuau aauacaagca caggcagcuu ccccgacguu 780
caccaauguu uaugcagcuu uaguugcagu cauaaauuca aaauucccca acauuggaga 840
acuguuacug aaaagguugg uuuugcaguu uaaaaggggu uucaagcaga acaacaaguc 900
uaucuguaua ucggcugcua ccuuugucgc gcauuuagua aaccaaagag uggcucauga 960
aauuuuagca uuggaaauuc uuacuuuacu uguugagucc cccacagaug auucaguaga 1020
agugagcaga acaacaaguc uaucuguaua ucggcugcua ccuuugucgc gcauuuagua 1080
aaccaaagag uggcccacga aauuuuagca uuggaaauuc uuacuuuacu uguugagucc 1140
cccacagaug auucagugga aguagcaauu ucguuuuuga aggaaagugg ucaaaaacuc 1200
acugaggugu cgaguaaagg uaucaaugcc auauuugaga uguugaggaa uauucugcau 1260
gaaggacagu uggagaagag aauacaguac augauugaag ucauguucca aguucggaaa 1320
gaugguuuca aggaucaugc ugcuguuacu gaagaacuag auauuguuga agaggaagau 1380
caguuuacuc accuaaucac auuggaugau guuaaacaag cuaacucaga ggauauauug 1440
aauguguuua aauuugauga uaaauaugag gaaaaugagg guaaauacaa aacuuuaagu 1500
aaggaaauuc uccagucaga cagugaauca ggcgaaucug guucagaggg gucugaagaa 1560
gacucggaag augaagaagg ugaagaagau gaaaccaaaa aucaaaccau uauugauaac 1620
acagaaacua auuuaaucac cuuaaggaga accaucuauc ucacaauaca auccaguuug 1680
gauuuugagg aaugugccca uaaauugaug aaaauggaga ucaaaccugg acaagagauu 1740
gaauuguguc acauguuccu ugauuguugc gcugaacagc guaccuacga aaaauucuuc 1800
ggccuccucu cgcagcgcuu cugccaaaua aacaagacuu ucaucgaacc guuccaacaa 1860

auuuucaaag auaccuauuc cacaacucac agacuugacg ccaaucgauu gagaaacguu 1920

agcaaauucu ucgcgcauuu auuguucacc gacgccaucg gcugggaagu gcucgauauc 1980

augaaauuaa acgaggaaga caccaacagu uccagcagga uuuucaucaa gauuuuguuc 2040

caggaguugu ccgaauauau gggauuagcg aaguugaaua aaaggcuaaa ggaugaaacu 2100

uuacaggaau auuucgcggg gcuauuuccg agggauaacc cgaagaacac gcguuucgcc 2160

aucaauuuuu ucacgucgau cgguuuagga ggucuaacgg acgaguugag ggagcacuug 2220

aaaaacgugc caaaacaucu ggaagugaug gcuuugaaag cagauucgag cagcucuagc 2280

agcaguagca gcaguuccag uaacgauucc agcagcaguu cagauucuuc cgaugacgag 2340

gguuccagga agaagaaaac aaaaaaauug aaaaccccgg acaaaaagaa gaaacagaaa 2400

gaagaugaaa aacccaaaaa gaaaagcgag gauaaaccga ggaacaaagu aaauggugau 2460

aagaauauag aaacagaaga uacacaagga guugaggaca caaaaaagac ug 2512

<210> 98
<211> 489
<212> RNA
<213> Meligethes aeneus

<400> 98

guuccuugau uguugcgcug aacagcguac cuacgaaaaa uucuucggcc uccucucgca 60

gcgcuucugc caaauaaaca agacuuucau cgaaccguuc caacaaauuu ucaaagauac 120

cuauuccaca acucacagac uugacgccaa ucgauugaga aacguuagca aauucuucgc 180

gcauuuauug uucaccgacg ccaucggcug ggaagugcuc gauaucauga aauuaaacga 240

ggaagacacc aacaguucca gcaggauuuu caucaagauu uuguuccagg aguuguccga 300

auauauggga uuagcgaagu ugaauaaaag gcuaaaggau gaaacuuuac aggaauauuu 360

cgcggggcua uuuccgaggg auaacccgaa gaacacgcgu uucgccauca auuuuuucac 420

gucgaucggu uuaggagguc uaacggacga guugagggag cacuugaaaa acgugccaaa 480

acaucugga 489

<210> 99
<211> 2599
<212> RNA
<213> Meligethes aeneus

<400> 99

```
uuuuuguuac caaccaaacg gccuaaauuu cccuagauaa ccuaaaauaa aaacaacacu      60
ucgucauuug uguaaauuca aacaaaugua gucacuacca uuguaguguu auucguuucu     120
ugugcuuauu uuuaauuaac cagucaauuc guguggugua gugaacaaag guuauaguua     180
ugacgacgga uuccgagaga gguuccccua cagcugcggc uccacgcaga agcgccucga     240
```

```
aaucgccaga accaaaaaaa gcaaaguacg auaagaaaga gaagggcgau aaagaucgca     300
agaggagauc ccacagaucc agaucuagau ccagggauag agaccauagg gacaaacaug     360
guggaaaaaa acguuaccac gaccuggacg acccuucuga agacuaccca agauauuaug     420
gcgaggauag aaaacagaac agugacagau auuggucca guacccaaag aaagacaggg      480
acgaauaugu uauugguagc cgguauuaug auguugagga aaagaaggag aaaaaggaaa     540
aagaggauga aaauaaggau aaauccguca uuacuccaag ggaaaggaaa acaguggacu     600
uacuaacauc ucgaacaggu ggggcuuaua uaccuccagc uaaauuacgu augaugcagg     660
cugagauaac ugauaaauca ucagcugcau aucaaagaau ugccugggaa gcuuuaaaaa     720
aguccauuca ugguuacauc aacaaaauua acacuuccaa uauuggucuu auugcuagag     780
aauuacugca ugaaaacauu guaagaggua gagguuugcu guguaaaucu auaauacaag     840
cacaggcagc uuccccgaca uucaccaaug uuuaugcagc uuuaguugca gucauaaauu     900
caaaauuccc caacauugga gaacuguuac ugaaaagguu gguuuugcag uuuaaaaggg     960
guuucaagca gaacaacaag ucuaucugua uaucggcugc uaccuuuguc gcgcauuuag    1020
uaaaccaaag aguggcccau gaaauuuuag cauuggaaau ucuuacuuua cuuguugagu    1080
cccccacaga ugauucagug gaaguagcaa uuucguuuuu gaaggaaagu ggucaaaaac    1140
ucacugaggu gucgaguaaa gguaucaaug ccauauuuga gauguugagg aauauucugc    1200
augaaggaca guuggagaag agaauacagu acaugauuga agucauguuc caaguucgga    1260
aagaugguuu caaggaucau gcugcuguua cugaagaacu agauauuguu gaagaggaag    1320
aucaguuuac ucaccuaauc acauuggaug auguuaaaca agcuaacuca gaggauauau    1380
ugaauguguu uaaauuugau gauaaauaug aggaaaauga ggguaaauac aaaacuuuaa    1440
guaaggaaau ucuccaguca gacagugaau caggcgaauc ugguucagag gggucugaag    1500
aagacucgga agaugaagaa ggugaagaag augaaaccaa aaaucaaacc auuauugaua    1560
acacagaaac uaauuuaauc accuuaagga gaaccaucua ucucacaaua caauccaguu    1620
uggauuuuga ggaaugugcc cauaaauuga ugaaaaugga gaucaaaccu ggacaagaga    1680
uugaauugug ucacauguuc cuugauuguu gcgcugaaca gcguaccuac gaaaaauucu    1740
ucggccuccu cucgcagcgc uucugccaaa uaaacaagac uuucaucgaa ccguuccaac    1800
aaauuucaa agauaccuau uccacaacuc acagacuuga cgccaaucga uugagaaacg     1860
uuagcaaauu cuucgcgcau uuauuguuca ccgacgccau cggcugggaa gugcucgaua    1920
ucaugaaauu aaacgaggaa gacaccaaca guuccagcag gauuuucauc aagauuuugu    1980
uccaggaguu guccgaauau augggauuag cgaaguugaa uaaaaggcua aaggaugaaa    2040
cuuuacagga auauuucgcg ggcuauuuc cgagggauaa cccgaagaac acgcguuucg     2100
ccaucaauuu uuucacgucg aucgguuuag gaggucuaac ggacgaguug agggagcacu    2160
```

ugaaaaacgu gccaaaacau cuggaaguga uggcuuugaa agcagauucg agcagcucua 2220

gcagcaguag cagcaguucc aguaacgauu ccagcagcag uucagauucu uccgaugacg 2280

aggguuccag gaagaagaaa acaaaaaaau ugaaaacccc ggacaaaaag aagaaacaga 2340

aagaagauga aaaacccaaa aagaaaagcg aggauaaacc gaggaacaaa ccagacuaua 2400

gagauagaag aaacgacgac agggaaaagu uuaaaaaaua cagaaacaac gacgaagaaa 2460

gccacagaag aagcagagaa gaugcaagag aaaaauacag aggucacgag gaaagaagaa 2520

gcgaccacag agaagaauac cggccgagag aacauagagg uagagauaga cguuaguugu 2580

auaauaaugu auauuuuuu 2599